# EUROPEAN PATENT APPLICATION

(11) **EP 2 168 960 A1**
(43) Date of publication of application: **31.03.2010**
(21) Application number: 08777308.1
(22) Date of filing: 18.06.2008
(51) Int. Cl.: C07D 401/14, A61K 31/4155, A61K 31/4188, A61K 31/4196, A61K 31/428, A61K 31/4709, A61K 31/501, A61P 1/04, A61P 3/04, A61P 3/06, A61P 3/10, A61P 9/04, A61P 9/12, A61P 11/06, A61P 17/00, A61P 25/00, A61P 25/14, A61P 25/16, A61P 25/18, A61P 25/24

(54) **PYRAZOLONE DERIVATIVE AND PDE INHIBITOR CONTAINING THE SAME AS ACTIVE INGREDIENT**

(30) Priority: 19.06.2007 JP 2007161195
(71) Applicant: Kyorin Pharmaceutical Co., Ltd., Tokyo 101-8311 (JP)
(72) Inventor: KOHNO, Yasushi, Shimotsuga-gun Tochigi 329-0114 (JP); OCHIAI, Koji, Shimotsuga-gun Tochigi 329-0114 (JP); TAKITA, Satoshi, Shimotsuga-gun Tochigi 329-0114 (JP); KISHI, Tetsuya, Shimotsuga-gun Tochigi 329-0114 (JP)
(74) Representative: Vossius & Partner
(86) International application number: PCT/JP2008/061104
(87) International publication number: WO 2008/156102

(57) **Abstract**

It is to provide a novel pyrazolone derivative represented by the following general formula (1), which is useful as a pharmaceutical and has a phosphodiesterase inhibitory action: 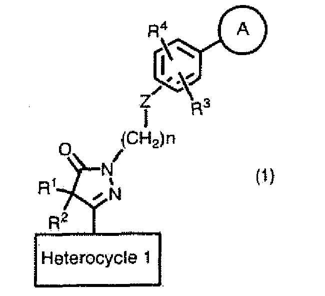 wherein R¹,R²: C₁₋₆ alkyl; R³,R⁴: H, X, C₁₋₆ alkoxy; Z:O, S; A:AA, BB, wherein AA represents 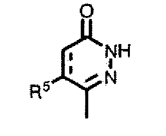 wherein BB represents 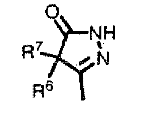 wherein R⁵: H, C₁₋₆ alkyl ; R⁶,R⁷: C₁₋₆ alkyl.

## Description

### Technical Field

The present invention relates to a pyrazolone derivative, a salt thereof, or a hydrate thereof, which is useful as a phosphodiesterase (PDE) inhibitor.

### Background Art

Phosphodiesterases (PDE) are enzymes that breakdown cyclic AMP (cAMP) and cyclic GMP (cGMP) which are second messengers in the living body. To date, type 1 to 11 of PDEs have been identified and each type specifically breaks down either cAMP or cGMP, or both. There are differences in the tissue distribution of each type of PDE. It is thought that cellular reactions are controlled by various types of PDEs according to the type of organ.

Up to the present, a large number of PDE inhibitors have been developed. For example, PDE3 inhibitors are anticipated as agents for treating angina pectoris, cardiac failure, hypertension, or the like, or as platelet aggregation inhibitors or antiasthmatic agents; and PDE4 inhibitors are anticipated as agents for treating bronchial asthma, chronic obstructive pulmonary disease (COPD), interstitial pneumonia, allergic rhinitis, atopic dermatitis, rheumatoid arthritis, multiple sclerosis, Crohn's disease, inflammatory colitis, Alzheimer, dementia, Parkinson's disease, depression, or the like. PDE5 inhibitors are already in clinical use as agents for treating male erectile dysfunction. Moreover, it has been recently reported that the use of minocycline as a PDE10A modulator is effective for patients with Huntington's disease (Patent Document 1), and a patent laid-open publication has been disclosed, which describes PDE10 inhibitors as effective as agents for treating various psychiatric disorders such as Huntington's disease, Alzheimer, dementia, Parkinson's disease, schizophrenia, and the like (Patent Document 2). In addition, recently, the pamphlet of International Publication (Patent Document 3) which describes that the inhibitors are also effective for obesity and metabolic syndrome has also been disclosed.

Pyrazolone derivatives having PDE inhibitory action have been reported (Patent Documents 4 and 5, and Non-Patent Documents 1 and 2). In addition, a compound having an alkyl group at position 2 of a phthalazinone ring and thus having PDE inhibitory action has been disclosed (Patent Documents 6 and 7). However, a compound which has the characteristics of the present invention, which is a compound with a pyridazinone ring or pyrazolone ring via an alkyl group at position 2 of the pyrazolone ring, wherein various hetero ring compounds are linked to the pyrazolone rings is not known.
[Patent Document 1] Pamphlet of WO 01024781
[Patent Document 2] JP-A-2002-363103
[Patent Document 3] Pamphlet of WO 2005120514
[Patent Document 4] JP-A-2006-169138
[Patent Document 5] JP-A-2007-91597
[Patent Document 6] Pamphlet of WO 2001019818
[Patent Document 7] Pamphlet of WO 9947505
[Non-Patent Document 1] Sircar I et al., J. Med. Chem., 30, 1724(1987)
[Non-Patent Document 2] Scott D. Edmonson et al., Bio. Med. Chem. Lett., 13, 3983(2003)

### Disclosure of the Invention

### Problem that the Invention is to Solve

The present invention aims to provide a pyrazolone derivative having excellent phosphodiesterase inhibitory action with few side effects.

### Means for Solving the Problem

The present inventors have conducted extensive studies on a highly safe compound having phosphodiesterase inhibitory activity, and, as a result, have found that a novel pyrazolone derivative structurally different from any of the existing PDE inhibitors has PDE inhibitory action. Thus, the present invention has been completed.

Namely, the present invention relates to:
1) a pyrazolone derivative, an optically active compound thereof, a pharmaceutically acceptable salt thereof, or a hydrate thereof, wherein the pyrazolone derivative is represented by the following general formula (1):

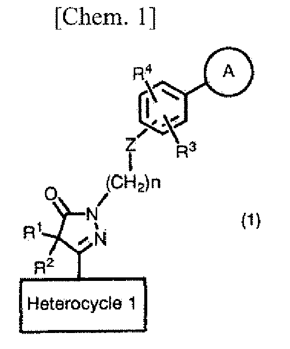

[wherein R¹ and R² are the same as or different from each other and represent an alkyl group having 1 to 6 carbon atoms,
R³ and R⁴ are the same as or different from each other and represent a hydrogen atom, a halogen atom, or an alkoxy group having 1 to 6 carbon atoms,
Z represents an oxygen atom or a sulfur atom,
A represents a substituent represented by the general formula:

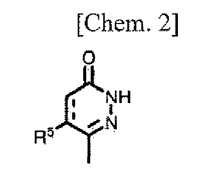

(wherein R⁵ represents a hydrogen atom or an alkyl group having 1 to 6 carbon atoms, and

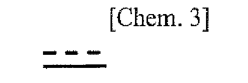

represents a single bond or a double bond) or a substituent represented by the general formula:

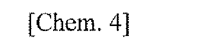

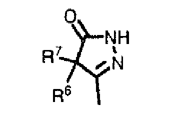

(wherein R⁶ and R⁷ are the same as or different from each other and represent an alkyl group having 1 to 6 carbon atoms),
Heterocycle 1 represents a substituent represented by the following general formula (2):

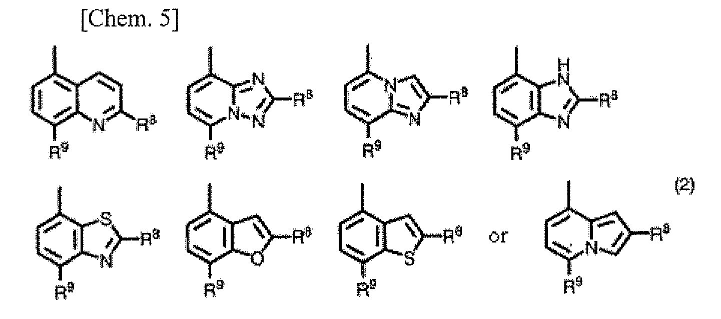

(wherein R⁸ represents a hydrogen atom or an alkyl group having 1 to 6 carbon atoms which may be substituted with halogen atom(s), and R⁹ represents an alkoxy group having 1 to 6 carbon atoms), and
n represents an integer of 1 to 5];

2) the pyrazolone derivative, optically active compound thereof, pharmaceutically acceptable salt thereof, or hydrate thereof according to 1), wherein the compound represented by the general formula (1) is represented by the general formula (1a):

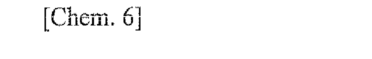

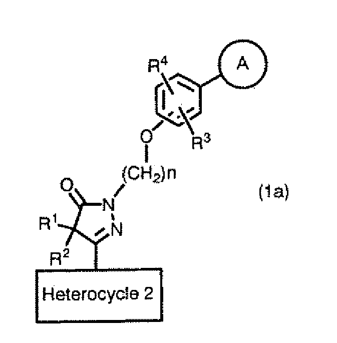

[wherein Heterocycle 2 represents the following general formula (2a):

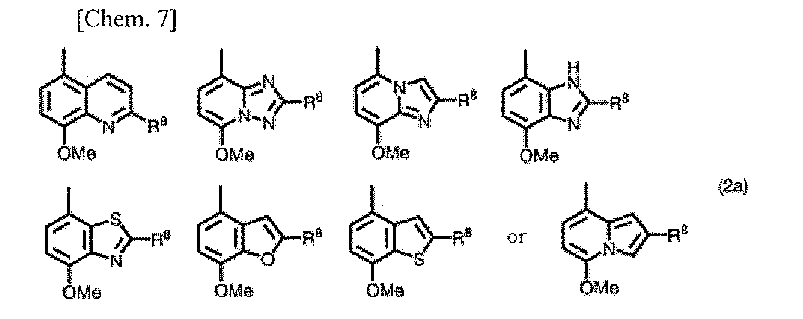

(wherein R⁸ is as defined above), and R¹, R², R³, R⁴, A, and n are as defined above];

3) the pyrazolone derivative, optically active compound thereof, pharmaceutically acceptable salt thereof, or hydrate thereof according to 1), wherein the compound represented by the general formula (1) is
5-(8-methoxy-2-methylquinotin-5-yl)-2-[4-[4-(4-methyl-6-oxo-1,4,5,6-tetrahydropyridazin-3-yl)phenoxy]butyl]-4,4-dimethyl-4,4-dihydro-pyrazol-3-one,
5-(2-ethyl-8-methoxyquinolin-5-yl)-2-[4-[2-fluoro-4-(4,4-dimethyl-5-oxo-4,5-dihydro-1H-pyrazol-3-yl)phenaxy]butyl]-4,4-dimethyl-2,4-dihydro-pyrazol-3-one,
5-(2-ethyl-8-methoxyquinolin-5-yl)-2-[4-[2,3-difluoro-4-(4,4-dimethyl-5-oxo-4,5-dihydro-1H-pyrazol-3-yl)phenoxy]butyl]-4,4-dimethyl-2,4-dihydro-pyrazol-3-one,
5-(2-ethyl-8-methoxyquinolin-5-yl)-2-[4-[2-fluoro-4-(4-methyl-6-oxo-1,4,5,6-tetrahydropyridazin-3-yl)phenoxy]butyl]-4,4-dimethyl-2,4-dihydro-pyrazol-3-one,
5-(2-ethyl-8-methoxyqumolin-5-yl)-2-[4-[2,3-dinuoro-4-(4-methyl-6-oxo-1,4,5,6-tetrahydropyridazin-3-yl)phenoxy]butyl]-4,4-dimethyl-2,4-dihydro-pyrazol-3-one,
(-)-5-(2-ethyl-8-methoxyquinohn-5-yl)-2-[4-[2,3-difluoro-4-(4-methyl-6-oxo-1,4,5,6-tetrahydropyridazin-3-yl)phenoxy]butyl]-4,4-dimethyl-2,4-dihydro-pyrazol-3-one,
2-[4-[2,3-difluoro-4-(6-oxo-1,4,5,6-tetrahydropyridazin-3-ylphenoxy]butyl]-5-(4-methoxy-2-trifluoromethyl-1H-benzo[d]imidazol-7-yl)-4,4-dimethyl-2,4-dihydro-pyrazol-3-one,
2-[4-[2,3-difluoro-4-(4,4-dimethyl-5-oxo-4,5-dihydro-1H-pyrazol-3-yl)phenoxy]butyl]-5-(4-methoxy-2-trifluoromethyl-1H-benzo[d]imidazol-7-yl)-4,4-dimethyl-2,4-dihydro-pyrazol-3-one,
2-[4-[2,3-difluoro-4-(4-methyl-6-oxo-1,4,5,6-tetrahydropyridazin-3-yl)phenoxy]butyl]-5-(4-methoxy-2-trifluoromethyl-1H-benzo[d]imidazol-7-yl)-4,4-dimethyl-2,4-dihydro-pyrazol-3-one,
2-[4-[2,3-difluoro-4-(6-oxo-1,4,5,6-tetrahydropyridazm-3-yl)phenoxy]butyl]-5-(4-methoxy-2-trifluoromethylbenzo[d]thiazol-7-yl)-4,4-dimethyl-2,4-dihydro-pyrazol-3-one,
2-[4-[2,3-difluoro-4-(6-oxo-1,4,5,6-tetrahydropyridazin-3-yl)phenoxy]butyl]-5-(8-methoxy-2-trifluoromethylimidazo[1,2-a]pyridin-5-yl)-4,4-dimethyl-2,4-dihydro-pyrazol-3-one,
2-[4-[2,3-difluoro-4-(4,4-dimethyl-5-oxa-4,5-dihydro-1H-pyrazol-3-yl)phenoxy]butyl]-5-(4-methoxy-2-trifluoromethylbenzo[d]thiazol-7-yl)-4,4-dimethyl-2,4-dihydro-pyrazol-3-one, or
5-(2-ethyl-8-methoxyquinolin-5-yl)-2-[4-[2,3-difluoro-4-(4-methyl-6-oxo-1,4,5,6-tetrahydropyridazin-3-yl)phenoxy]propyl]-4,4-dimethyl-2,4-dihydro-pyrazol-3-one;

4) a phosphodiesterase (PDE) inhibitor comprising, as an active ingredient, the pyrazolone derivative, optically active compound thereof, pharmaceutically acceptable salt thereof, or hydrate thereof according to any one of 1) to 3);

5) a pharmaceutical agent comprising, as an active ingredient, the pyrazolone derivative, optically active compound thereof, pharmaceutically acceptable salt thereof, or hydrate thereof according to any one of 1) to 3); and

6) the pharmaceutical agent according to 5), which is an agent for preventing or treating angina pectoris, cardiac failure, hypertension, bronchial asthma, chronic obstructive pulmonary disease (COPD), interstitial pneumonitis, allergic rhinitis, atopic dermatitis, rheumatoid arthritis, multiple sclerosis, Crohn's disease, inflammatory bowel disease, Huntington's disease, Alzheimer, dementia, Parkinson's disease, depression, schizophrenia, obesity, or metabolic syndrome.

### Advantage of the Invention

According to the present invention, it has been found that a novel pyrazolone derivative and an addition salt thereof have excellent PDE inhibitory action. Such a compound having PDE inhibitor action is useful as an agent for treating angina pectoris, cardiac failure, hypertension, or the like, as a platelet aggregation inhibitor, as an agent for preventing or treating bronchial asthma, chronic obstructive pulmonary disease (COPD), interstitial pneumonitis, allergic rhinitis, atopic dermatitis, rheumatoid arthritis, multiple sclerosis, Crohn's disease, or inflammatory bowel disease, as an agent for preventing or treating various psychiatric disorders such as Huntington's disease, Alzheimer, dementia, Parkinson's disease, depression, schizophrenia, and the like, as an agent for preventing or treating obesity, metabolic syndrome, and the like, and as an agent for treating male erectile dysfunction.

### Best Mode for Carrying out the Inveniton

In the present invention, the alkyl group having 1 to 6 carbon atoms means a linear chained or branched alkyl group having 1 to 6 carbon atoms, and preferably an alkyl group having 1 to 4 carbon atoms. Examples thereof include a methyl group, an ethyl group, a propyl group, an isopropyl group, a butyl group, an isobutyl group, a sec-butyl group, a t-butyl group, and the like.

The alkyl group having 1 to 6 carbon atoms which may be substituted with halogen atom(s) is preferably an alkyl group having 1 to 6 carbon atoms, in which all of the hydrogen atoms are substituted with fluorine atoms; more preferably an alkyl group having 1 to 4 carbon atoms, in which all of the hydrogen atoms are substituted with fluorine atoms; and particularly preferably a trifluoromethyl group.

The alkoxy group having 1 to 6 carbon atoms means a linear chained or branched alkoxy group having 1 to 6 carbon atoms, and is preferably an alkoxy group having 1 to 4 carbon atoms. Examples thereof include a methoxy group, an ethoxy group, a propoxy group, an isopropoxy group, a butoxy group, an isobutoxy group, a sec-butoxy group, a t-butoxy group, and the like.

Examples of the halogen atom include a fluorine atom, a chlorine atom, a bromine atom, and an iodine atom.

Examples of the pharmaceutically acceptable salt in the present invention include acid addition salts such as hydrochloride salt, hydrobromide salt, acetate salt, trifluoroacetate salt, methanesulfonate salt, citrate salt, and tartrate salt.

From the viewpoint of phosphodiesterase inhibitory activity, R¹ and R² are preferably alkyl groups having 1 to 4 carbon atoms, and more preferably a methyl group. n is preferably an integer of 2 to 4, and more preferably 4. R³ and R⁴ are preferably hydrogen atoms or fluorine atoms.

According to the present invention, the compound represented by the general formula (1) can be prepared via, for example, Synthesis Pathway A as shown below.

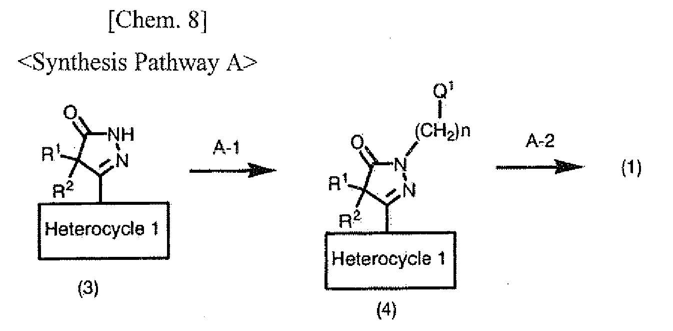

In Synthesis Pathway A, a compound represented by the general formula (4) can be prepared by allowing a compound represented by the general formula (3) to act on a compound represented by the general formula (5) in the presence of a base (Step A-1):

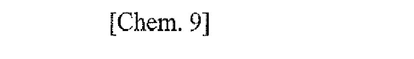

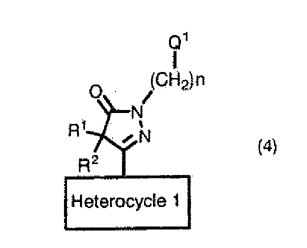

[wherein Q¹ represents a chlorine atom, a bromine atom, an iodine atom, a methanesulfonyloxy group, a trifluoromethanesulfonyloxy group, a benzenesulfonyloxy group, a paratoluenesulfonyloxy group, a t-butyldimethylsilyloxy group, a t-butyldiphenylsilyloxy group, a triisopropylsilyloxy group, a tetrahydropyranyloxy group, a methoxymethyloxy group, or a hydroxyl group, and R¹, R², n, and Heterocycle 1 are as defined above]

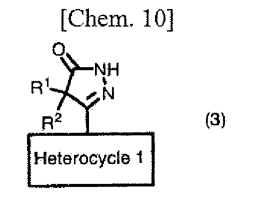

[wherein R¹, R², and Heterocycle 1 are as defined above]

[Chem. 11] Q²**-**(CH₂)n-Q¹ (5)

[wherein Q² represents a chlorine atom, a bromine atom, an iodine atom, a methanesulfonyloxy group, a trifluoromethanesulfonyloxy group, a benzenesulfonyloxy group, or a paratoluenesulfonyloxy group, and n and Q¹ are as defined above].

The reaction can be carried out at 0°C to 100°C using n-butyl lithium, sodium hydride, lithium alkoxide, sodium alkoxide, potassium alkoxide, or the like as a base and using tetrahydrofuran (THF), N,N-dimethyl formamide (DMF), or the like as a reaction solvent.

In Synthesis Pathway A, the compound represented by the general formula (1) can be prepared by reacting the compound represented by the general formula (4) with a compound represented by the general formula (6) (Step A-2):

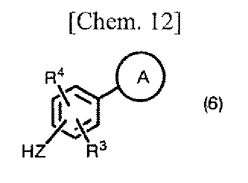

[wherein R³, R⁴, A, and Z are as defined above].

In the case where Q¹ of the compound represented by the general formula (4) is a chlorine atom, a bromine atom, an iodine atom, a methanesulfonyloxy group, a trifluoromethanesulfonyloxy group, a benzenesulfonyloxy group, or a paratoluenesulfonyloxy group, the reaction can be carried out at 0°C to 100°C using n-butyl lithium, sodium hydride, lithium alkoxide, sodium alkoxide, potassium alkoxide, lithium carbonate, sodium carbonate, potassium carbonate, cesium carbonate, or the like as a base and using THF, DMF, or the like as a reaction solvent.

Further, in the case where Q¹ of the compound represented by the general formula (4) is a t-butyldimethylsilyloxy group, a t-butyldiphenylsilyloxy group, or a triisopropylsilyloxy group, it is necessary to allow tetrabutyl ammonium fluoride, hydrofluoric acid, hydrofluoride pyridinium, or the like to act thereon at 0°C to room temperature using THF or the like as a solvent, thereby first transforming a hydroxyl group thereinto. In the case where Q¹ is a tetrahydropyranyloxy group or a methoxymethyloxy group, it is necessary to allow an acid such as, for example, concentrated hydrochloric acid, hydrobromic acid, or the like to act thereon at 0°C to 100°C in a solvent such as acetic acid, thereby transforming a hydroxyl group thereinto. In the case where a chlorine atom, a bromine atom, or an iodine atom is transformed from the obtained hydroxide form, this step can be carried out by allowing a chlorinating agent such as chlorine, carbon tetrachloride, N-chlorosuccinimide (NCS), or the like, a brominating agent such as bromine, carbon tetrabromide, N-bromosuccinimide (NBS), or the like, or an iodinating agent such as iodine, N-iodosuccinimide (NBS), or the like to act thereon at 0°C to room temperature in a solvent such as toluene, methylene chloride, THF, or the like, in the presence oftributylphosphine, triphenylphosphine, triphenoxyphosphine, or the like. Further, in the case where a methanesulfonyloxy group, a trifluoromethanesulfonyloxy group, a benzenesulfonyloxy group, or a paratoluenesulfonyloxy group is transformed from the obtained hydroxide form, this step can be carried out at 0°C to room temperature in a solvent such as methylene chloride, THF, or the like, in the presence of a base such as diisopropylethylamine, triethylamine, pyridine, or the like, using the corresponding sulfonyl chloride or sulfonyl anhydride. The compound having such an introduction can be reacted with the compound represented by the general formula (6) at 0°C to 100°C, using THF, DMF, or the like as a solvent, in the presence of a base such as n-butyl lithium, sodium hydride, lithium alkoxide, sodium alkoxide, potassium alkoxide, lithium carbonate, sodium carbonate, potassium carbonate, cesium carbonate, or the like.

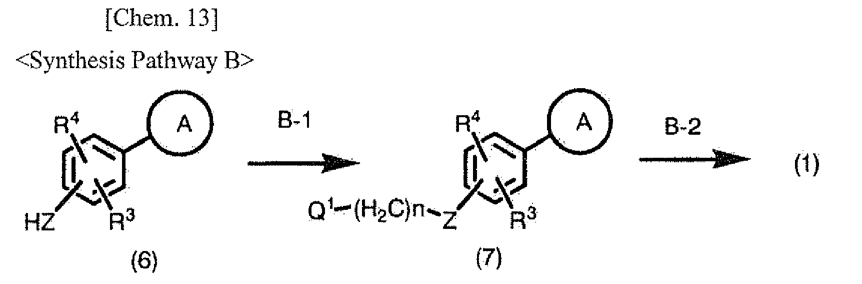

In Synthesis Pathway B, a compound represented by the general formula (7) can be prepared by allowing the compound represented by the general formula (6) to act on the compound represented by the general formula (5) in the presence of a base (Step B-1):

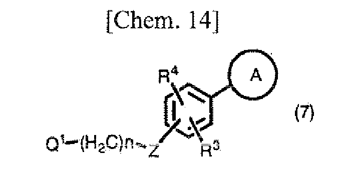

[wherein R³, R⁴, A, Z, Q¹, and n are as defined above].

The reaction can be carried out in the same manner as in Step A-1.

In Synthesis Pathway B, the compound represented by the general formula (1) can be prepared by reacting the compound represented by the general formula (7) with the compound represented by the general formula (3) (Step B-2).

In the case where Q¹ of the compound represented by the general formula (7) is a chlorine atom, a bromine atom, an iodine atom, a methanesulfonyloxy group, a trifluoromethanesulfonyloxy group, a benzenesulfonyloxy group, or a paratoluenesulfonyloxy group, the reaction can be carried out at 0°C to 100°C using n-butyl lithium, sodium hydride, lithium alkoxide, sodium alkoxide, potassium alkoxide, lithium carbonate, sodium carbonate, potassium carbonate, cesium carbonate, or the like as a base and using THF, DMF, or the like as a reaction solvent.

Further, in the case where Q¹ of the compound represented by the general formula (7) is a t-butyldimethylsilyloxy group, a t-butyldiphenylsilyloxy group, or a triisopropylsilyloxy group, it is necessary to allow tetrabutyl ammonium fluoride, hydrofluoric acid, hydrofluoride pyridinium, or the like to act thereon at 0°C to room temperature using THF or the like as a solvent, thereby first transforming a hydroxyl group thereinto. In the case where Q¹ is a tetrahydropyranyloxy group or a methoxymethyloxy group, it is necessary, for example, to allow acids such as concentrated hydrochloric acid, hydrobromide, or the like to act thereon at 0°C to 100°C in a solvent such as acetic acid, thereby transforming a hydroxyl group thereinto. In the case where a chlorine atom, a bromine atom, or an iodine atom is transformed from the obtained hydroxide form, this step can be carried out by allowing a chlorinating agent such as chlorine, carbon tetrachloride, NCS, or the like, a brominating agent such as bromine, carbon tetrabromide, NBS, or the like, or an iodinating agent such as iodine, NIS, or the like to act thereon at 0°C to room temperature in a solvent such as toluene, methylene chloride, THF, or the like, in the presence of tributylphosphine, triphenylphosphine, triphenoxyphosphine, or the like. Further, in the case where a methanesulfonyloxy group, a tritluoromethanesulfonyloxy group, a benzenesulfonyloxy group, or a paratoluenesulfonyloxy group is transformed from the obtained hydroxide form, this step can be carried out at 0°C to room temperature in a solvent such as methylene chloride, THF, or the like in the presence of a base such as diisopropylethylamine, triethylamine, pyridine, or the like, using the corresponding sulfonyl chloride or sulfonyl anhydride. The compound which transformed by the above can be reacted with the compound represented by the general formula (3) at 0°C to 100°C, using THF, DMF, or the like as a solvent, in the presence of a base such as n-butyl lithium, sodium hydride, lithium alkoxide, sodium alkoxide, potassium alkoxide, lithium carbonate, sodium carbonate, potassium carbonate, cesium carbonate, or the like.

The compound represented by the general formula (1), wherein A is

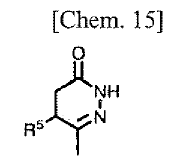

[wherein R⁵ is as defined above], that is, a compound represented by the general formula (1b)

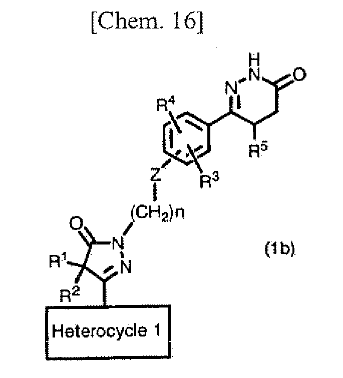

[wherein R¹, R², R³, R⁴, R⁵, Z, Heterocycle 1, and n are as defined above], and the compound in which A is represented by

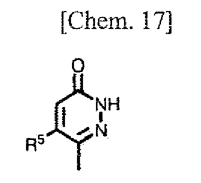

[wherein R⁵ is as defined above], that is, a compound represented by the general formula (1c)

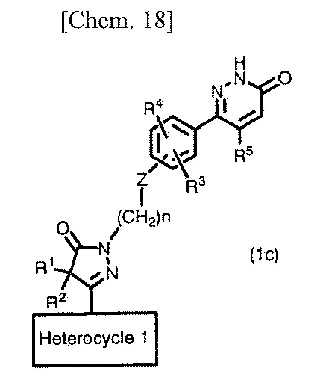

[wherein R¹, R², R³, R⁴, R⁵, Z, Heterocycle 1, and n are as defined above] can be converted to each other as shown in Synthesis Pathways below.

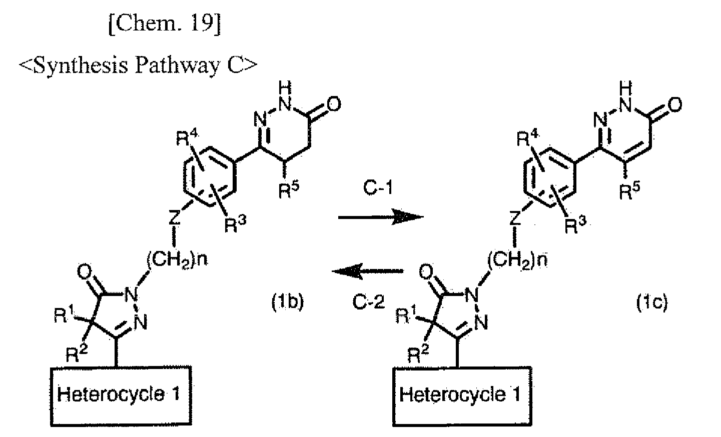

In Synthesis Pathway C, the compound represented by the general formula (1b) can be prepared by reducing the compound represented by the general formula (1c) (Step C-2).

The reaction can be carried out at 80°C to 90°C with the addition of zinc in acetic acid.

In Synthesis Pathway C, the compound represented by the general formula (1c) can be prepared by oxidizing the compound represented by the general formula (1b) (Step C-1).

The reaction can be carried out at from 50°C to 60°C with the addition of bromine in acetic acid, or the reaction can be carried out at from room temperature to heating temperature under reflux, using copper (II) chloride in acetonitrile. Further, the reaction can also be carried out by allowing sodium m-nitrobenzene sulfonate to act thereon at from room temperature to heating temperature under reflux in an aqueous sodium hydroxide solution.

In Synthesis Pathways A and B, the compound represented by the general formula (3), wherein Heterocycle 1 is a quinoline ring, that is, a compound represented by the general formula (3a) can be prepared via, for example, Synthesis Pathway D as shown below:

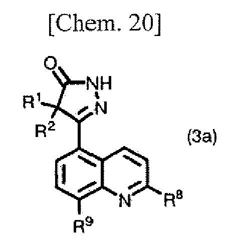

[wherein R¹, R², R⁸, and R⁹ are as defined above]

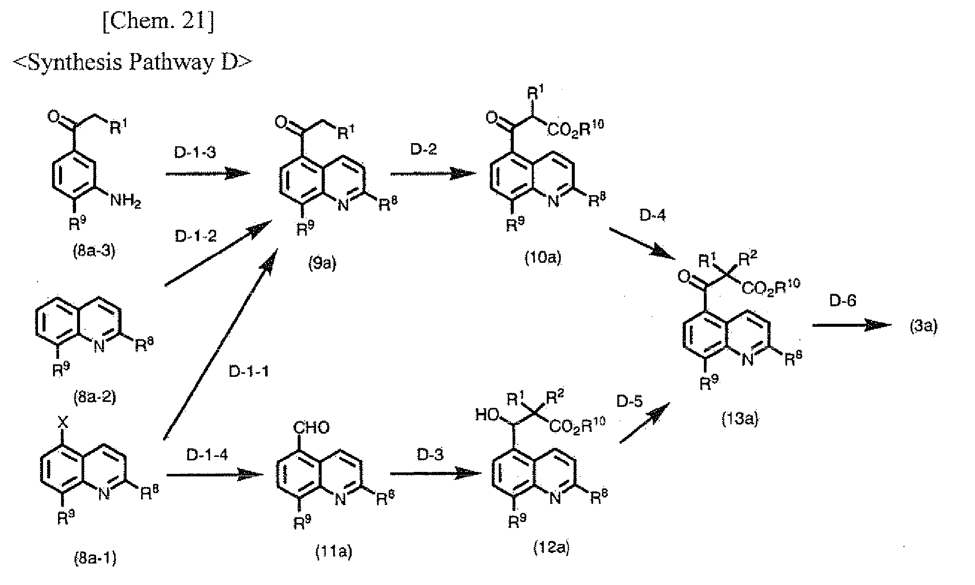

In Synthesis Pathway D, a compound represented by the general formula (9a) can be prepared by treating a compound represented by the general formula (8a-1) with an organometallic reagent, followed by reacting with a compound represented by the general formula (14) or a compound represented by the general formula (15) (Step D-1-1):

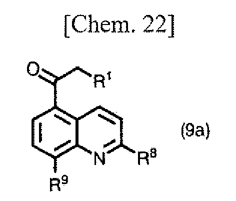

[wherein R¹, R⁸, and R⁹ are as defined above]

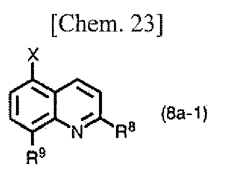

[wherein X represents a halogen atom, and R⁸ and R⁹ are as defined above]

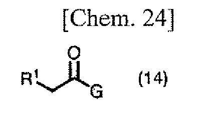

[wherein G represents a halogen atom, an amino group, a dimethyl amino group, or an alkoxy group having 1 to 6 carbon atoms, and R¹ is as defined above]

[Chem. 25] (R¹CH₂CO)₂O (15)

[wherein R¹ is as defined above].

The reaction is preferably carried out by dissolving the compound represented by the general formula (8a-1) in THF, ether, 1,4-dioxane, or the like, and performing a reaction at from -78°C to 0°C, using an organomagnesium reagent such as methyl magnesium chloride, ethyl magnesium chloride, isopropyl magnesium chloride, methyl magnesium bromide, ethyl magnesium bromide, isopropyl magnesium bromide, methyl magnesium iodide, ethyl magnesium iodide, isopropylmagnesium iodide, or the like or an organolithium reagent such as n-butyl lithium, s-butyl lithium, t-butyl lithium, or the like, as an organometallic reagent, and preferably n-butyl lithium, and then allowing the compounds represented by the general formula (14) or the general formula (15) to act thereon, followed by slowly warming to room temperature.

Further, the compound represented by the general formula (9a) can also be prepared by allowing a compound represented by the general formula (8a-2) to act on a compound represented by the general formula (16) (Step D-1-2):

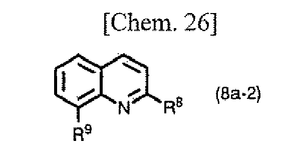

[wherein R⁸ and R⁹ are as defined above]

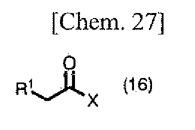

[wherein R¹ and X are as defined above].

The reaction can be carried out by adding a Lewis acid such as aluminum chloride, iron chloride, titanium tetrachloride, tin chloride, or the like, and preferably aluminum chloride, followed by warming to a temperature from room temperature to 120°C, using a solvent such as dichlorobenzene, methylene chloride, dichloroethane, tetrachloroethane, nitromethane, benzene, chlorobenzene, or the like, and preferably dichlorobenzene.

Further, the compound represented by the general formula (9a) can also be prepared by reacting a compound represented by the general formula (8a-3) with a compound represented by the general formula (S) (Step D-1-3):

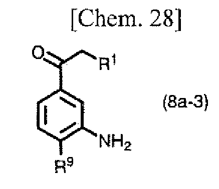

[wherein R¹ and R⁹ are as defined above]

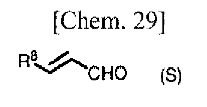

[wherein R⁸ is as defined above].

The reaction can be carried out at from 50°C to 100°C using 70% sulfuric acid or 6 mol/L hydrochloric acid also as a solvent or using methanol, ethanol, propanol, or butanol, and preferably butanol as a solvent, with the addition of 50% sulfuric acid or concentrated hydrochloric acid. Further, sodium iodide can also be added to the reaction system.

In Synthesis Pathway D, a compound represented by the general formula (11a) can be prepared by treating a compound represented by the general formula (8a-1) with an organometalic reagent, followed by reacting with DMF or formic ester (Step D-1-4):

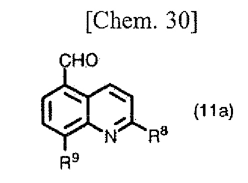

[wherein R⁸ and R⁹ are as defined above].

The reaction is preferably carried out by dissolving the compound represented by the general formula (8a-1) in THF, ether, 1,4-dioxane, or the like, and performing a reaction at from -78°C to 0°C, using an organomagnesium reagent such as methyl magnesium chloride, ethyl magnesium chloride, isopropyl magnesium chloride, methyl magnesium bromide, ethyl magnesium bromide, isopropyl magnesium bromide, methyl magnesium iodide, ethyl magnesium iodide, isopropylmagnesium iodide, or the like or an organolithium reagent such as n-butyl lithium, s-butyl lithium, t-butyl lithium, or the like, and preferably n-butyl lithium, and then allowing DMF or formic ester to act thereon, followed by slowly warming to room temperature.

In Synthesis Pathway D, a compound represented by the general formula (10a) can be prepared by reacting the compound represented by the general formula (9a) with a compound represented by the general formula (17) in the presence of a base (Step D-2):

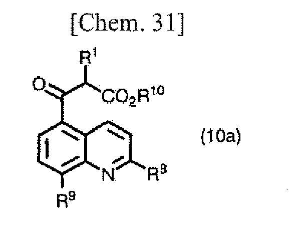

[wherein R¹⁰ represents an alkyl group having 1 to 6 carbon atoms or a benzyl group, and R¹, R⁸, and R⁹ are as defined above]

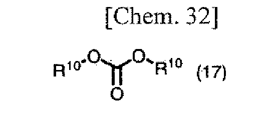

[wherein R¹⁰ is as defined above].

The reaction is preferably carried out with heating under reflux, using a solvent amount of the compound represented by the general formula (17) in the presence of an inorganic base such as sodium alkoxide, potassium alkoxide, sodium hydride, potassium hydride, or the like, and preferably sodium hydride.

In Synthesis Pathway D, a compound represented by the general formula (12a) can be prepared by allowing the compound represented by the general formula (11a) to act on a compound represented by the general formula (18) in the presence of a Lewis acid (Step D-3):

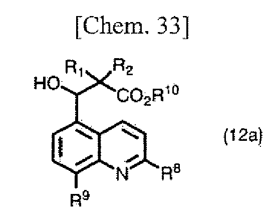

[wherein R¹, R², R⁸, R⁹, and R¹⁰ are as defined above]

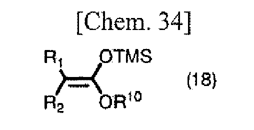

[wherein TMS represents a trimethylsilyl group, and R¹, R², and R¹⁰ are as defined above] (Step D-3).

The reaction is preferably carried out at room temperature with the addition of a Lewis acid such as a boron trifluoride-diethyl ether complex, iron chloride, titanium tetrachloride, aluminum chloride, or the like, and preferably a boron trifluoride-diethyl ether complex, using diethyl ether, THF, 1,4-dioxane, dichloromethane, chloroform, or the like as a solvent.

In Synthesis Pathway D, a compound represented by the general formula (13a) can be prepared by treating the compound represented by the general formula (10a) with a base, followed by reacting with a compound represented by the general formula (19) (Step D-4):

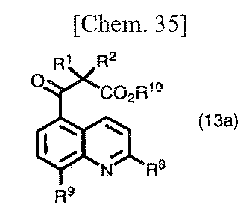

[wherein R¹, R², R⁸, R⁹, and R¹⁰ are as defined above]

[Chem. 36] R²X (19)

[wherein R² and X are as defined above].

The reaction is preferably carried out by treating the compound represented by the general formula (10a) at from -78°C to 0°C using sodium hydride, potassium hydride, sodium alkoxide, potassium alkoxide, lithium diisopropylamide (LDA), lithium-2,2,6,6-tetramethyl piperidide, lithium bistrimethylsilyl amide, sodium bistrimethylsilyl amide, potassium bistrimethylsilyl amide, or the like as a base and using THF, 1,4-dioxane, 1,2-dimethoxyethane, or the like as a reaction solvent, followed by reacting with the compound of the general formula (19), followed by slowly warming to room temperature.

Further, the compound represented by the general formula (13a) can be prepared by oxidizing the compound represented by the general formula (12a) (Step D-5).
The reaction can employ a means for oxidizing a generally used alcohol to a ketone, and examples of the means include a chromium oxide-pyridine complex such as pyridinium chlorochromate, pyridinium dichromate, or the like, a metal oxidant such as chromium oxide, silver carbonate, manganese dioxide, or the like, DMSO oxidation using various DMSO activators including a sulfur trioxide-pyridine complex, oxalyl chloride, anhydrous trifluoroacetic acid, acetic anhydride, DCC, or the like, and hypervalency iodine oxidation using 2-iodoxybenzoic acid (IBX), Dess-Martin periodinane, or the like.

In Synthesis Pathway D, the compound represented by the general formula (3a) can be prepared by allowing a hydrazine derivative to act on the compound represented by the general formula (13a) (Step D-6).

As the hydrazine derivative, a hydrazine or a salt of the hydrazine such as hydrazine acetate, hydrazine hydrochloride, or the like, or a carbazic ester such as t-butyl carbazate, methyl carbazate, benzyl carbazate, or the like can be used.

In the case of using a hydrazine or a salt thereof, the reaction can be carried out at room temperature or with heating under reflux, and preferably with heating under reflux, using benzene, toluene, acetic acid, or ethanol as a reaction solvent.

Further, in the case of using a carbazic ester, the reaction can be carried out with heating under reflux using benzene, toluene, xylene, or the like as a reaction solvent and using paratoluenesulfonic acid, pyridinium paratoluenesulfonate, or the like as an acid catalyst, and preferably under a dehydration condition using a Dean-Stark trap, and if necessary, after the reaction, the obtained compound is preferably deprotected under an acidic condition using trifluoroacetic acid, hydrogen chloride-containing methanol, ethanol, ethyl acetate, diethyl ether, or the like.

Further, in Synthesis Pathway D, the compound represented by the general formula (13a) can also be prepared by the method shown in Synthesis Pathway D' below.

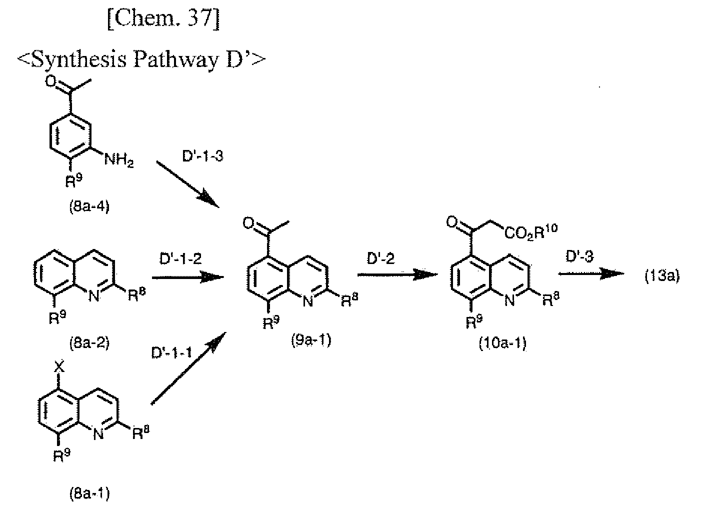

In Synthesis Pathway D', a compound represented by the general formula (9a-1) can be prepared by treating a compound represented by the general formula (8a-1) with an organometallic reagent, followed by reacting with a compound represented by the general formula (20) or acetic anhydride (Step D'-1-1):

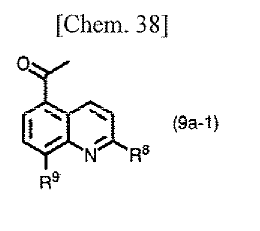

[wherein R⁸ and R⁹ are as defined above]

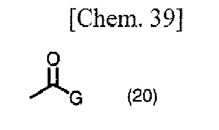

[wherein G is as defined above].

The reaction can be carried out in the same manner as in Step D-1-1.

Further, the compound represented by the general formula (9a-1) can also be prepared by reacting the compound represented by the general formula (8a-2) with a compound represented by the general formula (21) (Step D'-1-2):

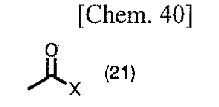

[wherein X is as defined above].

The reaction can be carried out in the same manner as in Step D-1-2.

Further, the compound represented by the general formula (9a-1) can also be prepared by reacting a compound represented by the general formula (8a-4) with the compound represented by the general formula (S) (Step D'-1-3):

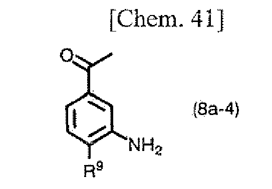

[wherein R⁹ is as defined above,

The reaction can be carried out in the same manner as in Step D-1-3.

In Synthesis Pathway D', a compound represented by the general formula (10a-1) can be prepared by reacting the compound represented by the general formula (9a-1) with the compound represented by the general formula (17) in the presence of a base (Step D'-2):

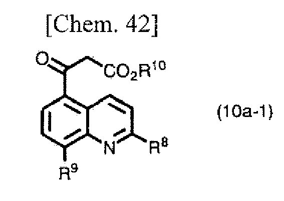

[wherein R⁸, R⁹, and R¹⁰ are as defined above].

The reaction can be carried out in the same manner as in Step D-2.

In Synthesis Pathway D', a compound represented by the general formula (13a) can be prepared by treating the compound represented by the general formula (10a-1) with a base, followed by reacting with a compound represented by the general formula (22), and further treating it with a base and then allowing the compound represented by the general formula (19) to act thereon (Step D'-3):

[Chem. 43] R¹X (22)

[wherein R¹ and X are as defined above].

The reaction is preferably carried out by treating the compound represented by the general formula (10a-1) at from -78°C to 0°C using sodium hydride, potassium hydride, sodium alkoxide, potassium alkoxide, LDA, lithium-2,2,6,6-tetramethylpiperizide, lithium bistrimethylsilyl amide, sodium bistrimethylsilyl amide, potassium bistrimethylsilyl amide, or the like as a base and using THF, 1,4-dioxane, 1,2-dimethoxyethane, or the like as a reaction solvent, followed by reacting with the compound represented by the general formula (22), followed by slowly warming to room temperature, and thereafter, treating at from -78°C to 0°C using sodium hydride, potassium hydride, sodium alkoxide, potassium alkoxide, LDA, lithium-2,2,6,6-tetramethylpiperizide, lithium bistrimethylsilyl amide, sodium bistrimethylsilyl amide, potassium bistrimethylsilyl amide, or the like as a base, followed by reacting with the compound represented by the general formula (19), followed by slowly warming to room temperature.

The compound represented by the general formula (3), wherein Heterocycle 1 is a triazolopyridine ring, that is, a compound represented by the general formula (3b) can be prepared by Synthesis Pathway E below:

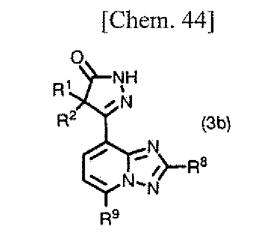

[wherein R¹, R², R⁸, and R⁹ are as defined above].

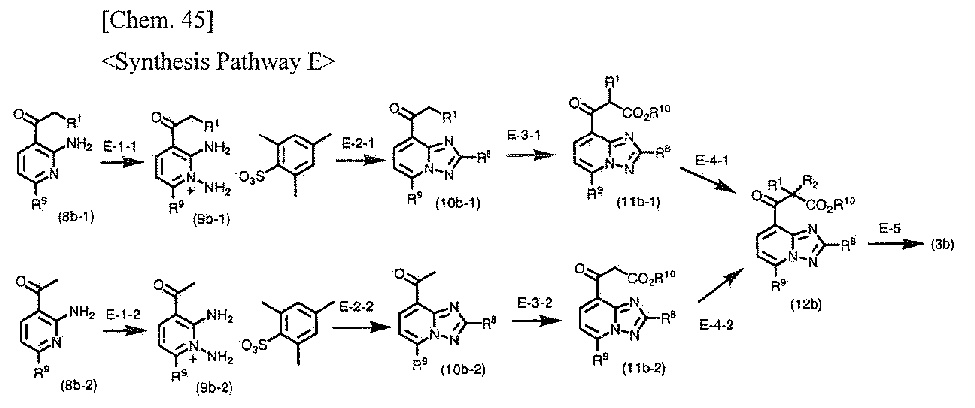

In Synthesis Pathway E, a compound represented by the general formula (9b-1) can be prepared by allowing a compound represented by the general formula (8b-1) to act on O-mesitylensulfonyl hydroxyamine (hereinafter referred to as MSH) (Step E-1-1):

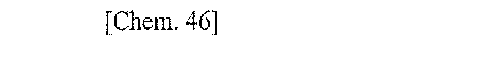

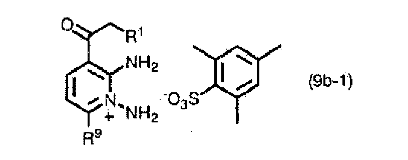

[wherein R¹ and R⁹ are as defined above]

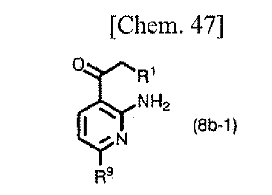

[wherein R¹ and R⁹ are as defined above].

The reaction is preferably carried by dissolving the compound represented by the general formula (8b-1) in methylene chloride, and allowing a solution of MSH in methylene chloride to act thereon at from 0°C to room temperature.

In Synthesis Pathway E, a compound represented by the general formula (10b-1) can be prepared by allowing a compound represented by the general formula (23) to act on the compound represented by the general formula (9b-1) in the presence of a base (Step E-2-1):

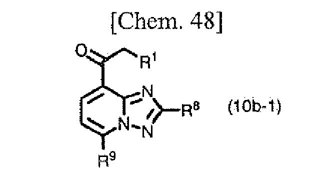

[wherein R¹, R⁸, and R⁹ are as defined above]

[Chem. 49] (R⁸CO)₂O (23)

[wherein R⁸ is as defined above].

The reaction can be carried out at from room temperature to heating temperature under reflux, using benzene, toluene, xylene, methanol, ethanol, or the like as a solvent and using a base such as triethylamine, sodium hydroxide, potassium hydroxide, potassium carbonate, or the like, and preferably triethylamine.

In Synthesis Pathway E, a compound represented by the general formula (11b-1) can be prepared by reacting the compound represented by the general formula (10b-1) with the compound represented by the general formula (17) in the presence of a base (Step E-3-1):

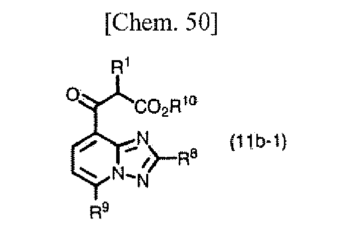

[wherein R¹, R⁸, R⁹, and R¹⁰ are as defined above].

The reaction can be carried out in the same manner as in Step D-2.

In Synthesis Pathway E, a compound represented by the general formula (9b-2) can be prepared by allowing a compound represented by the general formula (8b-2) to act on MSH (Step E-1-2):

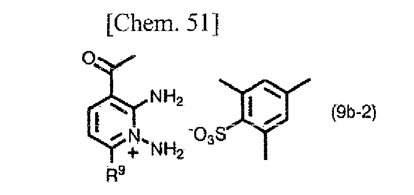

[wherein R⁹ is as defined above]

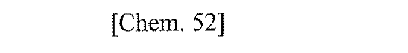

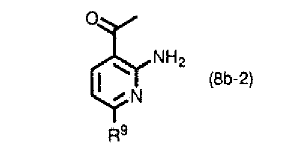

[wherein R⁹ is as defined above],

The reaction can be carried out in the same manner as in Step E-1-1.

In Synthesis Pathway E, a compound represented by the general formula (10b-2) can be prepared by allowing the compound represented by the general formula (9b-2) to act on the compound represented by the general formula (23) in the presence of a base (Step E-2-2):

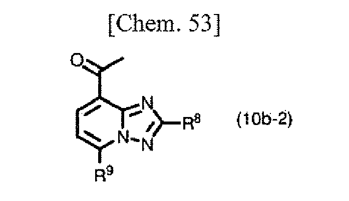

[wherein R⁸ and R⁹ are as defined above].

The reaction can be carried out in the same manner as in Step E-2-1.

In Synthesis Pathway E, a compound represented by the general formula (11b-2) can be prepared by allowing the compound represented by the general formula (10b-2) to react with the compound represented by the general formula (17) in the presence of a base (Step E-3-2):

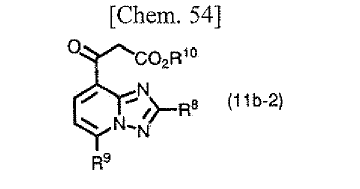

[wherein R⁸, R⁹, and R¹⁰ are as defined above]_{.}

The reaction can be carried out in the same manner as in Step D-2.

In Synthesis Pathway E, a compound represented by the general formula (12b) can be prepared by treating the compound represented by the general formula (11b-1) with a base, followed by reacting with the compound represented by the general formula (19) (Step E-4-1):

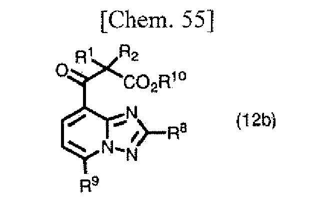

[wherein R¹, R², R⁸, R⁹, and R¹⁰ are as defined above].

The reaction can be carried out in the same manner as in Step D-4.

Further, the compound represented by the general formula (12b) can be prepared by treating the compound represented by the general formula (11b-2) with a base, followed by reacting with the compound represented by the general formula (22), and thereafter, further treating it with a base and then allowing the compound represented by the general formula (19) to act thereon (Step E-4-2).

The reaction can be carried out in the same manner as in Step D'-3.

In Synthesis Pathway E, the compound represented by the general formula (3b) can be prepared by allowing the compound represented by the general formula (12b) to act on a hydrazine derivative (Step E-5). The reaction can be carried out in the same manner as in Step D-6.

In Synthesis Pathway E, the compounds represented by the general formulae (10b-1) and (12b) can also be prepared by Synthesis Pathway E' below.

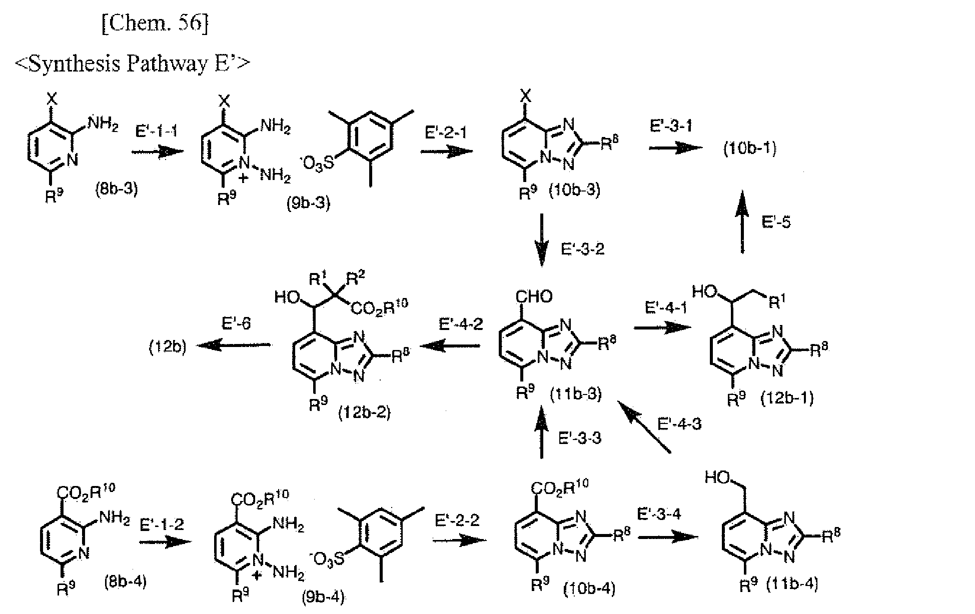

In Synthesis Pathway E', a compound represented by the general formula (9b-3) and a compound represented by the general formula (9b-4) can be prepared by allowing a compound represented by the general formula (8b-3) or a compound represented by the general formula (8b-4) to act on MSH, respectively, (Steps E'-1-1 and E'-1-2):

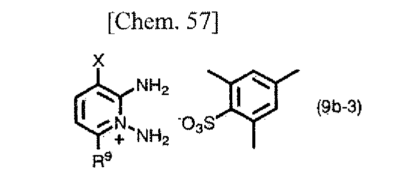

[wherein R⁹ and X are as defined above]

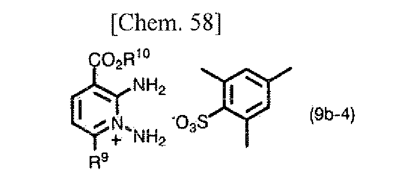

[wherein R⁹ and R¹⁰ are as defined above]

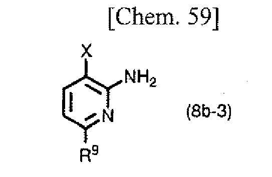

[wherein R⁹ and X are as defined above]

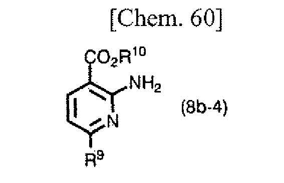

[wherein R⁹ and R¹⁰ are as defined above].

The reaction can be carried out in the same manner as in Step E-1-1.

In Synthesis Pathway E', a compound represented by the general formula (10b-3) or a compound represented by the general formula (10b-4) can be prepared by reacting the compound represented by the general formula (9b-3) or (9b-4) with the compound represented by the general formula (23), respectively, in the presence of a base (Steps E'-2-1 and E'-2-2):

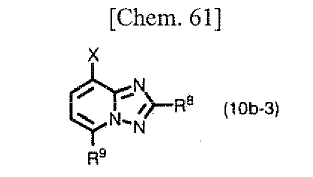

[wherein R⁸, R⁹, and X are as defined above]

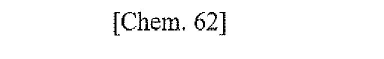

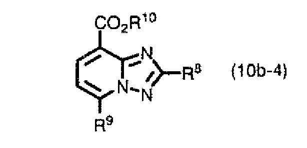

[wherein R⁸, R⁹, and R¹⁰ are as defined above].

The reaction can be carried out in the same manner as in Step E-2-1.

In Synthesis Pathway E', a compound represented by the general formula (11b-4) can be prepared by reducing the compound represented by the general formula (10b-4) (Step E'-3-4):

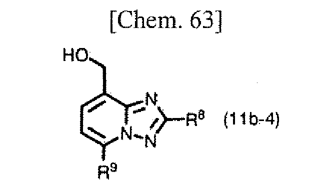

[wherein R⁸ and R⁹ are as defined above].

The reaction can be carried out at a reaction temperature from 0°C to underheating, and preferably at room temperature, using borane (BH₃), an alkylborane derivative such as 9-borabicyclo[3,3,1]nonane (9-BBN) or a metal hydrogen complex compound such as diisobutylaluminum hydride (DIBAL), lithium borohydride (LiBH₄), sodium borohydride (NaBH₄), lithium aluminum hydride (LiAlH₄), or the like, and preferably LiBH₄ and using THF, ethanol, methanol, or the like as a reaction solvent.

In Synthesis Pathway E', a compound represented by the general formula (11b-3) can be prepared by oxidizing the compound represented by the general formula (11b-4) (Step E'-4-3):

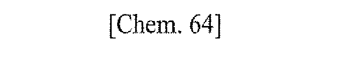

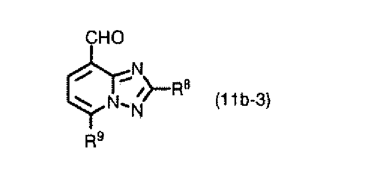

[wherein R⁸ and R⁹ are as defined above].

The reaction can be carried out in the same manner as in Step D-5.

Further, a compound represented by the general formula (11b-3) can also be prepared by treating the compound represented by the general formula (10b-3) with an organometalic reagent, followed by reacting with DMF or formic ester (Step E'-3-2).

The reaction can be carried out in the same manner as in Step D-1-4.

Further, the compound represented by the general formula (11b-3) can also be prepared by reducing the compound represented by the general formula (10b-4) (Step E'-3-3).

The reaction is preferably carried out at from -78°C to 0°C using DIBAL or RedAl as a reducing agent with dissolution in THF, ether, 1,4-dioxane, or the like.

In Synthesis Pathway E', a compound represented by the general formula (12b-1) can be prepared by reacting the compound represented by the general formula (11b-3) with a compound represented by the general formula (24) (Step E'-4-1):

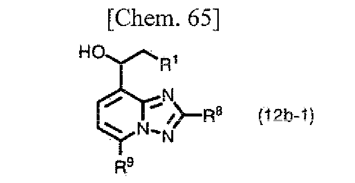

[wherein R¹, R⁸, and R⁹ are as defined above]

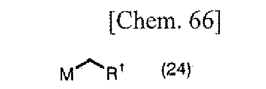

[wherein M represents lithium atom, MgCl, MgBr, or MgI, and R¹ is as defined above].

The reaction is preferably carried out by mixing both the compounds at from -78°C to 0°C, followed by, if necessary, warming to room temperature, using THF, ether, 1,4-dioxane, or the like as a solvent.

In Synthesis Pathway E', the compound represented by the general formula (10b-1) can be prepared by oxidizing the compound represented by the general formula (12b-1) (Step E'-5).

The reaction can be carried out in the same manner as in Step D-5.

Further, the compound represented by the general formula (10b-1) can also be prepared by treating the compound represented by the general formula (10b-3) with an organometalic reagent, followed by reacting with the compound represented by the general formula (14) or the compound represented by the general formula (15) (Step E'-3-1).

The reaction can be carried out in the same manner as in Step D-1-1.

In Synthesis Pathway E', a compound represented by the general formula (12b-2) can be prepared by allowing the compound represented by the general formula (18) to act on the compound represented by the general formula (11b-3) in the presence of a Lewis acid (Step E'-4-2):

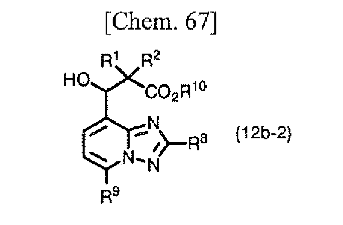

[wherein R¹, R², R⁸, R⁹, and R¹⁰ are as defined above].

The reaction can be carried out in the same manner as in Step D-3.

In Synthesis Pathway E', the compound represented by the general formula (12b) can be prepared by oxidizing the compound represented by the general formula (12b-2) (Step E'-6)
The reaction can be carried out in the same manner as in Step D-5.

In Synthesis Pathways E and E', the compound represented by the general formula (10b-1), wherein R⁹ is an alkoxy group having 1 to 6 carbon atoms, that is, a compound represented by the general formula (10b-5) can also be prepared by the method shown in Synthesis Pathway E" below:

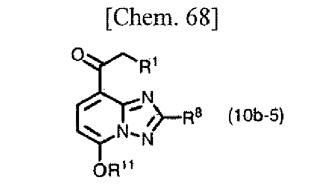

[wherein R¹¹ represents an alkyl group having 1 to 6 carbon atoms, and R¹ and R⁸ are as defined above].

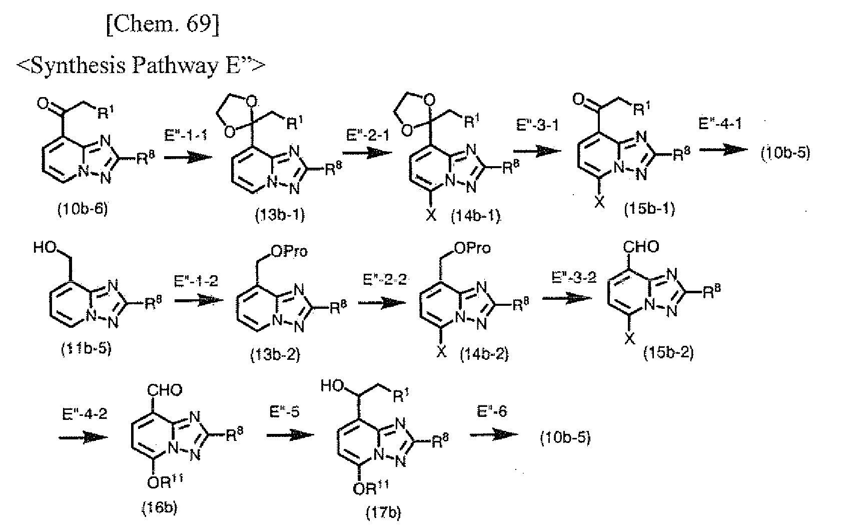

In Synthesis Pathway E", a compound represented by the general formula (13b-1) can be prepared by reacting the compound represented by the general formula (10b-1) in which R⁹ is a hydrogen atom, that is, a compound represented by the general formula (10b-6) with ethylene glycol (Step E"-1-1):

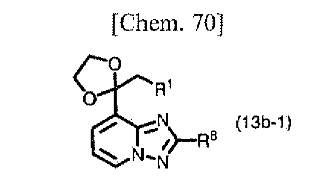

[wherein R¹ and R⁸ are as defined above]

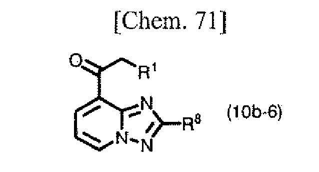

[wherein R¹ and R⁸ are as defined above].

The reaction is preferably carried out with heating under reflux in a solvent such as benzene, toluene, xylene, or the like, preferably under dehydration using a Dean-Stark trap using a catalytic amount of paratoluenesulfonic acid or pyridinium paratoluenesulfonate.

In Synthesis Pathway E", a compound represented by the general formula (14b-1) can be prepared by halogenating the compound represented by the general formula (13b-1) (Step E"-2-1):

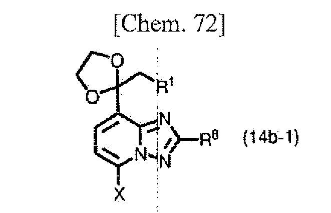

[wherein R¹, R⁸, and X are as defined above].

The reaction is preferably carried out by performing a reaction at from -78°C to 0°C in THF as a solvent using a base such as butyl lithium, lithium hexamethyl disilazide, LDA, or the like, and preferably LDA, and then allowing NCS, NBS, NIS, bromine, iodine, 1,2-dibromoethane, or 1,2-diiodoethane to act thereon.

In Synthesis Pathway E", a compound represented by the general formula (15b-1) can be prepared by deprotecting the compound represented by the general formula (14b-1) (Step E"-3-1):

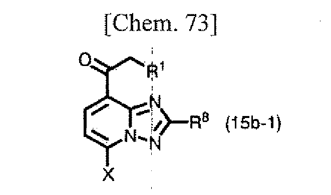

[wherein R¹, R⁸, and X are as defined above].

The reaction is preferably carried out by allowing paratoluenesulfonic acid to act thereon at from room temperature to heating temperature under reflux in acetone as a solvent, or by performing a reaction at from 0°C to room temperature using hydrogen chloride-containing methanol, ethanol, ethyl acetate, or diethyl ether.

In Synthesis Pathway E", a compound represented by the general formula (13b-2) can be prepared by subjecting the compound of the general formula (11b-4) in which R⁹ is a hydrogen atom, that is, a compound represented by the general formula (11b-5) to various reactions for introducing an alcohol protecting group (Step E"-1-2):

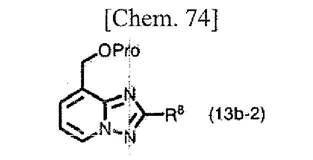

[wherein Pro represents an alcohol protecting group, such as a methoxymethylyl group, a t-butyldimethylsilyl group, a t-butyldiphenylsilyl group, a triisopropylsilyl group, a tetrahydropyranyl group, an acetyl group, or the like, and R⁸ is as defined above].

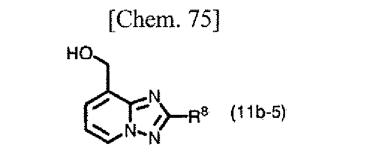

[wherein R⁸ is as defined above].

In the case of the introduction of a methoxymethyl group, it is preferable to allow methoxymethyl chloride or methoxymethyl bromide to act thereon at from 0°C to room temperature in a solvent such as THF, dichloromethane, acetonitrile, or the like, and preferably dichloromethane, in the presence of sodium hydride, triethylamine, ethyldiisopropylamine, or the like. Further, in the case of the introduction of a t-butyldimethylsilyl group, a t-butyldiphenylsilyl group, or a triisopropylsilyl group, the reaction is preferably carried out by allowing the corresponding silyl chloride, silyl bromide, or silyl trifluoromethanesulfonate to act thereon at from 0°C to room temperature in a solvent such as THF, DMF, acetonitrile, methylene chloride, or the like in the presence of triethylamine, imidazole, or the like. For the introduction of a tetrahydropyranyl group, it is preferable to add an acidic catalyst such as paratoluenesulfonic acid, or the like in the presence of dihydropyrane, and allow it to act thereon in methylene chloride. Further, in the case of the introduction of an acetyl group, acetyl chloride, acetyl bromide, or acetic anhydride can be allowed to act thereon at from 0°C to room temperature in a solvent such as THF, 1,4-dioxane, or methylene chloride in the presence of an organic base such as triethylamine, ethyldiisopropylamine, pyridine, or the like, or the reaction can be carried out using pyridine also as a solvent from 0°C to room temperature.

In Synthesis Pathway E", a compound represented by the general formula (14b-2) can be prepared by halogenating the compound represented by the general formula (13b-2) (Step E"-2-2):

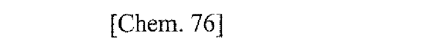

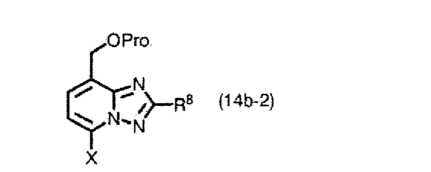

[wherein R⁸, X, and Pro are as defined above].

The reaction can be carried out in the same manner as in Step E"-2-1.

In Synthesis Pathway E", a compound represented by the general formula (15b-2) can be prepared by deprotecting and oxidizing the compound represented by the general formula (14b-2) (Step E"-3-2):

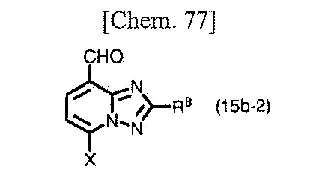

[wherein R⁸ and X are as defined above].

In the case of a methoxymethyl group or a tetrahydropyranyl group, the deprotection reaction is preferably carried out at from 0°C to room temperature using hydrogen chloride-containing methanol, ethanol, ethyl acetate, or diethyl ether. In the case of a silyl protecting group, the reaction is preferably carried out at from 0°C to room temperature in acetonitrile or THF as a solvent, using potassium fluoride, cesium fluoride, or tetrabutyl ammonium fluoride. Further, in the case of an acetyl group, the reaction is preferably carried out at from 0°C to room temperature using an aqueous sodium hydroxide solution, an aqueous potassium hydroxide solution or an aqueous lithium hydroxide solution and using THF, methanol, ethanol, 1,4-dioxane, or the like as a solvent. Examples of the oxidation reaction include a chromium oxide-pyridine complexes such as pyridinium chlorochromate, pyridinium dichromate, or the like, a metal oxidant such as chromium oxide, silver carbonate, manganese dioxide, or the like, DMSO oxidation using various DMSO activators including, a sulfur trioxide-pyridine complex, oxalyl chloride, anhydrous trifluoroacetic acid, acetic anhydride, DCC, or the like, and hypervalency iodine oxidation such as IBX, Dess-Martin periodinane oxidation, or the like.

In Synthesis Pathway E", a compound represented by the general formula (16b) can be prepared by reacting the compound represented by the general formula (15b-2) with a compound represented by the general formula (25) (Step E"-4-2):

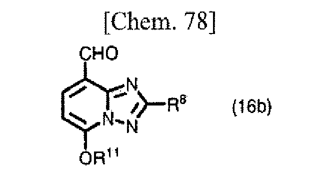

[wherein R⁸ and R¹¹ are as defined above]

[Chem. 79] R¹¹OQ³ (25)

[wherein Q³ represents a sodium atom, a potassium atom, or a lithium atom, and R¹¹ is as defined above].

The reaction can be carried out at from room temperature to heating temperature under reflux, using methanol, ethanol, propanol, or butanol.

In Synthesis Pathway E", a compound represented by the general formula (17b) can be prepared by reacting the compound represented by the general formula (16b) with the compound represented by the general formula (24) (Step E"-5);

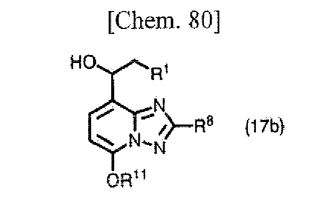

[wherein R¹, R⁸, and R¹¹ are as defined above].

The reaction can be carried out in the same manner as in Step E'-4-1.

In Synthesis Pathway E", the compound represented by the general formula (10b-5) can be prepared by oxidizing the compound represented by the general formula (17b) (Step E"-6).

The reaction can be carried out in the same manner as in Step D-5.

Further, the compound represented by the general formula (10b-5) can be prepared by reacting the compound represented by the general formula (15b-1) with the compound represented by the general formula (25) (Step E"-4-1).

The reaction can be carried out in the same manner as in Step E"-4-2.

The compound represented by the general formula (3), wherein Heterocycle 1 is an imidazopyridine ring, that is, a compound represented by the general formula (3c) can be prepared via Synthesis Pathway F below:

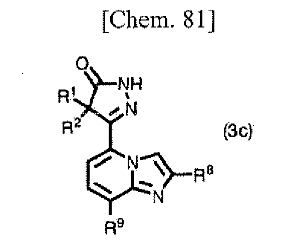

[wherein R¹, R², R⁸, and R⁹ are as defined above].

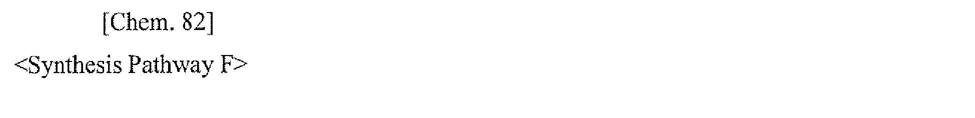

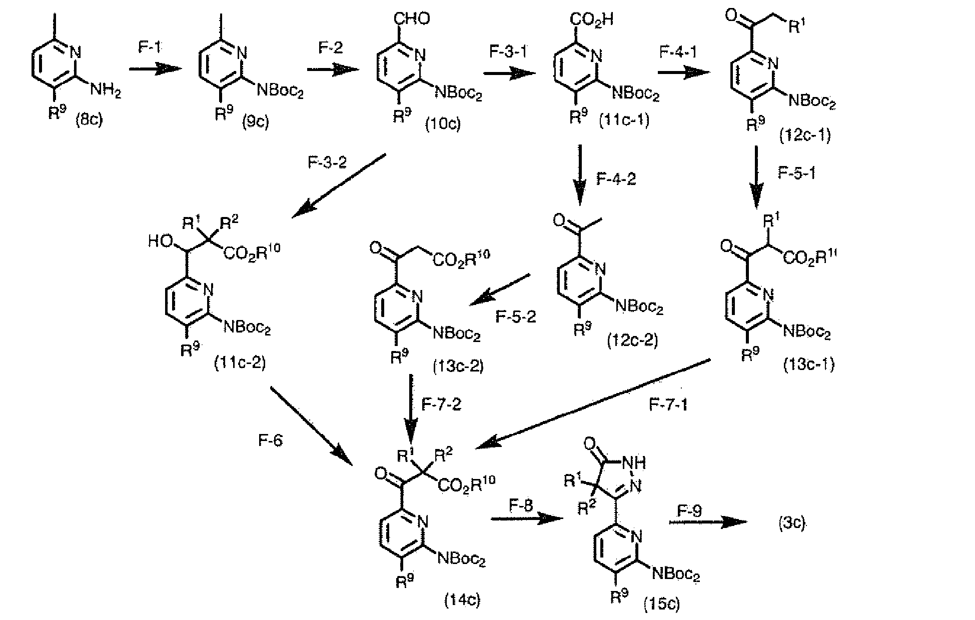

In Synthesis Pathway F, a compound represented by the general formula (9c) can be prepared by the Boc-addition of the compound represented by the general formula (8c) (Step F-1):

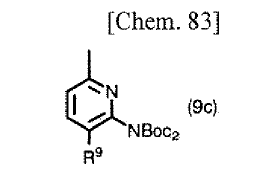

[wherein Boc represents a t-butoxycarbonyl group and R⁹ is as defined above]

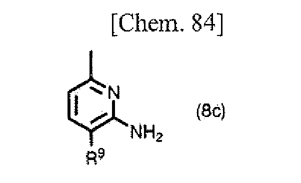

[wherein R⁹ is as defined above].

The reaction can be carried out at room temperature with the addition of Boc₂O, triethylamine, and 4-dimethyl aminopyridine (DMAP), using a solvent such as acetonitrile, t-butanol, or the like, and preferably acetonitrile.

In Synthesis Pathway F, a compound represented by the general formula (10c) can be prepared by halogenating the compound represented by the general formula (9c), followed by reacting with N-methylmorpholine-N-oxide (NMO) (Step F-2):

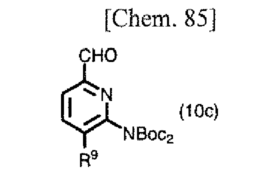

[wherein R⁹ and Boc are as defined above].

The reaction is preferably carried out with heating under reflux in a solvent such as methylene chloride, chloroform, carbon tetrachloride, or the like, and preferably carbon tetrachloride, with the addition of a catalytic amount of a radical initiator such as benzoyl peroxide, azoisobutyronitrile, or the like, using NCS, NBS, or NIS. Further, the reaction can be efficiently completed by light irradiation instead of heating under reflux.

By adding a Molecular Sieve 4A, NMO to the halogeno form obtained by the reaction as described above, and carrying out the reaction using acetonitrile as a solvent under an inert gas atmosphere at room temperature, its conversion into an aldehyde can be attained.

In Synthesis Pathway F, a compound represented by the general formula (11c-1) can be prepared by oxidizing the compound represented by the general formula (10c) (Step F-3-1):

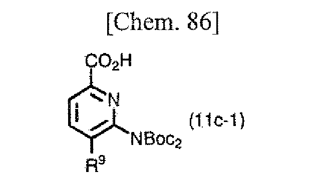

[wherein R⁹ and Boc are as defined above].

The reaction is preferably carried out at room temperature with the addition of sodium chlorite, sodium dihydrogen phosphate, 2-methyl-2-butene, and water, using t-butanol as a solvent.

In Synthesis Pathway F, a compound represented by the general formula (12c-1) can be prepared by condensing the compound represented by the general formula (11c-1) with N,O-dimethylhydroxyamine, followed by reacting with the compound represented by the general formula (24) (Step F-4-1):

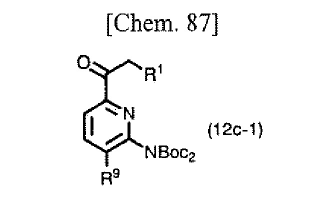

[wherein R¹, R⁹, and Boc are as defined above].

The condensation reaction with N,O-dimethylhydroxyamine is preferably carried out using a condensing agent such as DCC, diisopropylcarbodiimide (DIPC), diphenylphosphoryl azide (DPPA), diethylphosphoryl cyanide (DEPC), 1-ethyl-3-(3-dimethylaminopropyl)-carbodiimide (WSC), or the like in the presence of an organic base such as triethylamine, pyridine, or the like, the reaction is preferably carried out from 0°C to room temperature, using THF, DMF, DMSO, methylene chloride, or the like as a reaction solvent or as the occasion demands, with the addition of a catalytic amount of DMAP. It is preferable that the amide form thus obtained is dissolved in a solvent such as THF, diethyl ether, 1,4-dioxane, or the like, and the compound represented by the general formula (24) is added at from -78°C to 0°C, followed by, if necessary, heating to room temperature.

In Synthesis Pathway F, a compound represented by the general formula (12c-2) can be prepared by condensing the compound represented by the general formula (11c-1) with N,O-dimethylhydroxyamine, followed by reacting with a compound represented by the general formula (26) (Step F-4-2):

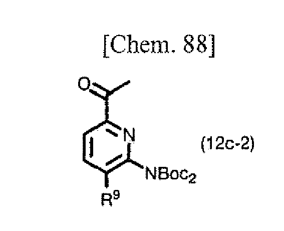

[wherein R⁹ and Boc are as defined above]

[Chem. 89] **M-CH₃** (26)

[wherein M is as defined above].

The reaction can be carried out in the same manner as in Step F-4-1.

In Synthesis Pathway F, a compound represented by the general formula (11c-2) can be prepared by allowing the compound represented by the general formula (10c) to act on the compound represented by the general formula (18) in the presence of a Lewis acid (Step F-3-2):

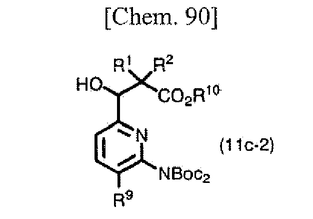

[wherein R¹, R², R⁹, R¹⁰, and Boc are as defined above].

The reaction can be carried out in the same manner as in Step D-3.

In Synthesis Pathway F, a compound represented by the general formula (13c-1) can be prepared by reacting the compound represented by the general formula (12c-1) with the compound represented by the general formula (17) in the presence of a base (Step F-5-1):

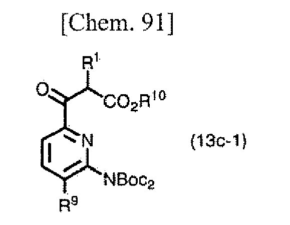

[wherein R¹, R⁹, R¹⁰, and Boc are as defined above].

The reaction can be carried out in the same manner as in Step D-2.

In Synthesis Pathway F, a compound represented by the general formula (13c-2) can be prepared by reacting the compound represented by the general formula (12c-2) with the compound represented by the general formula (17) in the presence of a base (Step F-5-2):

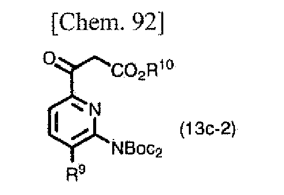

[wherein R⁹, R¹⁰, and Boc are as defined above].

The reaction can be carried out in the same manner as in Step D-2.

In Synthesis Pathway F, a compound represented by the general formula (14c) can be prepared by oxidizing the compound represented by the general formula (11c-2) (Step F-6):

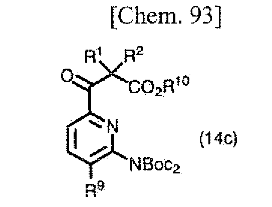

[wherein R¹, R², R⁹, R¹⁰, and Boc are as defined above].
The reaction can be carried out in the same manner as in Step D-5.

Further, the compound represented by the general formula (14c) can be prepared by treating the compound represented by the general formula (13c-1) with a base, followed by reacting with the compound represented by the general formula (19) (Step F-7-1).

The reaction can be carried out in the same manner as in Step D-4.

Further, the compound represented by the general formula (14c) can be prepared by treating the compound represented by the general formula (13c-2) with a base, followed by reacting with the compound represented by the general formula (22), and thereafter, further treating it with a base and then allowing the compound represented by the general formula (19) to act thereon (Step F-7-2).

The reaction can be carried out in the same manner as in Step D'-3.

In Synthesis Pathway F, a compound represented by the general formula (15c) can be prepared by allowing a hydrazine derivative to act on the compound represented by the general formula (14c) (Step F-8):

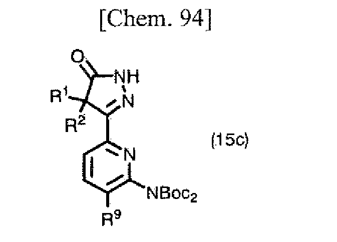

[wherein R¹, R², R⁹, and Boc are as defined above].

The reaction can be carried out in the same manner as in Step D-6.

In Synthesis Pathway F, the compound represented by the general formula (3c) can be prepared by deprotecting the compound represented by the general formula (15c), followed by reacting with a compound represented by the general formula (27) (Step F-9):

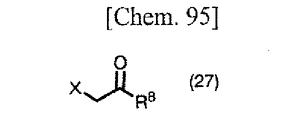

[wherein R⁸ and X are as defined above].

The deprotection reaction can be carried out at room temperature by the addition of hydrochloric acid -containing methanol, ethanol, diethyl ether, 1,4-dioxane, ethyl acetate, or trifluoroacetic acid. Subsequently, the reaction with the compound represented by the general formula (27) is preferably carried out at from 70°C to heating temperature under reflux, using a solvent such as methanol, ethanol, and the like, and preferably ethanol.

The compound represented by the general formula (3), wherein Heterocycle 1 is a benzothiazole ring, and is linked at its position 7, that is, a compound represented by the general formula (3d) can be prepared via Synthesis Pathway G below:

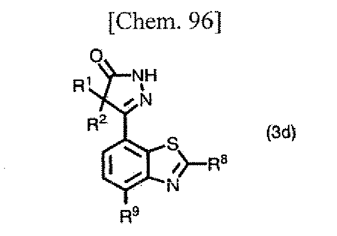

[wherein R¹, R², R⁸, and R⁹ are as defined above].

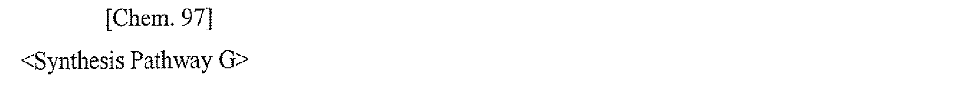

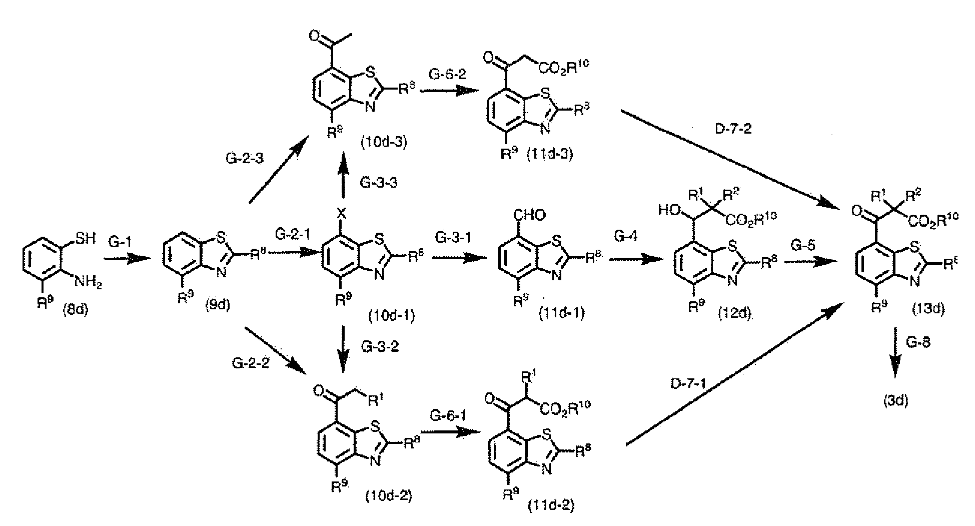

In Synthesis Pathway G, a compound represented by the general formula (9d) can be prepared by reacting a compound represented by the general formula (8d) and a compound represented by the general formula (28) (Step G-1):

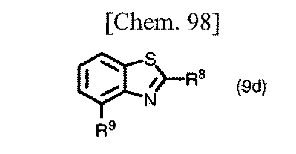

[wherein R⁸ and R⁹ are as defined above]

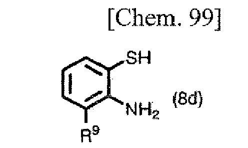

[wherein R⁹ is as defined above]

[Chem. 100] **R⁸CO²H** (28)

[wherein R⁸ is as defined above].

The reaction is preferably carried out using the compound represented by the general formula (28) as a solvent as well as a reactive agent, with the addition of trimethylsilyl polyphosphate (from room temperature to 120C), particularly at 90°C.

In Synthesis Pathway G, a compound represented by the general formula (10d-1) can be prepared by halogenating the compound represented by the general formula (9d) (Step G-2-1):

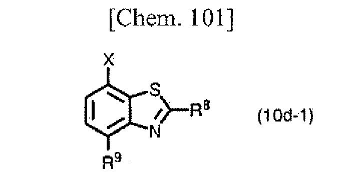

[wherein R⁸, R⁹, and X are as defined above].
The reaction can be carried out at from room temperature to heating temperature under reflux, and preferably at 70°C in a solvent such as dichloromethane, chloroform, carbon tetrachloride, acetonitrile, DMF, and the like, and preferably acetonitrile, with the addition of NCS, NBS, or NIS.

In Synthesis Pathway G, a compound represented by the general formula (10d-2) can be prepared by reacting the compound represented by the general formula (9d) with the compound represented by the general formula (16) (Step G-2-2):

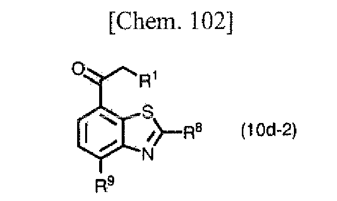

[wherein R¹, R⁸, and R⁹ are as defined above].

The reaction can be carried out in the same manner as in Step D-1-2.

Further, the compound represented by the general formula (10d-2) can also be prepared by treating the compound represented by the general formula (10d-1) with an organometalic reagent, followed by reacting with the compound represented by the general formula (14) or the compound represented by the general formula (15) (Step G-3-2).

The reaction can be carried out in the same manner as in Step D-1-2.

In Synthesis Pathway G, a compound represented by the general formula (10d-3) can be prepared by reacting the compound represented by the general formula (9d) with the compound represented by the general formula (21) (Step G-2-3):

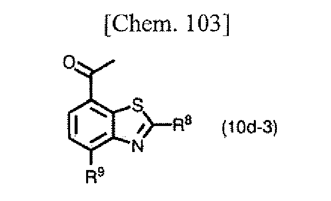

[wherein R⁸ and R⁹ are as defined above].

The reaction can be carried out in the same manner as in Step D'-1-2.

Further, the compound represented by the general formula (10d-3) can also be prepared by treating the compound represented by the general formula (10d-1) with an organometalic reagent, followed by reacting with the compound represented by the general formula (20) or acetic anhydride (Step G-3-3).

The reaction can be carried out in the same manner as in Step D'-1-1.

In Synthesis Pathway G, a compound represented by the general formula (11d-1) can be prepared by treating the compound represented by the general formula (10d-1) with an organometalic reagent, followed by reacting with DMF or formic ester (Step G-3-1):

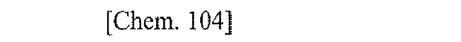

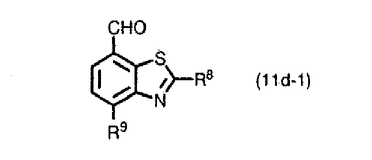

[wherein R⁸ and R⁹ are as defined above].

The reaction can be carried out in the same manner as in Step D-1-4.

In Synthesis Pathway G, a compound represented by the general formula (12d) can be prepared by allowing the compound represented by the general formula (11d-1) to act on the compound represented by the general formula (18) in the presence of a Lewis acid (Step G-4):

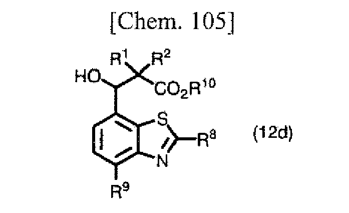

[wherein R¹, R², R⁸, R⁹, and R¹⁰ are as defined above].

The reaction can be carried out in the same manner as in Step D-3.

In Synthesis Pathway G, a compound represented by the general formula (11d-2) can be prepared by reacting the compound represented by the general formula (10d-2) with the compound represented by the general formula (17) in the presence of a base (Step G-6-1):

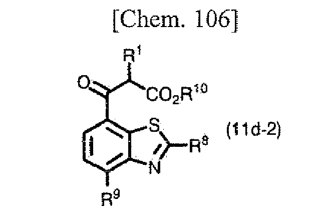

[wherein R¹, R⁸, R⁹, and R¹⁰ are as defined above].

The reaction can be carried out in the same manner as in Step D-2.

In Synthesis Pathway G, a compound represented by the general formula (11d-3) can be prepared by reacting the compound represented by the general formula (10d-3) with the compound represented by the general formula (17) in the presence of a base (Step G-6-2):

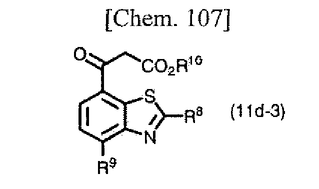

[wherein R⁸, R⁹, and R¹⁰ are as defined above].

The reaction can be carried out in the same manner as in Step D-2.

In Synthesis Pathway G, a compound represented by the general formula (13d) can be prepared by oxidizing the compound represented by the general formula (12d) (Step G-5):

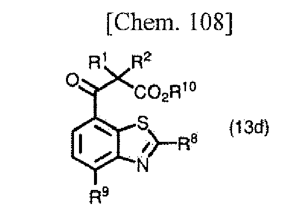

[wherein R¹, R², R⁸, R⁹, and R¹⁰ are as defined above].
The reaction can be carried out in the same manner as in Step D-5.

Further, the compound represented by the general formula (13d) can be prepared by treating the compound represented by the general formula (11d-2) with a base, followed by reacting with the compound represented by the general formula (19) (Step D-7-1).

The reaction can be carried out in the same manner as in Step D-4.

Further, the compound represented by the general formula (13d) can be prepared by treating the compound represented by the general formula (11d-3) with a base, followed by reacting with the compound represented by the general formula (22), and thereafter, further treating it with a base and then allowing the compound represented by the general formula (19) to act thereon (Step D-7-2).

The reaction can be carried out in the same manner as in Step D'-3.

In Synthesis Pathway G, the compound represented by the general formula (3d) can be prepared by allowing a hydrazine derivative to act on the compound represented by the general formula (13d) (Step G-8).

The reaction can be carried out in the same manner as in Step D-6.

The compound represented by the general formula (3), wherein Heterocycle 1 is a benzimidazole ring, that is, a compound represented by the general formula (3e) can be prepared by Synthesis Pathway H below:

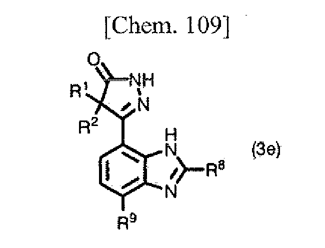

[wherein R¹, R², R⁸, and R⁹ are as defined above]

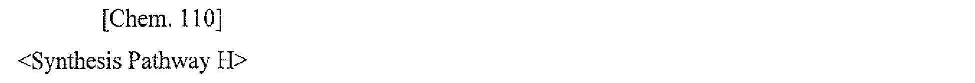

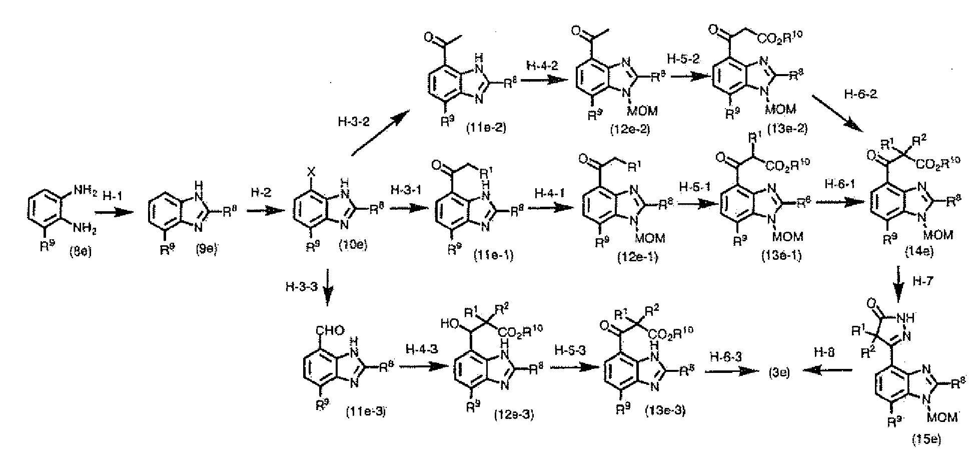

In Synthesis Pathway H, a compound represented by the general formula (9e) can be prepared by reacting a compound represented by the general formula (8e) with the compound represented by the general formula (28) (Step H-1):

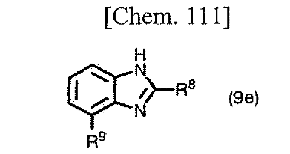

[wherein R⁸ and R⁹ are as defined above]

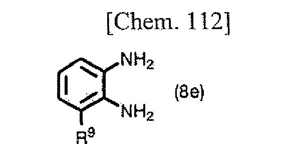

[wherein R⁹ is as defined above]

The reaction can be carried out in the same manner as in Step G-1.

In Synthesis Pathway H, a compound represented by the general formula (10e) can be prepared by halogenating the compound represented by the general formula (9e) (Step H-2):

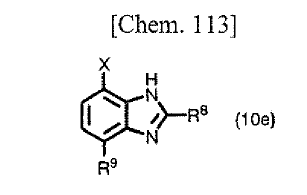

[wherein R⁸, R⁹, and X are as defined above].

The reaction can be carried out in the same manner as in Step G-2-1.

In Synthesis Pathway H, a compound represented by the general formula (11e-1) can be prepared by treating the compound represented by the general formula (10e) with an organometalic reagent, followed by reacting with the compound represented by the general formula (14) or the compound represented by the general formula (15) (Step H-3-1):

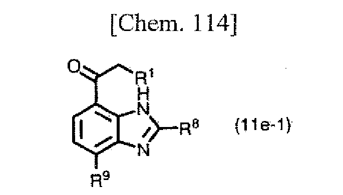

[wherein R¹, R⁸, and R⁹ are as defined above].

The reaction can be carried out in the same manner as in Step D-1-1.

In Synthesis Pathway H, a compound represented by the general formula (11e-2) can be prepared by treating a compound represented by the general formula (10e) with an organometalic reagent, followed by reacting with the compound represented by the general formula (20) or acetic anhydride (Step H-3-2):

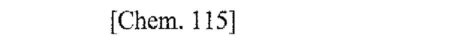

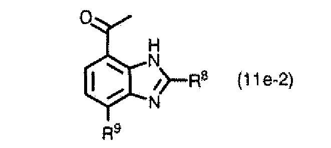

[wherein R⁸ and R⁹ are as defined above].

The reaction can be carried out in the same manner as in Step D'-1-1.

In Synthesis Pathway H, a compound represented by the general formula (12e-1) can be prepared by protecting the compound represented by the general formula (11e-1) with a MOM group (Step H-4-1):

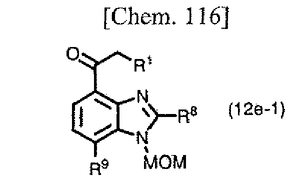

[wherein MOM represents a methoxymethyl group, and R¹, R⁸, and R⁹ are as defined above].

For the reaction, the reaction is preferably carried out at from 0°C to room temperature by reacting the compound represented by the general formula (11e-1) with methoxymethylchloride or methoxymethylbromide in a solvent such as THF, acetonitrile, DMF, dichloromethane, and the like, and preferably DMF in the presence of a base such as triethylamine, diisopropylethylamine, pyridine, and the like.

In Synthesis Pathway H, a compound represented by the general formula (12e-2) can be prepared by protecting the compound represented by the general formula (11e-2) with a MOM group (Step H-4-2):

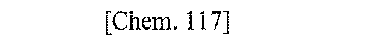

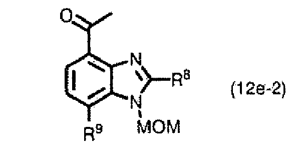

[wherein R⁸, R⁹, and MOM are as defined above].

The reaction can be carried out in the same manner as in Step H-4-1.

In Synthesis Pathway H, a compound represented by the general formula (13e-1) can be prepared by reacting the compound represented by the general formula (12e-1) with the compound represented by the general formula (17) in the presence of a base (Step H-5-1):

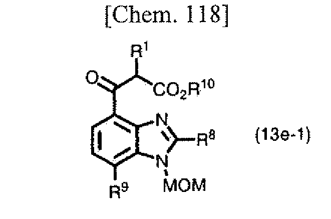

[wherein R¹, R⁸, R⁹, R¹⁰, and MOM are as defined above].

The reaction can be carried out in the same manner as in Step D-2.

In Synthesis Pathway H, a compound represented by the general formula (13e-2) can be prepared by reacting the compound represented by the general formula (12e-2) with the compound represented by the general formula (17) in the presence of a base (Step H-5-2):

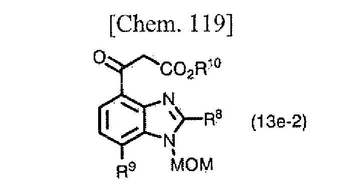

[wherein R⁸, R⁹, R¹⁰ and MOM are as defined above].

The reaction can be carried out in the same manner as in Step D-2.

In Synthesis Pathway H, a compound represented by the general formula (14e) can be prepared by treating the compound represented by the general formula (13e-1) with a base, followed by reacting with the compound represented by the general formula (19) (Step H-6-1):

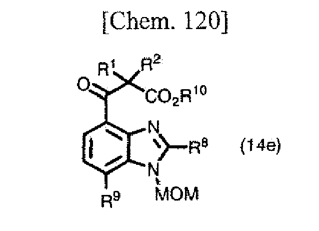

[wherein R¹, R², R⁸, R⁹, R¹⁰, and MOM are as defined above].

The reaction can be carried out in the same manner as in Step D-4.

Further, the compound represented by the general formula (14e) can be prepared by treating the compound represented by the general formula (13e-2) with a base, followed by reacting with the compound represented by the general formula (22), and thereafter, further treating it with a base and then allowing the compound represented by the general formula (19) to act thereon (Step H-6-2).

The reaction can be carried out in the same manner as in Step D'-3.

In Synthesis Pathway H, a compound represented by the general formula (15e) can be prepared by allowing a hydrazine derivative to act on the compound represented by the general formula (14e) (Step H-7):

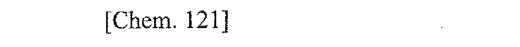

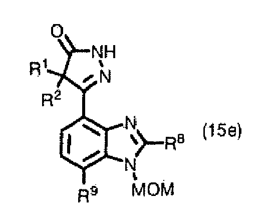

[wherein R¹, R², R⁸, R⁹, and MOM are as defined above].

The reaction can be carried out in the same manner as in Step D-6.

In Synthesis Pathway H, a compound represented by the general formula (11e-3) can be prepared by treating the compound represented by the general formula (10e) with an organometalic reagent, followed by reacting with DMF or formic ester (Step H-3-3):

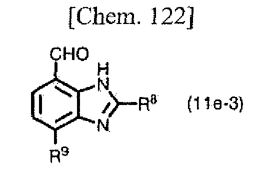

[wherein R⁸ and R⁹ are as defined above].

The reaction can be carried out in the same manner as in Step D-1-4.

In Synthesis Pathway H, a compound represented by the general formula (12e-3) can be prepared by allowing the compound represented by the general formula (11e-3) to act on the compound represented by the general formula (18) in the presence of a Lewis acid (Step H-4-3):

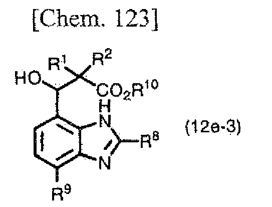

[wherein R¹, R², R⁸, R⁹, and R¹⁰ are as defined above].

The reaction can be carried out in the same manner as in Step D-3.

In Synthesis Pathway H, a compound represented by the general formula (13e-3) can be prepared by oxidizing the compound represented by the general formula (12e-3) (Step H-5-3):

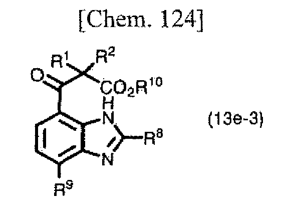

[wherein R¹, R², R⁸, R⁹, and R¹⁰ are as defined above].
The reaction can be carried out in the same manner as in Step D-5.

In Synthesis Pathway H, the compound represented by the general formula (3e) can be prepared by deprotecting the compound represented by the general formula (15e) (Step H-8).

The reaction is preferably carried out at room temperature in a solvent such as methanol, ethanol, diethyl ether, THF, 1,4-dioxane, and the like, with the addition of hydrochloric acid.

Further, the compound represented by the general formula (3e) can be prepared by allowing a hydrazine derivative to act on the compound represented by the general formula (13e-3) (Step H-6-3).

The reaction can be carried out in the same manner as in Step D-6.

The compound represented by the general formula (3), wherein Heterocycle 1 is a benzofuran ring or a benzothiophene ring, that is, a compound represented by the general formula (3f) can be prepared by Synthesis Pathway J below:

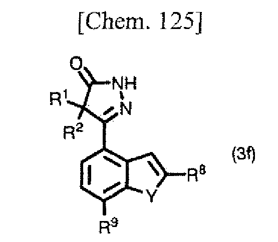

[wherein Y represents an oxygen atom or a sulfur atom, and R¹, R², R⁸, and R⁹ are as defined above].

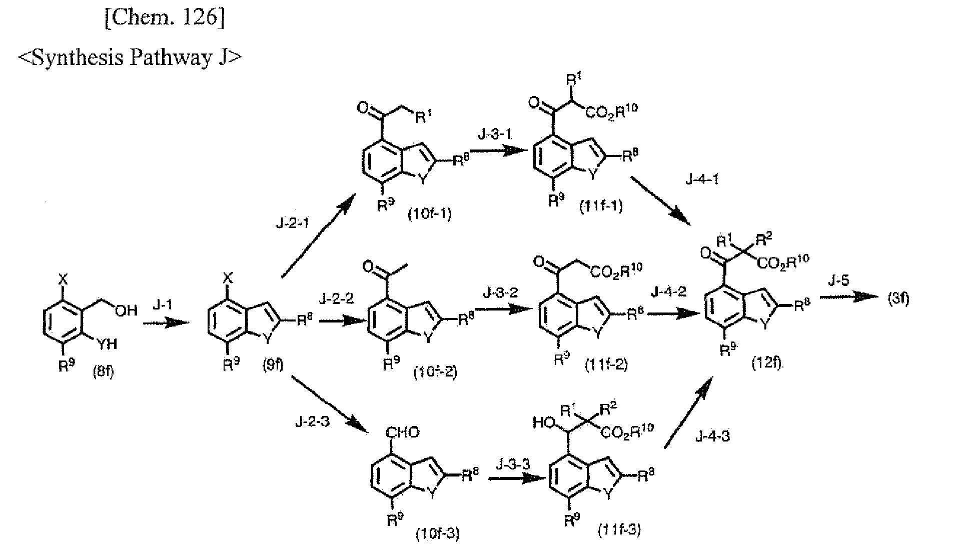

In Synthesis Pathway J, a compound represented by the general formula (9f) can be prepared by reacting the compound represented by the general formula (8f) with triphenylphosphonium bromide, followed by reacting with the compound represented by the general formula (23) (Step J-1):

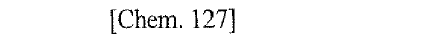

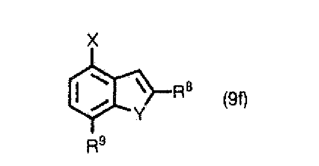

[wherein R⁸, R⁹, X, and Y are as defined above]

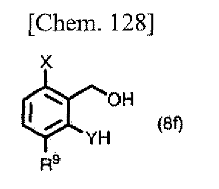

[wherein R⁹, X, and Y are as defined above].

The reaction is preferably carried out by performing the reaction with heating under reflux with the addition of triphenylphosphonium bromide, using a solvent such as acetonitrile, THF, 1,4-dioxane, ethyl acetate, and the like, and preferably acetonitrile, and then performing the reaction with heating under reflux with the addition of triethylamine and the compound represented by the general formula (23), with changing the reaction solvent to toluene, benzene, or xylene, and preferably toluene.

In Synthesis Pathway J, a compound represented by the general formula (10f-1) can be prepared by treating the compound represented by the general formula (9f) with an organometalic reagent, followed by reacting with the compound represented by the general formula (14) or the compound represented by the general formula (15) (Step J-2-1):

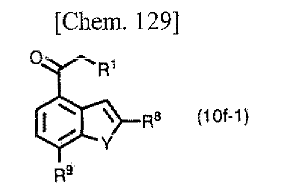

[wherein R¹, R⁸, R⁹ and Y are as defined above].

The reaction can be carried out in the same manner as in Step D-1-1.

In Synthesis Pathway J, a compound represented by the general formula (10f-2) can be prepared by treating the compound represented by the general formula (9f) with an organometalic reagent, followed by reacting with the compound represented by the general formula (20) or acetic anhydride (Step J-2-2):

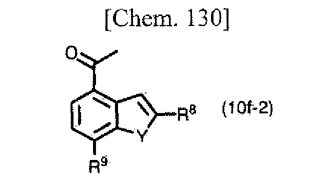

[wherein R⁸, R⁹, and Y are as defined above].

The reaction can be carried out in the same manner as in Step D'-1-1.

In Synthesis Pathway J, a compound represented by the general formula (11f-1) can be prepared by reacting the compound represented by the general formula (10f-1) with the compound represented by the general formula (17) in the presence of a base (Step J-3-1):

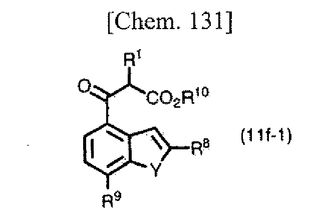

[wherein R¹, R⁸, R⁹, R¹⁰, and Y are as defined above].

The reaction can be carried out in the same manner as in Step D-2.

In Synthesis Pathway J, a compound represented by the general formula (11f-2) can be prepared by reacting the compound represented by the general formula (10f-2) with the compound represented by the general formula (17) in the presence of a base (Step J-3-2):

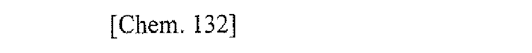

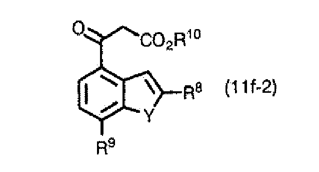

[wherein R⁸, R⁹, R¹⁰, and Y are as defined above].

The reaction can be carried out in the same manner as in Step D-2.

In Synthesis Pathway J, a compound represented by the general formula (10f-3) can be prepared by treating the compound represented by the general formula (9f) with an organometalic reagent, followed by reacting with DMF or formic ester (Step J-2-3):

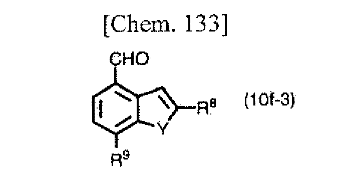

[wherein R⁸, R⁹, and Y are as defined above].

The reaction can be carried out in the same manner as in Step D-1-4.

In Synthesis Pathway J, a compound represented by the general formula (11f-3) can be prepared by allowing the compound represented by the general formula (10f-3) to act on the compound represented by the general formula (18) in the presence of a Lewis acid (Step J-3-3):

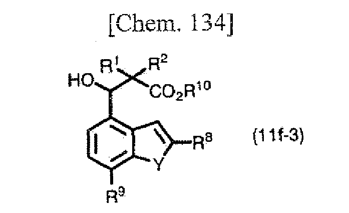

[wherein R¹, R², R⁸, R⁹, R¹⁰, and Y are as defined above].

The reaction can be carried out in the same manner as in Step D-3.

In Synthesis Pathway J" a compound represented by the general formula (12f) can be prepared by treating the compound represented by the general formula (11f-1) with a base, followed by reacting with the compound represented by the general formula (19) (Step J-4-1):

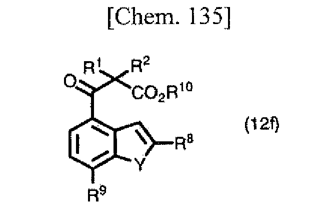

[wherein R¹, R², R⁸, R⁹, R¹⁰, and Y are as defined above].

The reaction can be carried out in the same manner as in Step D-4.

Further, the compound represented by the general formula (12f) can be prepared by treating the compound represented by the general formula (11f-2) with a base, followed by reacting with the compound represented by the general formula (22), and thereafter, further treating it with a base and then allowing the compound represented by the general formula (19) to act thereon (Step J-4-2).

The reaction can be carried out in the same manner as in Step D'-3.

Further, the compound represented by the general formula (12f) can be prepared by oxidizing the compound represented by the general formula (11f-3) (Step J-4-3)
The reaction can be carried out in the same manner as in Step D-5.

In Synthesis Pathway J, the compound represented by the general formula (3f) can be prepared by allowing a hydrazine derivative to act on the compound represented by the general formula (12f) (Step J-5).

The reaction can be carried out in the same manner as in Step D-6.

The compound represented by the general formula (3), wherein Heterocycle 1 is an indolidine ring, that is, a compound represented by the general formula (3g) can be prepared by Synthesis Pathway K below:

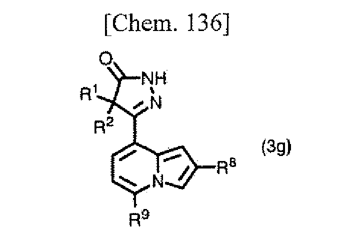

[wherein R¹, R², R⁸, and R⁹ are as defined above].

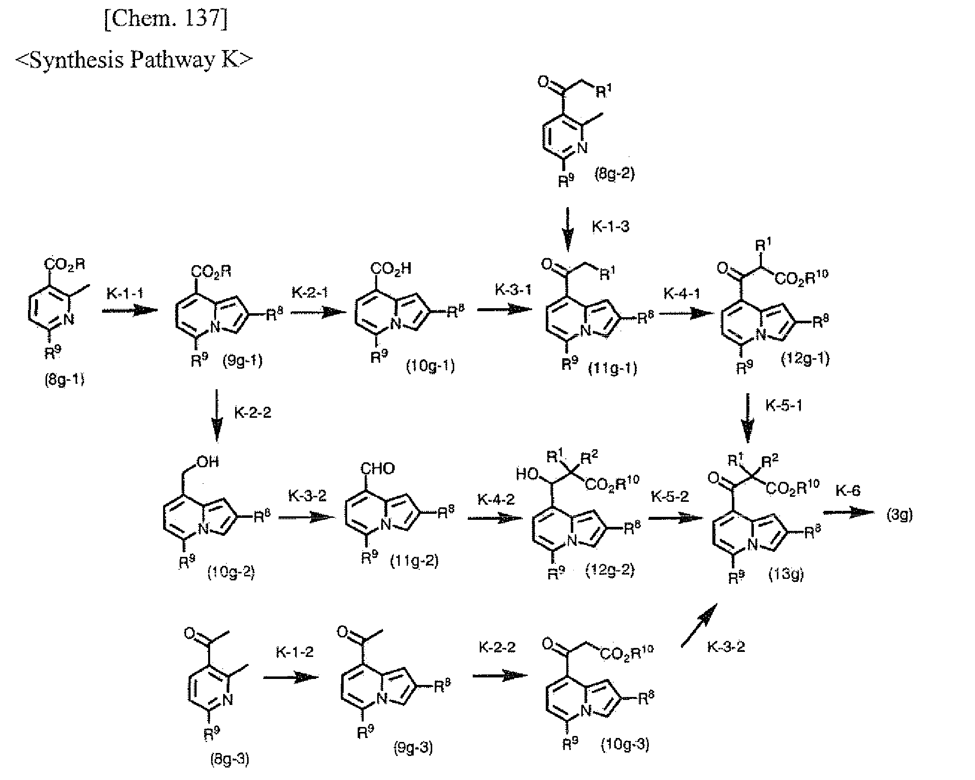

In Synthesis Pathway K, a compound represented by the general formula (9g-1) can be prepared by reacting a compound represented by the general formula (8g-1) with the compound represented by the general formula (27), followed by treating with a base (Step K-1-1):

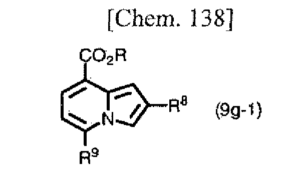

[wherein R represents an alkyl group having 1 to 6 carbon atoms or a benzyl group, and R⁸ and R⁹ are as defined above]

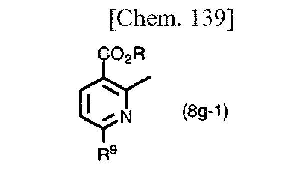

[wherein R⁹ and R are as defined above].

The reaction is preferably carried out by heating at from 50°C to 100°C without a solvent or using THF, benzene, toluene, methylene chloride, chloroform, ethyl acetate, or the like as a solvent, and then heating at from 80°C to 130°C with the addition of 1,8-diazabicyclo[5,4,0]undec-7-ene.

In Synthesis Pathway K, a compound represented by the general formula (10g-1) can be prepared by hydrolyzing the compound represented by the general formula (9g-1) (Step K-2-1):

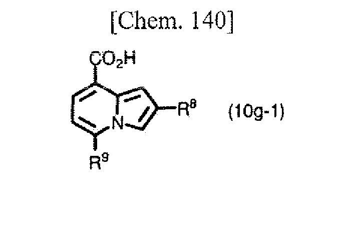

[wherein R⁸ and R⁹ are as defined above].

The reaction can be carried out at from 0°C to 80°C using methanol, ethanol, THF, DMF, DMSO, or the like as a solvent with the addition of an aqueous solution of sodium hydroxide, potassium hydroxide, or lithium hydroxide.

In Synthesis Pathway K, a compound represented by the general formula (11g-1) can be prepared by condensing the compound represented by the general formula (10g-1) with N,O-dimethylhydroxyamine, followed by reacting with the compound represented by the general formula (24) (Step K-3-1):

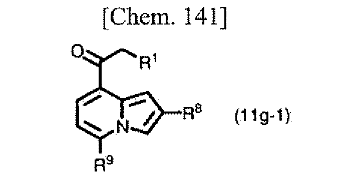

[wherein R¹, R⁸, and R⁹ are as defined above].

The reaction can be carried out in the same manner as in Step F-4-1.

Further, the compound represented by the general formula (11g-1) can also be prepared by reacting a compound represented by the general formula (8g-2) with the compound represented by the general formula (27), followed by treating with a base (Step K-1-3):

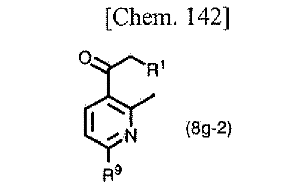

[wherein R¹ and R⁹ are as defined above].

The reaction can be carried out in the same manner as in Step K-1-1.

In Synthesis Pathway K, a compound represented by the general formula (12g-1) can be prepared by reacting the compound represented by the general formula (11g-1) with the compound represented by the general formula (17) in the presence of a base (Step K-4-1):

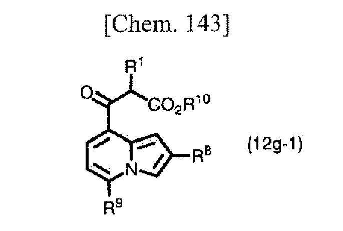

[wherein R¹, R⁸, R⁹, and R¹⁰ are as defined above].

The reaction can be carried out in the same manner as in Step D-2.

In Synthesis Pathway K, a compound represented by the general formula (10g-2) can be prepared by reducing the compound represented by the general formula (9g-1) (Step K-2-2):

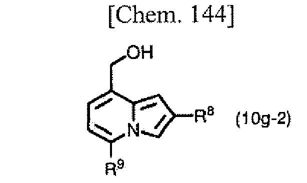

[wherein R⁸ and R⁹ are as defined above].

The reaction can be carried out in the same manner as in Step E'-3-4.

In Synthesis Pathway K, a compound represented by the general formula (11g-2) can be prepared by oxidizing the compound represented by the general formula (10g-2) (Step K-3-2):

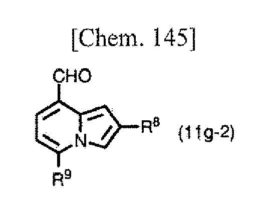

[wherein R⁸ and R⁹ are as defined above].

The reaction can be carried out in the same manner as in Step D-5.

In Synthesis Pathway K, a compound represented by the general formula (12g-2) can be prepared by allowing the compound represented by the general formula (11g-2) and the compound represented by the general formula (18) to act thereon in the presence of a Lewis acid (Step K-4-2):

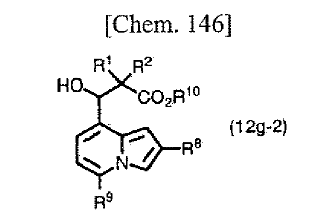

[wherein R¹, R², R⁸, R⁹, and R¹⁰ are as defined above].

The reaction can be carried out in the same manner as in Step D-3.

In Synthesis Pathway K, a compound represented by the general formula (9g-3) can also be prepared by reacting a compound represented by the general formula (8g-3) with the compound represented by the general formula (27); followed by treating with a base (Step K-1-2):

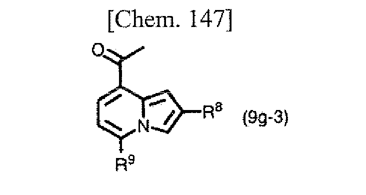

[wherein R⁸ and R⁹ are as defined above]

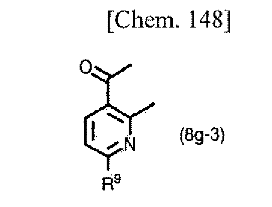

[wherein R⁹ is as defined above].

The reaction can be carried out in the same manner as in Step K-1-1.

In Synthesis Pathway K, a compound represented by the general formula (10g-3) can be prepared by reacting a compound represented by the general formula (9g-3) with the compound represented by the general formula (17) in the presence of a base (Step K-2-2):

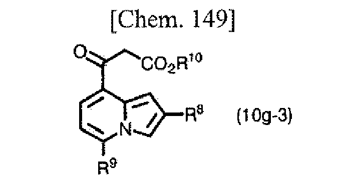

[wherein R⁸, k⁹, and R¹⁰ are as defined above].

The reaction can be carried out in the same manner as in Step D-2.

In Synthesis Pathway K, a compound represented by the general formula (13g) can be prepared by treating the compound represented by the general formula (12g-1) with a base, followed by reacting with the compound represented by the general formula (19) (Step K-5-1):

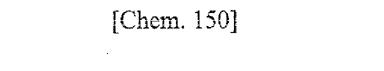

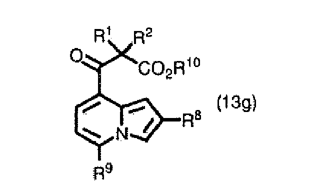

[wherein R¹, R², R⁸, R⁹, and R¹⁰ are as defined above].

The reaction can be carried out in the same manner as in Step D-4.

Further, the compound represented by the general formula (13 g) can be prepared by treating the compound represented by the general formula (10g-3) with a base, followed by reacting with the compound represented by the general formula (22), and thereafter, further treating it with a base and then allowing the compound represented by the general formula (19) to act thereon (Step K-3-2).

The reaction can be carried out in the same manner as in Step D'-3.

Further, the compound represented by the general formula (13g) can be prepared by oxidizing the compound represented by the general formula (12g-2) (Step K-5-2).
The reaction can be carried out in the same manner as in Step D-5.

In Synthesis Pathway K, the compound represented by the general formula (3g) can be prepared by allowing a hydrazine derivative to act on the compound represented by the general formula (13g) (Step K-6).

The reaction can be carried out in the same manner as in Step D-6.

In Synthesis Pathway K, the compound represented by the general formula (11g-1), wherein R⁹ is an alkoxy group having 1 to 6 carbon atoms, that is, a compound represented by the general formula (15g') can be prepared by Synthesis Pathway K' below:

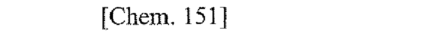

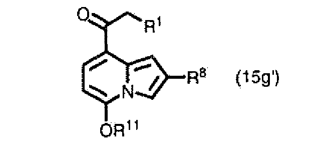

[wherein R¹, R⁸, and R¹¹ are as defined above]

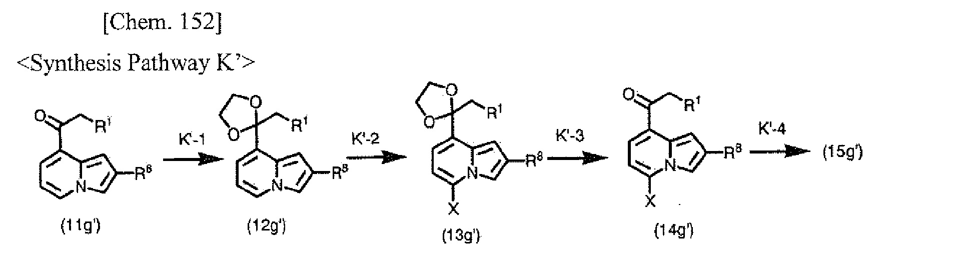

In Synthesis Pathway K', a compound represented by the general formula (12g') can be prepared by reacting the compound represented by the general formula (11g-1), wherein R⁹ is a hydrogen atom, that is, a compound represented by the general formula (11g') with ethylene glycol (Step K'-1):

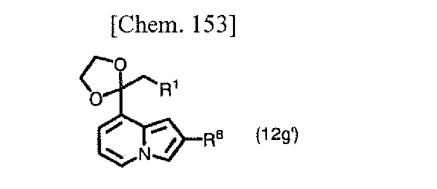

[wherein R¹ and R⁸ are as defined above]

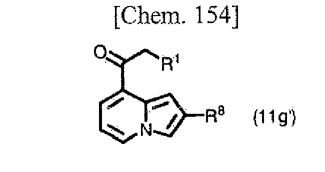

[wherein R¹ and R⁸ are as defined above].

The reaction can be carried out in the same manner as in Step E"-1-1.

In Synthesis Pathway K', a compound represented by the general formula (13g') can be prepared by halogenating the compound represented by the general formula (12g') (Step K'-2):

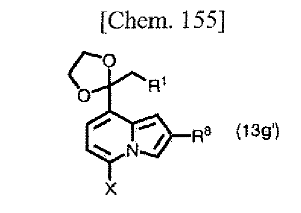

[wherein R¹, R⁸, and X are as defined above].

The reaction can be carried out in the same manner as in Step E"-2-1.

In Synthesis Pathway K', a compound represented by the general formula (14g') can be prepared by deprotecting the compound represented by the general formula: (13g') (Step K'-3):

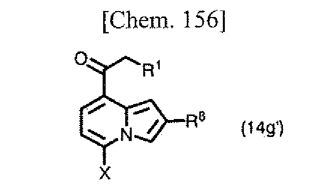

[wherein R¹, R⁸, and X are as defined above].

The reaction can be carried out in the same manner as in Step E"-3-1.

In Synthesis Pathway K', the compound represented by the general formula (15g') can be prepared by reacting the compound represented by the general formula (14g') with the compound represented by the general formula (25) (Step K'-4).

The reaction can be carried out in the same manner as in Step E"-4-2.

In Synthesis Pathway K, the compound represented by the general formula (11g-2), wherein R⁹ is an alkoxy group having 1 to 6 carbon atoms, that is, a compound represented by the general formula (14g") can be prepared by Synthesis Pathway K" below:

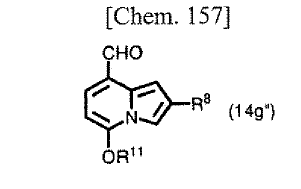

[wherein R⁸ and R¹¹ are as defined above].

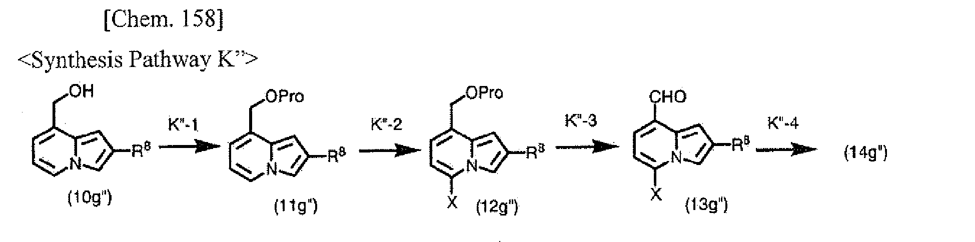

In Synthesis Pathway K", a compound represented by the general formula (11g") can be prepared by subjecting the compound represented by the general formula (10g-2), wherein R⁹ is a hydrogen atom, that is, a compound represented by the general formula (10g") to various reactions for introducing an alcohol protecting group (Step K"-1):

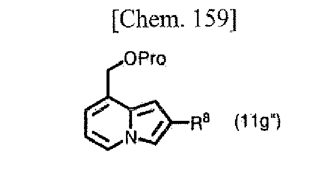

[wherein R⁸ and Pro are as defined above]

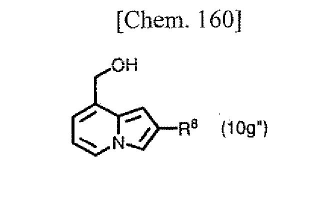

[wherein R⁸ is as defined above].

The reaction can be carried out in the same manner as in Step E"-1-2.

In Synthesis Pathway K", a compound represented by the general formula (12g") can be prepared by halogenating the compound represented by the general formula (11g") (Step K"-2):

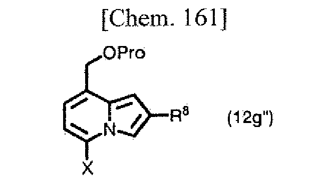

[wherein R⁸, X, and Pro are as defined above].

The reaction can be carried out in the same manner as in Step E"-2-1.

In Synthesis Pathway K", a compound represented by the general formula (13g") can be prepared by deprotecting and oxidizing the compound represented by the general formula (12g") (Step K"-3):

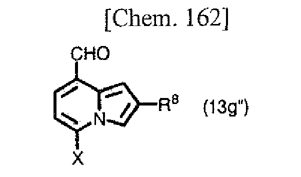

[wherein R⁸ and X are as defined above].

The reaction can be carried out in the same manner as in Step E"-3-2.

In Synthesis Pathway K", the compound represented by the general formula (14g") can be prepared by reacting the compound represented by the general formula (13g") with the compound represented by the general formula (25) (Step K"-4).

The reaction can be carried out in the same manner as in Step E"-4-2.

### EXAMPLES

Hereinbelow, the present invention will be described with reference to specific Examples, but the present invention is not limited to these Examples.

### <Example 1>

### 5-Bromo-8-methoxy-2-methylquinoline

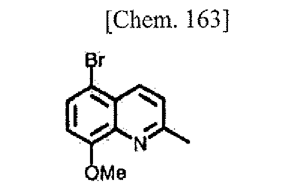

Commercially available 8-methoxy-2-methylquinoline (7.92 g) was dissolved in methanol (80 mL), and bromine (2.37 mL) was added dropwise thereto under ice cooling, followed by stirring at room temperature for 1.5 hours. To the reaction liquid was added a saturated aqueous sodium thiosulfate solution and further added a saturated aqueous sodium hydrogen carbonate solution, followed by evaporating methanol under reduced pressure. This aqueous solution was extracted with ethyl acetate, and the organic layer was washed with water and saturated brine in that order, and then dried over anhydrous sodium sulfate. The solvent was evaporated under reduced pressure to obtain the desired product (47.6 g) as a yellow powder.
¹H-NMR (CDCl₃, 400 MHz): δ 2.83 (3H, s), 4.07 (3H, s), 6.92 (1H, d, J=8.6 Hz), 7.42 (1H, d, J=8.6 Hz), 7.66 (1H, d, J=8.6 Hz), 8.38 (1H, d, J=8.6 Hz).

### <Example 2>

### 2-Ethyl-8-methoxyquinoline

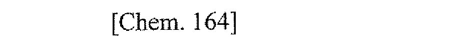

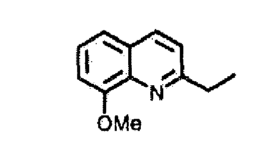

Commercially available 8-hydroxy-2-methylquinoline (7.00 g) was dissolved in THF (100 mL), and tetrabutyl ammonium bromide (700 mg), iodomethane (8.20 mL), and a 50% aqueous sodium hydroxide solution (8.8 mL) were added thereto in that order, followed by stirring at room temperature for 6 hours. After evaporating THF under reduced pressure, the residue was extracted three times with ethyl acetate, and the combined extracted layer was washed with saturated brine, dried over anhydrous sodium sulfate, and filtered. After evaporating the solvent under reduced pressure, the residue was then purified by silica gel column chromatography (hexane:ethyl acetate=1:2) to obtain 8-methoxy-2-methylquinoline (7.29 g) as a colorless powder.

The obtained 8-methoxy-2-methylquinoline (7.29 g) was dissolved in THF (210 mL) under an argon atmosphere, and a solution (2.71 mol/L, 17.1 mL) of n-butyl lithium in hexane was added thereto at -78°C, followed by stirring at 0°C for 30 minutes. Iodomethane (2.88 mL) was added thereto at -78°C, followed by stirring at room temperature for 3.5 hours. To the reaction liquid was added a saturated aqueous ammonium chloride solution, followed by extraction three times with ethyl acetate, and the combined extracted layer was washed with saturated brine, and then dried over anhydrous sodium sulfate and filtrated. After evaporating the solvent under reduced pressure, the residue was then purified by silica gel column chromatography (hexane:ethyl acetate=2:1) to obtain the desired product (7.36 g) as a colorless powder.
¹H-NMR (CDCl₃, 400 MHz): δ 1.40 (3H, t, J=7.3 Hz), 3.08 (2H, q, J=7.3 Hz), 4.08 (3H, s), 7.03 (1H, dd, J=7.3, 1.2 Hz), 7.34-7.40 (3H, m), 8.05 (1H, d, J=8.6 Hz).

### <Example 3>

### 5-Bromo-2-ethyl-8-methoxyquinoline

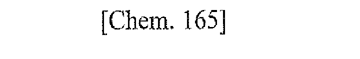

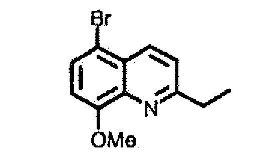

The compound of Example 2 (7.36 g) was dissolved in methanol (80 mL), and bromine (2.10 mL) was added thereto, followed by stirring at room temperature for 45 minutes. A saturated aqueous sodium hydrogen carbonate solution was added to the reaction liquid, followed by extraction three times with ethyl acetate, and the combined extracted layer was washed with saturated brine, and then dried over anhydrous sodium sulfate and filtered. After evaporating the solvent under reduced pressure, the residue was purified by silica gel column chromatography (hexane:ethyl acetate=2:1 to obtain the desired product (9.55 g) as a yellow powder.
¹H-NMR (CDCl₃, 400 MHz): δ 1.41 (3H, t, J=7.3 Hz), 3.12 (2H, q, J=7.3 Hz), 4.08 (3H, s), 692 (1H, d, J=8.6 Hz), 7.47 (1H, d, J=8.6 Hz), 7.66 (1H, d, J=8.6 Hz), 8.42 (1H, d, J=8.6 Hz).

### <Example 4>

### 5-Bromo-8-methoxy-2-isopropylquinoline

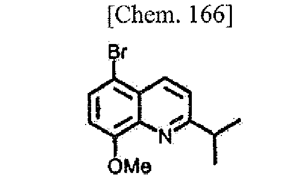

8-Methoxy-2-isopropylquinoline (J. Org. Chem., 1965, 30,4311-4313.) (4.09 g) was dissolved in methanol (35.2 mL), and bromine (1.15 mL) was added dropwise thereto under ice cooling, followed by stirring at room temperature for 2 hours. To the reaction liquid was added a saturated aqueous sodium thiosulfate solution and further added a saturated aqueous sodium hydrogen carbonate solution, followed by evaporating methanol under reduced pressure. This aqueous solution was extracted with ethyl acetate, and the organic layer was washed with water and saturated brine in that order, and then dried over anhydrous sodium sulfate. After evaporating the solvent under reduced pressure, the residue was purified by silica gel column chromatography (hexane:ethyl acetate=2:1) to obtain the desired product (5.00 g) as a yellow powder.
¹H-NMR (CDCl₃, 400 MHz): δ 1.39 (6H, d, J=8.0 Hz), 3.36-3.46 (1H, m), 4.06 (3H, s), 6.90 (1H, d, J=8.6 Hz), 7.49 (1H, d, J=8.6 Hz), 7.64 (1H, d, J=8.6 Hz), 8.43 (1H, d, J=8.6 Hz).

### <Examples 5>

### 8-Methoxy-2-triSuoromethylquinolin-4-one

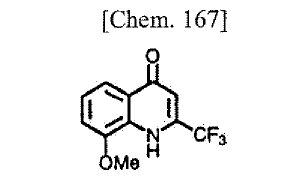

Commercially available 2-methoxyaniline (5.00 mL) was dissolved in diphenyl ether (100 mL), and ethyl 3-trifluoromethylpropionate (8.10 g) was added thereto, followed by stirring at 100°C for I hour and at 250°C for 1 hour. After leaving to be cooled, hexane was added thereto, and the precipitated crystal was collected by filtration to obtain the desired product (3.78 g) as a yellow powder.
¹H-NMR (CDCl₃, 400 MHz): δ 4.05 (3H, s), 6.63 (1H, s), 7.13 (1H, dd, J=8.2, 1.2 Hz), 7.34 (1H, t, J=8.2 Hz), 7.90 (1H, dd, J=8.2, 1.2 Hz), 8.71(1H, brs).

### <Example 6>

### 4-Chloro-8-methoxy-2-trifluoromethylquinoline

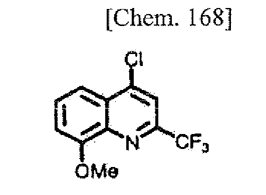

The compound of Example 5 (5.0 g) was dissolved in phosphorous oxychloride (100 mL) under an argon atmosphere, followed by stirring for 2 hours in the condition of heating under reflux. The phosphorous oxychloride was evaporated under reduced pressure, and a saturated aqueous sodium hydrogen carbonate solution was added thereto, followed by extraction three times with ethyl acetate. The combined extracted layer was washed with saturated brine, dried over anhydrous sodium sulfate, and filtered. After evaporating the solvent of the filtrate under reduced pressure, the residue was purified by silica gel column chromatography (hexane:ethyl acetate=10:1) to obtain the desired product (5.17 g) as a colorless powder.
¹H-NMR (CDCl₃, 400 MHz): δ 4.12 (3H, s), 7.20 (1H, d, J=7.9 Hz), 7.70 (1H, t, J=7.9 Hz), 7.85-7.87 (2H, m).

### <Example 7>

### 8-Methoxy-2-trifluoromethylquinoline

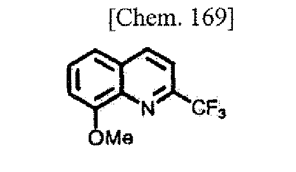

The compound of Example 6 (5.17 g) was dissolved in ethanol (100 mL), and 10% palladium-carbon (500 mg) was added thereto, followed by replacing with hydrogen and then stirring at room temperature for 2 hours. The reaction liquid was filtered, and after evaporating the solvent of the filtrate under reduced pressure, the residue was purified by silica gel column chromatography (hexane:ethyl acetate=15:1) to obtain a tetrahydro form (3.60 g) as a yellow oil and the desired product (814 mg) as a colorless powder. The tetrahydro form (3.60 g) was dissolved in acetic acid (70 mL), and bichromate potassium (2.75 g) was added thereto, followed by stirring at room temperature for 1 hour and at 90°C for 3 hours. It was neutralized with an aqueous sodium hydroxide solution and then extracted three times with ethyl acetate, and the combined extracted layer was washed with saturated brine, and then dried over anhydrous sodium sulfate and filtered. After evaporating the solvent of the filtrate under reduced pressure, the residue was purified by silica gel column chromatography (hexane:ethyl acetate=15:1) to obtain the desired product (2.24 g) as a colorless powder.
¹H-NMR (CDCl₃, 400 MHz): δ 4.11 (3H, s), 7.15 (1H, d, J=8.6 Hz), 7.47 (1H, d, J=8.6 Hz), 7.60 (1H, d, J=8.6 Hz), 7.77 (1H, d, J=8.6 Hz), 8.33 (1H, d, J=8.6 Hz).

### <Example 8>

### 5-Bromo-8-methoxy-2-trifluoromethylquinoline

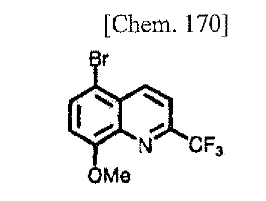

The compound of Example 7 (3.05 g) was dissolved in methanol (30 mL), and bromine (0.763 mL) was added thereto, followed by stirring at room temperature for 50 minutes. A saturated aqueous sodium hydrogen carbonate solution was added to the reaction liquid, followed by extraction three times with ethyl acetate, and the combined extracted layer was washed with saturated brine, and then dried over anhydrous sodium sulfate and filtered. After evaporating the solvent of the filtrate under reduced pressure, the residue was purified by silica gel column chromatography (hexane:ethyl acetate=15:1) to obtain the desired product (3.86 g) as a red powder.
¹H-NMR (CDCl₃, 400 MHz): δ 4.10 (3H, s), 7.03 (1H, d, J=8.6 Hz), 7.86 (1H, d, J=8.6 Hz), 7.87 (1H, d, J=8.6 Hz), 8.71 (1H, d, J=8.6 Hz).

### <Example 9>

### N-t-butoxycarbonyl-3-methoxy-2-nitroaniline

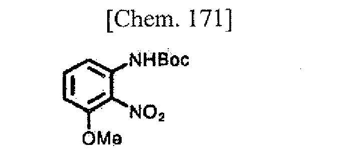

Commercially available 3-methoxy-2-nitrobenzoic acid (10.0 g) was dissolved in t-butanol (50.0 mL), and diphenylphosphoryl azide (11.5 mL) and triethylamine (7.40 mL) were added thereto, followed by stirring for 10 hours under the condition of heating under reflux. After evaporating the solvent under reduced pressure, the residue was diluted with ethyl acetate, washed with a saturated aqueous sodium hydrogen carbonate solution and saturated brine in this order, and then dried over anhydrous sodium sulfate. After evaporating the solvent under reduced pressure, the resulting solid was suspended in hexane and collected by filtration to obtain the desired product (13.3 g) as a yellow powder.
¹H-NMR (CDCl₃, 400 MHz): δ 1.50 (9H, s), 3.90 (3H, s), 6.71 (1H, dd, J=8.6, 1.2 Hz), 7.39 (1H, t, J=8.6, Hz), 7.55 (1H, brs), 7.77 (1H, dd, J=8.6, 1.2 Hz).

### <Examples 10>

### 3-Methoxy-2-nitroaniline

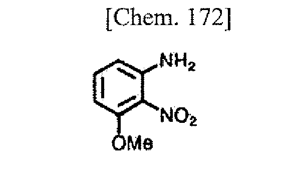

The compound of Example 9 (13.3 g) was dissolved in methylene chloride (100 mL), and trifluoroacetic acid (20.0 mL) was added thereto, followed by stirring at room temperature for 4 hours. After evaporating the solvent under reduced pressure, the residue was dissolved in ethyl acetate and poured into a saturated aqueous sodium hydrogen carbonate solution. The organic layer was separated out, washed with saturated brine, and then dried over anhydrous sodium sulfate. After evaporating the solvent under reduced pressure, the resulting solid was suspended in hexane and collected by filtration to obtain the desired product (7.55 g) as a yellow powder.
¹H-NMR (CDCl₃, 400 MHz): δ 3.88 (3H, s), 631 (1H, dd, J=8.6, 1.2 Hz), 7.36 (1H, dd, J=8.6, 1.2 Hz), 7.16 (1H, t, J=8.6, Hz).

### <Example 11>

### 2-Amino-3-methoxyaniline

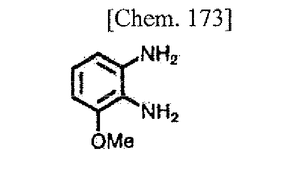

The compound of Example 10 (7.75 g) was dissolved in ethyl acetate (100 mL) and ethanol (100 mL), a few droplets of acetic acid were added thereto, and 10% palladium-activated carbon (775 mg) was added thereto, followed by stirring at room temperature for 11 hours under a hydrogen atmosphere. The insoluble materials were removed by filtration through Celite and the solvent of the filtrate was then evaporated under reduced pressure to obtain the desired product (6.49 g) as a yellowish brown oil. This was used in the next reaction without purification.

### <Examples 12>

### 4-Methoxy-2-trifluoromethyl-1H-benzimidazole

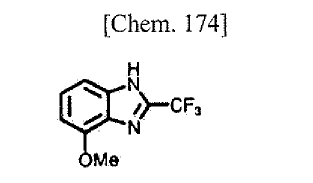

The compound of Example 11 (6.49 g) was dissolved in trifluoroacetic acid (75.0 mL) under ice cooling, followed by stirring for 5 hours under the condition of heating under reflux. After evaporating the solvent under reduced pressure, the residue was dissolved in ethyl acetate and poured into a saturated aqueous sodium hydrogen carbonate solution. The organic layer was separated out, washed with saturated brine, and then dried over anhydrous sodium sulfate. After evaporating the solvent under reduced pressure, the resulting solid was suspended in hexane and collected by filtration to obtain the desired product (8.69 g) as a yellowish brown powder.
¹H-NMR (CDOl₃, 400 MHz): δ 4.01 (3H, s), 6.89 (1H, d, J=8.0 Hz), 7.24 (1H, d, J=8.0 Hz), 7.32 (1H, t, J=8.0 Hz).

### <Example 13>

### 7-Bromo-4-methoxy-2-trifluoromethyl-1H-benzimidazole

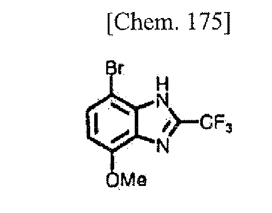

The compound of Example 12 (5.54 g) was dissolved in chloroform (130 mL), and NBS (5.02 g) was added thereto, followed by stirring at room temperature for 2 hours. To the reaction liquid was added a saturated aqueous sodium hydrogen carbonate solution, and the organic layer was separated out and then dried over anhydrous sodium sulfate. After evaporating the solvent under reduced pressure, the residue was purified by silica gel column chromatography (hexane:ethyl acetate=4:1) to obtain the desired product (47.2 mg) as a pale brown powder.
¹H-NMR (CDCl₃, 400 MHz): δ 4.00 (3H, s), 6.71 (1H, d, J=8.6 Hz), 7.46 (1H, d, J=8.6 Hz), 10.1 (1H, brs).

### <Example 14>

### 3-Bromo-2-hydroxymethyl-6-methoxyphenol

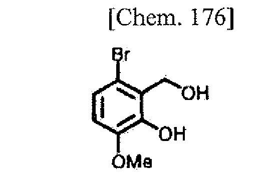

Commercially available 6-bromo-2-hydroxy-3-methoxybenzaldehyde (1.00 g) was dissolved in methanol (30 mL), and sodium borohydride (164 mg) was added thereto under stirring with ice cooling. After stirring at room temperature for 4 hours, a diluted hydrochloric acid was added thereto, followed by extraction with ethyl acetate. It was washed with water and saturated brine, and then dried over anhydrous sodium sulfate, and the solvent was evaporated under reduced pressure to obtain the desired product (911 mg) as a pale yellow powder.
¹H-NMR (CDCl₃, 400 MHz): δ 3.39(3H, s), 4.91(2H, s), 6.27(1H, s), 6.70(1H, d, J=8.6 Hz), 7.07(1H, d, J=8.6 Hz).

### <Example 15>

### (6-Bromo-2-hydroxy-3-methoxyphenyl)methyltriphenylphosphonium bromide

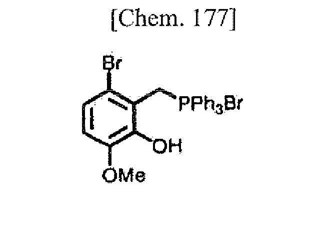

The compound of Example 14 (910 mg) was dissolved in acetonitrile (10 mL), and triphenyl phosphine hydrobromide (1.47 g) was added thereto, followed by heating under reflux for 5 hours. A half of the solvent was evaporated under reduced pressure, and ethyl acetate (50 mL) was added thereto. The precipitated crystal was collected by filtration and then dried to obtain the desired product (2.20 g) as a pale yellow powder.
¹H-NMR (DMSO-d₆, 400 MHz): δ 3.63(3H, s), 4.81(2H, d, J=14.1 Hz), 6.81(1H, dd, J=8.6, 1.8 Hz), 6.90(1H, dd, J=8.6, 0.6 Hz), 7.52-7.72(12H, m), 7.80-7.84(3H, m), 9.80(1H, s).

### <Example 16>

### 4-Bromo-7-methoxy-2-trifluoromethylbenzofuran

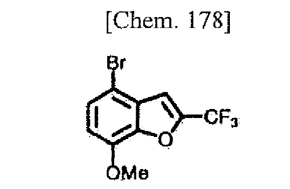

The compound of Example 15 (2.20 g) was suspended in toluene (20 mL) under an argon gas atmosphere, and trifluoroacetic anhydride (0.612 mL) and triethylamine (1.64 mL) were added thereto, followed by heating under reflux for 5 hours. To the reaction liquid was added water, followed by extraction with ethyl acetate and washing with saturated brine. After drying over anhydrous sodium sulfate, the solvent was evaporated under reduced pressure, and the residue was purified by silica gel column chromatography (hexane:ethyl acetate=10:1) to obtain the desired product (1.01 g) as a pale yellow powder.
¹H-NMR (CDCl₃, 440 MHz): δ 4.01(3H, s), 6.82(1H, d, J=8.6 Hz), 7.20-7.21(1H, m), 7.38(1H, d, J=8.6 Hz).

### <Example 17>

### O-(3-Bromo-2-formyl-6-methoxy)phenyl dimethyl thiocarbamate

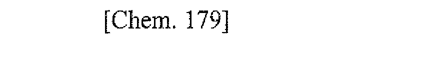

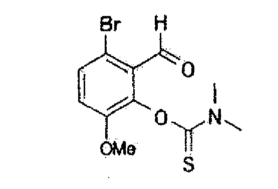

To a solution of 6-bromo-2-hydroxy-3-methoxybenzaldehyde (231 mg) in DMF (4.0 mL) were added triethylene diamine (224 mg) and dimethylthiocarbamoyl chloride (247 mg), followed by stirring at room temperature for 12 hours. The solvent was evaporated under reduced pressure, and then to the residue was added water, followed by extraction with ethyl acetate. The extracted layer was dried over anhydrous magnesium sulfate, and after evaporating the solvent under reduced pressure, the residue was washed with isopropyl ether to obtain the desired product (258 mg) as a pale yellow powder.
LRMS (EI⁺): 317 [M⁺]
¹H-NMR (CDCl₃, 400 MHz): δ 3.40 (3H, s), 3.45 (3H, s), 3.86 (3H, s), 7.05 (1H, d, J=8.6 Hz), 7.51 (1H, d, J=8.6 Hz), 10.20 (1H, s).

### <Example 18>

### S-(3-Bromo-2-formyl-6-methoxy)phenyl dimethyl thiocarbamate

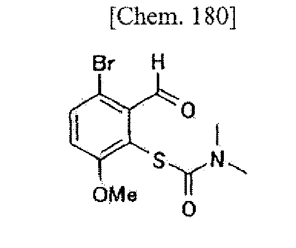

A solution of the compound of Example 17 (5.78 g) in diphenyl ether (57 mL) was stirred at 200°C for 30 minutes. The reaction liquid was cooled and then purified by silica gel column chromatography (hexane:ethyl acetate=1:1) to obtain the desired product (3.28 g) as a pale brown powder.
EIMS (+): 317 [M]⁺.
¹H-NMR (CDCl₃, 400 MHz): δ 3.00 (3H, brs), 3.16 (3H, brs), 3.89 (3H, s), 6.97 (1H, d, J=9.2 Hz), 7.64 (1H, d, J=9.2 Hz), 10.25 (1H, s).

### <Example 19>

### (6-Bromo-2-mercapto-3-methoxy)phenyl methanol

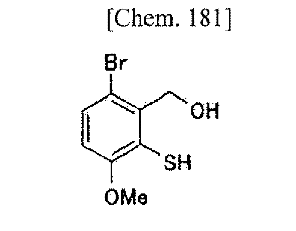

The compound of Example 18 (2.44 g) was suspended in isopropyl alcohol (60 mL), and 1 mol/L sodium hydroxide (15.3 mL) was added thereto, followed by stirring at 60°C for 30 minutes. The solvent was concentrated under reduced pressure, acidified by the addition of 5% hydrochloric acid, and then extracted with ethyl acetate. The extracted layer was washed with saturated brine and then dried over anhydrous magnesium sulfate, and the solvent was evaporated under reduced pressure. The residue was dissolved in methanol (60 mL), and sodium borohydride (580 mg) was added thereto under ice cooling, followed by stirring at room temperature for 30 minutes. The solvent was concentrated under reduced pressure, acidified by the addition of 5% hydrochloric acid, and then extracted with ethyl acetate. The extracted layer was washed with saturated brine and then dried over anhydrous magnesium sulfate, and the solvent was evaporated under reduced pressure to obtain the desired product (1.95 g) as a pale purple oil.
EIMS (+): 248 [M]⁺.
¹H-NMR (CDCl₃, 400 MHz): δ 1.99 (1H, brs), 3.90 (3H, s), 4.46 (1H, s), 4.93 (2H, s), 6.71 (1H, d, J=8.6 Hz), 7.33 (1H, d, J=8.6 Hz).

### <Example 20>

### 4-Bromo-7-methoxy-2-trifluoromethylbenzo[b]thiophene

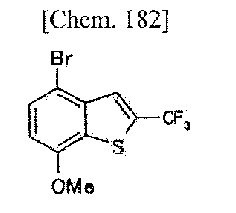

The compound of Example 19 (1.95 g) was dissolved in acetonitrile (15 mL), and triphenylphosphonium hydrobromide (2.90 g) was added thereto, followed by heating under reflux for 17 hours. The solvent was concentrated under reduced pressure and then washed with ethyl acetate to obtain a colorless powder (4.39 g). To the obtained solid (4.35 g) were added toluene (60 mL), trifluoroacetic anhydride (1.18 mL) and triethylamine (3.17 mL), followed by reflux for 3 hours. To the reaction liquid was added water, followed by extraction with ethyl acetate, the extracted layer was washed with saturated brine and then dried over anhydrous magnesium sulfate, and the solvent was evaporated under reduced pressure. The residue was purified by silica gel column chromatography (hexane:ethyl acetate=10:1) to obtain the desired product (2.00 g) as a colorless powder.
EIMS (+): 310 [M]⁺.
¹H-NMR (CDCl₃, 400 MHz): δ 4.00 (3H, s), 6.75 (1H, d, J=8.6 Hz), 7.53 (1H, d, J=8.6 Hz), 7.79 (1H, q, J=1.2 Hz).

### <Example 21>

### Ethyl 2-aminonicotinate

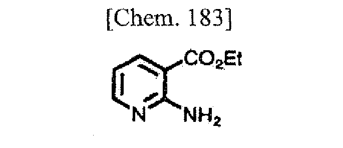

Commercially available 2-aminonicotinic acid (24.8 g) was dissolved in acetone (540 mL), and iodoethane (43.1 mL) and potassium carbonate (124 g) were added thereto, followed by stirring for 16 hours under the condition of heating under reflux, and iodoethane (29.0 mL) was added thereto, followed by further stirring for 15 hours. After removing the insoluble materials by filtration, the solvent of the filtrate was evaporated under reduced pressure, and the obtained residue was recrystallized with the addition of ethyl acetate to obtain the desired product (17.3 g). The mother liquid was purified by silica gel column chromatography (hexane:ethyl acetate=2:1 → 1:1) to obtain the desired product (1.60 g, total amount: 18.9 g) as a yellow powder.
¹H-NMR (CDCl_{3,} 400 MHz): δ 1.38 (3H, t, J=7.3 Hz), 4.34 (2H, q, J=7.3 Hz), 6.62 (1H, dd, J=7.9, 4.9 Hz), 8.13 (1H, dd, J=7.9, 1.8 Hz), 8.21 (1H, dd, J=4.9, 1.8 Hz).

### <Example 22>

### \N-Amino-2-amino-3-methoxycarbonylpyridiniummesitylenesulfonate

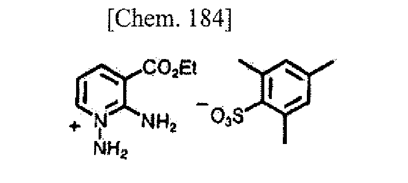

Ethyl mesitylsulfonylacetohydroxamate ester (28.3 g) was dissolved in 1,4-dioxane (40 mL), and 70% perchloric acid (14 mL) was added thereto at 0°C, followed by stirring for 30 minutes. To the reaction liquid was added cold water, and the precipitated solid was then collected by filtration and dissolved in methylene chloride. After removing the aqueous layer by a liquid separation operation, the methylene chloride layer was washed with saturated brine, dried over anhydrous sodium sulfate, and filtered. The filtrate was added to a solution of the compound of Example 21 (13.8 g) in methylene chloride (50 mL) at 0°C, followed by stirring at room temperature for 1 hour. The solvent was evaporated under reduced pressure, and diethyl ether was added thereto. The precipitated crystal was collected by filtration to obtain the desired product (29.0 g) as a yellow powder.
¹H-NMR (DMSO-d₆, 400 MHz): δ 1.34 (3H, t, J= 7.3 Hz), 2.17 (3H, s), 2.50 (6H, s), 4.37 (2H, q, J=7.3 Hz), 6.74 (2H, s), 6.96 (2H, brs), 7.00 (1H, t, J=6.7 Hz), 8.41 (1H, dd, J=6.7, 1.2 Hz), 8.53 (1H, d, J=6.7 Hz), 8.75 (2H, brs).

### <Example 23>

### Ethyl 2-trifluoromethyl-[1,2,4]triazolo[1,5-a]pyridine-8-carboxylate

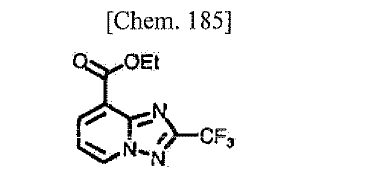

The compound of Example 22 (10.0 g) was dissolved in toluene (75 mL), and triethylamine (12.5 mL) and trifluoroacetic anhydride (5.60 mL) were added thereto, followed by stirring for 13 hours under the condition of heating under reflux. After evaporating the solvent under reduced pressure, a saturated aqueous sodium hydrogen carbonate solution was added thereto, followed by extraction three times with ethyl acetate, and the combined extracted layer was washed with saturated brine, and then dried over anhydrous sodium sulfate and filtered. After evaporating the solvent under reduced pressure, the residue was purified by silica gel column chromatography (hexane:ethyl acetate=3:2) to obtain the desired product (5.34 g) as a colorless powder.
¹H-NMR (CDCl₃, 400 MHz): δ 1.47 (3H, t, J=7.3 Hz), 4.54 (2H, q, J=7.3 Hz), 7.30 (1H, t, J=7.3 Hz), 8.39 (1H, dd, J=7.3, 1.2 Hz), 8.81 (1H, dd, J=7.3, 1.2 Hz).

### <Example 24>

### 8-t-Butyldimethylsiloxymethyl-2-trifluoromethyl-[1,2,4]triazolo[1,5-a]pyridine

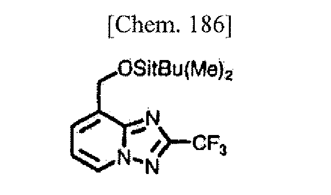

The compound of Example 23 (5.03 g) was dissolved in THF (150 mL) under an argon atmosphere, and a solution (0.95 mol/L, 40.9 mL) of diisobutylaluminum hydride in hexane was slowly added thereto at -10°C. 1 mol/L Hydrochloric acid was added thereto, followed by extraction three times with ethyl acetate, and the combined extracted layer was washed with saturated brine, and then dried over anhydrous sodium sulfate and filtered. After evaporating the solvent of the filtrate under reduced pressure, the residue was dissolved in DMF (100 mL) under an argon atmosphere, and imidazole (3.30 g) and chloro-t-butyldimethylsilane (3.51 g) were added thereto at 0°C, followed by stirring at room temperature for 1 hour. To the reaction liquid was added water, followed by extraction three times with acetic acid, and the combined extracted layer was washed with saturated brine, and then dried over anhydrous sodium sulfate and filtered. After evaporating the solvent of the filtrate under reduced pressure, the residue was purified by silica gel column chromatography (hexane:ethyl acetate=30:1) to obtain the desired product (5.90 g) as a colorless powder.
¹H-NMR (CDCl₃, 400 MHz): δ 0.17 (6H, s), 0.99 (9H, s), 5.17 (2H, s), 7.22 (1H, t, J= 6.7 Hz), 7.80 (1H, dd, J=6.7, 1.2 Hz), 8.53 (1H, dd, J=6.7, 1.2 Hz).

### <Example 25>

### 8-t-Butyldimethylsiloxymethyl-5-iodo-2-trifluoromethyl-[1,2,4]triazolo[1,5-a]pyridine

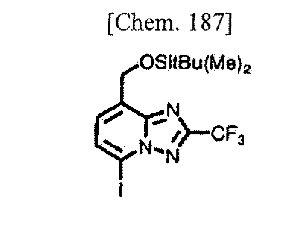

The compound of Example 24 (5.90 g) was dissolved in THF (120 mL) under an argon atmosphere, and a solution (2.71 mol/L, 7.23 mL) of n-butyl lithium in hexane was added thereto at -78°C, followed by stirring for 30 minutes. 1,2-Diiodoethane (5.52 g) was added thereto, followed by stirring at -78°C for 2.5 hours. A saturated aqueous sodium hydrogen carbonate solution was added thereto, followed by extraction three times with ethyl acetate, and the combined extracted layer was washed with saturated brine, and then dried over anhydrous sodium sulfate and filtered. After evaporating the solvent of the filtrate under reduced pressure, the residue was purified by silica gel column chromatography (hexane:ethyl acetate=30:1) to obtain the desired product (7.64 g) as a yellow powder.
¹H-NMR (CDCl₃, 400 MHz): δ 0.17 (6H, s), 0.96 (9H, s), 5.14 (2H, d, J=1.2 Hz), 7.55 (1H, dt, J= 7.9, 1.2 Hz), 7.68 (1H, d, J=7.9 Hz).

### <Example 26>

### N-Amino-2-amino-3-bromo-6-methoxypyridiniummesitylenesulfonate

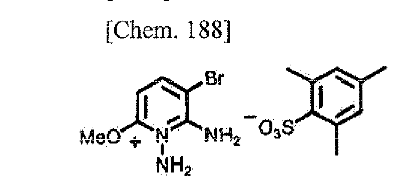

Ethyl mesitylsulfonylacetohydroxamate ester (25.3 g) was dissolved in 1,4-dioxane (35 mL), and 70% perchloric acid (13 mL) was added thereto at 0°C, followed by stirring for 30 minutes. To the reaction liquid was added cold water, and the precipitated solid was then collected by filtration and dissolved in methylene chloride. After removing the aqueous layer by a liquid separation operation, the methylene chloride layer was washed with saturated brine, dried over anhydrous sodium sulfate, and filtered. The filtrate was added to a solution of 2-amino-3-bromo-6-methoxypyridine (the pamphlet of WO03/031445) (15.0 g) in methylene chloride (100 mL) at 0°C, followed by stirring at room temperature for 1 hour. The solvent was evaporated under reduced pressure, and diethyl ether was added thereto. The precipitated crystal was collected by filtration to obtain the desired product (27.3 g) as a yellow powder.
¹H-NMR (DMSO-d₆, 400 MHz): δ 2.14 (3H, s), 2.47 (6H, s), 4.06 (3H, s), 5.73 (1H, s), 6.28 (2H, s), 6.45 (1H, d, J=8.6 Hz), 6.71 (2H, s), 8.20 (1H, d, J=8.6 Hz), 8.40 (2H, s).

### <Example 27>

### 8-Bromo-5-methoxy-2-trifluoromethyl-[1,2,4]triazolo[1,5-a]pyridine

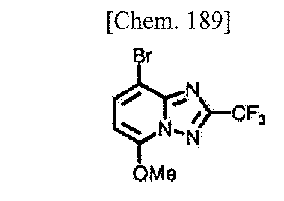

The compound of Example 26 (13.0 g) was dissolved in methanol (100 mL), triethylamine (13.0 mL), and a mixture of trifluoroacetic anhydride (6.6 mL) and methanol (20 mL) was added thereto at 0°C, followed by stirring at room temperature for 17.5 hours. To the reaction liquid was added water, followed by extraction three times with ethyl acetate, and the combined extracted layer was washed with saturated brine, and then dried over anhydrous sodium sulfate and filtered. After evaporating the solvent under reduced pressure, the residue was purified by silica gel column chromatography (hexane:ethyl acetate=1:1) to obtain the desired product (6.73 g) as a brown powder.
EIMS (+): 295 [M]⁺.
¹H-NMR (CDCl₃, 400 MHz): δ 4.23 (3H, s), 6.41 (1H, d, J=7.9 Hz), 7.87 (1H, d, J=7.9 Hz).

### <Example 28>

### 5-Methoxy-2-trifluoromethyl-[1,2,4]triazolo[1,5-a]pyridine-8-carboaldehyde

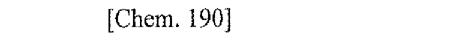

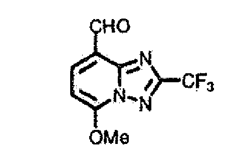

The compound of Example 25 (7.64 g) was dissolved in THF (100 mL) under an argon atmosphere, and a solution (1.0 mol/L, 33.4 mL) of tetrabutyl ammonium fluoride in THF was added thereto at 0°C, followed by stirring at room temperature for 1 hour. To the reaction liquid was added water, followed by extraction three times with ethytl acetate, and the combined extracted layer was washed with saturated brine, and then dried over anhydrous sodium sulfate and filtered. After evaporating the solvent of the filtrate under reduced pressure, to the residue were added methylene chloride (150 mL) and active manganese dioxide (14.5 g), followed by stirring at 60°C for 5 hours. The insoluble materials were removed by filtration through Celite, and after evaporating the solvent of the filtrate under reduced pressure, the residue was dissolved in methanol (100 mL) under an argon atmosphere. Sodium methoxide (3.61 g) was added thereto, followed by stirring for 2 hours under the condition of heating under reflux. A saturated aqueous ammonium chloride solution was added to the reaction liquid, followed by extraction three times with ethyl acetate, and the combined extracted layer was washed with saturated brine, and then dried over anhydrous sodium sulfate and filtered. After evaporating the solvent of the filtrate under reduced pressure, the residue was purified by silica gel column chromatography (hexane:ethyl acetate=1:1→2:3) to obtain the desired product (1.43 g) as a yellow powder. EIMS (+): 245 [M]⁺
¹H-NMR (CDCl₃, 400 MHz): δ 4.34 (3H, s), 6.66 (1H, d, J=7.9 Hz), 8.36 (1H, d, J=7.9 Hz), 10.59 (1H, s).

### <Example 29>

### 5-Methoxy-2-trifluoromethyl-[1,2,4]triazolo[1,5-a]pyridine-8-carboaldehyde

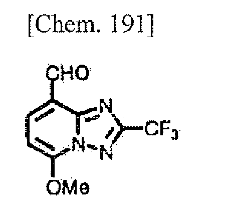

The compound of Example 27 (6.49 g) was dissolved in THF (200 mL) under an argon atmosphere, and a solution (2.55 mol/L, 9.02 mL) of n-butyl lithium in hexane was added thereto at -78°C, followed by stirring for 15 minutes. DMF (5.09 mL) was added thereto, followed by stirring for 1.5 hours. The reaction liquid was poured into a saturated aqueous ammonium chloride solution (cannulation), followed by extraction three times with ethyl acetate, and the combined extracted layer was washed with saturated brine, and then dried over anhydrous sodium sulfate and filtered. After evaporating the solvent of the filtrate under reduced pressure, the residue was purified by silica gel column chromatography (hexane:ethyl acetate=1:1) to obtain the desired product (2.43 g) as a yellow powder.

### <Example 30>

### 6-Amino-5-methoxy-2-picoline

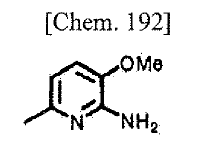

To a solution of commercially available 3-hydroxy-6-methyl-2-nitropyridine (9.76 g) in DMF (120 mL) were added potassium carbonate (14.0 g) and iodomethane (5.91 mL), followed by stirring at room temperature for 2 hours. Water (700 mL) was added thereto, followed by extraction with ethyl acetate (1.50 mL). The extract was washed with water and saturated brine, and then dried over anhydrous sodium sulfate. The extract was concentrated under reduced pressure and then purified by silica gel column chromatography (hexane:ethyl acetate=4:1) to obtain a methyl ether form (10.1 g). To a solution of the methyl ether form in ethyl acetate (300 mL) was added 10 % palladium-carbon (1.00 g), followed by stirring at room temperature for 4 hours under a hydrogen atmosphere. The reaction liquid was filtered through Celite and the filtrate was concentrated under reduced pressure to obtain the desired product (8.28 g) as a colorless powder.
¹H-NMR (CDCl₃, 400 MHz): δ 2.33 (3H, s), 3.81 (3H, s), 4.59 (2H, brs), 6.45 (1H, d, J=7.9 Hz), 6.82 (1H, d, J=7.9 Hz).

### <Example 31>

### 6-di(t-Butoxycarbonyl)amino-5-methoxy-2-picoline

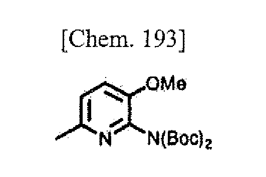

To the compound of Example 30 (3.00 g) in acetonitrile (100 mL) were added di-t-butyldicarbonate (28.4 g), triethylamine (4.39 g), and 4-dimethyl aminopyridine (100 mg), followed by stirring at room temperature for 8 hours. The reaction liquid was concentrated under reduced pressure and extracted with ethyl acetate (500 mL), and the extract was washed with water and saturated brine, and then dried over anhydrous sodium sulfate. The extract was concentrated under reduced pressure and then purified by silica gel column chromatography (hexane:ethyl acetate=3:1) to obtain the desired product (5.80 g) as a colorless powder.
¹H-NMR (CDCl₃, 400 MHz): δ 1.41 (18H, s), 2.48 (3H, s), 3.81 (3H, s), 7.07 (1H, d, J=8.6 Hz), 7.14 (1H, d, J=8.6 Hz).

### <Example 32>

### 6-Bromomethyl-2-di(t-butoxycarbonyl)amino-3-methoxypyridine

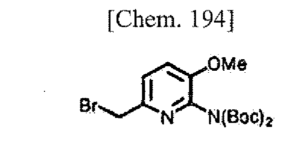

To a solution of compound of Example 31 (6.34 g) in carbon tetrachloride (50 mL) were added NBS (3.67 g) and benzoyl peroxide (20 mg), followed by heating under reflux for 4 hours under an argon atmosphere. The insoluble materials were removed by filtration and the filtrate was concentrated under reduced pressure. The residue was purified by recrystallization (ethyl acetate/hexane) to obtain the desired product (6.33 g) as a colorless powder.
¹H-NMR (CDCl₃,400 MHz): δ 1.40 (18H, s), 3.86 (3H, s), 4.53 (2H, s), 7.21 (1H, d, J=8,6 Hz), 7.37 (1H, d, J=8.6 Hz).

### <Example 33>

### 2-di(t-Butoxycarbonyl)amino-6-formyl-3-methoxypyridine

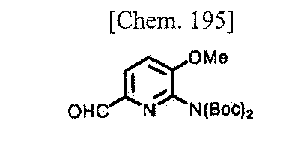

To a solution of N-methyl morpholine-N-oxide (3.55 g) and 4A Molecular Sieve powders (5.00 g) in acetonitrile (80 mL), a solution of compound Example 32 (6.33 g) in acetonitrile (20 mL) was added at room temperature under an argon atmosphere, followed by stirring for 4 hours. The reaction liquid was filtered through a silica gel pad and the filtrate was concentrated under reduced pressure. The residue was purified by silica gel column chromatography (hexane:ethyl acetate=5:1→3:1) to obtain the desired product (3.70 g) as a colorless powder.
¹H-NMR (CDCl₃, 400 MHz): δ 1.42 (18H, s), 3.96 (3H, s), 7.35 (1H, d, J=8.5 Hz), 8.00 (1H, d, J=8.5 Hz), 9.94 (1H, s).

### <Example 34>

### 8-Methoxy-5-propionylquinoline

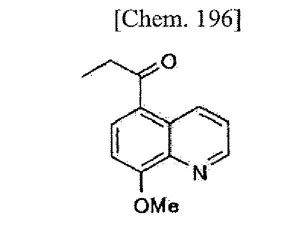

To a solution of commercially available 8-hydroxy quinoline (30.0 g) in 1,2-dichloroethane (207 mL) were added aluminum chloride (68.9 g) and propionyl chloride (19.9 mL), followed by stirring at 70°C for 3 hours. The reaction liquid was poured into 5% hydrochloric acid (1 L), followed by stirring for 30 minutes, and adjustment to pH 4 with sodium acetate, and the organic layer was collected by separation. The aqueous layer was extracted with chloroform, combined with the above organic layer, and dried over anhydrous sodium sulfate. The solvent was evaporated under reduced pressure to obtain crude 8-hydroxy-5-propionylquinoline as a pale yellow amorphous powder. To this amorphous powder were added THF (500 mL), a 50% aqueous sodium hydroxide solution, tetra-n-butylammonium bromide (3.00 g), and methyl iodide (38.6 mL), followed by stirring at room temperature for 29 hours. After concentrating the reaction liquid under reduced pressure, to the residue was added ice water (1 L), and the precipitated solid was collected by filtration. This solid was dissolved in ethyl acetate and dried over anhydrous sodium sulfate, and the solvent was evaporated under reduced pressure. To the residue was added ethyl acetate (1 L), followed by warming, and the insoluble materials were removed by filtration. The filtrate was concentrated under reduced pressure and the precipitated solid was collected by filtration to obtain the desired product (23.2 g) as a pale yellow powder.
EIMS (+): 215 [M]⁺
¹H-NMR (CDCl₃, 400 MHz): δ 1.28 (3H, t, J=7.3 Hz), 3.10 (2H, q, J=7.3 Hz), 4.10 (3H, s), 7.04 (1H, d, J=7.9 Hz), 7.55 (1H, dd, J=8.6, 4.3 Hz), 8.11 (1H, d, J=7.9 Hz), 8.96 (1H, dd, J=4.3, 1.8 Hz), 9.39 (1H, dd, J=8.6, 1.8 Hz).

### <Example 35>

### 8-Methoxy-2-methyl-5-propionylquinoline

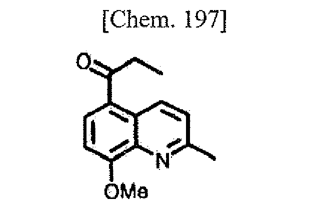

The compound of Example 1 (5.55 g) was dissolved in THF (220 mL) under an argon gas atmosphere, and a 1.58 mol/L n-butyl lithium/hexane solution (15.3 mL) was added dropwise thereto at -78°C, followed by stirring at the same temperature for 5 minutes. Thereafter, propionic anhydride (4.86 mL) was added thereto at -78°C, followed by stirring at -78°C for 10 minutes. To the reaction liquid was added a saturated aqueous ammonium chloride solution, followed by extraction with ethyl acetate. The organic layer was washed with water and saturated brine in that order, and then dried over anhydrous sodium sulfate. After evaporating the solvent under reduced pressure, the residue was purified by silica gel column chromatography (hexane:ethyl acetate= 1:1→1:2) to obtain the desired product (2.39 g) as a yellow powder.
¹H-NMR (CDCl₃, 400 MHz): δ 1.27 (3H, t, J=7.3 Hz), 2.80 (3H, s), 3.08 (2H, q, J=7.3 Hz), 4.14 (3H, s), 7.01 (1H, d, J=8.6 Hz), 7.44 (1H, d, J=9.2 Hz), 8.04 (1H, d, J=8.6 Hz), 9.26 (1H, d, J=9.2 Hz).

### <Example 36>

### 2-Ethyl-8-methoxy-5-proplonylquinoline

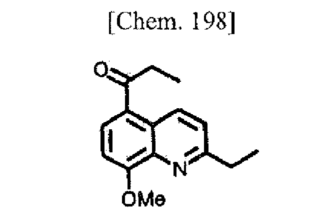

The compound of Example 3 (4.00 g) was dissolved in THF (150 mL) under an argon atmosphere, and a solution (2.71 mol/L, 6.1 mL) of n-butyl lithium in hexane was added thereto at -78°C, followed by stirring for 1 hour. Propionic anhydride (1.53 mL) was added thereto at the same temperature, followed by stirring for 2.5 hours, then warming to room temperature, and followed by further stirring for 1.5 hours. A saturated aqueous ammonium chloride solution was added to the reaction liquid, followed by extraction three times with ethyl acetate, and the combined extracted layer was washed with saturated brine, and then dried over anhydrous sodium sulfate and filtered. After evaporating the solvent of the filtrate under reduced pressure, the residue was purified by silica gel column chromatography (hexane:ethyl acetate=1:1) to obtain the desired product (2.08 g) as a yellow powder.
¹H-NMR (CDCl₃, 400 MHz): δ 1.27 (3H, t, J=7.3 Hz), 1.40 (3H, t, J=7.3 Hz), 3.05-3.11 (4H, m), 4.14 (3H, s), 7.01 (1H, d, J=8.6 Hz), 7.48 (1H, d, J=8.6 Hz), 8.03 (1H, d, J=8.6 Hz), 9.28 (1H, d, J=8.6 Hz).

### <Example 37>

### 2-Ethyl-8-methoxy-5-propionylquinoline

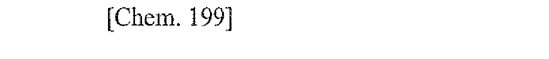

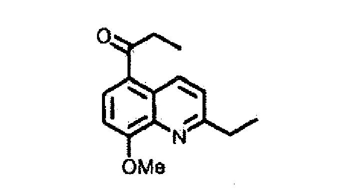

Aluminum chloride (214 mg) and 1,2,4-trichlorobenzene (1.0 mL) were mixed, and propionyl chloride (0.163 mL) was added thereto. The compound of Example 2 (100 mg) was added thereto, followed by stirring at an outer temperature of 70°C for 1 hour. 1 mol/L Hydrochloric acid was added thereto, followed by washing with ethyl acetate. The organic layer was extracted with 1 mol/L hydrochloric acid. The aqueous layer was combined, neutralized with sodium acetate, and then extracted twice with ethyl acetate. All the organic layers were combined, washed with saturated brine, dried over anhydrous sodium sulfate, filtered and concentrated under reduced pressure. The residue was subject to silica gel column chromatography (hexane:ethyl acetate=1:1) to obtain the desired product (70.9 mg) as a pale yellow powder.

### <Example 38>

### 2-Ethyl-8-methoxy-5-propionylquinoline

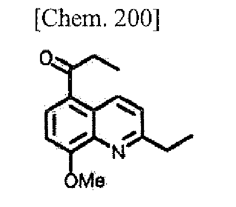

Commercially available 2-amino-4-propionylanisole (24.6 g), sodium iodide (20.6 g), 1-butanol (246 mL), and concentrated hydrochloric acid (246 mL) were mixed, and trans-1-heptenal (57.8 g) was added dropwise for 50 minutes while heating at 140°C, followed by heating under reflux at 140°C for 3 hours. To the residue obtained by evaporating the reaction liquid under reduced pressure were added ethyl acetate (500 mL) and water (250 mL), followed by liquid separation. The organic layer was extracted with water (250 mL), combined with the above aqueous layer, and adjusted to pH 8 with a 1 mol/L sodium hydroxide solution. The precipitated solid was collected by filtration and washed with water (75.0 mL). The obtained solid was dissolved in ethyl acetate (100 mL), and silica gel (1.23 g) was added thereto, followed by separation by filtration and concentration under reduced pressure. The residue was added with ethanol (98.5 mL), heated and dissolved at 50°C, and water (98.5 mL) was then added thereto, followed by being left to cool at room temperature. The precipitated solid was collected by filtration and washed with ethanol:water=1:5 (98.5 mL). It was dried at 60°C under reduced pressure to obtain the desired product (14.1 g) as a brown powder.

### <Example 39>

### 8-Methoxy-5-propionyl-2-isopropylquinoline

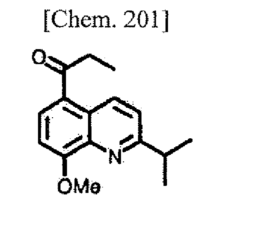

The compound of Example 4 (3.57 g) was dissolved in THF (120 mL) under an argon gas atmosphere, and a 1.60 mol/L n-butyl lithium/hexane solution (8.06 mL) was added dropwise thereto at -78°C, followed by stirring at the same temperature for 5 minutes. Thereafter, propionic anhydride (2.61 mL) was added thereto at -78°C, followed by stirring at -78°C for 25 minutes. To the reaction liquid was added a saturated aqueous ammonium chloride solution, followed by extraction with ethyl acetate. The organic layer was washed with water and saturated brine in this order, and then dried over anhydrous sodium sulfate. After evaporating the solvent under reduced pressure, the residue was purified by silica gel column chromatography (hexane:ethyl acetate=3:1) to obtain the desired product (1.20 g) as a colorless powder.
¹H-NMR (CDCl₃, 400 MHz): δ 1.27 (3H, t, J=7.3 Hz), 1.39 (6H, d, J=7.3 Hz), 3.08 (2H, q, J=7.3 Hz), 3.31-3.41 (1H, m), 4.14 (3H, s), 7.0 (1H, d, J=8.6 Hz), 7.52 (1H, d, J=9.2 Hz), 8.03 (1H, d, J=8.6 Hz), 9.29 (1H, d, J=9.2 Hz).

### <Example 40>

### 8-Methoxy-5-propionyl-2-trifluoromethylquinoline

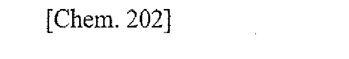

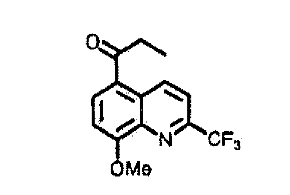

The compound of Example 8 (3.86 g) was dissolved in THF (100 mL) under an argon atmosphere, and a solution (2.71 mol/L, 5.2 mL) of n-butyl lithium in hexane was added thereto at -78°C, followed by stirring for I hour. Propionic anhydride (3.5 mL) was added thereto at the same temperature, followed by stirring for 3 hours. Then, a saturated aqueous ammonium chloride solution was added thereto, followed by extraction three times with ethyl acetate. The combined extracted layer was washed with saturated brine, and then dried over anhydrous sodium sulfate and filtered. After evaporating the solvent under reduced pressure, the residue was purified by silica gel column chromatography (hexane:ethyl acetate=5:1) to obtain the desired product (1.21 g) as a yellow powder.
¹H-NMR (CDCl₃, 400 MHz): δ 1.29 (3H, t, J=7.3 Hz), 3.11 (2H, q, J=7.3 Hz), 4.18 (3H, s), 7.12 (1H, d, J=8.6 Hz), 7.88 (1H, d, J=8.6 Hz), 8.23 (1H, d, J=8.6 Hz), 9.65 (1H, d, J=8.6 Hz).

### <Example 41>

### 4-Methoxy-7-propionyl-2-trifluoromethy-1H-benzimidazole

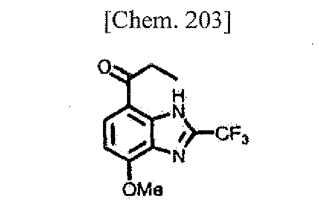

The compound of Example 13 (800 mg) was dissolved in THF (20.0 mL) under an argon gas atmosphere, and a 1.58 mol/L n-butyl lithium/hexane solution (3.90 mL) was added dropwise thereto at -78°C, followed by stirring for 1 hour as it was. Thereafter, N,N-dimethyl propionamide (890 µL) was added thereto at the same temperature, followed by stirring for 3 hours while warming to room temperature. To the reaction liquid was added a saturated aqueous ammonium chloride solution, followed by extraction with ethyl acetate. The organic layer was washed with water and saturated brine, and then dried over anhydrous sodium sulfate. After evaporating the solvent under reduced pressure, the residue was purified by silica gel column chromatography (chloroform) to obtain the desired product (342 mg) as a colorless powder.
EIMS (+): 272 [M]⁺
¹H-NMR (CDCl₃, 400 MHz): δ 1.29 (3H, t, J=7.3 Hz), 3.08 (2H, q, J=7.3 Hz), 4.15 (3H, s), 6.80 (1H, d, J=8.6 Hz), 7.96 (1H, d, J=8.6 Hz), 11.4 (1H, brs).

### <Example 42>

### 4-Methoxy-2-trifluoromethylbenzothiazole

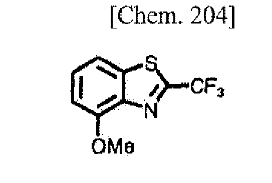

Commercially available 2-amino-4-methoxybenzothiazole (26.2 g) and a 60% aqueous sodium hydroxide solution were stirred under heating at 150°C for 22 hours. After cooling, ice was added thereto, followed by adjusting to pH 5 with concentrated hydrochloric acid, and the precipitate was removed by filtration. Then, the aqueous layer was extracted with toluene. The organic layer was dried over magnesium sulfate and then concentrated under reduced pressure to obtain a viscous oil (2.68 g). Alternatively, the precipitate as above was washed with a saturated aqueous sodium bicarbonate solution and toluene, and the organic layer was dried over anhydrous magnesium sulfate and then concentrated to obtain a viscous oil under reduced pressure in a similar manner. The obtained oil was combined, dissolved in trifluoroacetic acid (96 mL) and trimethylsilyl polyphosphate ester (53 mL), and reacted at 95°C for 6 hours. After cooling, the reaction liquid was added to water, adjusted to pH 8 with an aqueous sodium hydroxide solution, and extracted with anhydrous methylene chloride. The organic layer was dried over anhydrous magnesium sulfate and then concentrated under reduced pressure, and the residue was purified by silica gel column chromatography (hexane:ethyl acetate=3:1) to obtain the desired product (3.46 g) as a colorless powder.
¹H-NMR (acetone-d₆, 200 MHz): δ 3.86 (3H, s), 7.05 (1H, dd, J=8.0, 1.4 Hz), 7.25 (1H, dd, J=8.1, 1.4 Hz), 7.36 (1H, t, J=8.1 Hz).

### <Example 43>

### 4-Methoxy-7-propionyl-2-trifluoromethylbenzothiazole

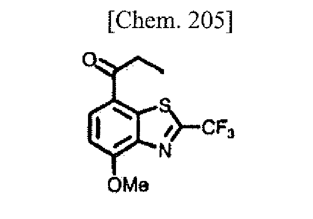

Titanium tetrachloride (7.46 mL) was dissolved in nitromethane (40 mL) under an argon atmosphere, and propionyl chloride (5.91 ml) was added thereto. Then, the compound of Example 42 (3.93 g) dissolved in nitromethane (30 mL) was added thereto, followed by stirring at room temperature for 30 minutes and at 75°C for 6 hours. Water was added to the reaction liquid, followed by extraction three times with ethyl acetate, and the combined organic layer was washed with saturated brine, and then dried over anhydrous sodium sulfate and filtered. After evaporating the solvent of the filtrate under reduced pressure, the residue was purified by silica gel column chromatography (hexane:ethyl acetate=9:1→4:1) to obtain the desired product (2.19 g) as a yellow powder.
¹H-NMR (CDCl₃, 400 MHz): δ 1.31 (3H, t, J=7.3 Hz), 3.14 (2H, q, J=7.3 Hz), 4.18 (3H, s), 7.08 (1H, d, J=8.6 Hz), 8.20 (1H, d, J=8.6 Hz).

### <Example 44>

### 7-Methoxy-4-propionyl-2-trifluoromethylbenzofuran

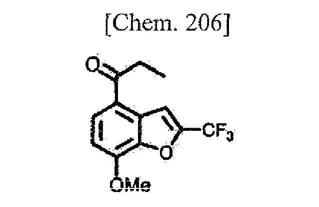

The compound of Example 16 (500 mg) was dissolved in THF (10 mL) under an argon gas flow, and an n-butyl lithium hexane solution (1.21 mL, 1.54 mol/L) was added dropwise thereto at -78°C, followed by stirring for 5 minutes. To this was added N,N-dimethyl propionic acid amide (513 mg), followed by slowly returning to room temperature. A saturated aqueous ammonium chloride solution was added to the reaction liquid, followed by extraction with ethyl acetate and washing with saturated brine. It was dried over anhydrous sodium sulfate, and the solvent was then evaporated under reduced pressure. The residue was purified by silica gel column chromatography (hexane:ethyl acetate=20:1→10:1) to obtain the desired product (162 mg) as a colorless powder.
¹H-NMR (CDCl₃, 400 MHz): δ 1.26 (3H, t, J=7.3 Hz), 3.05 (2H, q, J=7.3 Hz), 4.10 (3H, s), 6.93 (1H, d, J=8.6 Hz), 7.90 (1H, d, J=8.6 Hz), 8.01 (1H, d, J=1.2 Hz).

### <Example 45>

### 7-Methoxy-4-propionyl-2-trifluoromethylbenzo[b]thiophene

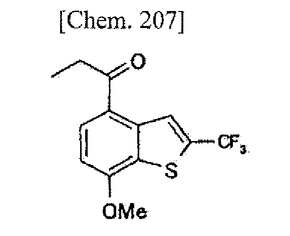

To a solution of the compound of Example 20 (1.70 g) in THF (27 mL) was added an n-butyl lithium (1.58 mol/L hexane solution, 3.80 mL) at -78°C, followed by stirring at the same temperature for 30 minutes, and then N,N-dimethyl propionamide (1.20 mL) was added thereto, followed by stirring at room temperature for 30 minutes. To the reaction liquid was added a saturated aqueous ammonium chloride solution, followed by extraction with ethyl acetate, the extracted layer was washed with saturated brine and then dried over anhydrous magnesium sulfate, and the solvent was evaporated. The residue was purified by silica gel column chromatography (hexane:ethyl acetate=4:1) to obtain the desired product (243 mg) as a colorless powder.
EIMS (+): 288 [M]⁺
¹H-NMR (CDCl₃, 400 MHz): δ 1.27 (3H, t, J=7.3 Hz), 3.07 (2H, q, J=7.03 Hz), 4.09 (3H, s), 6.89 (1H, d, J=8.6 Hz), 8.05 (1H, d, J=8.6 Hz), 8.80 (1H, q, J=1.2 Hz).

### <Example 46>

### Methyl 3-hydroxy-3-(5-methoxy-2-trifluoromethyl-[1,2,4]triazolo[1,5-a]pyridin-8-yl)-2,2-dimethyl-propionate

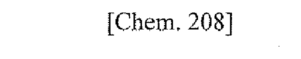

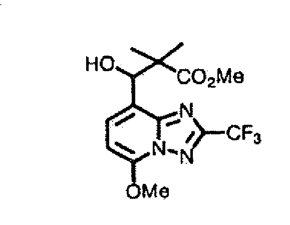

The compound of Example 28 (2.43 g) was dissolved in tetrahydrofuran (90 mL) under an argon atmosphere, and dimethylketene methyltrimethylsilyl acetal (3.03 mL) and a boron trifluoride-diethyl ether complex (1.89 mL) was added thereto, followed by stirring at room temperature for 35 minutes. To the reaction liquid was added a saturated aqueous sodium hydrogen carbonate solution, followed by extraction three times with ethyl acetate, and the combined extracted layer was washed with saturated brine, and then dried over anhydrous sodium sulfate and filtered. The solvent was evaporated under reduced pressure to obtain the desired product (3.41 g) as a yellow powder.
CIMS (+): 348 [M+H]⁺
¹H-NMR (CDCl₃, 400 MHz): δ 1.21 (3H, s), 1.23 (3H, s), 3.72 (3H, s), 4.21 (3H, s), 4.33 (1H, d, J=7.9 Hz), 5.37 (1H, d, J=7.9 Hz), 6.47 (1H, d, J=8.6 Hz), 7.62 (1H, d, J=8.6 Hz).

### <Example 47>

### Methyl 3-(3-di(t-butoxycarbonyl)amino-4-methoxyphenyl)-3-hydroxy-2,2-dimethylpropionate

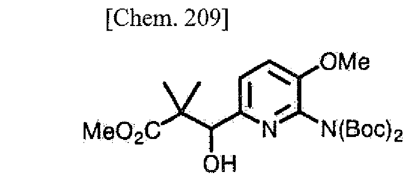

To a solution of the compound of Example 33 (8.43 g) and dimethylketene methyltrimethylsilyl acetal (6.25 g) in tetrahydrofuran (200 mL) was added a boron trifluoride-diethyl ether complex (4.55 mL) at 0°C under an argon atmosphere, followed by stirring at 0°C for 1 hour. To the reaction liquid was added a saturated aqueous sodium hydrogen carbonate solution, followed by extraction with ethyl acetate (500 mL). It was washed with water and saturated brine, and then dried over anhydrous sodium sulfate. The extract was concentrated under reduced pressure, and then purified by silica gel column chromatography (hexane:ethyl acetate=5:1→1:1) to obtain the desired product (8.63 g) as a colorless amorphous.
¹H-NMR (CDCl₃, 400 MHz): δ 1.08 (3H, s), 1.14 (3H, s), 1.37 (18H, s), 3.71 (3H, s), 3.85 (3H, s), 4.08 (1H, d, J=7.3 Hz), 4.90 (1H, d, J=7.3 Hz), 7.15 (1H, d, J=8.6 Hz), 7.21 (1H, d, J=8.6 Hz).

### <Example 48>

### Methyl 3-(8-methoxyquinolin-5-yl)-2-methyl-3-oxopropionate

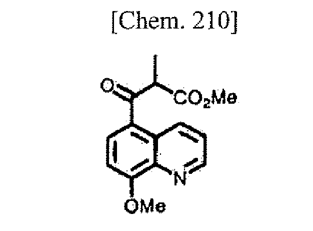

To the compound of Example 34 (700 mg) were added dimethyl carbonate (15 mL) and 60% sodium hydride (390 mg), followed by heating at 120°C for 9 hours. The reaction liquid was left to cool and then poured into a saturated aqueous ammonium chloride solution, followed by extraction with ethyl acetate, the extracted layer was dried over anhydrous magnesium sulfate, and the solvent was then evaporated under reduced pressure. The residue was purified by silica gel column chromatography (ethyl acetate:methanol=15:1) to obtain the desired product (890 mg) as a pale yellow oil.
EIMS (+): 273 [M]⁺
¹H-NMR (CDCl₃, 400 MHz): δ 1.55 (3H, d, J=7.3 Hz), 3.67 (3H, s), 4.17 (3H, s), 4.51 (1H, q, J=7.3 Hz), 7.06 (1H, d, J=8.6 Hz), 7.57 (1H, dd, J=8.6, 4.3 Hz), 8.13 (1H, d, J=8.6 Hz), 8.98 (1H, dd, J=4.3, 1.8 Hz), 9.29 (1H, dd, J=8.6, 1.8 Hz).

### <Example 49>

### Methyl 3-(8-methoxy-2-methylquinolin-5-yl)-2-methyl-3-oxopropionate

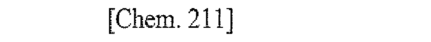

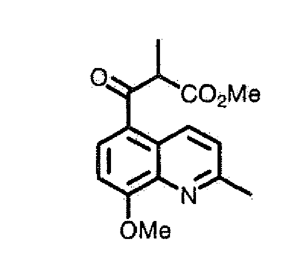

The compound of Example 35 (519 mg) was dissolved in dimethyl carbonate (10 mL) under an argon gas atmosphere, and 60% sodium hydride (272 mg) was added thereto at room temperature, followed by stirring for 9 hours under the condition of heating under reflux. To the reaction liquid was added a saturated aqueous ammonium chloride solution, followed by extraction with ethyl acetate. The organic layer was washed with water and saturated brine in this order, and then dried over anhydrous sodium sulfate. After evaporating the solvent under reduced pressure, the residue was purified by silica gel column chromatography (ethyl acetate:methanol=10:1) to obtain the desired product (465 mg) as a yellow oil.
¹H-NMR (CDCl₃, 400 MHz): δ 1.54 (3H, d, J=6.8 Hz), 2.80 (3H, s), 3.67 (3H, s), 4.15 (3H, s), 4.50 (1H, q, J=6.8 Hz), 7.03 (1H, d, J=8.6 Hz), 7.46 (1H, d, J=8.6 Hz), 8.06 (1H, d, J=8.6 Hz), 9.16 (1H, d, J=8.6 Hz).

### <Example 50>

### Methyl 3-(2-ethyl-8-methoxyquinolin-5-yl)-2-methyl-3-oxopropionate

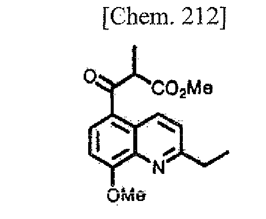

The compound of Example 36 (1.07 g) was dissolved in dimethyl carbonate (20 mL) under an argon atmosphere, and 60% sodium hydride (528 mg) and methanol (a few droplets) were added thereto, followed by stirring for 11 hours under the condition of heating under reflux. To the reaction liquid was added water, followed by extraction three times with ethyl acetate, and the combined extracted layer was washed with saturated brine, and then dried over anhydrous sodium sulfate and filtered. After evaporating the solvent under reduced pressure, the residue was purified by silica gel column chromatography (hexane:ethyl acetate=1:1) to obtain the desired product (1.04 g) as a yellow oil.
¹H-NMR (CDCl₃, 400 MHz): δ 1.40 (3H, t, J=7.3 Hz), 1.54 (3H, d, J=7.3 Hz), 3.07 (2H, q, J=7.3 Hz), 3.68 (3H, s), 4.15 (3H, s), 4.50 (1H, q, J=7.3 Hz), 7.03 (1H, d, J=8.6 Hz), 7.50 (1H, d, J=8.6 Hz), 8.05 (1H, d, J=8.6 Hz), 9.18 (1H, d, J=8.6 Hz).

### <Example 51>

### Methyl 3-(8-methoxy-2-isopropylquinolin-5-yl)-2-methyl-3-oxopropionate

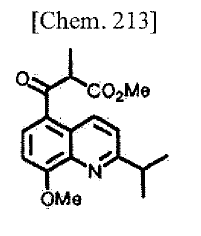

The compound of Example 39 (294 mg) was dissolved in dimethyl carbonate (5 mL) under an argon gas atmosphere, and 60% sodium hydride (137 mg) was added thereto at room temperature, followed by stirring for 10 hours under the condition of heating under reflux. To the reaction liquid was added a saturated aqueous ammonium chloride solution, followed by extraction with ethyl acetate. The organic layer was washed with water and saturated brine in that order, and then dried over anhydrous sodium sulfate. After evaporating the solvent under reduced pressure, the residue was purified by silica gel column chromatography (hexane:acetone=3:1) to obtain the desired product (257 mg) as a yellow oil.
¹H-NMR (CDCl₃, 400 MHz): δ 1.39 (6H, d, J=7.3 Hz), 1.54 (3H, d, J=6.7 Hz), 3.31-3.40 (1H, m) 3.67 (3H, s), 4.15 (3H, s), 4.50 (1H, q, J=6.7 Hz), 7.03 (1H, d, J=8.6 Hz), 7.53 (1H, d, J=8.6 Hz), 8.05 (1H, d, J=8.6 Hz), 9.20 (1H, d, J=8.6 Hz).

### <Example 52>

### Methyl 3-(8-methoxy-2-trifluoromethylquinolin-5-yl)-2-methyl-3-oxopropionate

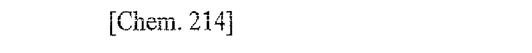

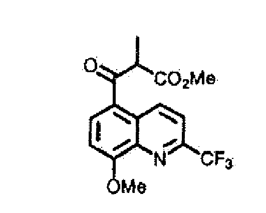

The compound of Example 40 (380 mg) was dissolved in dimethyl carbonate (15 mL) under an argon atmosphere, and 60% sodium hydride (161 mg) and methanol (a few droplets) were added thereto, followed by stirring for 5 hours under the condition of heating under reflux. To the reaction liquid was added water, followed by extraction three times with ethyl acetate, and the combined extracted layer was washed with saturated brine, and then dried over anhydrous sodium sulfate and filtered. After evaporating the solvent under reduced pressure, the residue was purified by silica gel column chromatography (hexane:ethyl acetate=5:1→3:1) to obtain the desired product (269 mg) as a yellow powder.
¹H-NMR (CDCl₃, 400 MHz): δ 1.55 (3H, d, J=6.7 Hz), 3.68 (3H, s), 4.18 (3H, s), 4.50 (1H, q, J=6.7 Hz), 7.13 (1H, d, J=8.6 Hz), 7.89 (1H, d, J=9.2 Hz), 8.24 (1H, d, J=8.6 Hz), 9.52 (1H, d, J=9.2 Hz).

### <Example 53>

### 1-(7-Methoxy-1-methoxymethyl-2-trifluoromethyl-1H-benzo[d]imidazol-4-yl)propan-1-one

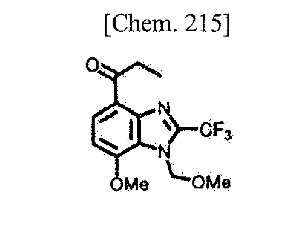

The compound of Example 41 (1.00 g) was dissolved in DMF (30.0 mL) under an argon atmosphere, and triethylamine (610 µL) and chloromethyl methyl ether (310 µL) were added thereto under ice cooling, followed by stirring at room temperature for 2 hours. To the reaction liquid was added water, followed by extraction with ethyl acetate. The organic layer was washed with water and saturated brine, and then dried over anhydrous sodium sulfate. After evaporating the solvent under reduced pressure, the residue was purified by aminated silica (Chromatorex) gel column chromatography (hexane:ethyl acetate=4:1) to obtain the desired product (1.01 g) as a colorless powder.
EIMS (+): 316 [M]⁺
¹H-NMR (CDCl₃, 400 MHz): δ 1.26 (3H, t, J=7.3 Hz), 3.37 (3H, s), 3.50 (2H, q, J=7.3 Hz), 4.07 (3H, s), 5.91 (2H, s), 6.94 (1H, d, J=8.6 Hz), 8.03 (1H, d, J=8.6 Hz).

### <Example 54>

### Methyl 3-(7-methoxy-1-methoxymethyl-2-trifluoromethyl-1H-benzo[d]imidazol-4-yl)-2-methyl-3-oxopropionate

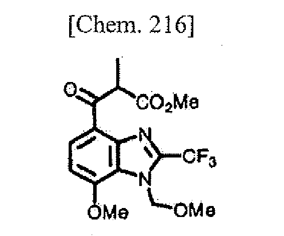

The compound of Example 53 (1.00 g) was dissolved in dimethyl carbonate (15 mL) under an argon atmosphere, and 60% sodium hydride (379 mg) was added thereto, followed by stirring for 30 minutes under the condition of heating under reflux. To the reaction liquid was added a saturated aqueous ammonium chloride solution, followed by extraction with ethyl acetate. The organic layer was washed with water and saturated brine in this order, and then dried over anhydrous sodium sulfate. After evaporating the solvent under reduced pressure, the residue was purified by silica gel column chromatography (hexane:ethyl acetate=2:1) to obtain the desired product (900 mg) as a pale yellow powder.
EIMS (+): 374 [M]⁺
¹H-NMR (CDCl₃, 400 MHz): δ 1.53 (3H, d, J=7.3 Hz), 3.38 (3H, s), 3.69 (3H, s), 4.08 (3H, s), 5.32 (1H, q, J=7.3 Hz), 5.88 (1H, d, J=11.0 Hz), 5.92 (1H, d, J=11.0 Hz), 6.97 (1H, d, J=8.6 Hz), 8.12 (1H, d, J=8.6 Hz).

### <Example 55>

### Methyl 3-(4-methoxy-2-trifluoromethylbenzo[d]thiazol-7-yl)-2-methyl-3-oxopropionate

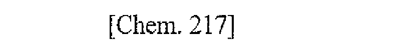

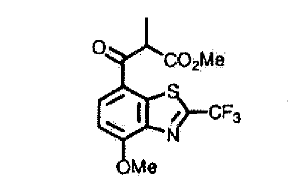

The compound of Example 43 (730 mg) was dissolved in dimethyl carbonate (20 mL) under an argon atmosphere, and 60% sodium hydride (303 mg) was added thereto, followed by stirring for 40 minutes under the condition of heating under reflux. To the reaction liquid was added a saturated aqueous ammonium chloride solution, followed by extraction three times with ethyl acetate, and the combined extracted layer was washed with saturated brine, and then dried over anhydrous sodium sulfate and filtered. After evaporating the solvent under reduced pressure, the residue was purified by silica gel column chromatography (ethyl acetate:hexane=4:1) to obtain the desired product (364 mg) as a yellow powder.
¹H-NMR (CDCl₃, 400 MHz): δ 1.60 (3H, d, J=7.3 Hz), 3.72 (3H, s), 4.21 (3H, s), 4.55 (1H, q, J=7.3 Hz), 7.12 (1H, d, J=8.6 Hz), 8.26 (1H, d, J=8.6 Hz).

### <Example 56>

### Methyl 3-(7-methoxy-2-trifluoromethylbenzofuran-4-yl)-2-methyl-3-oxopropionate

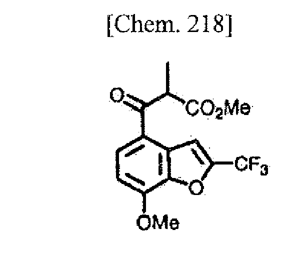

The compound of Example 44 (1.76 g) was dissolved in dimethyl carbonate (30.0 mL) under an argon atmosphere, and 60% sodium hydride (776 mg) was added thereto, followed by stirring for 30 minutes under the condition of heating under reflux. To the reaction liquid was added a saturated aqueous ammonium chloride solution, followed by extraction with ethyl acetate. The organic layer was washed with water and saturated brine in that order, and then dried over anhydrous sodium sulfate. After evaporating the solvent under reduced pressure, the residue was purified by silica gel column chromatography (hexane:ethyl acetate=3:1) to obtain the desired product (2.07 g) as a pale yellow powder. EIMS (+): 330 [M]⁺
¹H-NMR (CDCl₃, 400 MHz): δ 1.54 (3H, d, J=7.3 Hz), 3.70 (3H, s), 4.11 (3H, s), 4.46 (1H, q, J=7.3 Hz), 6.95 (1H, d, J=8.6 Hz), 7.93 (1H, d, J=8.6 Hz), 8.01 (1H, d, J=1.2 Hz).

### <Example 57>

### Methyl 3-(7-methoxy-2-trifluoromethylbenzo[b]thiophen-4-yl)-2-methyl-3-oxopropionate

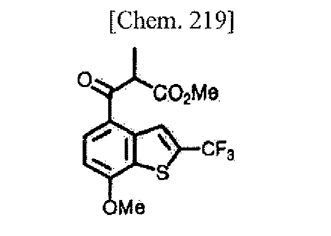

The compound of Example 45 (573 mg) was dissolved in dimethyl carbonate (15.0 mL) under an argon atmosphere, and 60% sodium hydride (239 mg) was added thereto, followed by stirring for 30 minutes under the condition of heating under reflux. To the reaction liquid was added a saturated aqueous ammonium chloride solution, followed by extraction with ethyl acetate. The organic layer was washed with water and saturated brine in that order, and then dried over anhydrous sodium sulfate. After evaporating the solvent under reduced pressure, the residue was purified by silica gel column chromatography (hexane:ethyl acetate=3:1) to obtain the desired product (625 mg) as a colorless powder. EIMS (+): 346 [M]⁺
¹H-NMR (CDCl₃, 400 MHz): δ 1.54 (3H, d, J=7.3 Hz), 3.69 (3H, s), 4.10 (3H, s), 4.50 (1H, q, J=7.3 Hz), 6.91 (1H, d, J=8.0 Hz), 8.09 (1H, d, J=8.0 Hz), 8.78 (1H q, J=1.2 Hz).

### <Example 58>

### Methyl 3-(8-methoxyquinolin-5-yl)-2,2-dimethyl-3-oxopropionate

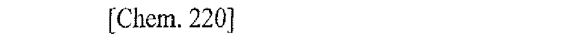

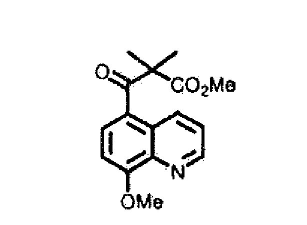

To a solution of the compound of Example 48 (890 mg) in DMF (30 mL) was added 60% sodium hydride (159 mg), followed by stirring at room temperature for 1 hour. To the reaction liquid was added methyl iodide (0.223 mL), followed by stirring at room temperature for 3 hours. Then, a saturated aqueous ammonium chloride solution was poured thereinto, followed by extraction with ethyl acetate, the extracted layer was dried over anhydrous sodium sulfate, and the solvent was then evaporated under reduced pressure. The residue was purified by silica gel column chromatography (ethyl acetate) to obtain the desired product (615 mg) as a colorless oil.
EIMS (+): 287 [M]⁺
¹H-NMR (CDCl₃, 400 MHz): δ 1.56 (3H, s), 1.61 (3H, s), 3.61 (3H, s), 4.14 (3H, s), 6.98 (1H, d, J=8.6 Hz), 7.54 (1H, dd, J=9.2, 4.3 Hz), 7.75 (1H, d, J=8.6 Hz), 8.90 (1H, dd, J=9.2, 1.8 Hz), 8.96 (1H, dd, J=4.3, 1.8 Hz).

### <Example 59>

### Methyl 3-(8-methoxy-2-methylquinolin-5-yl)-2,2-dimethyl-3-oxopropionate

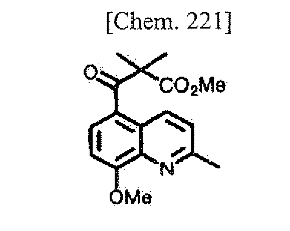

The compound of Example 49 (465 mg) was dissolved in DMF (16 mL) under an argon atmosphere, and 60% sodium hydride (84.2 mg) was added thereto under ice cooling, followed by stirring at room temperature for 30 minutes. Thereafter, iodomethane (0.131 mL) was added thereto under ice cooling, followed by stirring at room temperature for 2 hours. To the reaction liquid was added a saturated aqueous ammonium chloride solution, followed by extraction with ethyl acetate. The organic layer was washed with water and saturated brine in that order, and then dried over anhydrous sodium sulfate. After evaporating the solvent under reduced pressure, the residue was purified by silica gel column chromatography (hexane:ethyl acetate=1:2) to obtain the desired product (395 mg) as a yellow oil.
¹H-NMR (CDCl₃, 400 MHz): δ 1.60 (6H, s), 2.80 (3H, s), 3.60 (3H, s), 4.12 (3H, s), 6.95 (1H, d, J=8.6 Hz), 7.42 (1H, d, J=8.6 Hz), 7.67 (1H, d, J=8.6 Hz), 8.77 (1H, d, J=8.6 Hz).

### <Example 60>

### Methyl 3-(2-ethyl-8-methoxyquinolin-5-yl)-2,2-dimethyl-3-oxopropionate

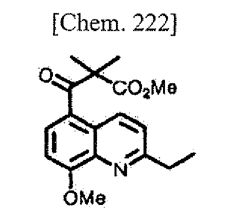

The compound of Example 50 (1.04 g) was dissolved in DMF (30 mL) under an argon atmosphere, and 60% sodium hydride (180 mg) was added thereto at 0°C, followed by stirring at room temperature for 30 minutes. Iodomethane (0.280 mL) was added thereto at 0°C, followed by stirring at room temperature for 2 hours. To the reaction liquid was added a saturated aqueous ammonium chloride solution, followed by extraction three times with ethyl acetate, and the combined extracted layer was washed with saturated brine, and then dried over anhydrous sodium sulfate and filtered. After evaporating the solvent under reduced pressure, the residue was purified by silica gel column chromatography (hexane:ethyl acetate=1.5:1) to obtain the desired product (893 mg) as a colorless powder.
¹H-NMR (CDCl₃, 400 MHz): δ 1.40 (3H, t, J=7.6 Hz), 1.60 (6H, s), 3.07 (2H, q, J=7.6 Hz), 3.60 (3H, s), 4.12 (3H, s), 6.94 (1H, d, J=8.6 Hz), 7.46 (1H, d, J=8.6 Hz), 7.66 (1H, d, J=8.6 Hz), 8.79 (1H, d, J=8.6 Hz).

### <Example 61>

### Methyl 3-(8-methoxy-2-isopropylquinolin-5-yl)-2,2-dimethyl-3-oxopropionate

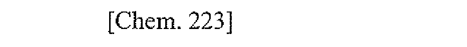

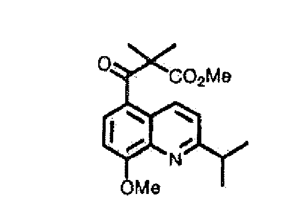

The compound of Example 51 (254 mg) was dissolved in DMF (8 mL) under an argon atmosphere, and 60% sodium hydride (42 mg) was added thereto under ice cooling, followed by stirring at room temperature for 30 minutes. Thereafter, iodomethane (0.065 mL) was added thereto under ice cooling, followed by stirring at room temperature for 45 minutes. To the reaction liquid was added a saturated aqueous ammonium chloride solution, followed by extraction with ethyl acetate. The organic layer was washed with water and saturated brine in this order, and then dried over anhydrous sodium sulfate. After evaporating the solvent under reduced pressure, the residue was purified by silica gel column chromatography (hexane:ethyl acetate=1:1) to obtain the desired product (254 mg) as a pale yellow oil.
¹H-NMR (CDCl₃, 400 MHz): δ 1.39 (6H, d, J=6.7 Hz), 1.60 (6H, s), 331-3.41 (1H, m), 3.60 (3H, s), 4.13 (3H, s), 6.95 (1H, d, J=8.6 Hz), 7.49 (1H, d, J=8.6 Hz), 7.66 (1H, d, J=8.6 Hz), 8.81 (1H, d, J=8.6 Hz).

### <Example 62>

### Methyl 3-(8-methoxy-2-trifluoromethylquinolin-5-yl)-2,2-dimethyl-3-oxopropionate

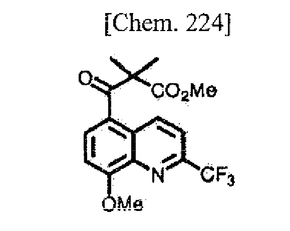

The compound of Example 52 (269 mg) was dissolved in DMF (8 mL) under an argon atmosphere, and 60% sodium hydride (38.0 mg) was added thereto at 0°C, followed by stirring at room temperature for 30 minutes. Iodomethane (0.591 mL) was added thereto at 0°C, followed by stirring at room temperature for 4 hours. To the reaction liquid was added a saturated aqueous ammonium chloride solution, followed by extraction three times with ethyl acetate, and the combined extracted layer was washed with saturated brine, and then dried over anhydrous sodium sulfate and filtered. After evaporating the solvent under reduced pressure, the residue was purified by silica gel column chromatography (hexane:ethyl acetate=4:1) to obtain the desired product (269 mg) as a yellow powder.
¹H-NMR (CDCl₃, 400 MHz): δ 1.61 (6H, s), 3.60 (3H, s), 4.15 (3H, s), 7.05 (1H, d, J=8.6 Hz), 7.85 (1H, d, J=9.2 Hz), 7.87 (1H, d, J=9.2 Hz), 9.14 (1H, d, J=8.6 Hz).

### <Example 63>

### Methyl 3-(7-methoxy-1-methoxymethyl-2-trifluoromethyl-1H-benzo[d]imidazol-4-yl)-2,2-dimethyl-3-oxopropionate

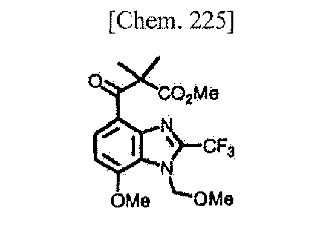

The compound of Example 54 (900 mg) was dissolved in DMF (20.0 mL) under an argon atmosphere, and 60% sodium hydride (106 mg) was added thereto under ice cooling, followed by stirring at room temperature for 30 minutes. Thereafter, iodomethane (10.179 mL) was added thereto under ice cooling, followed by stirring at room temperature for 2 hours. To the reaction liquid was added a saturated aqueous ammonium chloride solution, followed by further addition of water, and the resulting solid was collected by filtration and washed with water. The obtained solid was dissolved in ethyl acetate, washed with saturated brine, and then dried over anhydrous sodium sulfate. After evaporating the solvent under reduced pressure, the residue was purified by silica gel column chromatography (hexane:ethyl acetate=2:1) to obtain the desired product (893 mg) as a colorless oil.
EIMS (+): 388 [M]⁺
¹H-NMR (CDCl₃, 400 MHz): δ 1.58 (6H, s), 3.38 (3H, s), 3.64 (3H, s), 4.07 (3H, s), 5.88 (2H, s), 6.94 (1H, d, J=8.6 Hz), 8.04 (1H, d, J=8.6 Hz).

### <Example 64>

### Methyl 3-(4-methoxy-2-trifluoromethylbenzo [d]thiazol-7-yl)-2,2-dimethyl-3-oxopropionate

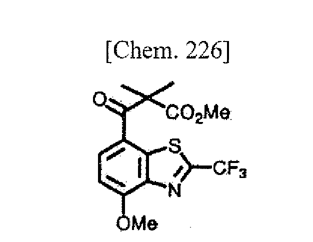

The compound of Example 55 (363 mg) was dissolved in DMF (10 mL) under an argon atmosphere, and 60% sodium hydride (46.0 mg) was added thereto at 0°C, followed by stirring at room temperature for 30 minutes. Iodomethane (0.716 mL) was added thereto at 0°C, followed by stirring at room temperature for 3 hours. To the reaction liquid was added a saturated aqueous ammonium chloride solution, followed by extraction three times with ethyl acetate, and the combined extracted layer was washed with saturated brine, and then dried over anhydrous sodium sulfate and filtered. After evaporating the solvent under reduced pressure, the residue was purified by silica gel column chromatography (ethyl acetate:hexane=3:1) to obtain the desired product (379 mg) as a colorless powder.
¹H-NMR (CDCl₃, 400 MHz): δ 1.64 (6H, s), 3.66 (3H, s), 4.17 (3H, s), 7.03 (1H, d, J=8.6 Hz), 7.99 (1H, d, J=8.6 Hz).

### <Example 65>

### Methyl 3-(7-methoxy-2-trifluoromethylbenzo-furan-4-yl)-2,2-dimethyl-3-oxopropionate

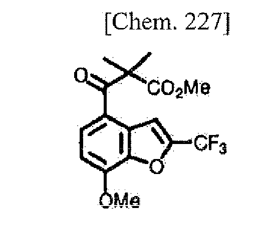

The compound of Example 56 (2.07 g) was dissolved in DMF (30.0 mL) under an argon atmosphere, and 60% sodium hydride (276 mg) was added thereto under ice cooling, followed by stirring at room temperature for 30 minutes. Thereafter, iodomethane (0.470 mL) was added thereto under ice cooling, followed by stirring at room temperature for 2 hours. To the reaction liquid was added a saturated aqueous ammonium chloride solution, followed by further addition of water, and the resulting solid was collected by filtration and washed with water. The obtained solid was dissolved in ethyl acetate, washed with saturated brine, and then dried over anhydrous sodium sulfate. After evaporating the solvent under reduced pressure, the desired product (2.11 g) was obtained as a pale yellow powder.
EIMS (+): 344 [M]⁺
¹H-NMR (CHCl₃, 400 MHz): δ 1.59 (6H, s), 3.64 (3H, s), 4.09 (3H, s), 6.87 (1H, d, J=8.6 Hz), 7.66 (1H, d, J=8.6 Hz), 8.02 (1H, d, J=1.2 Hz).

### <Example 66>

### Methyl 3-(7-methoxy-2-trifluoromethylbenzo[b]thiophen-4-yl)-2,2-dimethyl-3-oxopropionate

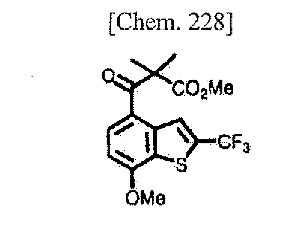

The compound of Example 57 (624 mg) was dissolved in DMF (15.0 mL) under an argon atmosphere, and 60% sodium hydride (79.3 mg) was added thereto under ice cooling, followed by stirring at room temperature for 30 minutes. Thereafter, iodomethane (0.150 mL) was added thereto under ice cooling, followed by stirring at room temperature for 2 hours. To the reaction liquid was added a saturated aqueous ammonium chloride solution, followed by further addition of water, and the resulting solid was collected by filtration and washed with water. The obtained solid was dissolved in ethyl acetate, washed with saturated brine, and then dried over anhydrous sodium sulfate. After evaporating the solvent under reduced pressure, the residue was purified by silica gel column chromatography (ethyl acetate:hexane=2:1) to obtain the desired product (568 mg) as a pale yellow powder.
EIMS (+): 360 [M]⁺
¹H-NMR (CDCl₃, 400 MHz): δ 1,60 (6H, s), 3.64 (3H, s), 4.08 (3H, s), 6.83 (1H, d, J=8.6 Hz), 7.79 (1H, d, J=8.6 Hz), 8.72 (1H, q, J=1.2 Hz).

### <Example 67>

### Methyl 3-(5-methoxy-2-trifluoromethyl-[1,2,4]triazolo[1,5-a]pyridin-8-yl)-2,2-dimethyl-3-oxopropionate

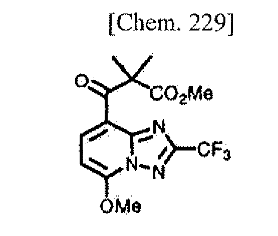

The compound of Example 46 (3.41 g) was dissolved in dichloromethane (90 mL), and a Dess-Martin reagent (4.99 g) was added thereto at 0°C, followed by stirring at room temperature for 1.5 hours. To the reaction liquid was added a saturated aqueous sodium hydrogen carbonate solution, followed by extraction three times with ethyl acetate, and the combined extracted layer was washed with saturated brine, and then dried over sodium sulfate and filtered. After evaporating the solvent under reduced pressure, the residue was purified by silica gel column chromatography (hexane:ethyl acetate=1:1) to obtain the desired product (3.26 g) as a yellow powder.
¹H-NMR (CDCl₃, 400 MHz): δ 1.61 (6H, s), 3.67 (3H, s), 4.30 (3H, s), 6.62 (1H, d, J=8.6 Hz), 8.54 (1H, d, J=8.6 Hz).

### <Example 68>

### Methyl 3-(2-di(t-butoxycarbonyl)amino)-3-methoxypyridin-6-yl)-2,2-dimethyl-3-oxopropionate

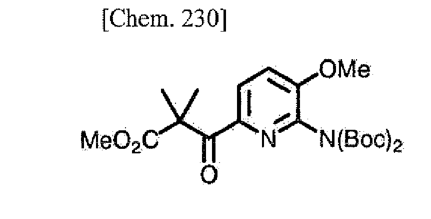

To a solution of the compound of Example 47 (8.63 g) in dichloromethane (150 mL) at 0°C, a Dess-Martin reagent(9.66 g) was added thereto, followed by stirring at 0°C for 1 hour. To the reaction liquid was added a saturated aqueous sodium hydrogen carbonate solution, followed by extraction with ethyl acetate (500 mL). It was washed with water and saturated brine, and then dried over anhydrous sodium sulfate. The extract was concentrated under reduced pressure and then purified by silica gel column chromatography (hexane:ethyl acetate= 3:1→2:1) to obtain the desired product (9.66 g) as a colorless powder.
¹H-NMR (CDCl₃, 400 MHz): δ 1.41 (18H, s), 1.48 (6H, s), 3.57 (3H, s), 3.91 (3H, s), 7.30 (1H, d, J=8.6 Hz), 8:10 (1H, d, J=8.6 Hz).

### <Example 69>

### 5-(8-Methoxyquinolin-5-yl)-4,4-dimethyl-1H-pyrazol-5(4H)-one

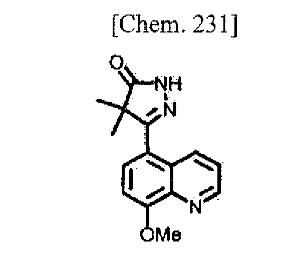

To a solution of the compound of Example 58 (520 mg) in n-propanol (18 mL) was added a hydrazine monohydrate (0.439 mL), followed by stirring at 120°C for 9 hours. The reaction liquid was ice cooled and the precipitated solid was collected by filtration to obtain the desired product (290 mg) as a pale yellow powder.
EIMS (+): 269 [M]⁺
¹H-NMR (CDCl₃, 400 MHz): δ 1.46 (3H, s), 1.59 (3H, s), 4.15 (3H, s), 7.08 (1H, d, J=8.6 Hz), 7.51 (1H, dd, J=8.6, 4.3 Hz), 7.64 (1H, d, J=8.6 Hz), 8.85 (1H, brs), 8.86 (1H, dd, J=8.6, 1.8 Hz), 8.79 (1H, dd, J=4.3, 1.8 Hz).

### <Example 70>

### 3-(8-Methoxy-2-methylquinolin-5-yl)-4,4-dimethyl-1H-pyrazol-5(4H)-one

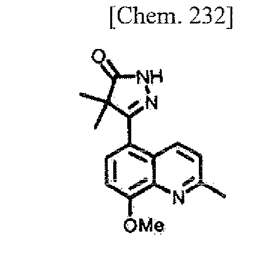

The compound of Example 59 (294 mg) was dissolved in ethanol (10 mL), and hydrazine monohydrate (0.473 mL) was added thereto, followed by stirring for 24 hours under the condition of heating under reflux. After evaporating the solvent under reduced pressure, the residue was purified by silica gel column chromatography (ethyl acetate) to obtain the desired product (197 mg) as a colorless powder.
Elemental analysis: Calculated value as C₁₆H₁₇N₃O₂ C 67.83 H 6.05 N 14.83 Found value C 67.73 H 6.06 N 14.75
EIMS (+): 283 [M]⁺
¹H-NMR (CDCl₃, 400 MHz): δ 1.44 (6H, s), 2.81 (3H, s), 4.13 (3H, s), 7.06 (1H, d, J=8.6 Hz), 7.39 (1H, d, J=8.6 Hz), 7.56 (1H, d, J=8.6 Hz), 8.61 (1H, brs), 8.70 (1H, d, J=8.6 Hz).

### <Example 71>

### 3-(2-Ethyl-8-methoxyquinolin-5-yl)-4,4-dimethyl-1H-pyrazol-5(4H)-one

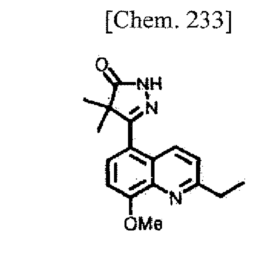

The compound of Example 60 (890 mg) was dissolved in ethanol (20 mL), and acetic acid (3.55 mL) and hydrazine monohydrate (1.37 mL) was added thereto, followed by stirring for 10 hours under the condition of heating under reflux. To the reaction liquid was added water, followed by extraction three times with ethyl acetate, and the combined extracted layer was washed with saturated brine, dried over anhydrous sodium sulfate, and filtered. After evaporating the solvent under reduced pressure, the residue was purified by silica gel column chromatography (hexane:ethyl acetate=1:2) to obtain the desired product (108 mg) as a yellow powder.
HREIMS (+): 297.1482 (Calculated value as C₁₇H₁₉N₃O₂ 297.1477)
¹H-NMR (CDCl₃, 400 MHz): δ 1.41 (3H, t, J=7.3 Hz), 1.44 (6H, s), 3.09 (2H, q, J=7.3 Hz), 4.13 (3H, s), 7.06 (1H, d, J=8.6 Hz), 7.43 (1H, d, J=8.6 Hz), 7.56 (1H, d, J=8.6 Hz), 8.64 (1H, brs), 8.72 (1H, d, J=8.6 Hz).

### <Example 72>

### 3-(8-Methoxy-2-isopropylquinolin-5-yl)-4,4-dimethyl-1H-pyrazol-5(4H)-one

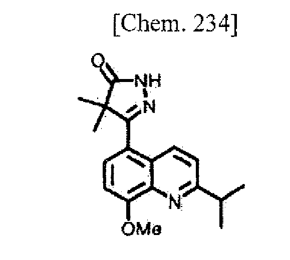

The compound of Example 61 (251 mg) was dissolved in ethanol (8 mL), and hydrazine monohydrate (0.111 mL) was added thereto, followed by stirring for 48 hours under the condition of heating under reflux. After evaporating the solvent under reduced pressure, the residue was purified by silica gel column chromatography (hexane:ethyl acetate=1:1→2:3) to obtain the desired product (187 mg) as a pale yellow powder. Elemental analysis: Found value C 69.22 H 6.77 N 13.39, Calculated value as C₁₈H₂₁N₃O₂ C 69.43 H 6.80 N 13.49
EIMS (+): 311 [M]⁺
¹H-NMR (CDCl₃, 400 MHz): δ 1.39 (6H, d, J=7.3 Hz), 1.45 (6H, s), 3.34-3.45 (1H, m), 4.13 (3H, s), 7.06 (1H, d, J=8.0 Hz), 7.46 (1H, d, J=9.2 Hz), 7.56 (1H, d, J=8.0 Hz), 8.74 (1H, d, J=9.2 Hz).

### <Example 73>

### 3-(8-Methoxy-2-trifluoromethylquinolin-5-yl)-4,4-dimethyl-1H-pyrazol-5(4H)-one

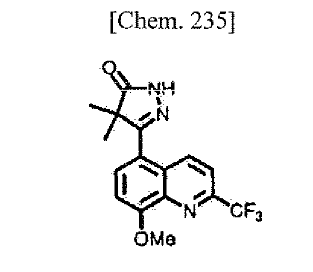

The compound of Example 62 (269 mg) was dissolved in ethanol (4.00 mL), and acetic acid (0.380 mL) and hydrazine monohydrate (0.147 mL) were added thereto, followed by stirring for 10 hours under the condition of heating under reflux. To the reaction liquid was added water, followed by extraction three times with ethyl acetate, and the combined extracted layer was washed with saturated brine, dried over anhydrous sodium sulfate, and filtered. After evaporating the solvent under reduced pressure, the residue was purified by aminated silica (Chromatorex) gel column chromatography (hexane:ethyl acetate=1:1) to obtain the desired product (45 mg) as a pale yellow amorphous.
HREIMS (+): 337.1022 (Calculated value as C₁₆H₁₄F₃N₃O₂ 337.1038)
¹H-NMR (CDCl₃, 400 MHz): δ 1.48 (6H, s), 4.16 (3H, s), 7.17 (1H, d, J=8.6 Hz), 7.78 (1H, d, J=8.6 Hz), 7.83 (1H, d, J=9.2 Hz), 8.66 (1H, brs), 9.16 (1H, d, J=9.2 Hz).

### <Example 74>

### 3-(7-Methoxy-1-methoxymethyl-2-trifluoromethyl-1H-benzo[d]imidazol-4-yl)-4,4-dimethyl-1H-pyrazol-5(4H)-one

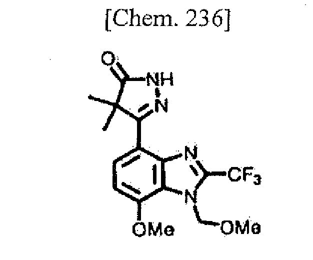

The compound of Example 63 (892 mg) was dissolved in ethanol (10.0 mL), and hydrazine monohydrate (0.335 mL) was added thereto, followed by stirring under the condition of heating under reflux for 5 hours. Thereafter, to the reaction liquid was added hydrazine monohydrate (0.112 mL), followed by stirring under the condition of heating under reflux for 1 hour. After evaporating the solvent under reduced pressure, the residue was purified by silica gel column chromatography (hexane:ethyl acetate =1:1) to obtain the desired product (740 mg) as a colorless powder.
EIMS (+): 370 [M]⁺
¹H-NMR (CDCl₃, 400 MHz): δ 1.61 (6H, s), 3.38 (3H, s), 4.07 (3H, s), 5.92 (2H, s), 6.93 (1H, d, J=8.6 Hz), 7.74 (1H, d, J=8.6 Hz), 8.80 (1H, brs).

### <Example 75>

### 3-(4-Methoxy-2-trifluoromethyl-1H-benzo[d]imidazol-7-yl)-4,4-dimethyl-1H-pyrazol-5(4H)-one

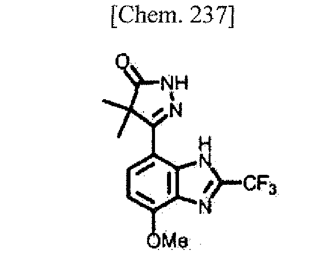

The compound of Example 74 (100 mg) was dissolved in THF (2.00 mL), and 3.00 mol/L hydrochloric acid (1.00 mL) was added thereto, followed by stirring at room temperature for 3 hours. To the reaction liquid was added a saturated aqueous sodium hydrogen carbonate solution, followed by extraction with ethyl acetate. The organic layer was washed with water and saturated brine, and then dried over anhydrous sodium sulfate. After evaporating the solvent under reduced pressure, the residue was purified by silica gel column chromatography (hexane:ethyl acetate =1:2) to obtain the desired product (60.7 mg) as a colorless powder.
HREIMS (+): 326.0961 (Calculated value as C₁₄H₁₃N₄O₂ 326.0991)
(as a tautomeric mixture)
¹H-NMR (CDCl₃, 400 MHz): δ 1.62 (9H, s, tautomer), 3.30 (1H, t, J=8.0 Hz, tautomer), 4.12 (6H, s), 5.38 (2H, d, J=8.0 Hz, tautomer), 6.81 (2H, d, J=8.6 Hz), 7.62 and 7.63 (2H, d, J=8.6 Hz, tautomer), 8.81 (2H, brs), 11.27 (2H, brs).

### <Example 76>

### 3-(4-Methoxy-2-trifluoromethylbenzo[d]thiazol-7-yl)-4,4-dimethyl-1H-pyrazol-5(4H)-one

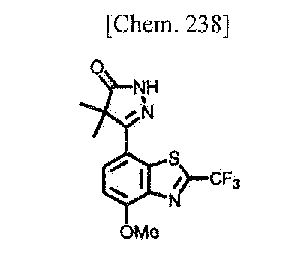

The compound of Example 64 (326 mg) was dissolved in ethanol (9.0 mL), and hydrazine monohydrate (0.175 mL) was added thereto, followed by stirring for 4.5 hours under the condition of heating under reflux. After evaporating the solvent under reduced pressure, the residue was purified by silica gel column chromatography (ethyl acetate:hexane=1:1) to obtain the desired product (250 mg) as a yellow powder.
Elemental analysis: Found value C 48.79% H 3.35% N 12.07%, Calculated value as C₁₄H₁₂F₃N₃O₂S C 48.98% H 3.52% N 12.24%
EIMS (+): 343 [M]⁺
¹H-NMR (CDCl₃, 400 MHz): δ 1.62 (6H, s), 4.15 (3H, s), 7.07 (1H, d, J=8.6 Hz), 7.82 (1H, d, J=8.6 Hz), 8.62 (1H, s).

### <Example 77>

### 3-(7-Methoxy-2-trifluoromethylbenzofuran-4-yl)-4,4-dimethyl-1H-pyrazol-5(4H)-one

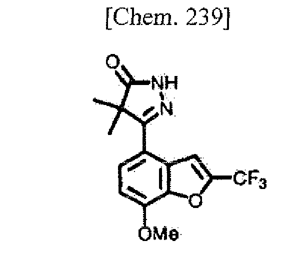

The compound of Example 65 (2.11 g) was dissolved in ethanol (30.0 mL), and hydrazine monohydrate (0.890 mL) was added thereto, followed by stirring for 4.5 hours under the condition of heating under reflux. After evaporating the solvent under reduced pressure, to the residue was added water, and the resulting solid was collected by filtration and washed with water. The obtained solid was dissolved in ethyl acetate, washed with saturated brine, and then dried over anhydrous sodium sulfate. After evaporating the solvent under reduced pressure, the solid was suspended in hexane and collected by filtration to obtain the desired product (1.61 g) as a colorless powder.
Elemental analysis: Found value C 54.96%, H 3.84%, N 8.58%, Calculated value as C₁₅H₁₃F₃N₂O₃ C 55.22%, H 4.02%, N 8.59%.
EIMS (+): 326 [M]⁺
¹H-NMR (CDCl₃, 400 MHz): δ 1.58 (6H, s), 4.08 (3H, s), 6.93 (1H, d, J=8.0 Hz), 7.52 (1H, d, J=8.0 Hz), 7.97-7.98 (1H, m), 8.73 (1H, brs).

### <Example 78>

### 3-(7-Methoxy-2-trifluoromethylbenzo[b]thiophen-4-yl)-4,4-dimethyl-1H-pyrazol-5(4H)-one

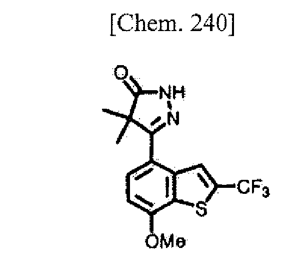

The compound of Example 66 (568 mg) was dissolved in ethanol (10.0 mL), and hydrazine monohydrate (0.230 mL) was added thereto, followed by stirring for 3 hours under the condition of heating under reflux. After evaporating the solvent under reduced pressure, to the residue was added water, and the resulting solid was collected by filtration and washed with water. The obtained solid was dissolved in ethyl acetate, washed with saturated brine, and then dried over anhydrous sodium sulfate. After evaporating the solvent under reduced pressure, the solid was suspended in diisopropyl ether, and collected by filtration to obtain the desired product (410 mg) as a colorless powder.
HREIMS (+): 342.0628 (Calculated value as C₁₅H₁₃F₃N₂O₂S 342.0650)
¹H-NMR (CDCl₃, 400 MHz): δ 1.57 (6H, s), 4.07 (3H, s), 6.90 (1H, d, J=8.6 Hz), 7.68 (1H, d, J=8.6 Hz), 8.64 (1H, brs), 8.70 (1H, d, J=1.2 Hz).

### <Example 79>

### 3-(5-Methoxy-2-trifluoromethyl-[1,2,4]triazolo[1,5-a]pyridin-8-yl)-4,4-dimethyl-1H-pyrazol-5(4H)-one

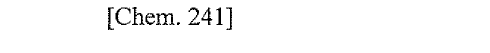

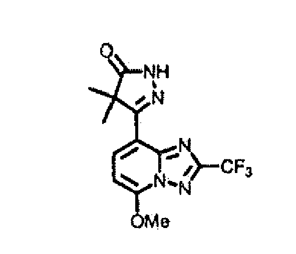

The compound of Example 67 (1.50 g) was dissolved in xylene (40 mL), and t-butyl carbazate (1.72 g) and pyridinium p-toluenesulfonate (109 mg) were added thereto, followed by stirring for 1 hour under the condition of heating under reflux (Dean-Stark). To the reaction liquid was added water, followed by extraction three times with ethyl acetate, and the combined extracted layer was washed with saturated brine, and then dried over anhydrous sodium sulfate and filtered. After evaporating the solvent under reduced pressure, the residue was purified by silica gel column chromatography (hexane:ethyl acetate=1:1→ethyl acetate) to obtain the desired product (214 mg) and an intermediate thereof (495 mg). The intermediate was dissolved in xylene (10 mL), and pyridinium p-toluenesulfonate (40 mg) was added thereto, followed by stirring for 1.5 hours under the condition of heating under reflux (Dean-Stark). To the reaction liquid was added water, followed by extraction three times with ethyl acetate, and the combined extracted layer was washed with saturated brine, and then dried over anhydrous sodium sulfate and filtered. After evaporating the solvent under reduced pressure, the residue was purified by silica gel column chromatography (hexane:ethyl acetate=1:3) to obtain the desired product (82.8 mg). It was combined to obtain the desired product (297 mg) as a colorless powder.
Elemental analysis: Found value C 47.56%, H 3.63%, N 20.89%, Calculated value as C₁₃H₁₂F₃N₅O₂ · 1/5H₂O C 47.19%, H 3.78%, N 21.27%.
HREIMS (+): 327.0924 (Calculated value as C₁₃H₁₂F₃N₅O₂ 327.0943)
¹H-NMR (CDCl₃, 400 MHz): δ 1.64 (6H, s), 4.29 (3H, s), 6.58 (1H, d, J=8.6 Hz), 8.19 (1H, d, J=8.6 Hz), 8.75 (1H, s).

### <Example 80>

### 3-(8-Methoxy-2-trifluoromethylimidazo[1,2-a]pyridin-5-yl)-4,4-dimethyl-1H-pyrazol-5(4H)-one

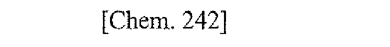

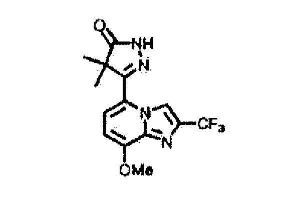

To a solution of the compound of Example 68 (8.03 g) in ethanol (120 mL) was added hydrazine monohydrate (4.30 mL), followed with heating under reflux for 16 hours. A part thereof was concentrated under reduced pressure and then added to water, and the precipitated crystal was collected by filtration. To a solution of the obtained crystal in dichloromethane (80 mL) was added trifluoroacetic acid (30 mL), followed by being left to stand at room temperature for 12 hours. It was concentrated under reduced pressure, neutralized with a saturated aqueous sodium hydrogen carbonate solution, and then extracted with ethyl acetate (500 mL). The extract was washed with water and saturated brine, and then dried over anhydrous sodium sulfate. The extract was concentrated under reduced pressure, the obtained crystal was dissolved in ethanol (30 mL), and 3-bromo-1,1,1-trifluoroacetone (8.82 g) was added thereto, followed by heating under reflux for 15 hours. A part was concentrated under reduced pressure and neutralized with saturated sodium hydrogen carbonate and water, and the precipitated crystal was then collected by filtration to obtain the desired product (2.33 g) as a colorless powder.
Elemental analysis: Found value C 51.33%, H 3.91%, N 16.79%, Calculated value as C₁₄H₁₃F₃N₄O₂ · 1/8H₂O C 51.18%, H 4.07%, N 17.05%.
EIMS (+): 326 [M]⁺
¹H-NMR (CDCl₃, 400 MHz): δ 1.62 (6H, s), 4.11 (3 H, s), 6.68 (1H, d, J=8.6 Hz), 7.29 (1H, d, J=8.6 Hz), 8.96 (1H, brs), 9.41 (1H, s).

### <Example 81>

### 2-Ethyl-8-ethoxycarbonylindolidine

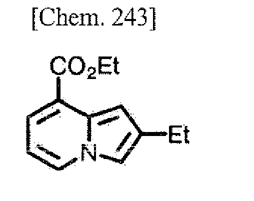

Ethyl 2-methyl nicotinate ester (3.77 mL) was dissolved in ethyl acetate (2.5 mL), and bromomethyl ethyl ketone (2.5 mL) was added thereto, followed by stirring at 70°C for 7 hours. After evaporating the solvent under reduced pressure, the residue was dissolved in toluene (25 mL), and 1,8-diazabicyclo[5,4,0]undec-7-ene (8.06 mL) was added thereto, followed by stirring for 1 hour under the condition of heating under reflux. Cold water was added thereto, followed by extraction three times with ethyl acetate, and the combined extracted layer was washed with saturated brine and then dried over sodium sulfate. After evaporating the solvent under reduced pressure, the residue was purified by silica gel chromatography (hexane-ethyl acetate: 9-1) to obtain the desired product (3.00 g) as a brown oil
¹H-NMR (CDCl₃, 400 MHz); δ 1.31 (3H, t, J=7.3 Hz), 1.44 (3H, t, J=7.3 Hz), 2.75 (2H, q, J=7.3 Hz), 4.42 (2H, q, J=7.3 Hz), 6.46 (1H, t, J=7.3 Hz), 6.96 (1H, s), 7.21 (1H, s), 7.54 (1H, d, J=7.3 Hz), 8.00 (1H, d, J=7.3 Hz).

### <Example 82>

### 2-Ethylindolidin-8-ylcarboxylic acid

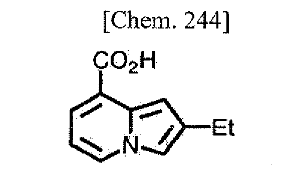

The compound of Example 81 (3.00 g) was dissolved in ethanol (100 mL), and water (50 mL) and potassium hydroxide (2.31 g) were added thereto, followed by stirring for 2.5 hours under the condition of heating under reflux. 1 mol/L Hydrochloric acid was added to adjust to pH 4 to 5, followed by extraction three times with ethyl acetate, and the combined extracted layer was washed with saturated brine and then dried over sodium sulfate. The solvent was evaporated under reduced pressure to obtain the desired product (2.52 g) as a yellow powder.
¹H-NMR (CDCl₃, 400 MHz): δ 1.33 (3H, t, J=7.3 Hz), 2.76 (2H, q, J=7.3 Hz), 6.50 (1H, t, J=6.7 Hz), 7.03 (1H, s), 7.26 (1H, s), 7.66 (1H, d, J=6.7 Hz), 8.06 (1H, d, J=6.7 Hz).

### <Example 83>

### 2-Ethyl-8-propionylindolidine

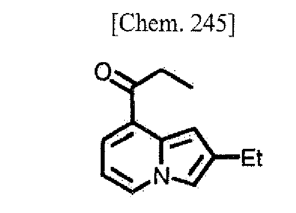

The compound of Example 82 (2.52 g) was dissolved in DMF (130 mL), and diisopropyl ethylamine (10.4 mL), N-(3-dimethylaminopropyl)-N'-ethylcarbodiimide hydrochloride (3.83 g), 1-hydxoxybenzotriazole (2.70 g), and N,O-dimethylhydroxylamine hydrochloride (1.95 g) were added thereto, followed by stirring at room temperature for 5 hours. Water was added thereto, followed by extraction three times with ethyl acetate, and the combined extracted layer was washed with saturated brine and then dried over sodium sulfate. After evaporating the solvent under reduced pressure, the residue was purified by silica gel chromatography (hexane-ethyl acetate: 1.5-1) to obtain amide form (2.76 g) as a yellow oil.
¹H-NMR (CDCl₃, 400 MHz): δ 1.28 (3H, t, J=7.3 Hz), 2.71 (2H, q, J=7.3 Hz), 3.35 (3H, s), 3.66 (3H, s), 6.39 (1H, s), 6.42 (1H, t, J=6.7 Hz), 6.80 (1H, d, J=6.7 Hz), 7.18 (1H, s), 7.86 (1H, d, J=6.7 Hz).
The obtained amide form (2.76 g) was dissolved in THF (60 mL), and a solution (0.97 mol/L, 36.7 mL) of ethyl magnesium bromide in THF was added thereto at 0°C, followed by stirring at room temperature for 1.5 hours. A saturated aqueous ammonium chloride solution was added thereto, followed by extraction three times with ethyl acetate, and the combined extracted layer was washed with saturated brine and then dried over sodium sulfate. After evaporating the solvent under reduced pressure, the residue was purified by silica gel chromatography (hexane-ethyl acetate: 10-1) to obtain the desired product (1.76 g) as a yellow oil.
¹H-NMR (CDCl₃, 400 MHz): δ 1.28 (3H, t, J=7.3 Hz), 1.33 (3H, t, J=7.3 Hz), 2.77 (2H, q, J=7.3 Hz), 3.04 (2H, q, J=7.3 Hz), 6.50 (1H, t, J=6.7 Hz), 7.19 (1H, s), 7.21 (1H, s), 7.43 (1H, d, J=6.7 Hz), 8.04 (1H, d, J=6.7 Hz).

### <Example 84>

### 2-Ethyl-8-(2-ethyl-1,3-dioxolan-2-yl)indolidine

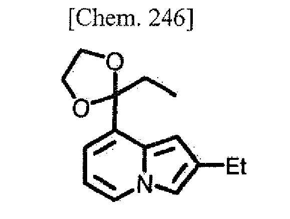

The compound of Example 83 (1.98 g) was dissolved in benzene (100 mL), and ethylene glycol (10 mL) and toluenesulfonic acid monohydrate (187 mg) were added thereto, followed by stirring for 9 hours under the condition of heating under reflux (Dean-stark). A saturated aqueous sodium hydrogen carbonate solution was added thereto, followed by extraction three times with ethyl acetate, and the combined extracted layer was washed with saturated brine, and then dried over anhydrous sodium sulfate and filtered. After evaporating the solvent under reduced pressure, the residue was purified by silica gel column chromatography (hexane-ethyl acetate:10-1) to obtain the desired product (2.02 g) as a yellow oil.
¹H-NMR (CDCl₃, 400 MHz): δ 0.89 (3H, t, J=7.3 Hz), 1.30 (3H, t, J=7.3 Hz), 2.12 (2H, q, J=7.3 Hz), 2.72 (2H, q, J=7.3 Hz), 3.83-3.87 (2H, m), 4.02-4.06 (2H, m), 6.36 (1H, t, J=6.7 Hz), 6.54 (1H, s), 6.72 (1H, d, J=6.7 Hz), 7.13 (1H, s), 7.77 (1H, d, J=6.7 Hz).

### <Example 85>

### 2-Ethyl-8-(2-ethyl-1, 3-dioxolan-2-yl)-5-iodoindolidine

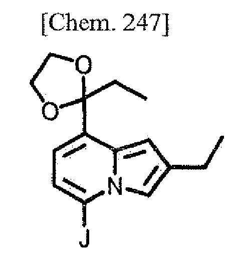

### [wherein J represents iodine]

The compound of Example 84 (2.02 g) and tetramethyl ethylene diamine (6.21 mL) were dissolved in THF (80 mL) under an argon atmosphere, and a solution (2.71 mol/L, 3.34 mL) of n-butyl lithium in hexane was added thereto at -40°C, followed by stirring at -40°C for 2 hours. 1,2-Diiodoethane (2.55 g) was added thereto at -40°C, followed by slowly warming to room temperature and stirring for 16 hours. A saturated aqueous ammonium chloride solution was added thereto, followed by extraction three times with ethyl acetate, and the combined extracted layer was washed with saturated brine, and dried over sodium sulfate. After evaporating the solvent under reduced pressure, the residue was purified by silica gel column chromatography (hexane-ethyl acetate: 30-1 to 15-1) to obtain the desired product (787 mg) as a yellow oil.
EIMS (+): 371 [M]⁺
¹H-NMR (CDCl₃, 400 MHz): δ 0.89 (3H, t, J=7.3 Hz), 1.32 (3H, t, J=7.3 Hz), 2.11 (2H, q, J=7.3 Hz), 2.75 (2H, q, J=7.3 Hz), 3.81-3.85 (2H, m), 4.02-4.04 (2H, m), 6.51 (1H, d, J=7.3 Hz), 6.85 (1H, s), 6.96 (1H, d, J=7.3 Hz), 7.42 (1H, s).

### <Example 86>

### 2-Ethyl-5-iodo-8-propionylindolidine

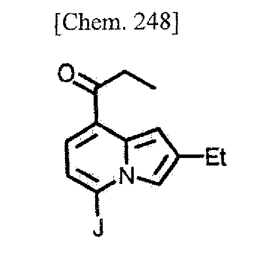

### [wherein J represents iodine]

The compound of Example 85 (787 mg) was dissolved in acetone (10 mL) and water (5.0 mL), and toluene sulfonic acid monohydrate (40.0 mg) was added thereto, followed by stirring at 80°C for 2 hours. A saturated aqueous sodium hydrogen carbonate solution was added thereto, followed by extraction three times with ethyl acetate, and the combined extracted layer was washed with saturated brine, and dried over sodium sulfate. After evaporating the solvent under reduced pressure, the residue was purified by silica gel column chromatography (hexane-ethyl acetate:30-1) to obtain the desired product (578 mg) as a yellow oil.
¹H-NMR (CHCl₃, 400 MHz): δ 1.26 (3H, t, J=7.3 Hz), 1.33 (3H, t, J=7.3 Hz), 2.75 (2H, q, J=7.3 Hz), 3.01 (2H, q, J=7.3 Hz), 7.08 (1H, d, J=7.3 Hz), 7.11 (1H, d, J=7.3 Hz), 7.47 (1H, s), 7.48 (1H, s).

### <Example 87>

### 2-Ethyl-5-methoxy-8-propionylindolidine

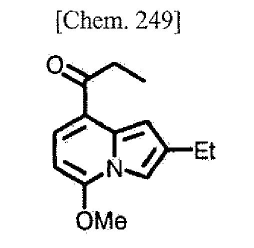

The compound of Example 86 (577 mg) was dissolved in methanol (17 mL) under an argon atmosphere, and sodium methoxide (381 mg) was added thereto, followed by stirring for 6 hours under the condition of heating under reflux. A saturated aqueous ammonium chloride solution was added thereto, followed by extraction three times with ethyl acetate, and the combined extracted layer was washed with saturated brine and then dried over sodium sulfate. After evaporating the solvent under reduced pressure, the residue was purified by silica gel chromatography (hexane-ethyl acetate: 1.5-1) to obtain the desired product (392 mg) as a yellow powder.
¹H-NMR (CDCl₃, 400 MHz): δ 1.24 (3H, t, J=7.3 Hz), 1.31 (3H, t, J=7.3 Hz), 2.75 (2H, q, J=7.3 Hz), 2.98 (2H, q, J=7.3 Hz), 4.11 (3H, s), 5.80 (1H, d, J=7.9 Hz), 7.22 (1H, s), 7.29 (1H, s), 7.57 (1H, d, J=7.9 Hz).

### <Example 88>

### Methyl 3-(2-ethyl-5-methoxyindolidin-8-yl)-2-methyl-3-oxopropionate

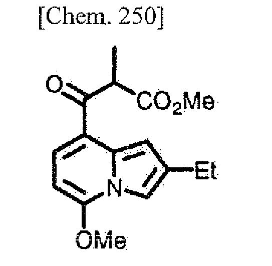

The compound of Example 87 (1.50 g) was dissolved in dimethyl carbonate (30 mL) under an argon atmosphere, and 60% sodium hydride (778 mg) was added thereto, followed by stirring for 2 hours under the condition of heating under reflux. To the reaction liquid was added ice water, followed by extraction three times with ethyl acetate. The extract was washed with saturated brine and then dried over anhydrous sodium sulfate. After evaporating the solvent under reduced pressure, the residue was purified by silica gel column chromatography (hexane:ethyl acetate=8: to 4:1) to obtain the desired product (1.73 g) as a yellow oil.
¹H-NMR (CDCl₃, 400 MHz): δ 1.30 (3H, t, J=7.3 Hz), 1.52 (3H, d, J=7.3 Hz), 2.74 (2H, q, J=7.3 Hz), 3.68 (3H, s), 4.13 (3H, s), 4.45 (1H, q, J=7.3 Hz), 5.84 (1H, d, J=7.9 Hz), 7.27 (1H, d, J=1.2 Hz), 7.30 (1H, d, J=1.2 Hz), 7.64 (1H, d, J=7.9 Hz).
EIMS (+): 289 [M]⁺.

### <Example 89>

### Methyl 3-(2-ethyl-5-methoxyindolidin-8-yl)-2,2-dimethyl-3-oxopropionate

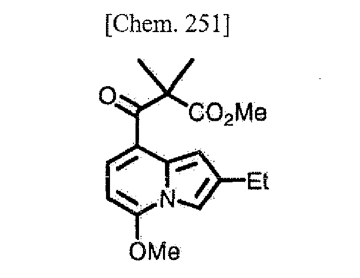

The compound of Example 88 (1.73 g) was dissolved in DMF (50 mL) under an argon atmosphere, and 60% sodium hydride (310 mg) was added thereto at 0°C, followed by stirring at room temperature for 30 minutes. Iodomethane (0.484 mL) was added thereto at 0°C, followed by stirring at room temperature for 1 hour. To the reaction liquid was added ice water, followed by extraction three times with ethyl acetate. The extract was washed with saturated brine and then dried over anhydrous sodium sulfate. After evaporating the solvent under reduced pressure, the residue was purified by silica gel column chromatography (hexane:ethyl acetate=8:1 to 4:1) to obtain the desired product (1.65 g) as a yellow powder. ¹H-NMR (CDCl₃, 400 MHz): δ 131 (3H, t, J=7.6 Hz), 1.58 (6H, s), 2.75 (2H, q, 1=7.6 Hz), 3.62 (3H, s), 4.11 (3H, s), 5.76 (1H, d, J=7.9 Hz), 7.28 (1H, d, J=1.2 Hz), 7.29 (1H, d, J=1.2 Hz), 7.37 (1H, d, J=7.9 Hz).
EIMS (+): 303 [M]⁺.

### <Example 90>

### 3-(2-Ethyl-5-methoxyindolidin-8-yl)-4,4-dimethyl-1H-pyrazol-5(4H)-one

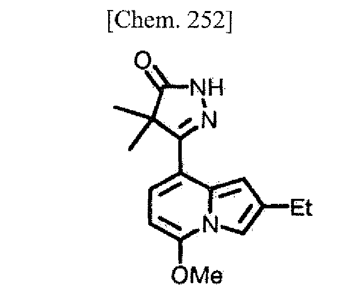

The compound of Example 89 (1.58 g) was dissolved in xylene (50 mL), and t-butyl carbazate (3.44 g) and pyridinium p-toluenesulfonate (131 mg) were added thereto, followed by stirring at 150°C for 41 hours. To the reaction liquid was added a saturated aqueous sodium hydrogen carbonate solution, followed by extraction three times with ethyl acetate. The extract was washed with saturated brine and then dried over anhydrous sodium sulfate. After evaporating the solvent under reduced pressure, the residue was purified by silica gel column chromatography (hexane:ethyl acetate=4:1 to 2:1) and then washed with diisopropyl ether to obtain the desired product (284 mg) as a yellow powder.
¹H-NMR (CDCl₃, 400 MHz): δ 1.32 (3H, t, J=7.6 Hz), 1.56 (6H, s), 2.75 (2H, q, J=7.6 Hz), 4.10 (3H, s), 5.82 (1H, d, J=7.3 Hz), 7.16 (1H, d, J=7.3 Hz), 7.17 (1H, s), 7.33 (1H, s), 8.51 (1H, s).
HREIMS (+): 285.1466 (Calculated value as C₁₆H₁₉N₃O₂ 285.1477).
Elemental analysis: Found value C 67.11%, H 6.74%, N 14.49%, Calculated value as C₁₆H₁₉N₃O₂ C 67.35%, H 6.71%, N 14.73%.

### <Example 91>

### 6-(4-Methoxyphenyl)-5-methyl-4,5-dihydro-2H-pyridazin-3-one

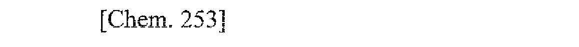

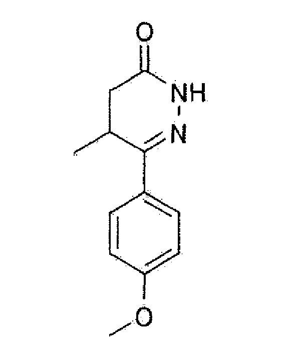

Commercially available 4-methoxypropiophenone (25.0 g) was dissolved in THF (750 mL) under an argon gas atmosphere, and a lithium bistrimethylsilyl amide (1.00 mol/L THF solution, 153 mL) was added dropwise under ice cooling, followed by stirring at the same temperature for 30 minutes. Thereafter, tert-butyl bromoacetate (33.7 mL) was added thereto at the same temperature, followed by stirring at room temperature for 3 hours. To the reaction liquid was added a saturated aqueous ammonium chloride solution, followed by extraction with ethyl acetate. The extracted layer was washed with water and saturated brine in this order, and then dried over anhydrous sodium sulfate. The yellowish brown oil obtained by evaporating the solvent under reduced pressure was dissolved in acetonitrile (250 mL), and montmorillonite KSF (30.0 g) was added thereto, followed by stirring for 7 hours under the condition of heating under reflux. The insoluble materials were removed by filtration and the solvent of the filtrate was evaporated under reduced pressure to obtain a yellowish brown oil. This was dissolved in ethanol (300 mL), and hydrazine monohydrate (22.0 mL) was added thereto, followed by stirring for 2.5 hours under the condition of heating under reflux. After evaporating the solvent under reduced pressure, to the residue was added ice water, and the resulting solid was collected by filtration. The obtained solid was washed with water, cold ethanol, and diisopropyl ether in that order to obtain the desired product (26.7 g) as a colorless powder.
¹H-NMR (CDCl₃, 400 MHz): δ 1.25 (3H, d, J=7.3 Hz), 2.46 (1H, d, J=17.1 Hz), 2.71 (1H, dd, J=17.1, 6.7 Hz), 3.32-3.36 (1H, m), 3.85 (3H, s), 6.93-6.95 (2H, m), 7.69-7.72 (2H, m), 8.44 (1H, brs).

### <Example 92>

### 6-(4-Hydroxyphenyl)-5-methyl-4,5-dihydro-2H-pyridazin-3-one

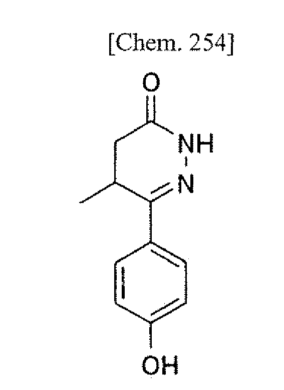

The compound of Example 91 (26.3 g) was dissolved in dichloromethane (500 mL), and aluminum chloride (323 g) was added thereto under ice cooling, followed by stirring at room temperature for 40 hours. The reaction liquid was poured into ice water, followed by extraction with THF, and the extracted layer was dried over anhydrous magnesium sulfate. After evaporating the solvent under reduced pressure, the resulting solid was suspended in diisopropyl ether and collected by filtration to obtain the desired product (20.9 g) as a pale yellow powder.
¹H-NMR (DMSO-d₆, 400 MHz): δ 1.04 (3H, d, J=7.3 Hz), 2.18 (1H, d, J=15.9 Hz), 2.63 (1H, dd, J=15.9, 1.8 Hz), 3.28-3.33 (1H, m), 6.78-6.80 (2H, m), 7.59-7.63 (2H, m), 9.78 (1H, s), 10.8 (1H, s).

### <Example 93>

### 6-(4-Methoxyphenyl)-5-methyl-2H-pyridazin-3-one

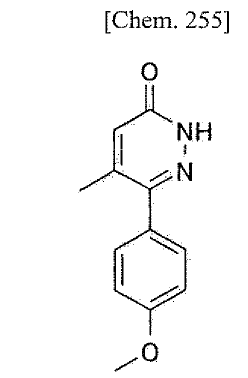

The compound of Example 91 (6.50 g) was dissolved in a 0.5 mol/L aqueous sodium hydroxide solution (350 mL), and sodium para-nitrobenzenesulfonate (6.70 g) was added thereto, followed by stirring for 4 hours under the condition of heating under reflux. The reaction liquid was neutralized with 6 mol/L hydrochloric acid, and the precipitated solid was then collected by filtration to obtain the desired product (3.90 g) as a white powder. ¹H-NMR (CDCl₃, 400 MHz): δ 2.20 (3H, s), 3.86 (3H, s), 6.83 (1H, s), 6.97 (2H, d, J=8.6 Hz), 7.35 (2H, d, J=8.6 Hz).

### <Example 94>

### 6-(4-Hydroxyphenyl)-5-methyl-2H-pyridazin-3-one

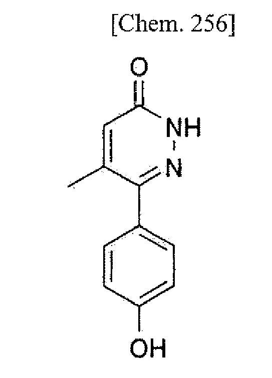

The compound of Example 93 (3.90 g) was dissolved in dichloromethane (180 mL), and aluminum chloride (24.1 g) was added thereto, followed by stirring at room temperature for 8 hours. To the reaction liquid was added water, followed by extraction with THF, the extracted layer was washed with saturated brine and then dried over anhydrous sodium sulfate, and the solvent was evaporated under reduced pressure to obtain the desired product (2.40 g) as a yellow powder.
LRMS (EI⁺):202 [M⁺].
¹H-NMR (DMSO-d₆, 400 MHz): δ 1.89 (3H, s), 6.56 (1H, s), 6.60 (2H, d, J=8.6 Hz), 7.05 (2H, d, J=8.6 Hz).

### <Example 95>

### 6-(4-t-Butyldimethylsilyloxyphenyl)-5-methyl-2H-pyridazin-3-one

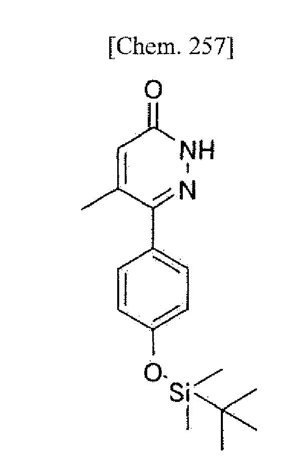

The compound of Example 94 (300 mg) was dissolved in DMF (8.0 mL), and imidazole (111 mg) and t-butyldimethylsilyl chloride (246 mg) were added thereto at 0°C, followed by stirring at room temperature for 4 hours. To the reaction liquid was added a saturated aqueous ammonium chloride solution, followed by extraction with ethyl acetate, and the extracted layer was washed with saturated brine and then dried over anhydrous sodium sulfate. The residue obtained by evaporating the solvent under reduced pressure was purified by silica gel chromatography (hexane:ethyl acetate=1:1) to obtain the desired product (208 mg) as a white powder.
¹H-NMR (CDCl₃, 400 MHz): δ 0.21 (6H, s), 0.95 (9H, s), 2.10 (3H, d, J=1.2 Hz), 6.79 (1H, d, J=1.2 Hz), 6.91 (2H, d, J=8.6 Hz), 7.36 (2H, d, J=8.6 Hz), 13.08 (1H, s).

### <Example 96>

### 2-t-Butoxycarbonyl-6-(4-t-butyldimethylsilyloxyphenyl)-5-methyl-2H-pyridazin-3-one

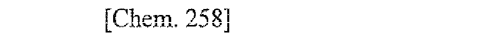

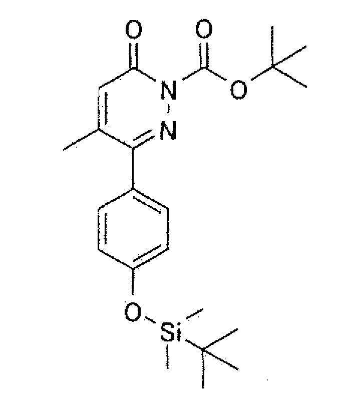

The compound of Example 95 (207 mg) was dissolved in acetonitrile (6.5 mL) under an argon atmosphere, and di-t-butyldicarbonate (170 mg) and 4-dimethyl aminopyridine (9.50 mg) were added thereto, followed by stirring at room temperature for 3 hours. To the reaction liquid was added water, followed by extraction with ethyl acetate, and the extracted layer was washed with saturated brine and then dried over anhydrous sodium sulfate. The solvent was evaporated under reduced pressure and the obtained residue was purified by silica gel chromatography (hexane:ethyl acetate=3:1) to obtain the desired product (159 mg) as a white powder.
¹H-NMR (CDCl₃, 400 MHz): δ 0.22 (6H, s), 0.99 (9H, s), 1.63 (9H, s), 2.15 (3H, d, J=1.2 Hz), 6.76 (1H, d, J=1.2 Hz), 6.89 (2H, d, J=8.6 Hz), 7.31 (2H, d, J=8.6 Hz).

### <Example 97>

### 2-t-Butoxycarbonyl-6-(4-hydroxyphenyl)-5-methyl-2H-pyridazin-3-one

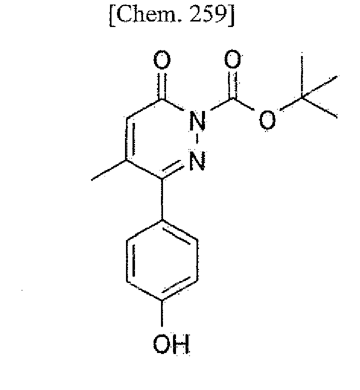

The compound of Example 96 (159 mg) was dissolved in THF (4.0 mL) under an argon atmosphere, and tetrabutyl ammonium fluoride (1.0 mol/L THF solution, 0.763 mL) was added thereto at 0°C, followed by stirring at room temperature for 40 minutes. To the reaction liquid was added a saturated aqueous ammonium chloride solution, followed by extraction with ethyl acetate, and the extracted layer was washed with saturated brine and then dried over anhydrous sodium sulfate. The solvent was evaporated under reduced pressure and the obtained residue was purified by silica gel chromatography (hexane-ethyl acetate, 2-1→1-1) to obtain the desired product (32.2 mg) as a white powder.
¹H-NMR (DMSO-d₆, 400 MHz): δ 1.53 (9H, s), 2.11 (3H, s), 6.83 (2H, d, J=8.6 Hz), 6.92 (1H, s), 7.30 (2H, d, J=8.6 Hz).

### <Example 98>

### t-Butyl 4-(4-methoxyphenyl)-4-oxobutanoate ester

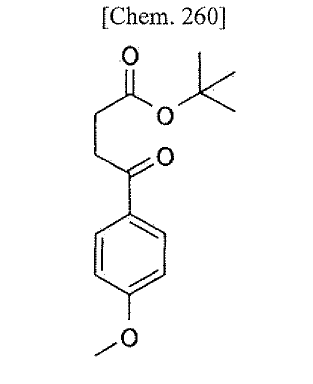

4-Methoxyacetophenone (15.0 g) was dissolved in THF (500 mL) under an argon atmosphere, and lithium hexamethyl disilazane (1.0 mol/L, THF solution, 119.9 mL) was added thereto at 0°C, followed by stirring at room temperature for 30 minutes. To the reaction liquid was added t-butyl bromoacetate (16.2 mL) at 0°C, followed by stirring at room temperature for 3 hours. Then, a saturated aqueous ammonium chloride solution was added thereto, followed by extraction with ethyl acetate. The extracted layer was washed with saturated brine and then dried over sodium sulfate, and the solvent was evaporated under reduced pressure to obtain the desired product (27.4 g) as a red oil.
¹H-NMR (CDCl₃, 400 MHz):δ 1.45 (9H, s), 2.67 (2H, t, J=6.7 Hz), 3.21 (2H, t, J=6.7 Hz), 3.87 (3H, s), 6.93(2H, d, J=8.9 Hz), 7.96 (2H, d, J=8.9 Hz).

### <Examples 99>

### 6-(4-Methoxyphenyl)-4,5-dihydro-2H-pyridazin-3-one

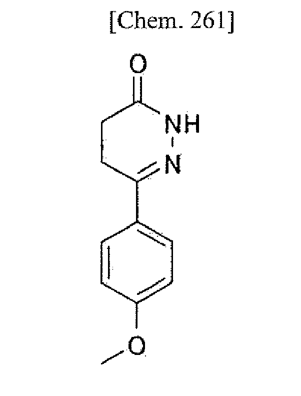

The compound of Example 98 (27.4 g) was dissolved in dichloromethane (100 mL), and trifluoroacetic acid (30 mL) was added thereto, and after being left to stand for 16 hours, the solvent was evaporated under reduced pressure. The obtained oil was dissolved in ethanol (200 mL), and hydrazine monohydrate (14.5 mL) was added thereto, followed by stirring for 2.5 hours under the condition of heating under reflux. After evaporating the solvent under reduced pressure, the residue was washed with diethyl ether, and the solid was collected by filtration to obtain the desired product (18.9 g) as a yellow powder.
EIMS (+): 204 [M]⁺.
¹H-NMR (CDCl₃, 400 MHz): δ 2.60 (2H, t, J=8.3 Hz), 2.97 (2H, t, J=8.3 Hz), 3.85 (3H, s), 6.93 (2H, d, J=9.2 Hz), 7.67 (2H, d, J=9.2 Hz), 8.47 (1H, brs).

### <Example 100>

### 6-(4-Hydroxyphenyl)-4,5-dihydro-2H-pyridazin-3-one

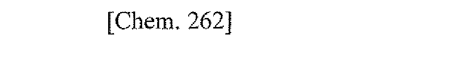

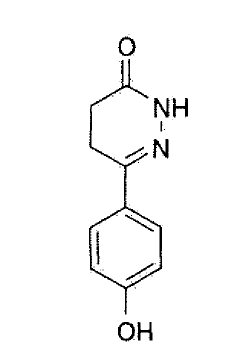

The compound of Example 99 (6.00 g) was dissolved in dichloromethane (300 mL), and aluminum chloride (78.4 g) was added thereto, followed by stirring at room temperature for 16 hours. To the reaction liquid was added water, followed by extraction with THF, the extracted layer was washed with saturated brine and then dried over anhydrous sodium sulfate, and the solvent was evaporated under reduced pressure to obtain the desired product (4.50 g) as a yellow powder.
EIMS (+): 190 [M]⁺.
¹H-NMR (DMSO-d₆, 400 MHz): δ 2.38 (2H, t, J=8.3 Hz), 2.86 (2H, t, J=8.3 Hz), 6.77 (2H, d, J=8.6 Hz), 7.57 (2H, d, J=8.6 Hz), 6.77 (1H, s), 10.73 (1H, s).

### <Example 101>

### 6-(4-Hydroxyphenyl)-2H-pyridazin-3-one

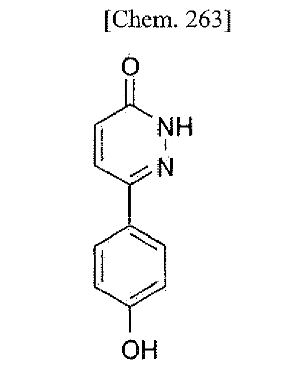

The compound of Example 100 (8.50 g) was dissolved in a 0.5 mol/L aqueous sodium hydroxide solution (500 mL), and sodium para-nitrobenzenesulfonate (10.3 g) was added thereto, followed by stirring for 1.5 hours under the condition of heating under reflux. The reaction liquid was neutralized with 6 mol/L hydrochloric acid and the precipitated solid was collected by filtration. The obtained solid was dissolved in dichloromethane (400 mL) and aluminum chloride (108 g) was added thereto, followed by stirring at room temperature for 19 hours. To the reaction liquid was added water, followed by extraction with THF, and the extracted layer was washed with saturated brine and then dried over anhydrous sodium sulfate. The residue obtained by evaporating the solvent under reduced pressure was washed with diethyl ether to obtain the desired product (3.29 g) as a yellow powder.
EIMS (+): 188 [M]⁺.
¹H-NMR (DMSO-d₆, 400 MHz): δ 6.82 (2H, d, J=8.6 Hz), 6.90 (1H, d, J=9.8 Hz), 7.66 (2H, d, J=8.6 Hz), 7.92 (1H, d, J=9.8 Hz), 9.79 (1H, brs).

### <Example 102>

### 6-(4-t-Butyldimethyisilyloxyphenyl)-2H-pyridazin-3-one

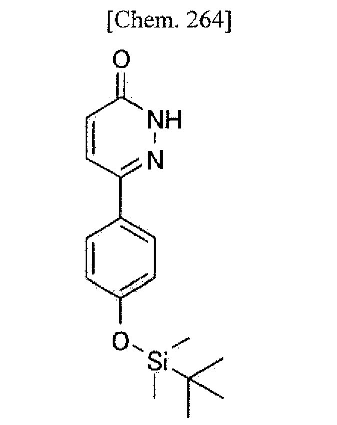

The reaction was carried out in the same manner as in Example 95 using the compound of Example 101 to obtain the desired product as a yellow powder.
¹H-NMR (CDCl₃, 400 MHz): δ 0.24 (6H, s), 1.02 (9H, s), 6.93 (2H, d, J=8.6 Hz), 7.05 (1H, d, J=10.0 Hz), 7.67 (2H, d, J=8.6 Hz), 7.72 (1H, d, J=10.0 Hz).

### <Example 103>

### 2-t-Butoxycarbonyl-6-(4-t-butyldimethylsilyloxyphenyl)-2H-pyridazin-3 -one

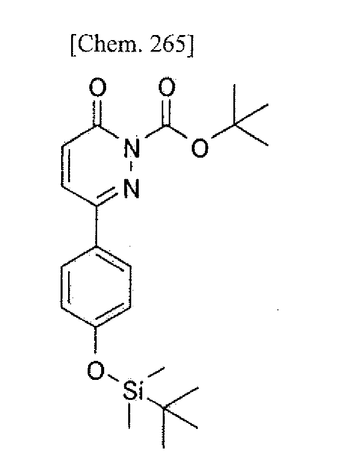

The reaction was carried out in the same manner as in Example 96 using the compound of Example 102 to obtain the desired product as a white powder.
¹H-NMR (CDCl₃, 400 MHz): δ 0.22 (6H, s), 0.99 (9H, s), 1.66 (9H, s), 6.91 (2H, d, J=8.6 Hz), 6.98 (1H, d, J=9.8 Hz), 7.62 (1H, d, J=9.8 Hz), 7.69 (2H, d, J=8.6 Hz).

### <Examples 104>

### 2-t-Butoxycarbonyl-6-(4-hydroxyphenyl)-2H-pyridazin-3-one

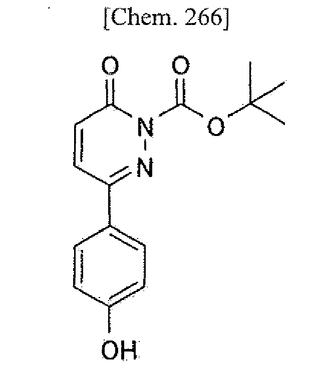

The reaction was carried out in the same manner as in Example 97 using the compound of Example 103 to obtain the desired product as a white powder.
¹H-NMR (CDCl₃, 400 MHz): δ 1.55 (9H, s), 6.84 (2H, d, J=8.6 Hz), 7.05 (1H, d, J=10.4 Hz), 7.70 (2H, d, J=8.6 Hz), 8.01 (1H, d, J=10.4 Hz), 9.93 (1H, s).

### <Example 105>

### Methyl 3-(4-Fnethnxyphenyl)-3-oxaprapzanate ester

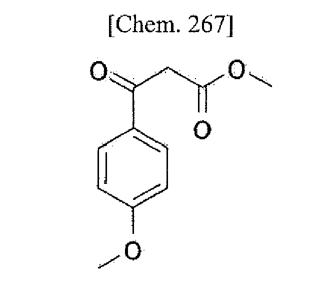

4-Methoxyacetophenone (7.00 g) was dissolved in dimethyl carbonate (100 mL), and a few droplets of 60% sodium hydride (5.60 g) and a few droplets of methanol were added thereto, followed by stirring for 1.5 hours under the condition of heating under reflux. To the reaction liquid was added a saturated aqueous ammonium chloride solution, followed by extraction with ethyl acetate, and the extracted layer was washed with saturated brine and then dried over anhydrous sodium sulfate. The solvent was evaporated under reduced pressure to obtain the desired product (9.70 g) as a colorless oil.
¹H-NMR (CDCl₃, 400 MHz): δ 3.75 (3H, s), 3.88 (3H, s), 3.97 (2H, s), 6.95 (2H, d, J=8.9 Hz), 7.93 (2H, d, J=8.9 Hz).

### <Example 106>

### 5-(4-Methoxyphenyl)-4,4-dimethyl-2,4-dihydropyrazol-3-one

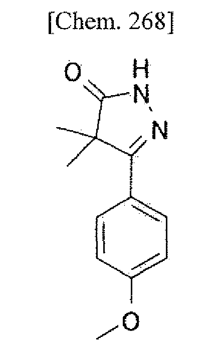

The compound of Example 105 (9.70 g) was dissolved in DMF (150 mL) under an argon atmosphere, and 60% sodium hydride (2.50 g) was added thereto at 0°C, followed by stirring at room temperature for 30 minutes. To the reaction liquid was added iodomethane (3.9 mL) at 0°C, followed by stirring at room temperature for 1 hour, 60% sodium hydride (2.50 g) was added thereto at 0°C again, followed by stirring at room temperature for 30 minutes, and then iodomethane (3.9 mL) was added thereto at 0°C, followed by stirring at room temperature for 1.5 hours. To the reaction liquid was added a saturated aqueous ammonium chloride solution, followed by extraction with ethyl acetate, and the extracted layer was washed with saturated brine and then dried over anhydrous sodium sulfate. The residue obtained by evaporating the solvent under reduced pressure was purified by silica gel chromatography (hexane:ethyl acetate=10:1) to obtain a solid. The obtained solid was dissolved in ethanol (100 mL) and hydrazine monohydrate (6.70 mL) was added thereto, followed by stirring for 9 hours under the condition of heating under reflux. After evaporating the solvent of the reaction liquid under reduced pressure, the residue was washed with hexane to obtain the desired product (6.60 g) as a white powder.
¹H-NMR (CDCl₃, 400 MHz): δ 1.51 (6H, s), 3.86 (3H, s), 6.95 (2H, d, J=9.2 Hz), 7.74 (2H, d, J=9.2 Hz).

### <Example 107>

### 5-(4-Hydroxyphenyl)-4,4-diznethyl-2,4-dihydropyrazol-3-one

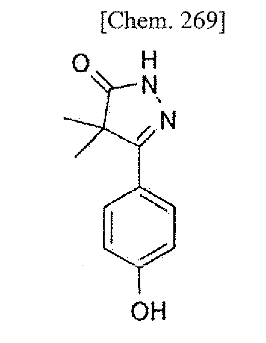

The compound of Example 106 (6.60 g) was dissolved in dichloromethane (300 mL), and aluminum chloride (80.6 g) was added thereto, followed by stirring at room temperature for 22 hours. To the reaction liquid was added water, followed by extraction with THF, and the extracted layer was washed with saturated brine and then dried over anhydrous sodium sulfate. The solvent was evaporated under reduced pressure and the residue was washed with diethyl ether to obtain the desired product (5.10 g) as a yellow powder.
¹H-NMR (DMSO-d₆, 400 MHz): δ 6.93 (6H, s), 6.42 (2H, d, J=8.2 Hz), 7.26 (2H, d, J=8.6 Hz), 9.48 (1H, s), 10.91 (1H, s).

### <Example 108>

### 3,N-dimethoxy-N-methylbenzamide

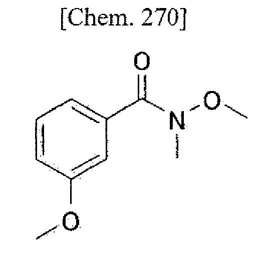

3-Methoxybenzoic acid (10.0 g) was dissolved in dichloromethane (300 mL), and N,O-dimethylhydroxylamine hydrochloride (7.00 g), triethylamine (11.9 mL), and N-(3-dimethylaminopropyl)-N'-ethylcarbodiimide hydrochloride (18.9 g) were added thereto at 0°C, followed by stirring at room temperature for 18 hours. To the reaction liquid was added a saturated aqueous sodium hydrogen carbonate solution, followed by extraction with ethyl acetate, and the extracted layer was washed with saturated brine and then dried over anhydrous sodium sulfate. The solvent was evaporated under reduced pressure to obtain the desired product (14.5 g) as a yellow oil.
¹H-NMR (CDCl₃, 400 MHz): δ 3.36 (3H, s), 3.58 (3H, s), 3.83 (3H, s), 6.98-7.01 (1H, m), 7.20-7.25 (2H, m), 7.31 (1H, t, J=7.9 Hz).

### <Example 109>

### 3-Methoxypropiophenone

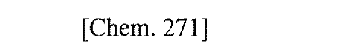

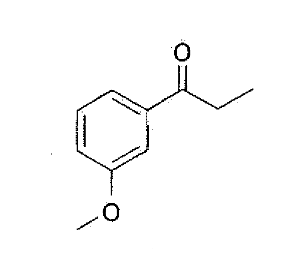

The compound of Example 108 (5.86 g) was dissolved in THF (150 mL) under an argon atmosphere, and ethyl magnesium bromide (0.96 mol/L, THF solution, 100 mL) was added thereto at 0°C, followed by stirring at room temperature for 3.5 hours. To the reaction liquid was added 1.0 mol/L hydrochloric acid, followed by extraction with ethyl acetate, and the extracted layer was washed with saturated brine and then dried over anhydrous sodium sulfate. The solvent was evaporated under reduced pressure to obtain the desired product (5.00 g) as a yellow oil.
¹H-NMR (CDCl₃, 400 MHz): δ 1.23 (3H, t, J=7.3 Hz), 3.00 (2H, q, J=7.3 Hz), 3.86 (3H, s), 7.10 (1H, dd, J= 2.4, 7.9 Hz), 7.37 (1H, t, J=7.9 Hz), 7.50 (1H, t, J=2.4 Hz), 7.54 (1H, d, J=7.9 Hz).

### <Example 110>

### 6-(3-Methoxyphenyl)-5-methyl-4,5-dihydro-2H-pyridazin-3-one

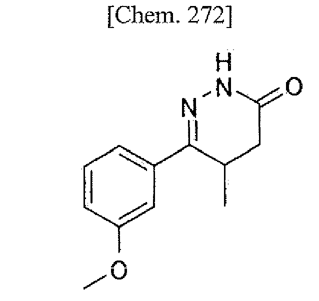

The compound of Example 109 (3.04 g) was dissolved in acetic acid (35 mL), and bromine (0.938 mL) was added thereto, followed by stirring at room temperature for 1.5 hours. The solvent of the reaction liquid was evaporated under reduced pressure, the residue was extracted with ethyl acetate, and the extracted layer was washed with 1.0 mol/L hydrochloric acid and a saturated aqueous sodium hydrogen carbonate solution in that order and then dried over anhydrous sodium sulfate. The solvent was evaporated under reduced pressure to obtain the residue. Diethyl malonate (3.1 mL) was dissolved in DMF (20 mL) under an argon atmosphere, and 60% sodium hydride (752 mg) was added thereto at 0°C, followed by stirring at room temperature for 3 hours. Then, the obtained residue was dissolved in DMF (10 mL) and then added thereto, followed by stirring at 110°C for 2.5 hours. To the reaction liquid was added a saturated aqueous ammonium chloride solution, followed by extraction with ethyl acetate, and the extracted layer was washed with saturated brine and then dried over anhydrous sodium sulfate. The residue obtained by evaporating the solvent under reduced pressure was purified by silica gel chromatography (hexane:ethyl acetate=8:1). The obtained oil was dissolved in 6.0 mol/L hydrochloric acid, followed by stirring for 8 hours under the condition of heating under reflux. The reaction liquid was extracted with ethyl acetate, the extracted layer was washed with saturated brine and then dried over anhydrous sodium sulfate, and the solvent was evaporated under reduced pressure. The obtained residue was dissolved in ethanol (75 mL), and hydrazine monohydrate (1.93 mL) was added thereto, followed by stirring for 5 hours under the condition of heating under reflux. The residue obtained by evaporating the solvent of the reaction liquid under reduced pressure was purified by silica gel chromatography (hexane:ethyl acetate=1:1) to obtain the desired product (1.44 g) as a yellow powder.
¹H-NMR (CDCl₃, 400 MHz). δ 1.26 (3H, d, J=7.3 Hz), 2.48 (1H, d, J=17.7 Hz), 2.72 (1H, dd, J=17.7, 7.0 Hz), 3.33-3.37 (1H, m), 3.85 (3H, s), 6.96-6.98 (1H, m), 7.30-7.35 (3H, m), 8.53 (1H, brs).

### <Example 111>

### 6-(3-Hydroxyphenyl)-5-methyl-4,5-dihydro-2H-pyridazin-3-one

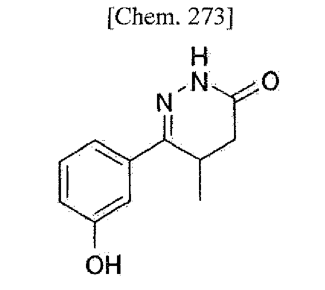

The compound of Example 110 (1.44 g) was dissolved in dichloromethane (50 mL) under an argon atmosphere, and borane tribromide (1.0 mol/L dichloromethane solution, 13.2 mL) was added thereto at 0°C, followed by stirring at room temperature for 5.5 hours. To the reaction liquid was added water, followed by extraction with THF, the extracted layer was washed with saturated brine and then dried over anhydrous sodium sulfate, and the solvent was evaporated under reduced pressure. The residue was washed with diethyl ether to obtain the desired product (630 mg) as a yellow powder. Further, after evaporating the solvent of the diethyl ether washing liquid under reduced pressure, the residue was purified by silica gel chromatography (hexane:ethyl acetate=4:1→1:1) to obtain the desired product (508 mg) as a white solid.
¹H-NMR (DMSO-d₆, 400 MHz): δ 0.68 (3H, d, J=7.3 Hz), 1.84 (1H, d, J=15.9 Hz), 2.30 (1H, dd, J=15.3, 6.7 Hz), 2.33-2.95 (1H, m), 6.41-6.44 (1H, m), 6.80-6.87 (3H, m), 9.16 (1H, s), 10.55 (1H, s).

### <Example 112>

### Methyl 3-(3-methoxyphenyl)-2-methyl-3-oxopropionate ester

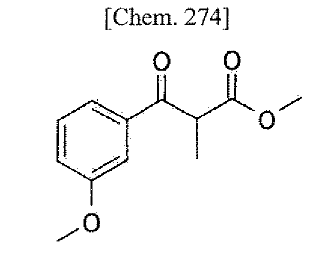

The compound of Example 109 (2.00 g) was dissolved in dimethyl carbonate (20 ml), and 60% sodium hydride (1.50 g) and a catalytic amount of methanol were added thereto, followed by stirring for 2.5 hours under the condition of heating under reflux. To the reaction liquid was added a saturated aqueous ammonium chloride solution, followed by extraction with ethyl acetate, and the extracted layer was washed with saturated brine and then dried over anhydrous sodium sulfate. After evaporating the solvent under reduced pressure, the residue was purified by silica gel chromatography (hexane:ethyl acetate=9:1) to obtain the desired product (2.00 g) as a yellow oil.
¹H-NMR (CDCl₃, 400 MHz): δ 1.50 (3H, d, J=6.7 Hz), 3.70 (3H, s), 3.86 (3H, s), 4.39 (1H, q, J=6.7 Hz), 7.14 (1H, dd, J=8.6, 2.4 Hz), 7.39 (1H, t, J=8.6 Hz), 7.51 (1H, t, J=2.4 Hz), 7.55 (1H, d, J=8.6 Hz).

### <Examples 113>

### Methyl 3-(3-Methoxyphenyl)-2,2-dimethyl-3-oxopropionate ester

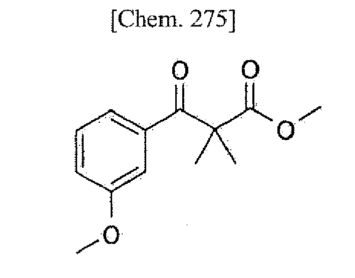

The compound of Example 112 (2.00 g) was dissolved in DMF (45 mL) under an argon atmosphere, and 60% sodium hydride (432 mg) was added thereto at 0°C, followed by stirring at room temperature for 30 minutes, and then iodomethane (0.673 mL) was added thereto at 0°C, followed by stirring at room temperature for 7 hours. To the reaction liquid was added a saturated aqueous ammonium chloride solution, followed by extraction with ethyl acetate, and the extracted layer was washed with saturated brine and then dried over anhydrous sodium sulfate. The residue obtained by evaporating the solvent under reduced pressure was purified by silica gel chromatography (hexane:ethyl acetate=9:1) to obtain the desired product (1.84 g) as a colorless oil.
¹H-NMR (CDCl₃, 400 MHz): δ 1.56 (6H, s), 3.65 (3H, s), 3.84 (3H, s), 7.06-7.09 (1H, m), 7.26-7.42 (3H, m).

### <Example 114>

### 5-(3-Methoxyphenyl)-4,4-dimethyl-2,4-dihydropyrazol-3-one

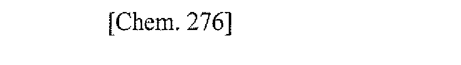

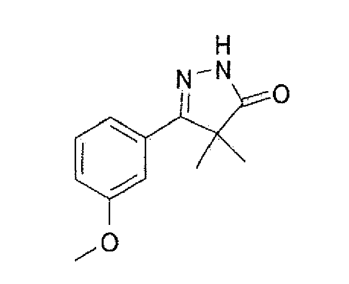

The compound of Example 113 (1.84 g) was dissolved in ethanol (50 mL), and hydrazine monohydrate (1.51 mL) was added thereto, followed by stirring for 6 hours under the condition of heating under reflux. The solvent of the reaction liquid was evaporated under reduced pressure to obtain the desired product (1.84 g) as a colorless oil.
¹H-NMR (CDCl₃, 400 MHz): δ 1.53 (6H, s), 3.87 (3H, s), 6.98-7.02 (1H, m), 7.34-7.38 (3H, m), 8.56 (1H, brs).

### <Examples 115>

### 5-(3-Hydroxyphenyl)-4,4-dimethyl-2,4-dihydropyrazol-3-one

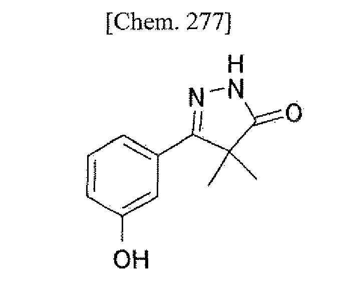

The compound of Example 114 (1.84 g) was dissolved in dichloromethane (80 mL) under an argon atmosphere, and aluminum chloride (22.7 g) was added thereto, followed by stirring for 26 hours under the condition of heating under reflux. To the reaction liquid was added water, followed by extraction with THF, and the extracted layer was washed with saturated brine and then dried over anhydrous sodium sulfate. The obtained residue was washed with diethyl ether to obtain the desired product (825 mg) as a yellow powder. Further, after evaporating the solvent of the diethyl ether washing liquid under reduced pressure, the residue was purified by silica gel chromatography (hexane:ethyl acetate=1:1) to obtain the desired product (290 mg) as a yellow powder.
¹H-NMR (DMSO-d₆, 400 MHz): δ 1.05 (6H, s), 6.52-6.55 (1H, m), 6.92-6.97 (3H, m), 9.31 (1H, s), 11.20 (1H, s).

### <Examples 116>

### 3-Fluoro-4-methoxypropiophenone

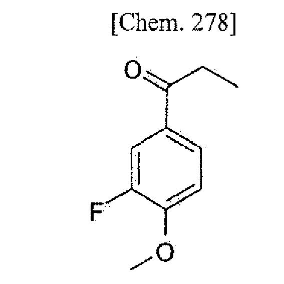

3-Fuoro-4-methoxybenzaldehyde (5.00 g) was dissolved in THF (150 mL), and ethyl magnesium bromide (0.96 mol/L THF solution 40.6 mL) was added thereto at -78°C, followed by stirring at room temperature for 5 hours. To the reaction liquid was added 1.0 mol/L hydrochloric acid, followed by extraction with ethyl acetate, and the extracted layer was washed with saturated brine and then dried over anhydrous sodium sulfate. The solvent was evaporated under reduced pressure to obtain an oil. DMSO (7.6 mL) was dissolved in dichloromethane (40 mL) under an argon atmosphere, and oxalyl chloride (6.2 mL) that had been dissolved in dichloromethane (20 mL) was added dropwise thereto at -78°C, followed by stirring at the same temperature for 15 minutes. To this reaction liquid was added dropwise a solution of the previously obtained oil in dichloromethane (40 mL) at -78°C, followed by stirring at the same temperature for 30 minutes, and then triethylamine (18 mL) was added dropwise thereto at the same temperature, followed by slowly warming to room temperature. To the reaction liquid was added water, followed by extraction with ethyl acetate, and the extracted layer was washed with saturated brine and then dried over anhydrous sodium sulfate. The residue obtained by evaporating the solvent under reduced pressure was purified by silica gel chromatography (hexane:ethyl acetate=15:1) to obtain the desired product (3.88 g) as a white powder.
¹H-NMR (CDCl₃, 400 MHz): δ 1.22 (3H, t, J=7.3 Hz), 2.94 (2H, q, J=7.3 Hz), 3.96 (3H, s), 7.00 (1H, t, J=8.6 Hz), 7.69-7.77 (2H, m).

### <Example 117>

### 4-(3-Fluoro-4-methoxyphenyl)-3-methyl-4-oxobutanoic acid

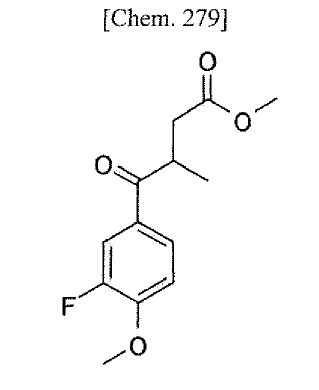

The compound of Example 116 (1.90 g) was dissolved in THF (75 mL) under an argon atmosphere, and a lithium hexamethyl disilazane (1.0 mol/L THF solution, 11.5 mL) was added thereto at -78°C, followed by stirring at room temperature for 30 minutes. To the reaction liquid was added methyl bromoacetate (0.825 mL) at -78°C, followed by stirring at room temperature for 5 hours. To the reaction liquid was added a saturated aqueous ammonium chloride solution, followed by extraction with ethyl acetate, and the extracted layer was washed with saturated brine and then dried over anhydrous sodium sulfate. The residue obtained by evaporating the solvent under reduced pressure was purified by silica gel chromatography (hexane:ethyl acetate=1:1) to obtain the desired product (1.98 g) as a yellow oil.
¹H-NMR (CDCl₃, 400 MHz): δ 1.22 (3H, d, J=7.3 Hz), 2.45 (1H, dd, J=16.5, 5.5 Hz), 2.95 (1H, dd, J=16.5, 8.6 Hz), 3.65 (3H, s), 3.83-3.89 (1H, m), 3.96 (3H, s), 7.02 (1H, t, J=8.2 Hz), 7.73 (1H, dd, J=11.9, 2.1 Hz), 7.79 (1H, m).

### <Example 118>

### 6-(3-Fluoro-4-methoxyphenyl)-5-methyl-4,5-dihydro-2H-pyridazin-3-one

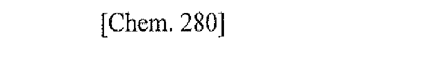

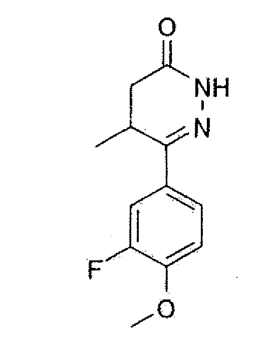

The compound of Example 117 (1.98 g) was dissolved in ethanol (50 mL), and hydrazine monohydrate (2.26 mL) and acetic acid (2.68 mmol) were added thereto, followed by stirring for 7.5 hours under the condition of heating under reflux. To the reaction liquid was added water, followed by extraction with ethyl acetate, and the extracted layer was washed with saturated brine and then dried over anhydrous sodium sulfate. The residue obtained by evaporating the solvent under reduced pressure was purified by silica gel chromatography (hexane:ethyl acetate=1:1) to obtain the desired product (1.70 g) as a yellow powder.
¹H-NMR (CDCl₃, 400 MHz): δ 1.24 (3H, d, J=7.3 Hz), 2.48 (1H, d, J=17.1 Hz), 2.71 (1H, dd, J=17.1, 6.7 Hz), 3.26-3.33 (1H, m), 3.94 (3H, s), 6.98 (1H, t, J=8.6 Hz), 7.44 (1H, dd, J=8.6, 1.2 Hz), 7.56 (1H, dd, J=11.6, 1.2 Hz), 8.52 (1H, brs).

### <Example 119>

### 6-(3-Fluoro-4-hydroxyphenyl)-5-methyl-4,5-dihydro-2H-pyridazin-3-one

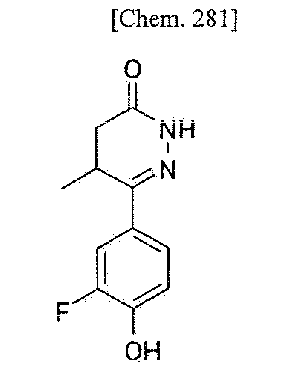

The compound of Example 118 (1.70 g) was dissolved in dichloromethane (50 mL) under an argon atmosphere, and aluminum chloride (19.2 g) was added thereto, followed by stirring at room temperature for 6 hours. To the reaction liquid was added water, followed by extraction with THF, the extracted layer was washed with saturated brine and then dried over anhydrous sodium sulfate, and the solvent was evaporated under reduced pressure. The obtained residue was washed with diethyl ether to obtain the desired product (1.33 g) as a yellow powder.
¹H-NMR (DMSO-d₆, 400 MHz): δ 0.68 (3H, d, J=7.3 Hz), 1.84 (1H, d, J=16.5 Hz), 2.28 (1H, dd, J=16.5, 6.7 Hz), 2.92-3.00 (1H, m), 6.63 (1H, t, J=8.6 Hz), 7.07 (1H, dd, J=8.6, 1.0 Hz), 7.16 (1H, dd, J=13.1, 2.1 Hz), 9.91 (1H, s), 10.51 (1H, s).

### <Example 120>

### 2-Fluoro-4-methoxypropiophenone

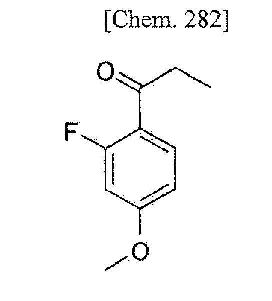

Commercially available 2-fluoro-4-methoxybenzaldehyde (5.00 g) was dissolved in THF (150 mL), and ethyl magnesium bromide (0.96 mol/L THF solution, 40.6 mL) was added thereto at -78°C, followed by stirring at room temperature for 4 hours. To the reaction liquid was added 1.0 mol/L hydrochloric acid, followed by extraction with ethyl acetate, and the extracted layer was washed with saturated brine and then dried over anhydrous sodium sulfate. The residue obtained by evaporating the solvent under reduced pressure was purified by silica gel chromatography (hexane:ethyl acetate=10:1) to obtain an oil. The obtained oil was dissolved in dimethyl sulfoxide (50 mL), and triethylamine (12.0 mL) and a sulfur trioxide-pyridine complex (6.80 g) were added thereto, followed by stirring at room temperature for 1 hour. To the reaction liquid was added water, followed by extraction with ethyl acetate:hexane (1:4), and the extracted layer was washed with saturated brine and then dried over anhydrous sodium sulfate. The residue obtained by evaporating the solvent under reduced pressure was purified by silica gel chromatography (hexane:ethyl acetate=9:1) to obtain the desired product (1.27 g) as a white powder.
¹H-NMR (CDCl₃, 400 MHz): δ 1.19 (3H, t, J=7.3 Hz), 2.92-2.99 (2H, m), 3.86 (3H, s), 6.61 (1H, dd, J=13.4, 2.4 Hz), 6.75 (1H, dd, J=8.6, 2.4 Hz), 7.90 (1H, t, J=8.6 Hz).

### <Examples 121>

### 6-(2-Fluoro-4-methoxyphenyl)-5-methyl-4,5-dihydro-2H-pyridazin-3-one

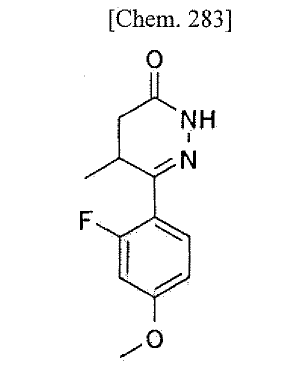

The compound of Example 120 (670 mg) was dissolved in THF (35 mL) under an argon atmosphere, and a lithium hexamethyl disilazane (1.0 mol/L, THF solution, 4.0 mL) was added thereto at 0°C, followed by stirring at room temperature for 30 minutes, and then t-butyl bromoacetate (0.591 mL) was added thereto at 0°C, followed by stirring at room temperature for 3 hours. To the reaction liquid was added a saturated aqueous ammonium chloride solution, followed by extraction with ethyl acetate, and the extracted layer was washed with saturated brine and then dried over anhydrous sodium sulfate. The residue obtained by evaporating the solvent under reduced pressure was dissolved in dichloromethane, (10 mL), and trifluoroacetic acid (5 mL) was added thereto, followed by stirring at room temperature for 16 hours. The solvent of the reaction liquid was evaporated under reduced pressure, the residue was dissolved in ethanol (35 mL), and acetic acid (1.85 mL) and hydrazine monohydrate (0.714 mL) were added thereto, followed by stirring for 6 hours under the condition of heating under reflux. To the reaction liquid was added water, followed by extraction with ethyl acetate, and the extracted layer was washed with saturated brine and then dried over anhydrous sodium sulfate. The residue obtained by evaporating the solvent under reduced pressure was purified by NH type silica gel chromatography (hexane:ethyl acetate=1:1) to obtain the desired product (585 mg) as a yellow powder.
¹H-NMR (CDCl₃, 400 MHz): δ 1.20 (3H, d, J=7.3 Hz), 2.43 (1H, dd, J=17.1, 3.0 Hz), 2.73 (1H, dd, J=17.1, 6.4 Hz), 3.27-3.31 (1H, m), 3.84 (3H, s), 6.64 (1H, dd, J=13.4, 2.4 Hz), 6.74 (1H, dd, J=8.6, 2.4 Hz), 7.55 (1H, t, J=8.6 Hz), 8.48 (1H, brs).

### <Example 122>

### 6-(2-Fluoro-4-hydroxyphenyl)-5-methyl-4,5-dihydro-2H-pyridazin-3-one

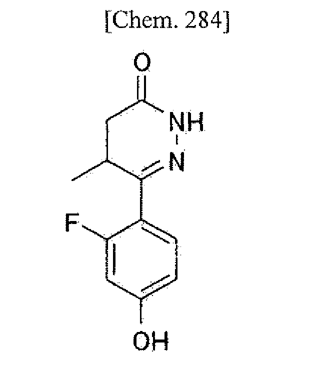

The compound of Example 121 (584 mg) was dissolved in dichloromethane (25 mL) under an argon atmosphere, and aluminum chloride (7.20 g) was added thereto, followed by stirring at room temperature for 15 hours. To the reaction liquid was added water, followed by extraction with THF and the extracted layer was washed with saturated brine and then dried over anhydrous sodium sulfate. The residue obtained by evaporating the solvent under reduced pressure was washed with diethyl ether to obtain the desired product (430 mg) as a yellow powder.
EIMS (+): 222 [M]⁺.
¹H-NMR (DMSO-d₆, 400 MHz): δ 1.02 (3H, d, J=6.7 Hz), 2.20 (1H, dd, J=16.5, 3.0 Hz), 2.63 (1H dd, J=17.1, 6.7 Hz), 3.08-3.13 (1H, m), 6.59 (1H, dd, J=13.4, 2.4 Hz), 6.65 (1H, dd, J=8.6, 2.4 Hz), 7.44 (1H, t, J=9.2 Hz), 10.23 (1H, s), 10.89 (1H, s).

### <Example 123>

### 4-(t-Butyldimethylsilyloxy)-3-methoxybenzaldehyde

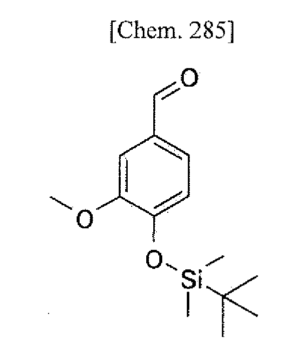

Vaniline (5.00 g) was dissolved in DMF (150 mL) under an argon atmosphere, and imidazole (3.36 g) and chloro-t-butyldimethylsilane (5.45 g) were added thereto at 0°C, followed by stirring at room temperature for 2.5 hours. The solvent of the reaction liquid was evaporated under reduced pressure and then to the residue was added water, followed by extraction with ethyl acetate. The extracted layer was washed with saturated brine and then dried over anhydrous sodium sulfate. After evaporating the solvent under reduced pressure, the residue was purified by silica gel chromatography (hexane:ethyl acetate=18:1) to obtain the desired product (8.30 g) as a colorless oil.
¹H-NMR (CDCl₃, 400 MHz): δ 0.21 (6H, s), 1.02 (9H, s), 3.89 (3H, s), 6.98 (1H, d, J=7.9 Hz), 7.38 (1H, dd, J=7.9, 1.8 Hz), 7.41 (1H, d, J=1.8 Hz), 9.86 (1H, s).

### <Example 124>

### 4-(t-Butyldimethylsilyloxy)-3-methoxypropiophenone

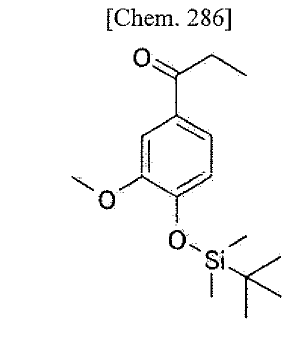

The compound of Example 123 (8.30 g) was dissolved in THF (200 mL) under an argon atmosphere, and ethyl magnesium bromide (0.97 mol/L, THF solution, 35.3 mL) was added thereto at -78°C, followed by stirring at room temperature for 3 hours. To the reaction liquid was added a saturated aqueous ammonium chloride solution, followed by extraction with ethyl acetate, and the extracted layer was washed with saturated brine and then dried over anhydrous sodium sulfate. After evaporating the solvent under reduced pressure, the residue was dissolved in DMSO (150 mL), and triethylamine (43.6 mL) and a sulfur trioxide-pyridine complex (24.8 g) were added thereto, followed by stirring at room temperature for 1 hour. To the reaction liquid was added 1 mol/L hydrochloric acid until the solution turned acidic, followed by extraction three times with hexane-ethyl acetate (1:4), and the combined organic layer was washed with saturated brine and then dried over anhydrous sodium sulfate. After evaporating the solvent under reduced pressure, the residue was purified by silica gel chromatography (hexane:ethyl acetate=9:1→2:1) to obtain the desired product (4.32 g) as a yellow oil.
¹H-NMR (CDCl₃, 400 MHz): δ 0.19 (6H, s), 1.01 (9H, s), 1.22 (3H, t, J=7.3 Hz), 2.97 (2H, q, J=7.3 Hz), 3.87 (3H, s), 6.88 (1H, d, J=7.9 Hz), 7.49 (1H, dd, J=7.9, 1.8 Hz), 7.54 (1H, d, J=1.8Hz).

### <Example 125>

### Methyl 4-[4-(t-Butyldimethylsilyloxy)-3-methoxyphenyl]-3-methyl-4-oxobutanoate ester

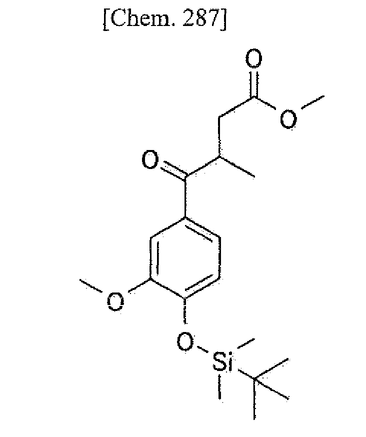

The compound of Example 124 (4.32 g) was dissolved in THF (100 mL) under an argon atmosphere, and a lithium hexamethyl disilazane (1.0 mol/L, THF solution, 16.9 mL) was added thereto at -78°C, followed by stirring at 0°C for 30 minutes, and then methyl bromoacetate (1.81 mL) was added thereto at -78°C, followed by stirring at room temperature for 2 hours. To the reaction liquid was added a saturated aqueous ammonium chloride solution, followed by extraction with ethyl acetate, and the extracted layer was washed with saturated brine and then dried over anhydrous sodium sulfate. After evaporating the solvent of the filtrate under reduced pressure, the residue was purified by silica gel chromatography (hexane:ethyl acetate=8:1→2:1) to obtain the desired product (3.27 g) as a yellow oil. ¹H-NMR (CDCl₃, 400 MHz): δ 0.18 (3H, s), 0.19 (3H, s), 1.00 (9H, s), 1.23 (3H, d, J=7.3 Hz), 2.45 (1H, dd, J=16.5, 5.8 Hz), 2.94 (1H, dd, J=16.5, 7.9 Hz), 3.65 (3H, s), 3.86 (3H, s), 3.90-3.92 (1H, m), 6.89 (1H, d, J=8.6 Hz), 7.52-7.55 (2H, m).

### <Examples 126>

### 6-(4-Hydroxy-3-methoxyphenyl)-5-methyl-4,5-dihydro-2H-pyridazm-3-one

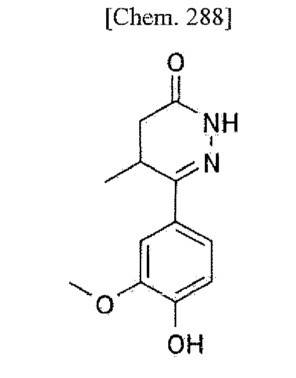

The compound of Example 125 (1.50 g) was dissolved in ethanol (40 mL), and acetic acid (0.718 mL) and hydrazine monohydrate (0.609 mL) were added thereto, followed by stirring for 2.5 hours under the condition of heating to reflux. To the reaction liquid was added water, followed by extraction with ethyl acetate, and the extracted layer was washed with saturated brine and then dried over anhydrous sodium sulfate. After evaporating the solvent under reduced pressure, the residue was dissolved in tetrahydrofuran (40 mL), and tetrabutyl ammonium fluoride (1.0 mol/L THF solution, 5.43 mL) was added thereto, followed by stirring at room temperature for 2 hours. To the reaction liquid was added 1 mol/L hydrochloric acid until the solution turned acidic, followed by extraction with ethyl acetate, and the extracted layer was washed with saturated brine and then dried over sodium sulfate. After evaporating the solvent under reduced pressure, the residue was purified by silica gel chromatography (hexane:ethyl acetate=1.3) to obtain the desired product (691 mg) as a yellow oil.
¹H-NMR (CDCl₃, 400 MHz): δ 1.23 (3H, d, J=7.3 Hz), 2.38 (1H, d, J=16.5 Hz), 2.82 (1H, dd, J=16.5, 6.7 Hz), 3.53-3.54 (1H, m), 3.97 (3H, s), 6.99 (1H, d, J=8.6 Hz), 7.37 (1H, dd, J=8.6, 1.8 Hz), 7.54 (1H, d, J=1.8 Hz), 9.60 (1H, s), 10.97 (1H, s).

### <Example 127>

### 6-(3-Fluoro-4-methoxyphenyl)-4,5-dihydro-2H-pyridazin-3-one

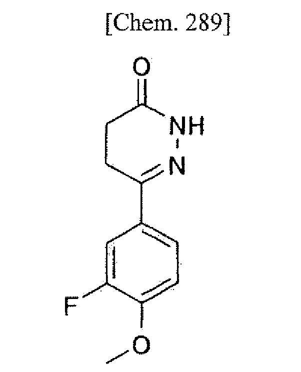

3-Fluoro-4-methoxyacetophenone (5.00 g) was dissolved in THF (150 mL) under an argon atmosphere, and lithium hexamethyl disilazane (1.0 mol/L THF solution, 31.2 mL) was added thereto at 0°C, followed by stirring at room temperature for 30 minutes, and then t-butyl bromoacetate (4.61 mL) was added thereto at 0°C, followed by stirring at room temperature for 3 hours. To the reaction liquid was added a saturated aqueous ammonium chloride solution, followed by extraction with ethyl acetate, and the extracted layer was washed with saturated brine and then dried over anhydrous sodium sulfate. After evaporating the solvent under reduced pressure, the residue was dissolved in dichloromethane (30 mL), and trifluoroacetic acid (10 mL) was added thereto, followed by stirring at room temperature for 7 hours. After evaporating the solvent under reduced pressure, the residue was dissolved in ethanol (150 mL), and hydrazine monohydrate (4.33 mL) was added thereto, followed by stirring for 3 hours under the condition of heating under reflux. The reaction liquid was concentrated under reduced pressure, and the obtained residue was then purified by silica gel chromatography (hexane:ethyl acetate= 1:1→ethyl acetate) to obtain the desired product (3.88 g, 57%) as a yellow powder.
¹H-NMR (CDCl₃, 400 MHz): δ 2.61 (2H, t, J=7.9 Hz), 2.95 (2H, t, J=7.9 Hz), 3.93 (3H, s), 6.97 (1H, dd, J=8.6, 8.6 Hz), 7.41 (1H, dd, J=8.6, 2.4 Hz), 7.53 (1H, dd, J,12.8, 2.4 Hz), 8.57 (1H, brs).

### <Example 128>

### 6-(3-Fluoro-4-hydroxyphenyl)-4,5-dihydro-2H-pyridazin-3-one

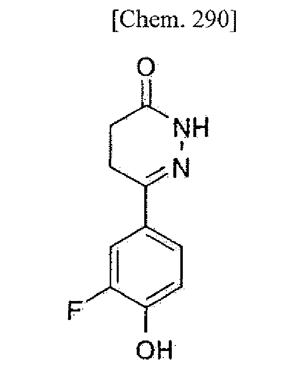

The compound of Example 127 (3.88 g) was dissolved in dichloromethane (100 mL) under an argon atmosphere, and aluminum chloride (45.3 g) was added thereto, followed by stirring at room temperature for 15 hours. To the reaction liquid was added ice water, followed by extraction with THF, and the extracted layer was washed with saturated brine and then dried over anhydrous sodium sulfate. After evaporating the solvent under reduced pressure, the residue was washed with diethyl ether to obtain the desired product (3.42 g) as a yellow powder.
¹H-NMR (DMSO-d₆, 400 MHz): δ 2.39 (2H, t, J=8.3 Hz), 2.87 (2H, t, J=8.3 Hz), 6.96 (1H, dd, J=8.6, 8.6 Hz), 7.39 (1H, dd, Z=8.6, 2.4 Hz), 7.49 (1H, dd, J=12.8, 2.4 Hz), 10.24 (1H, brs), 10.81 (1H, brs).

### <Example 129>

### t-Butyl 2,4,5-trifluorobenzoate ester

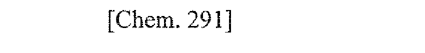

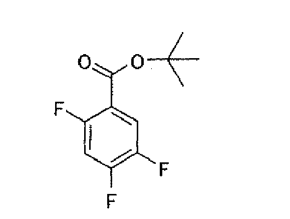

2,4,5-Trifluorobenzoic acid (5.00 g, 28.4 mmol) was dissolved in t-butanol (140 mL), and di-t-butyldicarbonate (12.4 g) and 4-dimethylaminopyridine (347 mg) were added thereto, followed by stirring at room temperature for 19 hours. To the reaction liquid was added ethyl acetate, and the organic layer was washed twice with 1 mol/L hydrochloric acid and twice with a saturated aqueous sodium hydrogen carbonate solution, and then dried over anhydrous sodium sulfate. After evaporating the solvent under reduced pressure, the residue was purified by silica gel chromatography (hexane:ethyl acetate=99:1) to obtain the desired product (6.04 g) as a colorless oil.
¹H-NMR (CDCl₃, 400 MHz): δ 1.59 (9H, s), 6.96 (1H, ddd, J=9.9, 6.3, 3.2 Hz), 7.71 (1H, ddd, J=12.8, 6.4, 4.0 Hz).

### <Example 130>

### t-Butyl 2,5-difluoro-4-methoxybenzoate ester

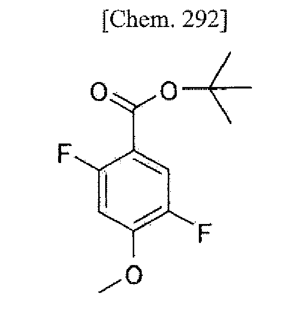

Methanol (0.996 mL) was dissolved in a 50% aqueous sodium hydroxide solution (70 mL), and a solution of tetrabutyl ammonium sulfate (2.78 g) and the compound of Example 129 (5.72 g) in toluene (170 mL) was added thereto, followed by stirring at room temperature for 1 hour. The reaction liquid was extracted with ethyl acetate, and the extracted layer was washed with saturated brine and then dried over anhydrous sodium sulfate. After evaporating the solvent under reduced pressure, the residue was purified by silica gel chromatography (hexane:ethyl acetate=20:1) to obtain the desired product (3.43 g) as a white powder.
¹H-NMR (CDCl₃, 400 MHz): δ 1.58 (9H, s), 3.92 (3H, s), 6.68 (1H, dd, J=11.6, 6.7 Hz), 7.59 (1H, dd, J=11.6, 6.7 Hz).

### <Examples 131>

### 2,5-Difluoro-4,N-dimethoxy-N-methylbenzamide

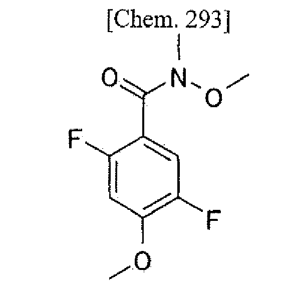

The compound of Example 130 (3.43 g) was dissolved in dichloromethane (50 mL), and trifluoroacetic acid (20 mL) was added thereto, followed by stirring at room temperature for 3 hours. The reaction liquid was concentrated under reduced pressure, the residue was then dissolved in dichloromethane (70 mL), and N,O-dimethylhydroxylamine hydrochloride (1.76 g), triethylamine (3.14 mL), and N-(3-dimethylaminopropyl)-N'-ethylearbodiimide hydrochloride (16.6 g) were added thereto at 0°C, followed by stirring at room temperature for 8 hours. To the reaction liquid was added water, followed by extraction with ethyl acetate, and the extracted layer was washed with saturated brine and then dried over anhydrous sodium sulfate. After evaporating the solvent under reduced pressure, the residue was purified by silica gel chromatography (ethyl acetate) to obtain the desired product (3.15 g) as a yellow oil.
¹H-NMR (CDCl₃, 400 MHz, 3.34 (3H, s), 3.59 (3H, s), 3.91 (3H, s), 6.71 (1H, dd, J=10.4, 7.5 Hz), 7.20 (1H, dd, J=10.4, 6.1 Hz).

### <Example 132>

### 2,5-Difluoro-4-methoxyacetophenone

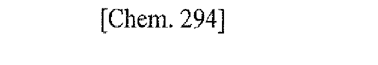

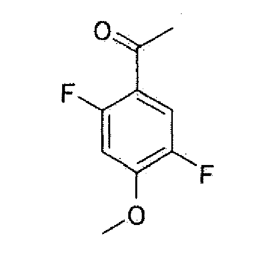

The compound of Example 131 (1.50 g) was dissolved in THF (30 mL) under an argon atmosphere, and methyl magnesium bromide (0.84 mol/L THF solution, 17.0 mL) was added thereto at 0°C, followed by stirring at room temperature for 1.5 hours. To the reaction liquid was added a saturated aqueous ammonium chloride solution, followed by extraction with ethyl acetate, and the extracted layer was washed with saturated brine and then dried over anhydrous sodium sulfate. After evaporating the solvent under reduced pressure, the residue was purified by silica gel chromatography (hexane:ethyl acetate=4:1) to obtain the desired product (1.12 g) as a white powder.
¹H-NMR (CDCl₃, 400 MHz): δ 2.60 (3H, d, J=5.5 Hz), 3.94 (3H, s), 6.70 (1H, dd, J=11.6, 6.7 Hz), 7.64 (1H, dd, J=11.6, 6.7 Hz).

### <Example 133>

### 6-(2,5-Difluoro-4-methoxyphenyl)-4,5-dihydro-2H-pyridazin-3-one

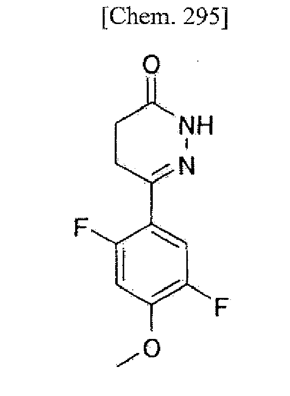

The compound of Example 132 (1.12 g) was dissolved in THF (30 mL) under an argon atmosphere, and lithium hexamethyl disilazane (1.0 mol/L THF solution, 6.32 mL) was added thereto at 0°C, followed by stirring at room temperature for 30 minutes, and then t-butyl bromoacetate (1.16 mL) was added thereto at 0°C, followed by stirring at room temperature for 5 hours. To the reaction liquid was added a saturated aqueous ammonium chloride solution, followed by extraction with ethyl acetate, and the extracted layer was washed with saturated brine and then dried over anhydrous sodium sulfate. After evaporating the solvent under reduced pressure, the residue was dissolved in dichloromethane (30 mL), and trifluoroacetic acid (10 mL) was added thereto, followed by stirring at room temperature for 2.5 hours. To the reaction liquid was added potassium carbonate, and a saturated aqueous sodium hydrogen carbonate solution was added thereto, followed by washing with ethyl acetate. Then, the aqueous layer was acidified with 1.0 mol/L hydrochloric acid and extracted three times with ethyl acetate. The combined extracted layer was washed with saturated brine and then dried over anhydrous sodium sulfate, and the solvent was evaporated under reduced pressure. The residue was dissolved in ethanol (20 mL), and hydrazine monohydrate (0.564 mL) and acetic acid (1.46 mL) were added thereto, followed by stirring for 10 hours under the condition of heating under reflux. To the reaction liquid was added water, followed by extraction with ethyl acetate, and the extracted layer was washed with saturated brine and then dried over anhydrous sodium sulfate. After evaporating the solvent under reduced pressure, the residue was purified by silica gel chromatography (hexane:ethyl acetate=4:1) to obtain the desired product (478 mg) as a white powder.
¹H-NMR (CDCl₃, 400 MHz): δ 2.57-2.59 (2H, m), 2.98-3.03 (2H, m), 3.91 (3H, s), 6.70 (1H, dd, J=12.2, 7.3 Hz), 7.43 (1H, dd, J=12.2, 7.3 Hz), 8.50 (1H, s).

### <Example 134>

### 6-(2,5-Difluoro-4-hydroxyphenyl)-4,5-dihydro-2H-pyridazin-3-one

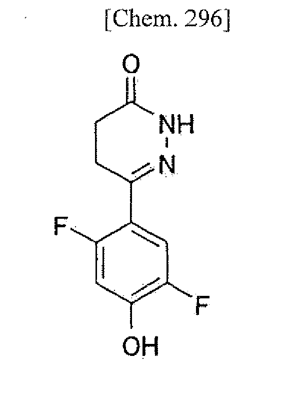

The compound of Example 133 (478 mg) was dissolved in dichloromethane (20 mL) under an argon atmosphere, and aluminum chloride (5.31 g) was added thereto, followed by stirring at room temperature for 17 hours. To the reaction liquid was added ice water, followed by extraction with THF, and the extracted layer was washed with saturated brine and then dried over anhydrous sodium sulfate. After evaporating the solvent under reduced pressure, the residue was washed with diethyl ether to obtain the desired product (391 mg) as a white powder.
¹H-NMR (DMSO-d₆, 400 MHz): δ 2.39 (2H, t, J=8.3 Hz), 2.83-2.85 (2H, m), 6.79 (1H, dd, J=12.2, 7.3 Hz), 7.39 (1H, dd, J=2.2, 7.3 Hz), 10.76 (1H, s), 10.94 (1H, s).

### <Example 135>

### (2,3 -Difluorophenoxy)triisopropylsilane

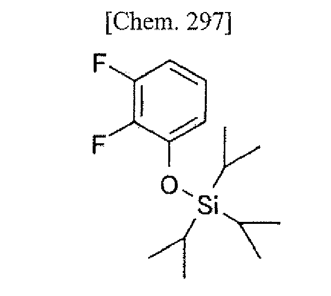

2,3-Difluorophenol (10.0 g) was dissolved in DMF (40 mL), and triisopropylsilyl chloride (16.5 mL) and imidazole (5.24 g) were added thereto, followed by stirring at room temperature for 18 hours. To the reaction liquid was added water, followed by extraction with ethyl acetate, and the extracted layer was washed with saturated brine and then dried over anhydrous sodium sulfate. After evaporating the solvent under reduced pressure, the residue was purified by silica gel chromatography (hexane:ethyl acetate=99:1) to obtain the desired product (21.0 g) as a colorless oil.
¹H-NMR (CDCl₃, 400 MHz): δ 1.12 (18H, d, J=3.7 Hz), 1.27-1.32 (3H, m), 6.72-6.75 (2H, m), 6.88-6.90 (1H, m).

### <Examples 136>

### 2,3-Difluoro-4-triisoprapylsalyloxy benzoic acid

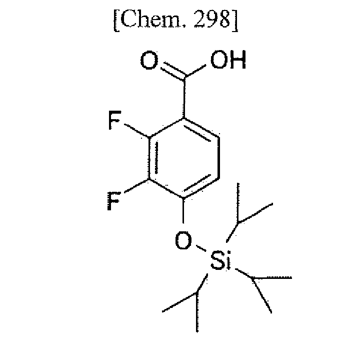

The compound of Example 135 (4.00 g) was dissolved in THF (20 mL) under an argon atmosphere, 2,2,6,6-tetramethylpiperidine (2.60 mL) was added thereto, and then n-butyl lithium (2.71 mol/L hexane solution, 5.41 mL) was added thereto at -78°C, followed by stirring at the same temperature for 2 hours. Carbon dioxide was injected into the reaction liquid at -78°C, followed by stirring for 30 minutes, and the liquid was acidified with the addition of 1 mol/L hydrochloric acid at room temperature, and extracted three times with ethyl acetate. The combined organic layer was washed with saturated brine and then dried over anhydrous sodium sulfate. After evaporating the solvent under reduced pressure, the residue was purified by silica gel chromatography (hexane:ethyl acetate=1:1) to obtain the desired product (3.94 g) as a white powder.
¹H-NMR (CDCl₃, 400 MHz): δ 1.11 (18H, d, J=7.3 Hz), 1.26-1.35 (3H, m), 6.77 (1H, ddd, J=8.6, 7.3, 1.8 Hz), 7.68-7.69 (1H, m).

### <Example 137>

### 2,3-Difluoro-4-triisopropylsilyloxyacetophenone

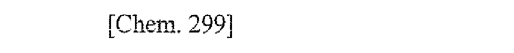

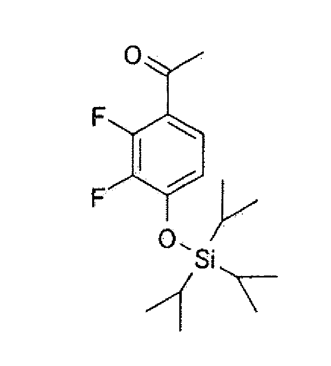

The compound of Example 136 (3.11 g) was dissolved in dichloromethane (50 mL), and N,O-dimethylhydroxyl amine hydrochloride (1.10 g), triethylamine (1.97 mL), and N-(3-dimethylaminopropyl)-N'-ethylcarbodiimide hydrochloride (2.71 g) were added thereto at 0°C, followed by stirring at room temperature for 4 hours. To the reaction liquid was added water, followed by extraction three times with ethyl acetate, and the combined organic layer was washed with saturated brine and then dried over anhydrous sodium sulfate. The solvent was evaporated under reduced pressure, the obtained residue was dissolved in tetrahydrofuran (50 mL), and methyl magnesium bromide (0.84 mol/L THF solution, 25.7 mL) was added thereto at 0°C under an argon atmosphere, followed by stirring at room temperature for 1.5 hours. To the reaction liquid was added a saturated aqueous ammonium chloride solution, followed by extraction three times with ethyl acetate, and the combined organic layer was washed with saturated brine and then dried over anhydrous sodium sulfate. After evaporating the solvent under reduced pressure, the residue was purified by silica gel chromatography (hexane:ethyl acetate=50:1) to obtain the desired product (1.80 g) as a colorless oil.
¹H-NMR (CDCl₃, 400 MHz): δ 1.11 (18H, d, J=7.3 Hz), 1.25-1.34 (3H, m), 2.61 (3H, d, J=4.9 Hz), 6.76 (1H ddd, J=9.2, 7.3, 1.8 Hz), 7.56-7.57 (1H, m).

### <Example 138>

### 6-(2,3-Difluoro-4-hydroxyphenyl)-4,5-dihydro-2H-pyridazin-3-one

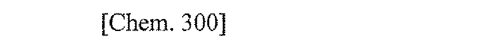

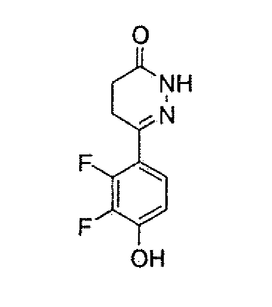

The compound of Example 137 (1.80 g) was dissolved in THF (30 mL) under an argon atmosphere, and lithium hexamethyl disilazane (1.0 mol/L THF solution, 6.30 mL) was added thereto at 0°C, followed by stirring at room temperature for 30 minutes, and then t-butyl bromoacetate (1.05 mL) was added thereto at 0°C, followed by stirring at room temperature for 3 hours. To the reaction liquid was added a saturated aqueous ammonium chloride solution, followed by extraction with ethyl acetate, and the extracted layer was washed with saturated brine and then dried over anhydrous sodium sulfate. After evaporating the solvent under reduced pressure, the residue was dissolved in dichloromethane (30 mL), and trifluoroacetic acid (10 mL) was added thereto, followed by stirring at room temperature for 16 hours. To the reaction liquid was added potassium carbonate, and a saturated aqueous sodium hydrogen carbonate solution was added thereto, followed by washing with ethyl acetate. The aqueous layer was acidified with 1.0 mol/L hydrochloric acid, followed by extraction with ethyl acetate, and the extracted layer was washed with saturated brine and dried over anhydrous sodium sulfate. After evaporating the solvent under reduced pressure, the residue was dissolved in ethanol (40 mL), and hydrazine monohydrate (0.799 mL) and acetic acid (2.07 mL) were added thereto, followed by stirring for 5.5 hours under the condition of heating under reflux. To the reaction liquid was added water, followed by extraction with ethyl acetate, and the extracted layer was washed with saturated brine, and then dried over anhydrous sodium sulfate and filtered. After evaporating the solvent of the filtrate under reduced pressure, the residue was purified by silica gel chromatography (hexane:ethyl acetate=4:1) to obtain the desired product (432 mg) as a white powder.
¹H-NMR (CDCl₃, 400 MHz): δ 2.40 (2H, t, J=8.3 Hz), 2.85 (2H, td, J=8.3, 1.6 Hz), 6.80 (1H, ddd, J=8.6, 8.6, 1.8 Hz), 7.24 (1H, ddd, J=8.6, 8.6, 2.4 Hz), 10.73 (1H, s), 10.94 (1H, s).

### <Example 139>

### Methyl 3-(3-fluoro-4-methoxyphenyl)-2-methyl-3-oxopropionate ester

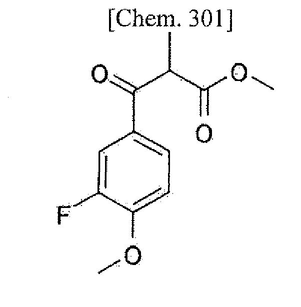

The compound of Example 116 (1.95 g) was dissolved in dimethyl carbonate (50 ml), and 60% sodium hydride (1.28 g) and a catalytic amount of methanol were added thereto, followed by stirring for 4 hours under the condition of heating under reflux. To the reaction liquid was added a saturated aqueous ammonium chloride solution, followed by extraction with ethyl acetate, and the extracted layer was washed with saturated brine and then dried over anhydrous sodium sulfate. After evaporating the solvent under reduced pressure, the residue was purified by silica gel chromatography (hexane:ethyl acetate=9:1→4:1) to obtain the desired product (2.20 g) as a yellow oil.
¹H-NMR (CDCl₃, 400 MHz): δ 1.48 (3H, d, J=7.3 Hz), 3.69 (3H, s), 3.96 (3H, s), 4.32 (1H, q, J=7.3 Hz), 7.01 (1H, t, J=8.2 Hz), 7.72 (1H, dd, J=12.2, 2.1 Hz), 7.76-7.79 (1H, m).

### <Example 140>

### Methyl 3-(3-fluoro-4-methoxyphenyl)-2,2-dimethyl-3-oxopropionate ester

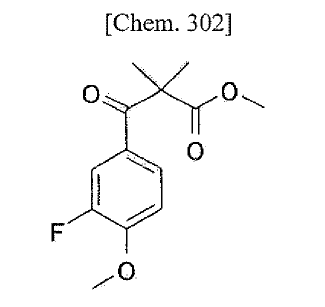

The compound of Example 139 (2.20 g) was dissolved in DMF (50 mL) under an argon atmosphere, and 60% sodium hydride (440 mg) was added thereto at 0°C, followed by stirring at room temperature for 30 minutes, and then iodomethane (0.685 mL) was added thereto at 0°C, followed by stirring at room temperature for 2 hours. To the reaction liquid was added a saturated aqueous ammonium chloride solution, followed by extraction with ethyl acetate, and the extracted layer was washed with saturated brine and then dried over anhydrous sodium sulfate. The residue obtained by evaporating the solvent under reduced pressure was purified by silica gel chromatography (hexane:ethyl acetate=9: 1) to obtain the desired product (2.10 g) as a colorless oil.
¹H-NMR (CDCl₃, 400 MHz): δ 1.53 (6H, s), 3.66 (3H, s), 3.94 (3H, s), 6.95 (1H, t, J=8.6 Hz), 7.58-7.65 (2H, m).

### <Example 141>

### 5-(3-Fluoro-4-methoxyphenyl)-4,4-dimethyl-2,4-dihydropyrazol-3-one

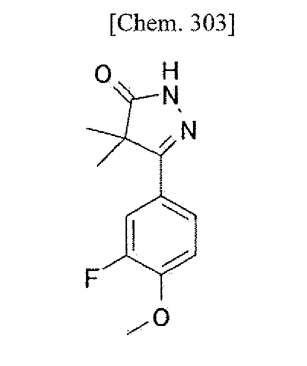

The compound of Example 140 (2.10 g) was dissolved in ethanol (40 mL), and hydrazine monohydrate (1.20 mL) was added thereto, followed by stirring for 8 hours under the condition of heating under reflux. The solvent of the reaction liquid was evaporated under reduced pressure and the residue was purified by silica gel chromatography (hexane:ethyl acetate=1:1) to obtain the desired product (1.59 g) as a white powder. ¹H-NMR (CDCl₃, 400 MHz): δ 1.50 (6H, s), 3.94 (3H, s), 6.99 (1H, t, J=8.6 Hz), 7.49 (1H, d, J=8.6 Hz), 7.57 (1H, dd, J=12.2, 1.8 Hz), 8.49 (1H, brs).

### <Example 142>

### 5-(3-Fluoro-4-hydroxyphenyl)-4,4-dimethyl-2,4-dihydropyrazol-3-one

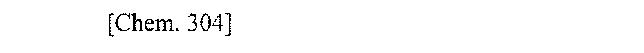

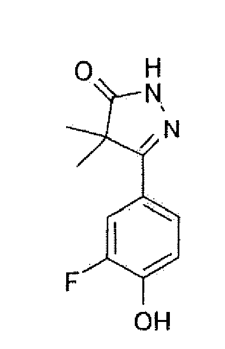

The compound of Example 141 (1.59 g) was dissolved in dichloromethane (50 mL) under an argon atmosphere, and aluminum chloride (17.9 g) was added thereto, followed by stirring at room temperature for 6 hours. To the reaction liquid was added water, followed by extraction with THF, the extracted layer was washed with saturated brine and then dried over anhydrous sodium sulfate. The residue obtained by evaporating the solvent under reduced pressure was washed with diethyl ether to obtain the desired product (1.30 g) as a yellow powder.
¹H-NMR (DMSO-d₆, 400 MHz): δ 1.32 (6H, s), 7.00 (1H, t, J=8.9 Hz), 7.45 (1H, dd, J=7.9, 2.1 Hz), 7.52 (1H, dd, J=12.5, 2.1 Hz), 10.37 (1H, s), 11.41 (1H, s).

### <Examples 143>

### Methyl 3-(2-fluoro-4-methoxyphenyl)-2,2-dimethyl-3-oxopropionate ester

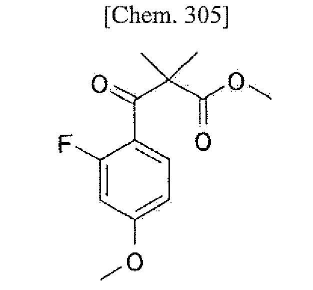

Diisopropyl amine (1.48 mL) was dissolved in THF (20 mL) under an argon atmosphere, and an n-butyl lithium (2.71 mol/L hexane solution, 3.87 mL)) was added thereto at -78°C, followed by stirring at 0°C for 25 minutes, and then methyl isobutyrate (1.12 mL, 9.73 mmol) was added thereto at -78°C, followed by stirring at room temperature for 30 minutes. To the reaction liquid was added a solution of commercially available 2-fluoro-4-methoxybenzaldehyde (1.25 g) in THF (20 mL) at -78°C, followed by stirring at 0°C for 4 hours. To the reaction liquid was added a saturated aqueous ammonium chloride solution, followed by extraction with ethyl acetate, and the extracted layer was washed with saturated brine and then dried over anhydrous sodium sulfate. The residue obtained by evaporating the solvent under reduced pressure was purified by silica gel chromatography (hexane:ethyl acetate=4:1). The obtained compound was dissolved in DMSO (40 mL), and triethylamine (11.2 mL) and a sulfur trioxide-pyridine complex (6.40 g) were added thereto, followed by stirring at room temperature for 1 hour. To the reaction liquid was added water, followed by extraction three times with ethyl acetate:hexane (1:4), and the combined extracted layer was washed with saturated brine and then dried over anhydrous sodium sulfate. The residue obtained by evaporating the solvent under reduced pressure was purified by silica gel chromatography (hexane:ethyl acetate=9:1) to obtain the desired product (1.23 g) as a yellow oil.
¹H-NMR (CDCl₃, 400 MHz): δ 1.49 (6H, s), 3.68 (3H, s), 3.85 (3H, s), 6.57 (1H, dd, J=13.4, 2.4 Hz), 6.76 (1H, dd, J=9.2, 2.4 Hz), 7.86 (1H, t, J=9.2 Hz).

### <Example 144>

### 5-(2-Fluoro-4-hydroxyphenyl)-4,4-dimethyl-2,4-dihydropyrazol-3-one

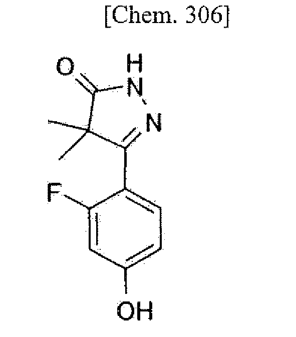

The compound of Example 143 (1.23 g) was dissolved in ethanol (25 mL), and acetic acid (2.43 mL) and hydrazine monohydrate (940 mL) were added thereto, followed by stirring for 8 hours under the condition of heating under reflux. The residue obtained by concentrating the reaction liquid under reduced pressure was purified by NH type silica gel chromatography (hexane:ethyl acetate=4:1). The obtained powder was dissolved in dichloromethane (40 mL), and aluminum chloride (10.9 g) was added thereto, followed by stirring at room temperature for 10 hours. To the reaction liquid was added water, followed by extraction with THF, and the extracted layer was washed with saturated brine and then dried over anhydrous sodium sulfate. The residue obtained by evaporating the solvent under reduced pressure was washed with diethyl ether to obtain the desired product (295 mg) as a white powder.
¹H-NMR (DMSO-d₆, 400 MHz, 1.19 (6H, s), 6.61 (1H, dd, J=13.1, 2.4 Hz), 6.66 (1H, dd, J=9.2, 2.4 Hz), 7.50 (1H, t, J=8.6 Hz), 10.32 (1H, s), 11.42 (1H, s).

### <Example 145>

### 2,5-Difluoro-4-methoxybenzyl alcohol

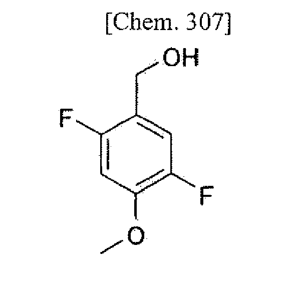

The compound of Example 130 (8.10 g) was dissolved in dichloromethane (100 mL), and trifluoroacetic acid (20 mL) was added thereto, followed by stirring at room temperature for 6 hours. The reaction liquid was concentrated under reduced pressure, and the residue was then dissolved in THF (250 mL) under an argon atmosphere, and a borane-tetrahydrofuran complex (1.0 mol/L THF solution, 42.6 mL) was added thereto at 0°C, followed by stirring at room temperature for 8 hours. To the reaction liquid was added ice water, followed by extraction with ethyl acetate, the extracted layer was washed with saturated brine and then dried over anhydrous sodium sulfate, and the solvent was evaporated under reduced pressure to obtain the desired product (5.74 g, 93%) as a white powder.
¹H-NMR (CDCl₃, 400 MHz): δ 3.88 (3H, s), 4.67 (2H, s), 6.70 (1H, dd, J=11.0, 6.7 Hz), 7.15 (1H, dd, J=11.3, 6.7 Hz).

### <Example 146>

### 2,5-Difluoro-4-methoxybenzaldehyde

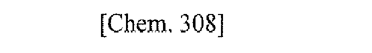

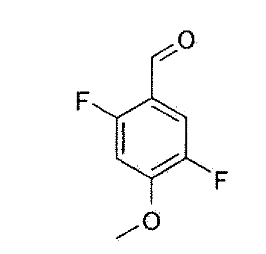

The compound of Example 145 (5.74 g) was dissolved in dichloromethane (300 mL), and active manganese dioxide (28.7 g) was added thereto, followed by stirring at 60°C for 3.5 hours. The insoluble materials were removed by filtration through Celite, and the solvent of the filtrate was evaporated under reduced pressure to obtain the desired product (5.48 g) as a yellow powder.
¹H-NMR (CDCl₃, 400 MHz): δ 3.97 (3H, s), 6.74 (1H, dd, J=11.0, 6.7 Hz), 7.57 (1H, dd, J=11.0, 6.7 Hz), 10.21 (1H, d, T=3.1 Hz).

### <Example 147>

### Methyl 3-(2,5-difluoro-4-methoxyphenyl)-3-hydroxy-2,2-dimethylpropionate ester

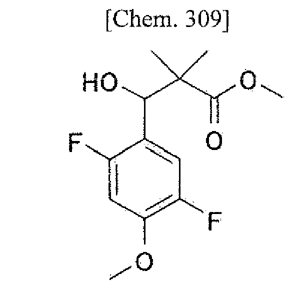

The compound of Example 146 (2.94 g) was dissolved in diethyl ether (150 mL) under an argon atmosphere, and dimethylketene methyltrimethylsilyl acetal (5.22 mL) and a boron trifluoride-diethyl ether complex (3.26 mL) were added thereto at room temperature, followed by stirring at room temperature for 1.5 hours. To the reaction liquid was added a 10% aqueous sodium hydroxide solution, followed by extraction with ethyl acetate, and the extracted layer was washed with saturated brine and then dried over anhydrous sodium sulfate. The residue obtained by evaporating the solvent under reduced pressure was purified by silica gel chromatography (hexane: ethyl acetate=6:1) to obtain the desired product (4.24 g) as a yellow powder.
¹H-NMR (CDCl₃, 400 MHz): δ 1.14 (6H, s), 3.36 (1H, brs), 3.74 (3H, s), 3.87 (3H, s), 5.19 (1H, s), 6.64 (1H, dd, J=11.3, 7.0 Hz), 7.14 (1H, dd, J=12.2, 7.0 Hz).

### <Examples 148>

### 5-(2,5-Difluoro-4-methoxyphenyl)-4,4-dimethyl-2,4-dzhydrapyrazoH3-one

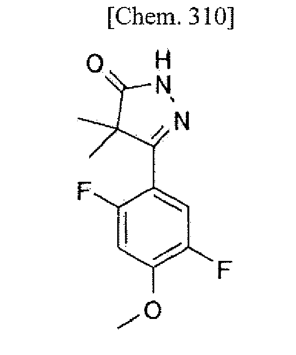

The compound of Example 147 (4.24 g) was dissolved in DMSO (100 mL) under an argon atmosphere, and triethylamine (22.9 mL) and a sulfur trioxide-pyridine complex (13.1 g) was added thereto, followed by stirring at room temperature for 1 hour. To the reaction liquid was added 1 mol/L hydrochloric acid, followed by extraction three times with a mixed solvent of hexane-ethyl acetate (4-1), and the combined organic layer was washed with saturated brine and then dried over anhydrous sodium sulfate. After evaporating the solvent under reduced pressure, the residue was purified by silica gel chromatography (hexane:ethyl acetate=7:1) to obtain a yellow oil. The obtained oil was dissolved in ethanol (100 mL), and acetic acid (10.3 mL) and hydrazine monohydrate (0.399 mL) were added thereto, followed by stirring for 8 hours under the condition of heating under reflux. To the reaction liquid was added water, followed by extraction with ethyl acetate, and the extracted layer was washed with saturated brine and then dried over anhydrous sodium sulfate. After evaporating the solvent under reduced pressure, the residue was purified by silica gel chromatography (hexane:ethyl acetate=3:1→2:1) to obtain the desired product (1.01 g) as a white powder.
¹H-NMR (CDCl₃, 400 MHz): δ 1.42 (6H, s), 3.93 (3H, s), 6.74 (1H, dd, J=12.2, 7.3 Hz), 7.51 (1H, dd, J=11.6, 6.7 Hz), 8.66 (1H, s).

### <Example 149>

### S-(2,5-Difluoro-4-hydroxyphenyl)-4,4-dimethyl-2,4-dihydropyrazol-3-one

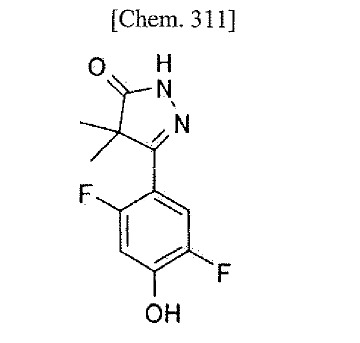

The compound of Example 148 (1.01 g) was dissolved in dichloromethane (40 mL) under an argon atmosphere, and aluminum chloride (11.9 g) was added thereto, followed by stirring at room temperature for 7 hours. To the reaction liquid was added ice water, followed by extraction with THF, and the extracted layer was washed with saturated brine and then dried over anhydrous sodium sulfate. After evaporating the solvent under reduced pressure, the residue was dissolved in dichloromethane (40 mL) again, and aluminum chloride (11.9 g) was added thereto, followed by stirring at room temperature for 22 hours. To the reaction liquid was added ice water, followed by extraction with THF, and the extracted layer was washed with saturated brine and then dried over anhydrous sodium sulfate. After evaporating the solvent under reduced pressure, the residue was washed with diethyl ether to obtain the desired product (430 mg) as a white powder.
¹H-NMR (DMSO-d₆, 400 MHz): δ 1.21 (3H, s), 1.21 (3H, s), 6.84 (1H, dd, J= 7.3, 12.2 Hz), 7.46 (1H, dd, J= 7.3, 12.2 Hz), 10.92 (1H, brs), 11.54 (1H, s).

### <Example 150>

### 2,3-Difluoro-4-triisopropylsilyloxybenzaldehyde

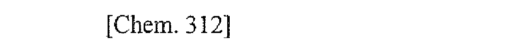

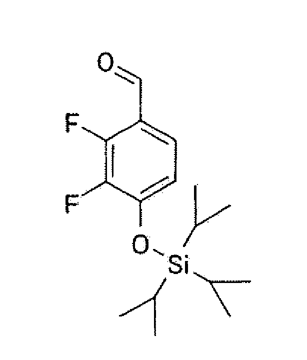

The compound of Example 136 (3.31 g) was dissolved in THF (75 mL) under an argon atmosphere, and a borane-tetrahydrofuran complex (1.4 mol/L THF solution, 12.4 mL) was added thereto at 0°C, followed by stirring at room temperature for 24 hours. To the reaction liquid was added ice water, followed by extraction with ethyl acetate, and the extracted layer was washed with saturated brine and then dried over anhydrous sodium sulfate. After evaporating the solvent under reduced pressure, the residue was dissolved in chloroform (75 mL), and active manganese dioxide (8.70 g) was added thereto, followed by stirring at 60°C for 6 hours. The insoluble materials were removed by filtration through Celite, and the solvent of the filtrate was evaporated under reduced pressure to obtain the desired product (3.49 g) as a yellow oil.
¹H-NMR (CDCl₃, 400 MHz): δ 1.11 (18H, d, J=7.3 Hz), 1.29-1.32 (3H, m), 6.80 (1H, ddd, J=8.6, 7.3, 1.8 Hz), 7.53 (1H, ddd, J=8.6, 7.3, 2.4 Hz), 10.19 (1H, s).

### <Example 151>

### 2,3-Difluoro-4-methoxybenzaldehyde

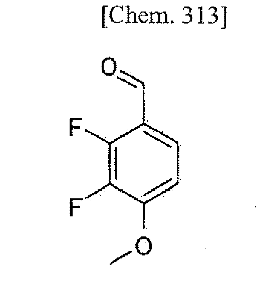

The compound of Example 150 (3.09 g) was dissolved in THF (50 mL) under an argon atmosphere, and tetrabutyl ammonium fluoride (1.0 mol/L THF solution, 12.8 mL) was added thereto, followed by stirring at room temperature for 20 minutes. To the reaction liquid was added 1.0 mol/L hydrochloric acid, followed by extraction with ethyl acetate, and the extracted layer was washed with saturated brine and then dried over anhydrous sodium sulfate. After evaporating the solvent under reduced pressure, the residue was dissolved in acetone (50 mL) and DMF (20 mL), and potassium carbonate (2.72 g) and iodomethane (0.918 mL) were added thereto, followed by stirring at room temperature for 5.5 hours. The insoluble materials were removed by filtration through Celite, the filtrate was concentrated under reduced pressure, and the obtained residue was purified by silica gel chromatography (hexane:ethyl acetate=3: 1) to obtain the desired product (1.30 g) as a white powder. ¹H-NMR (CDCl₃, 400 MHz): δ 4.00 (3H, s), 6.86 (1H, ddd, J=9.2, 6.7, 1.8 Hz), 7.64 (1H, ddd, J=9.2, 7.3, 1.8 Hz), 10.20 (1H, s).

### <Example 152>

### Methyl 3-(2,3-difluoro-4-methoxyphenyl)-3-hydroxy-2,2-dimethylpropionate ester

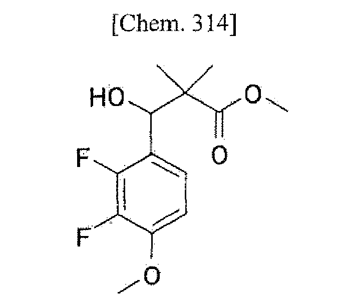

The compound of Example 151 (1.30 g) was dissolved in diethyl ether (70 mL) under an argon atmosphere, and dimethylketene methyltrimethylsilyl acetal (2.30 mL) and a boron trifluoride-diethyl ether complex (1.44 mL) were added thereto at room temperature, followed by stirring at room temperature for 1 hour. To the reaction liquid was added a 10% aqueous sodium hydroxide solution, followed by extraction with ethyl acetate, and the extracted layer was washed with saturated brine and then dried over anhydrous sodium sulfate. The residue obtained by evaporating the solvent under reduced pressure was purified by silica gel chromatography (hexane:ethyl acetate=3:1) to obtain the desired product (1.98 g) as a white powder.
¹H-NMR (CDCl₃, 400 MHz): δ 1.15 (3H, s), 1.16 (3H, d, J=2.4 Hz), 3.37 (1H, d, J=4.9 Hz), 3.75 (3H, s), 3.91 (3H, s), 5.20 (1H, d, J=4.9 Hz), 6.79 (1H, ddd, J=8,6, 7.9, 2.4 Hz), 7.13 (1H, ddd, J=8.6, 7.3, 2.4 Hz).

### <Example 153>

### Methyl 3-(2,3-diffuoro-4-methoxyphenyl)-2,2-dimethyl-3-oxopropionate ester

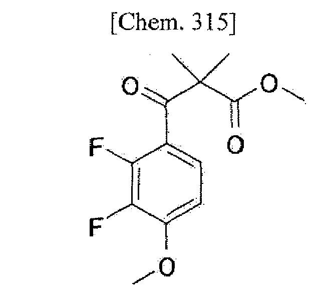

The compound of Example 152 (1.98 g) was dissolved in DMSO (50 mL) under an argon atmosphere, and triethylamine (10. mL) and a sulfur trioxide-pyridine complex (5.75 g) were added thereto, followed by stirring at room temperature for 1 hour. To the reaction liquid was added 1 mol/L hydrochloric acid, followed by extraction three times with hexane: ethyl acetate (4:1), and the combined organic layer was washed with saturated brine and then dried over anhydrous sodium sulfate. After evaporating the solvent under reduced pressure, the residue was purified by silica gel chromatography (hexane:ethyl acetate=6:1) to obtain the desired product (1.24 g) as a yellow oil.
¹H-NMR (CDCl₃, 400 MHz): δ 1.50 (6H, s), 3.70 (3H, s), 3.96 (3H, s), 6.82 (1H, ddd, J=9.2, 7.3, 1.8 Hz), 7.63 (1H, ddd, J=9.2, 7.3, 2.4 Hz).

### <Example 154>

### 5-(2,3-Difluoro-4-methoxyphenyl)-4,4-dimethyl-2,4-dihydropyrazol-3-one

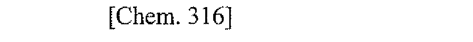

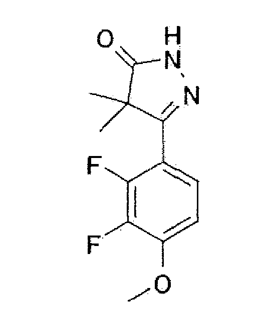

The compound of Example 153 (1.12 g) was dissolved in ethanol (20 mL), and acetic acid (1.55 mL) and hydrazine monohydrate (0.60 mL) were added thereto, followed by stirring for 10 hours under the condition of heating under reflux. To the reaction liquid was added water, followed by extraction with ethyl acetate, and the extracted layer was washed with saturated brine and then dried over anhydrous sodium sulfate. After evaporating the solvent under reduced pressure, the residue was purified by silica gel chromatography (hexane:ethyl acetate=2: 1) to obtain the desired product (425 mg) as a white powder. ¹H-NMR (CDCl₃, 400 MHz): δ 1.42 (3H, s), 1.43 (3H, s), 3.97 (3H, s), 6.82 (1H, ddd, J=9.2, 7.3, 1.8 Hz), 7.40 (1H, ddd, J=9.2, 7.3, 2.4 Hz), 8.64 (1H, s).

### <Example 155>

### 5-(2,3-Difluoro-4-hydroxyphenyl)-4,4-dimethyl-2,4-dihydropyrazol-3-one

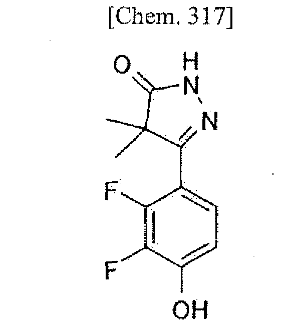

The compound of Example 154 (425 mg) was dissolved in dichloromethane (15 mL) under an argon atmosphere, and aluminum chloride (4.45 g) was added thereto, followed by stirring at room temperature for 15 hours. To the reaction liquid was added ice water, followed by extraction with THF, and the extracted layer was washed with saturated brine and then dried over anhydrous sodium sulfate. After evaporating the solvent under reduced pressure, the residue was washed with diethyl ether to obtain the desired product (210 mg) as a white powder.
¹H-NMR (DMSO-d₆, 400 MHz): δ 1.21 (6H, s), 6.83-6.84 (1H, m), 7.31 (1H, ddd, J=9.2, 8.6, 1.8 Hz), 10.87 (1H, s), 11.53 (1H, s).

### <Example 156>

### Methyl 3 -(4-fiuoro-3-methoxyphenyl)-3-hydroxy-2,2-dimethylpropionate ester

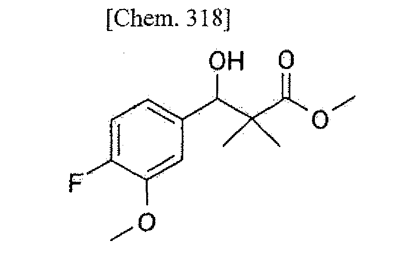

Diisopropyl amine (2.96 mL) was dissolved in THF (40 mL) under an argon atmosphere, and n-butyl lithium (2.71 mol/L hexane solution, 7.79 mL) was added thereto at -78°C, followed by stirring at 0°C for 30 minutes, and then methyl isobutyrate ester (2.33 mL) was added thereto at -78°C, followed by stirring at room temperature for 1 hour. To the reaction liquid was added a solution of 4-fluoro-3-methoxybenzaldehyde (2.50 g) in THF (40 mL) at -78°C, followed by stirring at room temperature for 1.5 hours. To the reaction liquid was added a saturated aqueous ammonium chloride solution, followed by extraction with ethyl acetate, and the extracted layer was washed with saturated brine and then dried over anhydrous sodium sulfate. After evaporating the solvent under reduced pressure, the residue was purified by silica gel column chromatography (hexane:ethyl acetate=4:1) to obtain the desired product (4.26 g) as a colorless oil.
¹H-NMR (CDCl₃, 400 MHz): δ 1.11 (3H, s), 1.15 (3H, s), 3.15 (1H, d, J=3.1 Hz), 3.73 (3H, s), 3.88 (3H, s), 4.86 (1H, d, J=3.1 Hz), 6.78-6.81 (1H, m), 6.95-7.03 (2H, m).

### <Example 157>

### Methyl 3-(4-fluoro-3-methoxyphenyl)-2,2-dimethyl-3-oxopropionate ester

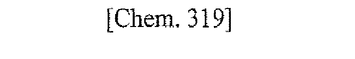

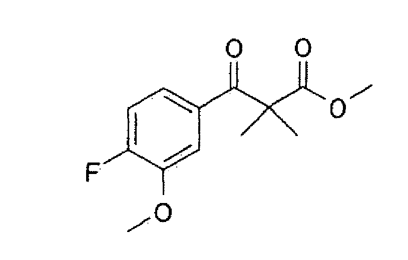

The compound of Example 156 (4.26 g) was dissolved in DMSO (80 mL) under an argon atmosphere, and triethylamine (23.2 mL) and a sulfur trioxide-pyridine complex (13.2 g) were added thereto, followed by stirring at room temperature for 1 hour. To the reaction liquid was added water, followed by extraction three times with hexane:ethyl acetate (4:1), and the combined organic layer was washed with saturated brine and then dried over anhydrous sodium sulfate. After evaporating the solvent under reduced pressure, the residue was purified by silica gel column chromatography (hexane:ethyl acetate=30:1→4:1) to obtain the desired product (2.50 g) as a colorless oil.
¹H-NMR (CDCl₃, 400 MHz): δ 1.55 (6H, s), 3.65 (3H, s), 3.92 (3H, s), 7.08 (1H, dd, J=10.4, 8.6 Hz), 7.33-7.37 (1H, m), 7.57 (1H, dd, J=2.4, 8.6 Hz).

### <Example 158>

### 5-(4-Fluoro-3-methoxyphenyl)-4,4-dimethyl-2,4-dihydropyrazol-3-one

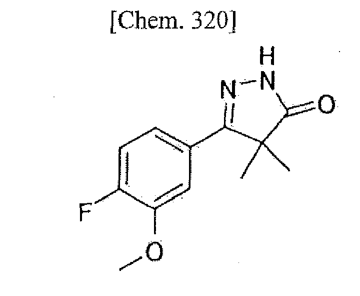

The compound of Example 157 (2.50 g) was dissolved in ethanol (50 mL), and hydrazine monohydrate (1.28 mL) was added thereto, followed by stirring for 5 hours under the condition of heating under reflux. The solvent of the reaction liquid was evaporated under reduced pressure to obtain the desired product (2.23 g) as a white powder.
¹H-NMR (CDCl₃, 400 MHz): δ 1.52 (6H, s), 3.94 (3H, s), 7.11 (1H, dd, J=11.0, 8.6 Hz), 7.21-7.25 (1H, m), 7.52 (1H, dd, J=8.6, 2.4 Hz), 8.95 (1H, brs).

### <Examples 159>

### 5-(4-Fluoro-3-hydroxyphenyl)-4,4-dimethyl-2,4-dihydropyrazol-3-one

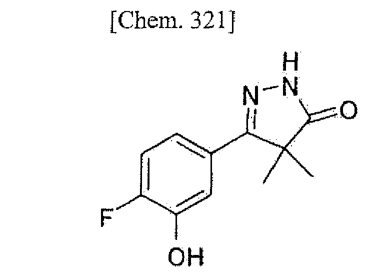

The compound of Example 158 (2.23 g) was dissolved in dichloromethane (50 mL) under an argon atmosphere, and boron tribromide (1.0 mol/L dichloromethane solution, 14.2 mL) was added thereto at 0°C, followed by stirring at 0°C for 1 hour and then stirring at room temperature for 4 hours. To the reaction liquid was added water, followed by extraction three times with ethyl acetate:THF (1:1), and the combined organic layer was washed with saturated brine and then dried over anhydrous sodium sulfate. After evaporating the solvent under reduced pressure, the obtained residue was washed with diethyl ether to obtain the desired product (1.91 g) as a yellow powder.
¹H-NMR (DMSO-d₆, 400 MHz): δ 1.32 (6H, s), 7.12-7.21 (2H, m), 7.43 (1H, dd, J=8.9, 2.1 Hz), 10.09 (1H, s), 11.48 (1H, s).

### <Example 160>

### 2-Fluoro-3-methaxybenzaldehyde

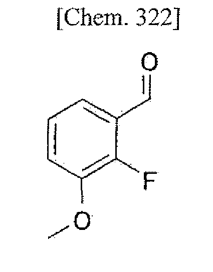

2-Fluoroanisole (2.24 mL) was dissolved in THF (25 mL) under an argon atmosphere, and an n-butyl lithium, (2.71 mol/L hexane solution, 7.38 mL) and N,N,N', N", N"-pentamethyldiethylene triamine (4.20 mL) were added thereto at -78°C, followed by stirring at -78C for 2 hours. Then, DMF (2.41 mL) was added thereto at -78°C, followed by stirring at room temperature for 1.5 hours. To the reaction liquid was added water, followed by extraction with ethyl acetate, and the extracted layer was washed with saturated brine and then dried over anhydrous sodium sulfate. After evaporating the solvent under reduced pressure, the residue was purified by silica gel column chromatography (hexane:ethyl acetate=15:1) to obtain the desired product (2.36 g) as a yellow powder.
¹H-NMR (CDCl₃, 400 MHz): δ 3.94 (3H, s), 7.16-7.44 (3H, m), 10.39 (1H, s).

### <Example 161>

### Methyl 3-(2-fluoro-3-methoxyphenyl)-3-hydroxy-2,2-dimethylpropionate ester

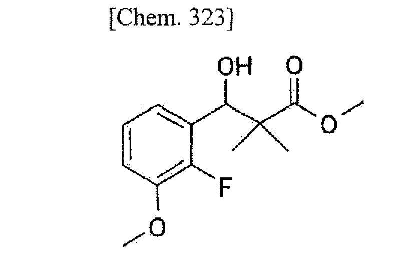

Diisopropyl amine (2.48 mL) was dissolved in THF (40 mL) under an argon atmosphere, and n-butyl lithium (2.71 mol/L hexane solution, 6.52 mL) was added thereto at -78°C, followed by stirring at 0°C for 30 minutes, and then methyl isobutyrate ester (1.87 mL) was added thereto at -78 °C, followed by stirring at room temperature for 1 hour. To the reaction liquid was added a solution of the compound of Example 121 (2.50 g) in THF (40 mL) at -78°C, followed by stirring at room temperature for 1.5 hours. To the reaction liquid was added a saturated aqueous ammonium chloride solution, followed by extraction with ethyl acetate, and the extracted layer was washed with saturated brine and then dried over anhydrous sodium sulfate. After evaporating the solvent under reduced pressure, the residue was purified by silica gel column chromatography (hexane:ethyl acetate=10.1→5:1) to obtain the desired product (1.73 g) as a yellow oil.
¹H-NMR (CDCl₃, 400 MHz): δ 1.16 (3H, s), 1.17 (3H, d, J=2.4 Hz), 3.33 (1H, d, J=3.7 Hz), 3.74 (3H, s), 3.88 (3H, s), 5.30 (1H, d, J=3.7 Hz), 6.89 (1H, td, J=7.9, 1.8 Hz), 7.00-7.07 (2H, m).

### <Example 162>

### Methyl 3-(2-fluoro-3-methoxyphenyl)-2,2-dimethyl-3-oxopropionate ester

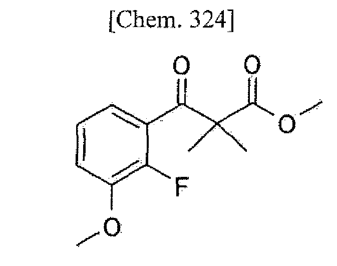

The compound of Example 161 (1.73 g) was dissolved in DMSO (35 mL) under an argon atmosphere, and triethylamine (9.43 mL) and a sulfur trioxide-pyridine complex (5.40 g) were added thereto, followed by stirring at room temperature for 1 hour. To the reaction liquid was added water, followed by extraction three times with hexane:ethyl acetate (4:1), and the combined organic layer was washed with saturated brine and then dried over anhydrous sodium sulfate. After evaporating the solvent under reduced pressure, the residue was purified by silica gel column chromatography (hexane:ethyl acetate=1: 1) to obtain the desired product (970 mg) as a yellow oil.
¹H-NMR (CDCl₃, 400 MHz): δ 1.50 (6H, s), 3.71 (3H, s), 3.90 (3H, s), 7.08-7.15 (2H, m), 7.19-7.23 (1H,m).

### <Example 163>

### 5-(2-Fluoro-3-hydroxyphenyl)-4,4-dimethyl-2,4-dihydropyrazol-3-one

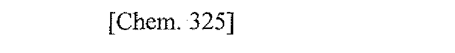

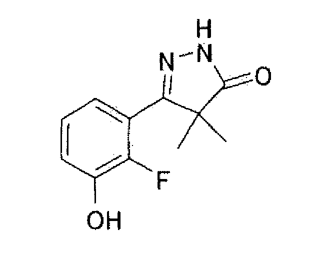

The compound of Example 162 (970 mg) was dissolved in ethanol (30 mL), and hydrazine monohydrate (0.552 mL) was added thereto, followed by stirring for 11 hours under the condition of heating under reflux. After evaporating the solvent of the reaction liquid under reduced pressure, the residue was dissolved in dichloromethane (30 mL), and boron tribromide (1.0 mol/L dichloromethane solution, 8.40 mL) was added thereto at 0°C under argon atmosphere, followed by stirring at room temperature for 1 hour. To the reaction liquid was added water, followed by extraction with THF, and the extracted layer was washed with saturated brine and then dried over anhydrous sodium sulfate. After evaporating the solvent under reduced pressure, the obtained residue was washed with diethyl ether to obtain the desired product (601 mg) as a yellow powder.
¹H-NMR (DMSO-d₆, 400 MHz): δ 1.24 (6H, s), 7.01-7.09 (3H, m), 10.09 (1H, s), 11.61 (1H, s).

### <Examples 164>

### (2-Bromoethoxy)-t-butyldiphenylsilane

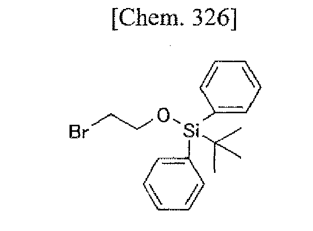

2-Bromoethanol (3.00 mL) was dissolved in DMF (40.0 mL) under an argon gas atmosphere, and imidazole (4.32 g) and tert-butyl diphenylsilyl chloride (11.6 mL) were added thereto under ice cooling, followed by stirring at room temperature for 1.5 hours. To the reaction liquid was added water, followed by extraction with ethyl acetate, and the extracted layer was washed with water and saturated brine, and then dried over anhydrous sodium sulfate. After evaporating the solvent under reduced pressure, the residue was purified by silica gel column chromatography (hexane:ethyl acetate=50:1) to obtain the desired product (15.1 g) as a colorless oil.
¹H-NMR (CDCl₃, 400 MHz): δ 1.07 (9H, s), 3.42 (2H, t, J=6.1 Hz), 3.92 (2H, t, J=6.1 Hz), 7.39-7.44 (6H, m), 7.66-7.68 (4H, m).

### <Example 165>

### 6-[4-[2-(t.Butyldiphenylsilyloxy)ethoxy]phenyl]-5-mcthyl-4,5-dihydro-2H-pyridazin-3-one

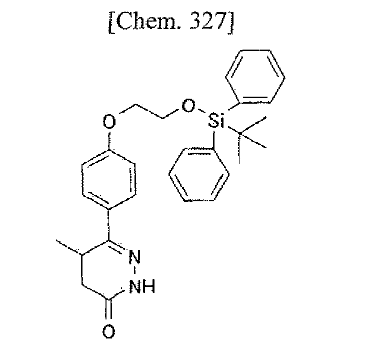

The compound of Example 92 (3.00 g) was dissolved in DMF (50.0 mL) under an argon gas atmosphere, and the compound of Example 164 (5.87 g) and potassium carbonate (4.06 g) were added thereto, followed by stirring at room temperature for 1.5 hours and at 60°C for 6.5 hours. To the reaction liquid was added water, followed by extraction with ethyl acetate, and the extracted layer was washed with water and saturated brine, and then dried over anhydrous sodium sulfate. After evaporating the solvent under reduced pressure, the residue was purified by silica gel column chromatography (hexane:ethyl acetate= 2:1) to obtain the desired product (5.73 g) as a colorless powder.
¹H-NMR (CDCl₃, 400 MHz): δ 1.69 (9H, s), 1.25 (3H, d, J=7.3 Hz), 2.46 (1H, d, J=17.1 Hz), 2.71 (1H, dd, J=17.1, 7.3 Hz), 3.31-3.35 (1H, m), 4.01 (2H, t, J=5.5 Hz), 4.12 (2H, t, J=5.5 Hz), 6.59-6.91 (2H, m), 7.37-7.44 (6H, m), 7.66-7.72 (6H, m), 8.48 (1H, brs).

### <Examples 166>

### 2-t-Butoxycarbonyl-6-[4-[2-(t-butyldiphenylsilyloxy)ethoxy]phenyl]-5-methyl-4,5-dihydro-2H-pyridazin-3 -one

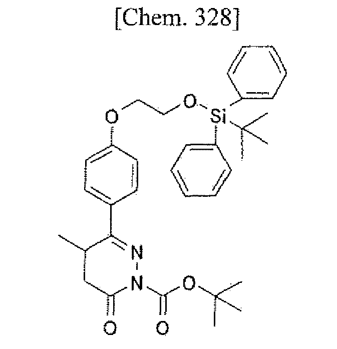

The compound of Example 165 (5.73 g) was dissolved in acetonitrile (100 mL) under an argon gas atmosphere, and di-tert-butyl-di-carbonate (3.08 g) and a catalytic amount of 4-dimethyl aminopyridine were added thereto, followed by stirring at room temperature for 2 hours. To the reaction liquid was added water, followed by extraction with ethyl acetate, and the extracted layer was washed with water and saturated brine, and then dried over anhydrous sodium sulfate. After evaporating the solvent under reduced pressure, the residue was purified by silica gel column chromatography (hexane:ethyl acetate= 4:1) to obtain the desired product (6.90 g) as a colorless amorphous.
¹H-NMR (CDCl₃, 400 MHz): δ 1.07 (9H, s), 1.25 (3H, d, J=6.7 Hz), 1.66 (9H, s), 2.57 (1H, dd, J=16.5, 1.2 Hz), 2.78 (1H, dd, J=16.5, 6.7 Hz), 3.35-3.38 (1H, m), 4.01 (2H, t, J=5.5 Hz), 4.12 (2H, t, J=5.5 Hz), 6.89-6.91 (2H, m), 7.37-7.44 (6H, m), 7.69-7.72 (4H, m), 7.75-7.78 (2H,m).

### <Example 167>

### 2-t-Butoxycarbonyl-6-[4-(2-hydroxyethoxy)phenyl]-5-methyl-4,5-dihydro-2H-pyridazin-3-one

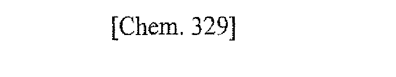

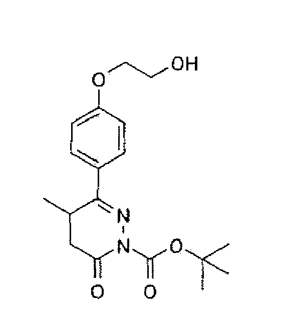

The compound of Example 166 (6.90 g) was dissolved in THF (60.0 mL) under an argon gas atmosphere, and tetrabutyl ammonium fluoride (1.00 mol/L THF solution, 14.0 mL) was added thereto under ice cooling, followed by stirring at room temperature for 4 hours. To the reaction liquid was added water, followed by extraction with ethyl acetate, and the extracted layer was washed with water and saturated brine, and then dried over anhydrous sodium sulfate. After evaporating the solvent under reduced pressure, the residue was purified by silica gel column chromatography (hexane:ethyl acetate=1:2) to obtain the desired product (3.33 g) as a colorless powder.
¹H-NMR (CDCl₃, 400 MHz): δ 1.25 (3H, d, J=7.3 Hz), 1.62 (9H, s), 2.57 (1H, dd, J=16.5, 1.8 Hz), 2.78 (1H, dd, J=16.5, 6.7 Hz), 3.33-3.40 (1H, m), 3.98-4.01 (2H, m), 4.11-4.15 (2H, m), 6.95-6.97 (2H, m), 7.78-7.81 (2H, m).

### <Example 168>

### 2-t-Butoxycarbonyl-6-[4-(2-iodoethoxy)phenyl]-5-methyl-4,5-dihydro-2H-pyridazin-3-one

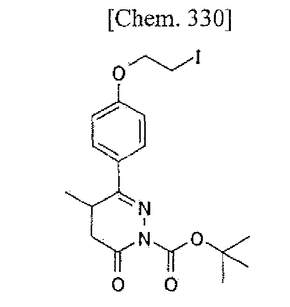

The compound of Example 167 (500 mg) was dissolved in THF (10.0 mL), and imidazole (147 mg), triphenyl phosphine (567 mg), and iodine (402 mg) were added thereto, followed by stirring at room temperature for 1 hour. To the reaction liquid was added a saturated aqueous sodium thiosulfate solution, followed by extraction with ethyl acetate, and the extracted layer was washed with water and saturated brine in this order, and then dried over anhydrous sodium sulfate. After evaporating the solvent under reduced pressure, the residue was purified by silica gel column chromatography (hexane:ethyl acetate= 2:1) to obtain the desired product (645 mg) as a colorless powder.
¹H-NMR (CDCl₃, 400 MHz): δ 1.25 (3H, d, J=7.3 Hz), 1.62 (9H, s), 2.57 (1H, dd, J=16.5, 1.8 Hz), 2.78 (1H, dd, J=16.5, 6.7 Hz), 3.355-3.39 (1H, m), 3.44 (2H, t, J=6.1 Hz), 4.30 (2H, t, J=6.1 Hz), 6.92-6.95 (2H, m), 7.78-7.81 (2H, m).

### <Example 169>

### 3-Methoxy-4-methoxymethyloxybenzaldehyde

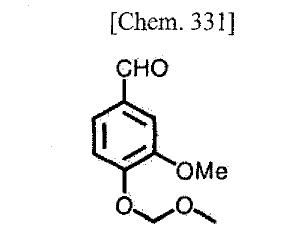

Vaniline (7.00 g) was dissolved in dichloromethane (200 mL) under an argon atmosphere, and diisopropyl ethylamine (16.4 mL) and chloromethylmethylether (4.54 mL) were added thereto at 0°C, followed by stirring at room temperature for 4 hours. To the reaction liquid was added water, followed by extraction three times with ethyl acetate, and the combined organic layer was washed with saturated brine and then dried over sodium sulfate. After evaporating the solvent under reduced pressure, the obtained residue was purified by silica gel chromatography (hexane:ethyl acetate=3:1) to obtain the desired product (9.45 g) as a colorless oil.
¹H-NMR (CDCl₃, 400 MHz): δ 3.53 (3H, s), 3.96 (3H, s), 5.34 (2H, s), 7.28 (1H, d, J=8.6 Hz), 7.43-7.44 (2H, m), 9.88 (1H, s).

### <Example 170>

### Methyl 3-(3-methoxy-4-methoxymethyloxyphenyl)-3-hydroxy-2,2-dimethylpropionate ester

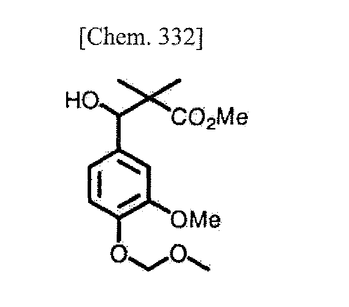

The compound of Example 169 (2.00 g) was dissolved in diethyl ether (100 mL) under an argon atmosphere, and dimethylketene methyltrimethylsilyl acetal (3.11 mL) and a boron trifluoride diethyl ether complex (1.94 mL) were added thereto, followed by stirring at room temperature for 1 hour. To the reaction liquid was added a saturated aqueous sodium hydrogen carbonate solution, followed by extraction three times with ethyl acetate, and the combined organic layer was washed with saturated brine and then dried over sodium sulfate. After evaporating the solvent under reduced pressure, the obtained residue was purified by silica gel chromatography (hexane:ethyl acetate2:1) to obtain the desired product (3.00 g) as a colorless oil.
¹H-NMR (CDCl₃, 400 MHz): δ 1.12 (3H, s), 1.16 (3H, s), 3.02 (1H, d, J=4.3 Hz), 3.52 (3H, s), 3.73 (3H, s), 3.88 (3H, s), 4.85 (1H, d, J=4.3 Hz), 5.22 (2H, s), 6.80 (1H, dd, J=8.6, 1.8 Hz), 6.90 (1H, d, J=1.8 Hz), 7.09 (1H, d, J=8.6 Hz).

### <Example 171>

### Methyl 3-(3-methoxy-4-methoxymethyloxyphenyl)-2,2-dimethyl-3-oxopropionatc ester

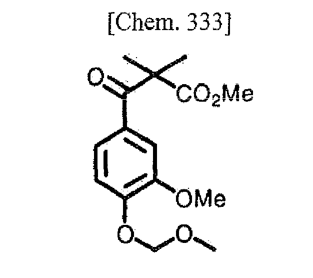

The compound of Example 170 (3.00 g) was dissolved in DMSO (70 mL) under an argon atmosphere, and triethylamine (14.1 mL) and a sulfur trioxide-pyridine complex (8.04 g) were added thereto, followed by stirring at room temperature for 1 hour. To the reaction liquid was added 1 mol/L hydrochloric acid, followed by extraction three times with hexane-ethyl acetate (4-1), and the combined organic layer was washed with saturated brine and then dried over anhydrous sodium sulfate. After evaporating the solvent under reduced pressure, the obtained residue was purified by silica gel chromatography (hexane:ethyl acetate=3:1) to obtain the desired product (2.41 g) as a colorless oil.
¹H-NMR (CDCl₃, 400 MHz): δ 1.54 (6H, s), 3.51 (3H, s), 3.65 (3H, s), 3.92 (3H, s), 5.29 (2H, s), 7.13 (1H, d, J=8.6 Hz), 7.37 (1H, dd, J=8.6, 2.4 Hz), 7.52 (1H, d, J=2.4 Hz).

### <Fxample 172>

### 5-(3-Methoxy-4-mcthoxymethyloxyphenyl)-4,4-dimethyl-2,4-dihydropyrazol-3-one

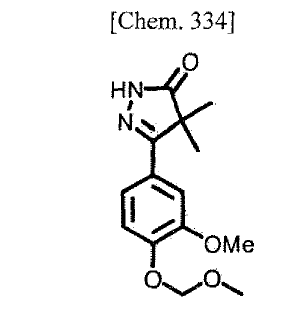

The compound of Example 171 (2.41 g) was dissolved in ethanol (50 mL), and hydrazine monohydrate (1.18 mL) was added thereto, followed by stirring for 10 hours under the condition of heating under reflux. To the reaction liquid was added water, followed by extraction three times with ethyl acetate, and the combined extracted layer was washed with saturated brine and then dried over anhydrous sodium sulfate. The solvent was evaporated under reduced pressure to obtain the desired product (2.17 g) as a colorless powder.
¹H-NMR (CDCl₃, 400 MHz): δ 1.52 (6H, s), 3.53 (3H, s), 3.94 (3H, s), 5.28 (2H, s), 7.17 (1H, d, J=8.6 Hz), 7.23 (1H, dd, J=8.6, 1.8 Hz), (1H, d, J=1.8 Hz), 8.62 (1H, s).

### <Example 173>

### 5-(3-Methoxy-4-hydroxyphenyl)-4,4-dimethyl-2,4-dihydropyrazol-3-one

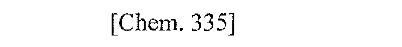

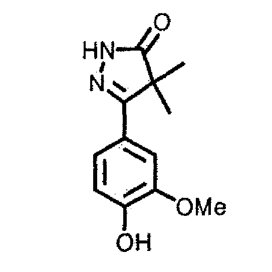

The compound of Example 172 (2.17 g) was dissolved in methanol (30 mL) and THF (30 mL), and 3 mol/L hydrochloric acid was added thereto, followed by stirring for 4 hours under the condition of heating under reflux. To the reaction liquid was added water, followed by extraction three times with ethyl acetate, and the combined extracted layer was washed with saturated brine and then dried over anhydrous sodium sulfate. After evaporating the solvent under reduced pressure, the obtained residue was washed with diisopropyl ether to obtain the desired product (1.60 g) as a colorless powder.
¹H-NMR (DMSO-d₆, 400 MHz): δ 1.32 (6H, s), 3.75 (3H, s), 6.80 (1H, d, J=7.9 Hz), 7.19 (1H, dd, J=7.9, 1.8 Hz), 7.31 (1H, d, J=1.8 Hz), 9.50 (1H, s), 11.32 (1H, s).

### <Examples 174>

### 2,3-Difluoro-4-methoxy-1-propionylbenzene

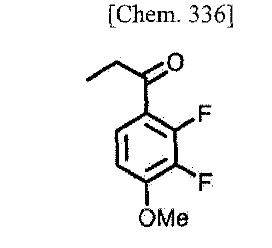

Aluminum chloride (9.25 g) was dissolved in nitromethane (100 mL) under an argon atmosphere, propionyl chloride (6.06 mL), and 2,3-difluoroanisole (4.0 g) that had been dissolved in nitromethane (30 mL) were added thereto, followed by stirring at room temperature for 18 hours. It was added to ice water, extracted three times with ethyl acetate, washed with saturated brine, dried over anhydrous sodium sulfate, and then passed through a silica gel. The solvent was evaporated under reduced pressure to obtain the desired product (5.68 g) as a white powder.
¹H-NMR (CDCl₃, 400 MHz): δ 1.20 (3H, t, J=7.6 Hz), 2.95-2.99 (2H, m), 3.96 (3H, s), 6.81 (1H, ddd, J=1.8, 6.7, 6.7 Hz), 7.69 (1H, ddd, J=2.4, 7.9, 7.9 Hz).

### <Examples 175>

### 6-(2,3-Difluoro-4-methoxyphenyl)-5-methyl-4,5-dihydro-2H-pyridazin-3-one

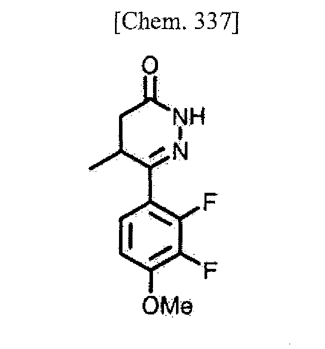

The compound of Example 174 (5.68 g) was dissolved in THF (200 mL) under an argon atmosphere, and a solution (1.0 mol/L, 31.2 mL) of lithium hexamethyl disilazane in THF was added thereto at 0°C, followed by stirring at 0°C for 30 minutes, and then t-butyl bromoacetate (5.41 mL) was added thereto, followed by stirring at room temperature for 1.5 hours. A saturated aqueous ammonium chloride solution was added thereto, followed by evaporation of tetrahydrofuran under reduced pressure. Then, the residue was extracted three times with ethyl acetate, washed with saturated brine, and then dried over anhydrous sodium sulfate and filtered. The solvent of the filtrate was evaporated under reduced pressure, and the obtained residue was dissolved in dichloromethane (50 mL), and trifluoroacetic acid (20 mL) was added thereto, followed by being left to stand at room temperature for 15 hours. After evaporating the solvent under reduced pressure, the obtained residue was dissolved in ethanol (250 mL), and acetic acid (18.0 mL, 315 mmol) and hydrazine monohydrate (6.92 mL) were added thereto, followed by stirring for 7 hours under the condition of heating under reflux. After evaporating ethanol under reduced pressure, water was added to precipitate the crystal, collected by filtration, and then washed with diisopropyl ether to obtain the desired product (4.05 g) as a white powder.
¹H-NMR (CDCl₃, 400 MHz): δ 1.22 (3H, d, J=7.3 Hz), 2.46 (1H, dd, J=3.1, 17.1 Hz), 2.75 (1H, dd, J=6.7, 17.1 Hz), 3.27-3.29 (1H, m), 3.95 (3H, s), 6.79 (1H, ddd, J=1.8, 7.3, 8.6 Hz), 7.34 (1H, ddd, J=2.4, 7.9, 8.6 Hz), 8.51 (1H, s).

### <Example 176>

### 6-(2,3-Difluoro-4-hydroxyphenyl)-5-methyl-4,5-dihydro-2H-pyridazin-3-one

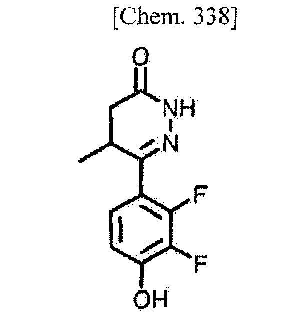

The compound of Example 175 (4.05 g) was dissolved in dichloromethane (170 mL) under an argon atmosphere, and aluminum chloride (45.9 g) was added thereto, followed by stirring at room temperature for 15 hours. Ice water was added thereto, followed by extraction three times with tetrahydrofuran, and the combined organic layer was washed with saturated brine and then dried over anhydrous sodium sulfate. After evaporating the solvent of the filtrate under reduced pressure, diisopropyl ether was added thereto, and the precipitated crystal was collected by filtration to obtain the desired product (3.42 g) as a yellow powder.
¹H-NMR (CDCl₃, 400 MHz): δ 1.02 (3H, d, J=7.3 Hz), 2.20 (1H, dd, J=3.1, 17.1 Hz), 2.65 (1H, dd, J=6.7, 17.1 Hz), 3.10-3.12 (1H, m), 6.80-6.81 (1H, m), 7.23-7.24 (1H, m), 10.75 (1H, s), 10.95 (1H, s).

### <Example 177>

### t-Butyl 3-[2,3-difluoro-4-(2-iodoethoxy)phenyl]-4-methyl-6-oxo-5,6-dihydropyridazine-1(4H)-carboxylate

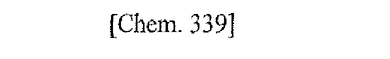

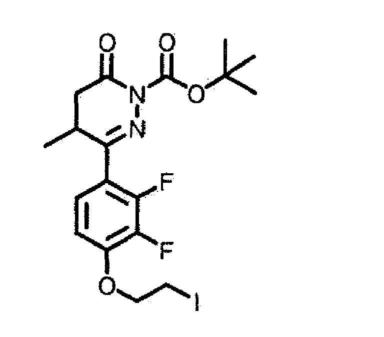

The operations were sequentially carried out in the same manner as in Example 165 to Example 168 using the compound of Example 176 to obtain the desired products as colorless powders.
¹H-NMR (CDCl₃, 400 MHz): δ 1.22 (3H, d, J=7.3 Hz), 2.54 (1H, dd, J=16.2, 3.4 Hz), 2.82 (1H, dd, J=16.2, 6.1 Hz), 3.30-3.32 (1H, m), 3.45 (2H, t, J=7.0 Hz), 4.36 (2H, t, J=7.0 Hz), 6.76-6.80 (1H, m), 7.45-7.47 (1H, m).
ESIMS (+): 495 [M+H]⁺.

### <Examples 178 and 179>

### (+)-6-(4-Methoxyphenyl)-5-methyl-4,5-dihydro-2H-pyridazin-3-one

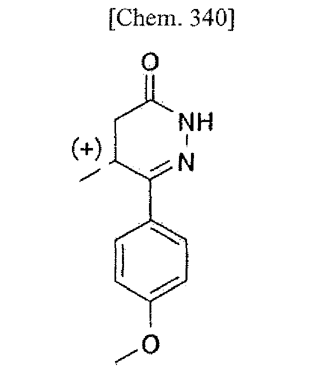

The compound of Example 91 was subjected to optical resolution by high performance liquid chromatography (Daicel Chiralpak AS-H column, Eluent: hexane/ethanol=40/60, Flow rate: 3.00 ml/min, Detection: 293 nm) to obtain a (+) form as a colorless powder from the earlier elution portion (Example 178).
Optical rotation: [α]D²³ +449 (c0.53, DMSO).

(-)-6-(4-Methoxyphenyl)-5-methyl-4,5-dihydro-2H-pyridazin-3 -one

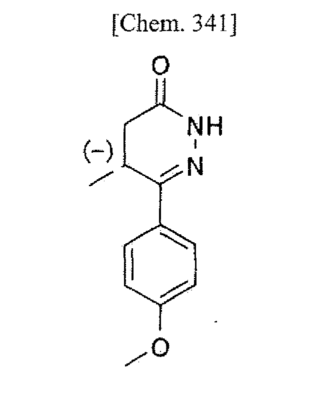

A (-) form was obtained as a colorless powder from the later elution portion (Example 179).
Optical rotation: [α]_{D}²³ -467 (c 0.52, DMSO).

### <Example 180>

### (+)-6-(4-Hydroxyphenyl)-5-methyl-4,5-dihydro-2H-pyridazin-3-one

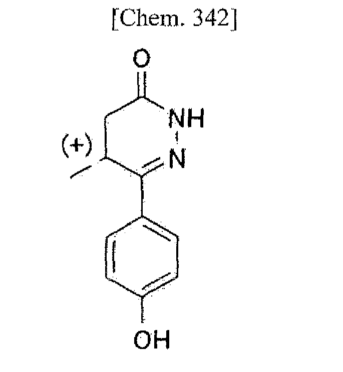

The compound of Example 178 (150 mg) was dissolved in dichloromethane (5.00 mL), and aluminum chloride (1.83 g) was added thereto under ice cooling, followed by stirring at room temperature for 22 hours. The reaction liquid was poured into ice water, followed by extraction with THF, and the extracted layer was dried over anhydrous magnesium sulfate. After evaporating the solvent under reduced pressure, the resulting powder was suspended in diisopropyl ether and collected by filtration to obtain the desired product (117 mg) as a white powder.
¹H-NMR (DMSO-d₆, 400 MHz): δ 1.04 (3H, d, J=7.3 Hz), 2.18 (1H, d, J=15.9 Hz), 2.63 (1H, dd, J=1.8, 15.9 Hz), 3.28-3.33 (1H, m), 6.78-6.80 (2H, m), 7.59-7.63 (2H, m), 9.78 (1H, s), 10.8 (1H, s).
The optical purity was measured by means of HPLC.
Analysis condition: Column; Daicel Chiralpak AS column (0.46cmφ x 25 cm), Developing solvent: hexaue/ethanol=40/60, Flow rate: 0.5 ml/min., Detection: UV (293 mn).
Retention time: 12.1 minutes (98%ee).

### <Example 181>

### (-)-6-(4-Hydroxyphenyl)-5-methyl-4,5-dihydro-2H-pyridazin-3 -one

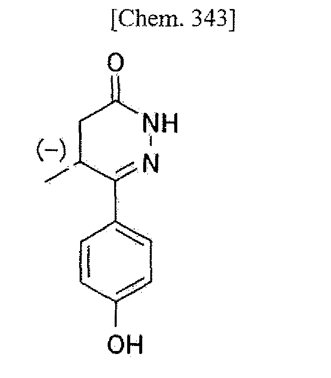

The reaction was carried out in the same manner as in Example 180 using the compound (150 mg) of Example 179 to obtain the desired product (112 mg) as a white powder.
¹H-NMR (DMSO-d₆, 400 MHz): δ 1.04 (3H, d, J=7.3 Hz), 2.18 (1H, d, J=15.9 Hz), 2.63 (1H, dd, J=1.8, 15.9 Hz), 3.28-3.33 (1H, m), 6.78-6.80 (2H, m), 7.59-7.63 (2H, m), 9.78 (1H, s), 10.8 (1H, s).
The optical purity was measured by means of HPLC.
Analysis conditions: column; Daicel Chiralpak AS column (0.46cmφ x 25 cm), Developing solvent: hexane/ethanol=40/60, Flow rate: 0.5 ml/min., Detection: UV (293 nm).
Retention time: 21.5 minutes (>99%ee),

### <Example 182>

### 2-(4-Bromobutyl)-5-(8-methoxy-2-methylquinolin-5-yl)-4,4-dimethyl-4,4-dihydro-pyrazol-3-one

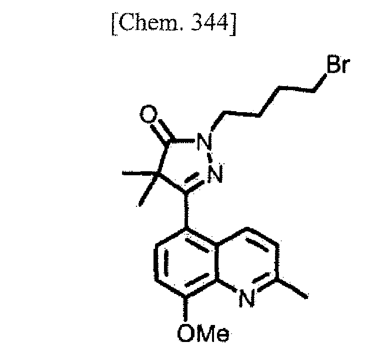

The compound of Example 70 (211 mg) was dissolved in DMF (8.0 mL) under an argon gas atmosphere, and 60% sodium hydride (34.4 mg) was added thereto under ice cooling, followed by stirring at room temperature for 30 minutes. Thereafter, 1,4-dibromobutane (0.467 mL) was added thereto under ice cooling, followed by stirring at room temperature for 1.5 hours. To the reaction liquid was added a saturated aqueous ammonium chloride solution, followed by extraction with ethyl acetate. The organic layer was washed with water and saturated brine in that order, and then dried over anhydrous sodium sulfate. After evaporating the solvent, the residue was purified by silica gel column chromatography (hexane:ethyl acetate=1:5) to obtain the desired product (249 mg) as a colorless oil.
¹H-NMR (CDCl₃, 400 MHz): δ 1.43 (6H, s), 1.95-2.01 (4H, m), 2.81 (3H, s), 3.49 (2H, t, J=6.1 Hz), 3.88 (2H, t, J=6.1 Hz), 4.13 (3H, s), 7.06 (1H, d, J=8.0 Hz), 7.41 (1H, d, J=8.6 Hz), 7.57 (1H, d, J=8.0 Hz), 8.74 (1H, d, J=8.6 Hz).

### <Example 183>

### 2-(4-Bromobutyl)-5-(2-ethyl-8-methoxyquinolin-5-yl)-4,4-dimethyl-4,4-dihydro-pyrazol-3-one

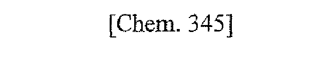

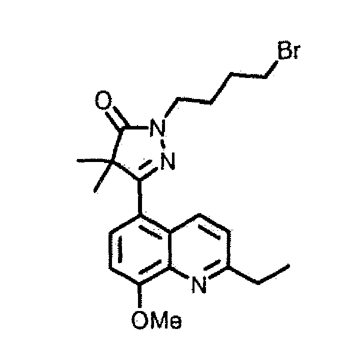

The reaction was carried out in the same manner as in Example 182 using the compound of Example 71 to obtain the desired product as a yellow powder.
¹H-NMR (CDCl₃, 400 MHz): δ 1.41 (3H, t, J=7.3 Hz), 1.43 (6H, s), 1.96-2.01 (4H, m), 3.09 (2H, q, J=7.3 Hz), 3.48-3.51 (2H, m), 3.87-3.90 (2H, m), 4.13 (3H, s), 7.06 (1H, d, J=8.0 Hz), 7.45 (1H, d, J=8.6 Hz), 7.57 (1H, d, J=8.0 Hz), 8.76 (1H, d, J=8.6 Hz).

### <Example 184>

### 2-(4-Bromobutyl)-5-(8-methoxy-2-isopropylquinolin-5-yl)-4,4-dimethyl-4,4-dihydro-pyrazol-3-one

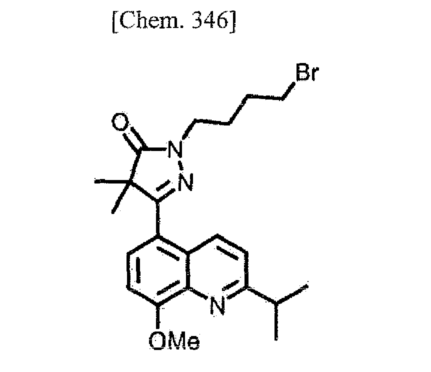

The reaction was carried out in the same manner as in Example 182 using the compound of Example 72 to obtain the desired product as a colorless oil.
¹H-NMR (CDCl₃, 400 MHz): δ 1.40 (6H, d, J=6.7 Hz), 1.43 (6H, s), 1.96-2.01 (4H, m), 3.36-3.45 (1H, m), 3.50 (2H, t, J=6.1 Hz), 3.88 (2H, t, J=6.7 Hz), 4.13 (3H, s), 7.06 (1H, d, J=8.0 Hz), 7.49 (1H, d, J=9.2 Hz), 7.57 (1H, d, J=8.0 Hz), 8.78 (1H, d, J=9.2 Hz).

### <Example 185>

### 2-(4-Bromobutyl)-5-(8-methoxyquinolin-5-yl)-4,4-dimethyl-4,4-dihydro-pyrazol-3-one

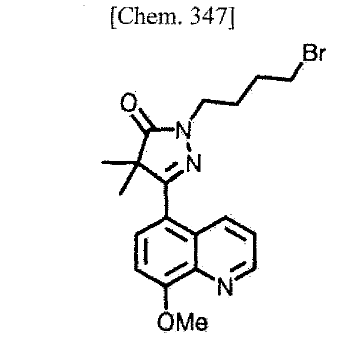

The reaction was carried out in the same manner as in Example 182 using the compound of Example 69 to obtain the desired product as a pale yellow oil.
EIMS (+): 403 [M]⁺
¹H-NMR (CDCl₃, 400 MHz): δ 1.44 (6H, s), 1.95-2.02 (4H, m), 3.49 (2H, t, J=6.1 Hz), 3.89 (2H, t, J=6.1 Hz), 4.15 (3H, s), 7.09 (1H, d, J=8.0 Hz), 7.53 (1H, dd, J=8.6, 4.3 Hz), 7.65 (1H, d, J=8.0 Hz), 8.86 (1H, dd, J=8.6, 1.8 Hz), 8.98 (1H, dd, J=4.3, 1.8 Hz).

### <Example 186>

### 2-(4-Bromobutyl)-5-(7-methoxy-2-trifluoromethylbenzofuran-4-yl)-4,4-dimethyl-4,4-dihydro-pyrazol-3-one

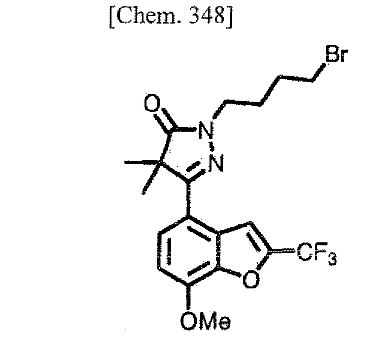

The reaction was carried out in the same manner as in Example 182 using the compound of Example 77 to obtain the desired product as a colorless powder.
ELMS (+): 460 [M]⁺.
¹H-NMR (CDCl₃, 400 MHz): δ 1.53 (6H, s), 1.95-1.99 (4H, m), 3.48 (2H, t, J=6.2 Hz), 3.87 (2H, t, J-6.1 Hz), 4.08 (3H, s), 6.93 (1H, d, J=8.6 Hz), 7.51 (1H, d, J=8.6 Hz), 7.95 (1H, d, 1=1.2 Hz).

### <Example 187>

### 2-(4-Bromobutyl)-5-(7-methoxy-1-methoxymethyl-2-trifluoromethyl-1H-benzo[d]imidazol-4-yl)-4,4-dimethyl-4,4-dihydro-pyrazol-3-one

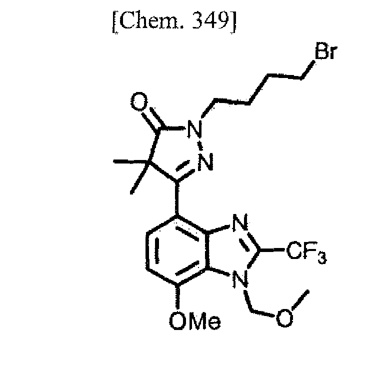

The reaction was carried out in the same manner as in Example 182 using the compound of Example 74 to obtain the desired product as a colorless powder.
EIMS (+): 504 [M]⁺.
¹H-NMR (CDCl₃, 400 MHz): δ 1.59 (6H, s), 1.96-1.99 (4H, m), 3.38 (3H, s), 3.48-3.51 (2H, m), 3.84-3.87 (2H, m), 4.06 (3H, s), 5.90 (2H, s), 6.93 (1H, d, J=8.6 Hz), 7.80 (1H, d, J=8.6 Hz)

### <Example 188>

### 2-(4-Bromobutyl)-5-(7-methoxy-2-trifluoromethylbenzo[b]thiophcn-4-yl)-4,4-dimethyl-4,4-dihydro-pyrazol-3-one

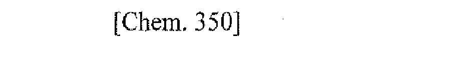

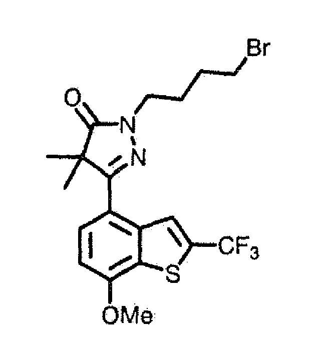

The reaction was carried out in the same manner as in Example 182 using the compound of Example 78 to obtain the desired product as a colorless powder.
ESIMS (+): 477 [M+H]⁺
¹H-NMR (400 MHz, CDCl₃): δ 1.54 (6H, s), 1.96-2.00 (4H, m), 3.48 (2H, t, J=6.1 Hz), 3.88 (2H, t, J=6.7 Hz), 4.07 (3H, s), 6.89 (1H, d, J=8.6 Hz), 7.67 (1H, d, J=8.6 Hz), 8.70 (1H, t, J=1.2 Hz).

### <Example 189>

### 2-(4-Bromobutyl)-5-(5-methoxy-2-trifluoromethyl-[1,2,4]triazolo[1,5-a]pyridin-8-yl)-4,4-dimethyl-4,4-dihydro-pyrazol-3-one

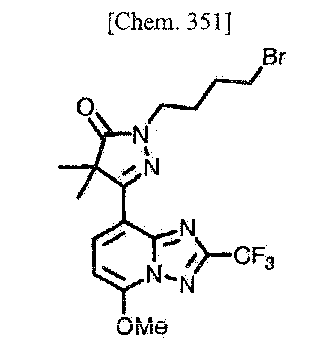

The reaction was carried out in the same manner as in Example 182 using the compound of Example 79 to obtain the desired product as a yellow powder.
ESIMS (+): 464 [M+H]⁺
¹H-NMR (CDCl₃, 400 MHz): δ 1.63 (6H, s), 1.96-1.97 (4H, m), 3.48-3.49 (2H, m), 3.85-3.87 (2H, m), 4.28 (3H, s), 6.58 (1H, d, J=8.6 Hz), 8.29 (1H, d, J=8.6 Hz).

### <Example 190>

### 2-(4-Bromobutyl)-5-(4-methoxy-2-trifluoromcthylbenzo[d]thiazol-7-yl)-4,4-dimethyl-4,4-dihydro-pyrazol-3-one

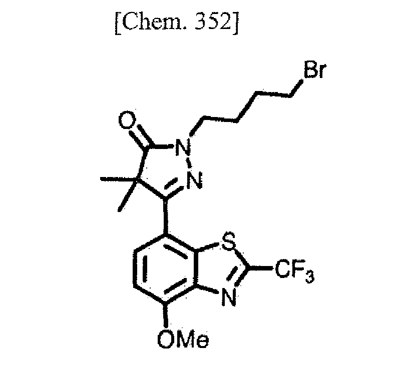

The reaction was carried out in the same manner as in Example 182 using the compound of Example 76 to obtain the desired product as a yellow powder.
ESIMS (+): 480 [M+H]⁺
¹H-NMR (CDCl₃, 400 MHz): δ 1.59 (6H, s), 1.95-2.06 (4H, m), 3.49 (2H, t, J=6.4 Hz), 3.90 (2H, t, J=6.4 Hz), 4.15 (3H, s), 7.07 (1H, d, J=7.9 Hz), 7.81 (1H, d, J=7.9 Hz).

### <Example 191>

### 2-(4-Bromobutyl)-5-(2-ethyl-5-methoxyindolidin-8-yl)-4,4-dimethyl-4,4-dihydro-pyrazol-3-one

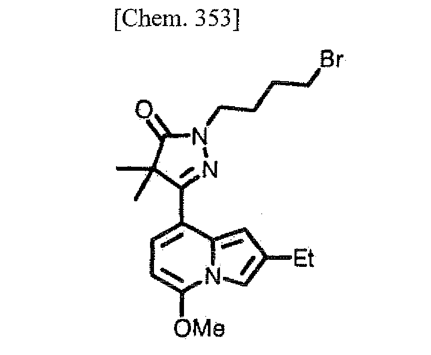

The reaction was carried out in the same manner as in Example 182 using the compound of Example 90 to obtain the desired product as a yellow oil.
¹H-NMR (CDCl₃, 400 MHz): δ 1.33 (3H, t, J=7.3 Hz), 1.53 (6H, s), 1.98-2.02 (4H, m), 2.77 (2H, q, J=7.3 Hz), 3.49 (2H, t, J=6.1 Hz), 3.88 (2H, t, J=6.4 Hz), 4.09 (3H, s), 5.81 (1H, d, J=7.9 Hz), 7.15 (1H, d, J=7.9 Hz), 7.19 (1H, d, J=1.8 Hz), 7.34 (1H, d, J=1.8 Hz).
EIMS (+): 419 [M]⁺.

### <Example 192>

### 2-(4-Bromobutyl)-5-(8-methoxy-2-tnfluoromethylimidazo[1,2-a]pyridin-5-yl)-4,4-dimethyl-4,4-dihydro-pyrazol-3-one

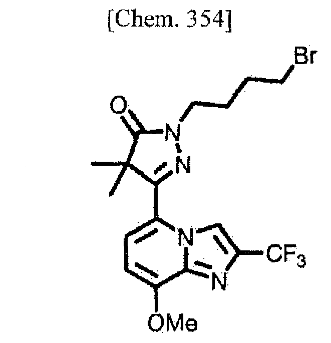

The reaction was carried out in the same manner as in Example 182 using the compound of Example 80 to obtain the desired product as a colorless powder.
¹H-NMR (CDCl₃, 400 MHz): δ 1.59 (6H, s), 1.90-2.08 (4H, m), 3.48 (2H, t, J=6.1 Hz), 3.93 (2H, t, J=6.7 Hz), 4.11 (3H, s), 6.68 (1H, d, J=8.6 Hz), 7.29 (1H, d, J=8.6 Hz), 9.40 (1H, s).

### <Example 193>

### 5-(8-Methoxy-2-methylquinolin-5-yl)-2-[4-[4-(4-methyl-6-oxo-1,4,5,6-tetrahydropyridazin-3-yl)phenoxy]lbutyl]-4,4-dimethyl-4,4-dihydro-pyrazol-3-one

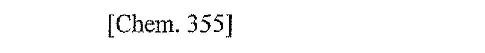

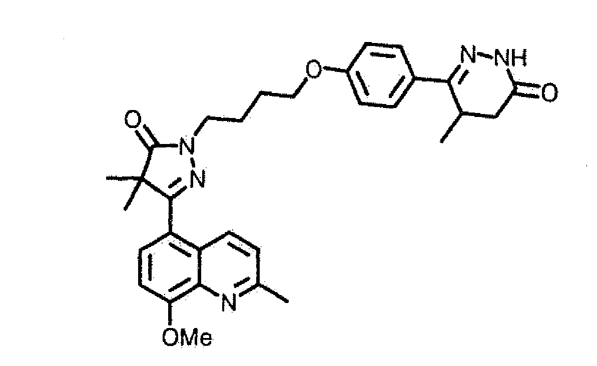

The compound of Example 182 (238 mg) was dissolved in DMF (3.5 mL) under an argon gas atmosphere, and the compound of Example 92 (116 mg) and potassium carbonate (157 mg) were added thereto, followed by stirring at 60°C for 6 hours. To the reaction liquid was added water, followed by extraction with ethyl acetate. The organic layer was washed with water and saturated brine in that order, and then dried over anhydrous sodium sulfate. After evaporating the solvent, the residue was purified by silica gel column chromatography (ethyl acetate→ethyl acetate:methanol=9:1) to obtain the desired product (269 mg) as a colorless amorphous.
Elemental analysis: Found value C 68.10%, H 6.51%, N 12.62%, Calculated value as C₃₁H₃₅N₅O₄ · 1/5H₂O C 68.29%, H 6.54%, N 12.84%.
ESIMS (+): 542 [M+H]⁺
¹H-NMR (CDCl₃, 400 MHz): δ 1.24 (3H, d, J=7.4 Hz), 1.43 (6H, s), 1.87-1.94 (2H, m), 1.99-2.06 (2H, m), 2.46 (1H, d, J=17.1 Hz), 2.70 (1H, dd, J=6.7, 17.1 Hz), 2.81 (3H, s), 3.32 (1H, m), 3.92 (2H, t, J=6.7 Hz), 4.08 (2H, t, J=6.1 Hz), 4.13 (3H, s), 6.90-6.93 (2H, m), 7.06 (1H, d, J=8.6 Hz), 7.37 (1H, d, J=8.6 Hz), 7.57 (1H, d, J=8.6 Hz), 7.65-7.68 (2H, m), 8.44 (1H, brs), 8.74 (1H, d, J=8.6 Hz).

### <Example 194>

### 5-(2-Ethyl-8-methoxyquinolin-5-yl)-2-[4-[4-(4-methyl-6-oxo-1,4,5,6-tetrahydropyridazin-3-yl)phenoxy]butyl]-4,4-dimethyl-2,4-dihydro-pyrazol-3-one

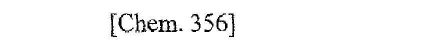

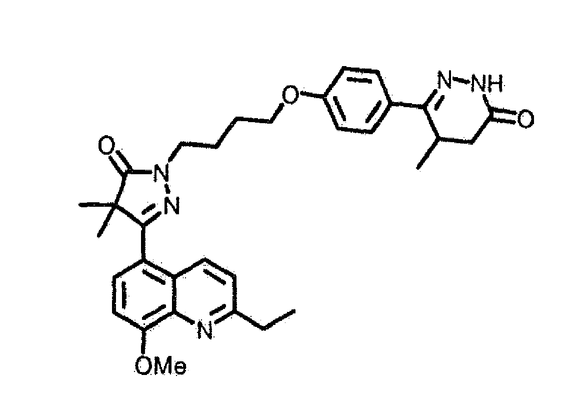

The reaction was carried out in the same manner as in Example 193 using the compound of Example 183 and the compound of Example 92 to obtain the desired product as a colorless amorphous.
HRFABMS (+): 556.2881 (Calculated value as C₃₂H₃₈N₅O₄ 556.2924)
¹H-NMR (CDCl₃, 400 MHz): δ 1.24 (3H, d, J=7.3 Hz), 1.40 (3H, t, J=7.3 Hz), 1.42 (6H, s), 1.89-2.05 (4H, m), 2.46 (1H, d, J=16.9 Hz), 2.70 (1H, dd, J=6.7, 16.9 Hz), 3.09 (2H, q, J=7.3 Hz), 3.29-3.34 (1H, m), 3.93 (2H, t, J=6.7 Hz), 4.08 (2H, t, J=6.1 Hz), 4.13 (3H, s), 6.91-6.94 (2H, m), 7.06 (1H, d, J=8.6 Hz), 7.42 (1H, d, J=8.6 Hz), 7.58 (1H, d, J=8.6 Hz), 7.66-7.68 (2H, m), 8.43 (1H, brs), 8.76 (1H, d, J=8.6 Hz).

### <Example 195>

### 5-(8-Methoxy-2-isopropylquinolin-5-yl)-2-[4-[4-(4-methyl-6-oxo-1,4,5,6-tetrahydropyridazin-3-yl)phenoxy]butyl]-4,4-dimethyl-2,4-dihydro-pyrazol-3-one

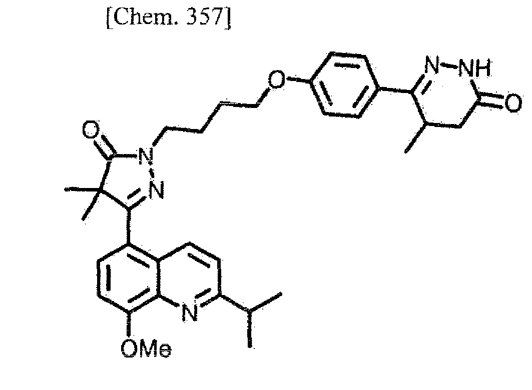

The reaction was carried out in the same manner as in Example 193 using the compound of Example 184 and the compound of Example 92 to obtain the desired product as a colorless amorphous.
Elemental analysis: Found value C 69.12%, H 6.82%, N 11.93%, Calculated value as C₃₃H₃₉N₅O₄ · 1/5H₂O C 69.14%, H 6.93%, N 12.22%.
ESIMS (+): 570 [M+H]⁺
¹H-NMR (CDCl₃, 400 MHz): δ 1.24 (3H, d, J=7.4 Hz), 1.39 (6H, d, J=6.7 Hz), 1.43 (6H, s), 1.89-1.94 (2H, m), 2.00-2.07 (2H, m), 2.46 (1H, d, J=17.8 Hz), 2.70 (1H, dd, J=6.7, 17.8 Hz), 3.30-3.43 (2H, m), 3.93 (2H, t, J=7.3 Hz), 4.08 (2H, t, J=6.1 Hz), 4.13 (3H, s), 6.93 (2H, d, J=9.2 Hz), 7.06 (1H, d, J=8.0 Hz), 7.46 (1H, d, J=8.6 Hz), 7.57 (1H, d, J=8.0 Hz), 7.67 (2H, d, J=9.2 Hz), 8.40 (1H, brs), 8.78 (1H, d, J=8.6 Hz).

### <Example 196>

### 2-[4-[2,3-Difluoro-4-(4-methyl-6-oxo-1,4,5,6-tetrahydropyridazin-3-yl)phenoxy]butyl]-5-(8-methoxy-2-methylquinolin-5-yl)-4,4-dimethyl-2,4-dihydro-pyrazol-3-one

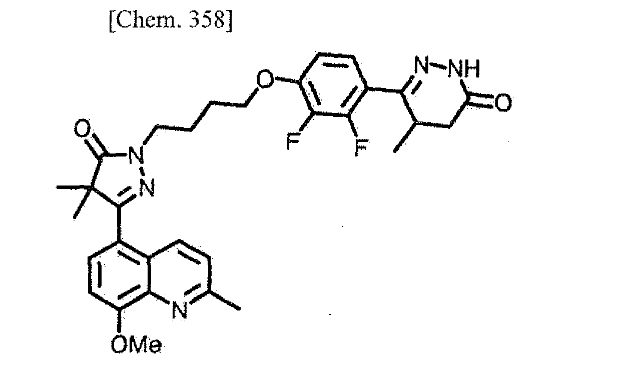

The reaction was carried out in the same manner as in Example 193 using the compound of Example 182 and the compound of Example 176 to obtain the desired product as a colorless amorphous.
Elemental analysis (%): Found value C 63.92, H 5.77, N 12.02, Calculated value as C₃₁H₃₃F₂N₅O₄ · 1/3H₂O C 63.80, H 5.81, N 12.00.
ESIMS (+): 578 [M+H]⁺
¹H-NMR (CDCl₃, 400 MHz): δ 1.21 (3H, d, J=7.4 Hz), 1.58 (6H, s), 1.91-1.98 (2H, m), 2.00-2.06 (2H, m), 2.44 (1H, dd, J=3.1, 16.8 Hz), 2.74 (1H, dd, J=6.7, 16.8 Hz), 2.81 (3H, s), 3.22-3.30 (1H, m), 3.93 (2H, t, J=6.7 Hz), 4.13 (3H, s), 4.16 (2H, t, J=6.1 Hz), 6.76-6.80 (1H, m), 7.06 (1H, d, J=8.0 Hz), 7.26-7.30 (1H, m), 7.40 (1H, d, J=8.6 Hz), 7.57 (1H, d, J=8.0 Hz), 8.51 (1H, brs), 8.74 (1H, d, J=8.6 Hz).

### <Example 197>

### 2-[4-[2,3-Difluoro-4-(4,4-dimethyl-5-oxo-4,5-dihydro-1H-pyrazol-3-yl)phenoxy]butyl]-5-(8-methoxy-2-methylquinolin-5-yl)-4,4-dimethyl-2,4-dihydro-pyrazol-3-one

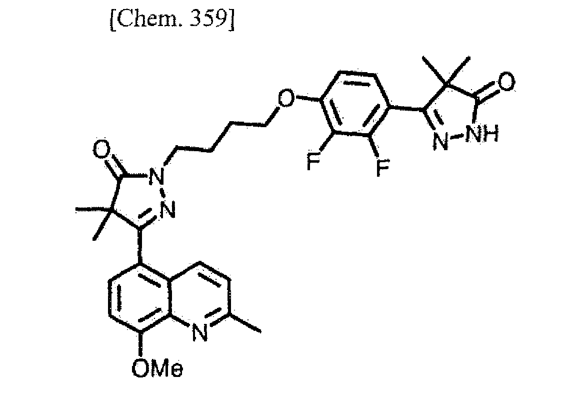

The reaction was carried out in the same manner as in Example 193 using the compound of Example 182 and the compound of Example 155 to obtain the desired product as a colorless amorphous.
Elemental analysis (%): Found value C 64.40%, H 5.77%, N 12.00%, Calculated value as C₃₁H₃₃F₂N₅O₄ C 64.46%, H 5.76%, N 12.12%.
ESIMS (+): 578 [M+H]⁺
¹H-NMR (CDCl₃, 400 MHz): δ 1.40 (6H, d, 1=1.2 Hz), 1.43 (6H, s), 1.92-1.98 (2H, m), 2.01-2.08 (2H, m), 2.81 (3H, s), 3.93 (2H, t, J=6.7 Hz), 4.13 (3H, s), 4.18 (2H, t, J=6.1 Hz), 6.78-6.82 (1H, m), 7.06 (1H, d, J=8.6 Hz), 7.32-7.37 (1H, m), 7.40 (1H, d, J=8.6 Hz), 7.57 (1H, d, J=8.6 Hz), 8.61 (1H, brs), 8.74 (1H, d, J=8.6 Hz).

### <Example 198>

### 5-(2-Ethyl-8-methoxyquinolin-5-yl)-2-[4-[2,3-difluoro-4-(6-oxo-1,4,5,6-tetrahydropyridazin-3-yl)phenoxy]butyl]-4,4-dimethyl-2,4-dihydro-pyrazol-3-one

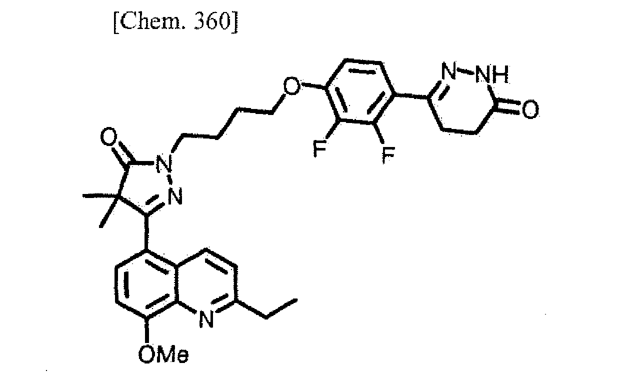

The reaction was carried out in the same manner as in Example 193 using the compound of Example 183 and the compound of Example 138 to obtain the desired product as a colorless amorphous.
HRESIMS (+): 578.25554 (Calculated value as C₃₁H₃₄F₂N₅O₄ 578.25788)
¹H-NMR (CDCl₃, 400 MHz): δ 1.41 (3H, t, J=7.3 Hz), 1.43 (6H, s), 1.93-2.06 (4H, m), 2.57-2.61 (2H, m), 2.96-3.00 (2H, m), 3.09 (2H, q, J=7.3 Hz), 3.93 (2H, t, J=6.7 Hz), 4.13 (3H, s), 4.16 (2H, t, J=6.1 Hz), 6.75-6.80 (1H, m), 7.06 (1H, d, J=8.0 Hz), 7.28-7.32 (1H, m), 7.45 (1H, d, J=8.6 Hz), 7.57 (1H, d, J=8.0 Hz), 8.56 (1H, brs), 8.77 (1H, d, J=8.6 Hz).

### <Example 199>

### 5-(2-Ethyl-8-methoxyquinolin-5-yl)-2-[4-[2-fluoro-4-(6-oxo-1,4,5,6-tetrahydropyridazin-3-yl)phenoxy]butyl]-4,4-dimethyl-2,4-dihydro-pyrazol-3-one

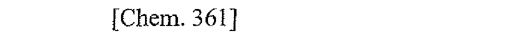

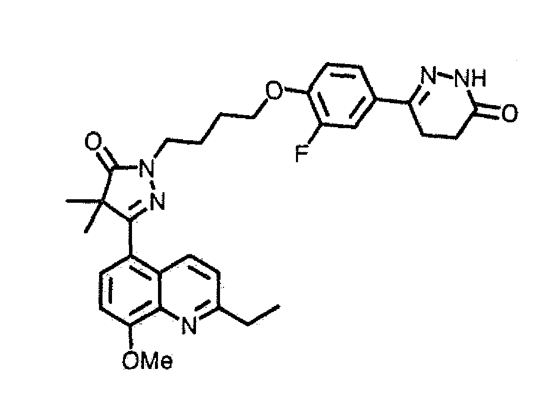

The reaction was carried out in the same manner as in Example 193 using the compound of Example 183 and the compound of Example 128 to obtain the desired product as a colorless amorphous.
HRESIMS (+): 560.26588 (Calculated value as C₃₁H₃₅FN₅O₄ 560.26731)
¹H-NMR (CDCl₃, 400 MHz): δ 1.40 (3H, t, J=7.3 Hz), 1.43 (6H, s), 1.93-2.07 (4H, m), 2.60 (2H, t, J=8.0 Hz), 2.89-2.96 (2H, m), 3.09 (2H, q, J=7.3 Hz), 3.93 (2H, t, J=6.7 Hz), 4.13 (3H, s), 4.15 (2H, t, J=6.1 Hz), 6.96 (1H, dd, J=8.6, 8.6 Hz), 7.06 (1H, d, J=8.0 Hz), 7.34-7.36 (1H, m), 7.44 (1H, d, J=8.6 Hz), 7.50 (1H, dd, J=1.8, 12.9 Hz), 7.57 (1H, d, J=8.0 Hz), 8.55 (1H, brs), 8.77 (1H, d, J=8.6 Hz).

### <Example 200>

### 5-(2-Ethyl-8-methoxyquinolin-5-yl)-2-[4-[4-(6-oxo-1,4,5,6-tetrahydropyridazin-3-yl)phenoxy]butyl]-4,4-dimethyl-2,4-dihydro-pyrazol-3-one

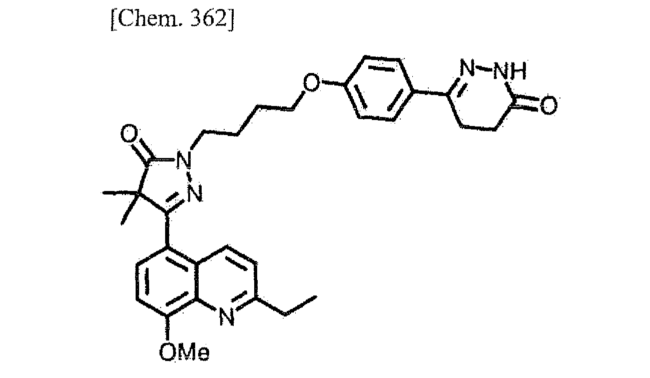

The reaction was carried out in the same manner as in Example 193 using the compound of Example 183 and the compound of Example 100 to obtain the desired product as a colorless amorphous.
HRESIMS (+): 542.27700 (Calculated value as C₃₁H₃₆N₅O₄ 542.27673)
¹H-NMR (CDCl₃, 400 MHz): δ 1.40 (3H, t, J=8.0 Hz), 1.43 (6H, s), 1.89-2.05 (4H, m), 2.59 (2H, t, J=8.0 Hz), 2.95 (2H, t, J=8.0 Hz), 3.08 (2H, q, J=8.0 Hz), 3.92 (2H, t, J=73 Hz), 4.08 (2H, t, J=6.1 Hz), 4.13 (3H, s), 6.90-6.93 (2H, m), 7.06 (1H, d, J=8.0 Hz), 7.42 (1H, d, J=8.6 Hz), 7.57 (1H, d, J=8.0 Hz), 7.62-7.65 (2H, m), 8.42 (1H, brs), 8.76 (1H, d, J=8.6 Hz).

### <Example 201>

### 5-(2-Ethyl-8-methoxyquinolin-5-yl)-2-[4-[4-(4,4-dimethyl-5-oxo-4,5-dihydro-1H-pyrazol-3-yl)phenoxy]buty1]-4,4-dimethyl-2,4-dihydro-pyrazol-3-one

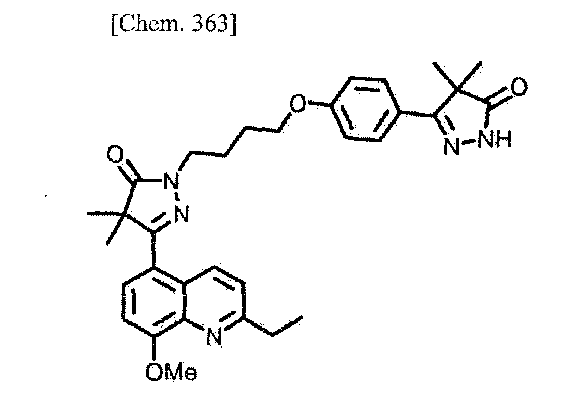

The reaction was carried out in the same manner as in Example 193 using the compound of Example 183 and the compound of Example 107 to obtain the desired product as a colorless amorphous.
HRESIMS (+): 556.28981 (Calculated value as C₃₂H₃₈N₅O₄ 556.29238)
¹H-NMR (CDCl₃, 400 MHz): δ 1.40 (3H, t, J=8.0 Hz), 1.43 (6H, s), 1.50 (6H, s), 1.89-2.05 (4H, m), 3.09 (2H, q, J=8.0 Hz), 3.93 (2H, t, J=6.7 Hz), 4.09 (2H, t, J=6.1 Hz), 4.13 (3H, s), 6.92-6.95 (2H, m), 7.06 (1H, d, J=8.0 Hz), 7.43 (1H, d, J=8.6 Hz), 7.57 (1H, d, J=8.0 Hz), 7.70-7.73 (2H, m), 8.51 (1H, brs), 8.76 (1H, d, J=8.6 Hz).

### <Example 202>

### 5-(2-Ethyl-8-methoxyquinolin-5-yl)-2-[4-[2-fluoro-4-(4,4-dimethyl-5-oxo-4,5-dihydro-1H-pyrazol-3-yl)phenoxy]butyl]-4,4-dimethyl-2,4-dihydro-pyrazol-3-one

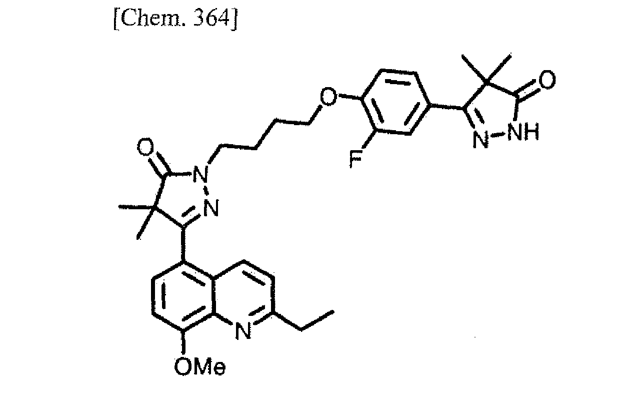

The reaction was carried out in the same manner as in Example 193 using the compound of Example 183 and the compound of Example 142 to obtain the desired product as a colorless amorphous.
HRESIMS (+): 574.28296 (Calculated value as C₃₂H₃₇FN₅O₄ 574.28296)
¹H-NMR (CDCl₃, 400 MHz): δ 1.40 (3H, t, J=8.0 Hz), 1.43 (6H, s), 1.49 (6H, s), 1.93-2.07 (4H, m), 3.09 (2H, q, J=8.0 Hz), 3.93 (2H, t, J=6.7 Hz), 4.13 (3H, s), 4.16 (2H, t, J=6.1 Hz), 6.98 (1H, dd, J=8.6, 8.6 Hz), 7.06 (1H, d, J=8.6 Hz), 7.43-7.45 (2H, m), 7.56 (1H, dd, J=2.5, 12.5 Hz), 7.57 (1H, d, J=8.6 Hz), 8.48 (1H, brs), 8.77 (1H, d, J=8.6 Hz).

### <Example 203>

### 5-(2-Ethyl-8-methoxyquinolin-5-yl)-2-[4-[2,3-difluoro-4-(4,4-dimethyl-5-oxo-4,5-dihydro-1H-pyrazol-3-yl)phenoxy]butyl]-4,4-dimethyl-2,4-dihydro-pyrazol-3-one

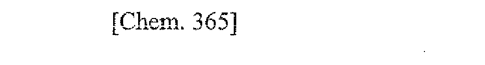

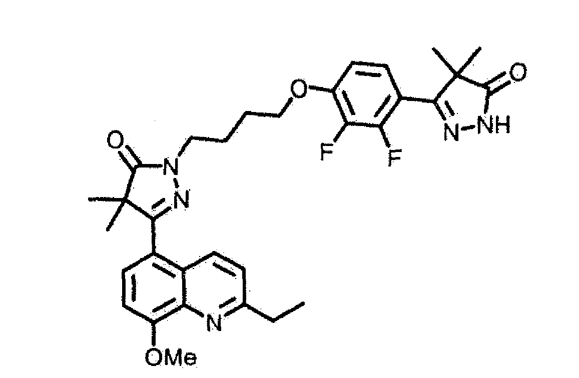

The reaction was carried out in the same manner as in Example 193 using the compound of Example 183 and the compound of Example 155 to obtain the desired product as a pale yellow amorphous.
HRESIMS (+): 592.27552 (Calculated value as C₃₂H₃₆F₂N₅O₄ 592.27353)
¹H-NMR (CDCl₃, 400 MHz): δ 1.40 (6H, s), 1.41 (3H, t, J=7.3 Hz), 1.43 (6H, s), 1.93-2.47 (4H, m), 3.09 (2H, q, J=7.3 Hz), 3.93 (2H, t, J=6.7 Hz), 4.13 (3H, s), 4.18 (2H, t, J=6.1 Hz), 6.78-6.83 (1H, m), 7.06 (1H, d, J=8.0 Hz), 7.32-7.35 (1H, m), 7.45 (1H, d, J=8.6 Hz), 7.57 (1H, d, J=8.0 Hz), 8.65 (1H, brs), 8.77 (1H, d, J=8.6 Hz).

### <Example 204>

### 5-(2-Ethyl-8-mcthoxyquinolin-5-yl)-2-[4-[2-methoxy-4-(4,4-dimethyl-5-oxo-4,5-dihydro-1H-pyrazol-3-yl)phenoxy]butyl]-4,4-dimethyl-2,4-dihydro-pyrazol-3-one

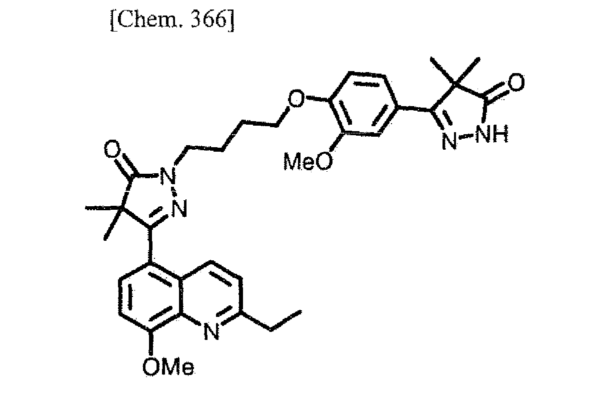

The reaction was carried out in the same manner as in Example 193 using the compound of Example 183 and the compound of Example 173 to obtain the desired product as a pale yellow powder.
HRESIMS (+): 586.29994 (Calculated value as C₃₃H₄₀N₅O₅ 586.30294)
¹H-NMR (CDCl₃, 400 MHz): δ 1.40 (3H, t, J=7.3 Hz), 1.43 (6H, s), 1.51 (6H, s), 1.97-2.05 (4H, m), 3.08 (2H, q, J=7.3 Hz), 3.88 (3H, s), 3.93 (2H, t, J=6.7 Hz), 4.12 (2H, t, J=6.7 Hz), 4.13 (3H, s), 6.88 (1H, d, J=8.6 Hz), 7.06 (1H, d, J=8.6 Hz), 7.22 (1H, dd, J=1.8, 8.6 Hz), 7.42 (1H, d, J=8.6 Hz), 7.43 (1H, d, J=1.8 Hz), 7.56 (1H, d, J=8.6 Hz), 8.49 (1H, brs), 8.77 (1H, d, J=8.6 Hz).

### <Example 205>

### 5-(2-Ethyl-8-methoxyquinolin-5-yl)-2-[4-[2-fluoro-4-(4-methyl-6-oxo-1,4,5,6-tetrahydropyridazin-3-yl)phenoxy]butyl]-4,4-dimethyl-2,4-dihydro-pyrazol-3-one

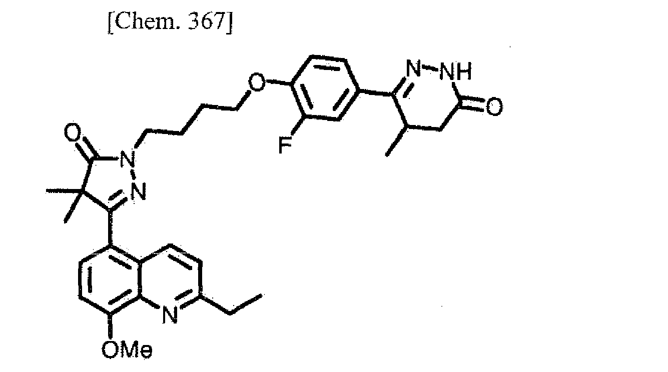

The reaction was carried out in the same manner as in Example 193 using the compound of Example 183 and the compound of Example 119 to obtain the desired product as a colorless amorphous.
HRESIMS(+): 574.28011 (Calculated value as C₃₂H₃₇FN₅O₄ 574.28296)
¹H-NMR (CDCl₃, 400 MHz): δ 1.23 (3H, d, J=7.3 Hz), 1.26 (3H, t, J=8.0 Hz), 1.43 (6H, s), 1.93-2.07 (4H, m), 2.46 (1H, d, J=16.5 Hz), 2.70 (1H, dd, J=6.7, 16.5 Hz), 3.09 (2H, q, J=8.0 Hz), 3.24-3.29 (1H, m), 3.93 (2H, t, J=6.7 Hz), 4.13 (3H, s), 4.16 (2H, t, J=6.1 Hz), 6.97 (1H, dd, J=8.6, 8.6 Hz), 7.06 (1H, d, J=8.0 Hz), 7.39 (1H, d, J=8.6 Hz), 7.44 (1H, d, J=8.6 Hz), 7.53 (1H, dd, J=1.8, 12.3 Hz), 7.57 (1H, d, J=8.0 Hz), 8.49 (1H, brs), 8.77 (1H, d, J=8.6 Hz).

### <Examples 206 and 207>

### (+)-5-(2-Ethyl-8-methoxyquinolin-5-yl)-2-[4-[2-fluoro-4-(4-methyl-6-oxo-1,4,5,6-tetrahydropyridazin-3-yl)phenoxy]butyl]-4,4-dimethyl-2,4-dihydro-pyrazol-3-one and (-)-5-(2-ethyl-8-methoxyquinolin-5-yl)-2-[4-[2-fluoro-4-(4-methyl-6-oxo-1,4,5,6-tetrahydropyridazin-3-yl)phenoxy]butyl]-4,4-dimethyl-2,4-dihydro-pyrazol-3-one

The compound of Example 205 was subjected to resolution by HPLC (Daicel Chiralpak AS-H column, Eluent: 100% EtOH, Flow: 3.0 mL/min, Detection: UV 293 nm) to obtain a (+) form (Example 206) and a (-) form (Example 207), respectively, as a colorless amorphous.
Example 206 [α]_{D}²³=+208 (c 0.47, CHCl₃)
Example 207 [α]_{D}²³=-197 (c 0.47, CHCl₃)

### <Example 208>

### 5-(2-Ethyl-8-methoxyquinolin-5-yl)-2-[4-[2-methoxy-4-(4-methyl-6-oxo-1,4,5,6-tetrahydropyridazin-3-yl)phenoxy]butyl]-4,4-dimethyl-2,4-dihydro-pyrazol-3-one

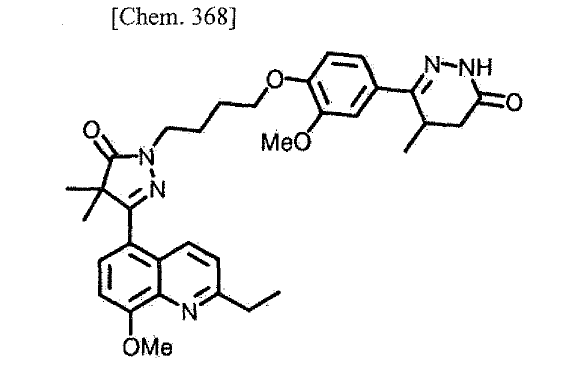

The reaction was carried out in the same manner as in Example 193 using the compound of Example 183 and the compound of Example 126 to obtain the desired product as a colorless powder.
HRESIMS(+): 586.30159 (Calculated value as C₃₃H₄₀N₅O₅ 586.30294)
¹H-NMR (CDCl₃, 400 MHz): δ 1.24 (3H, d, J=7.3 Hz), 1.40 (3H, t, J=7.3 Hz), 1.43 (6H, s), 1.97-2.04 (4H, m), 2.46 (1H, d, J=15.9 Hz), 2.70 (1H, dd, J=6.7, 15.9 Hz), 3.08 (2H, q, J=7.3 Hz), 3.30-3.35 (1H, m), 3.88 (3H, s), 3.92 (2H, t, J=6.7 Hz), 4.13 (3H, s), 4.14 (2H, t, J=6.1 Hz), 6.88 (1H, d, J=8.6 Hz), 7.03 (1H, d, J=8.6 Hz), 7.15 (1H, dd, J=2.5, 8.6 Hz), 7.41 (1H, d, J=9.2 Hz), 7.42 (1H, d, J=2.5 Hz), 7.56 (1H, d, J=8.6 Hz), 8.43 (1H, brs), 8.76 (1H, d, J=9.2 Hz).

### <Example 209>

### 5-(2-Ethyl-8-methoxyquinolin-5-yl)-2-[4-[2,3-difluoro-4-(4-methyl-6-oxo-1,4,5,6-tetrahydropyridazin-3-yl)phenoxy]butyl]-4,4-dimethyl-2,4-dihydro-pyrazol-3-one

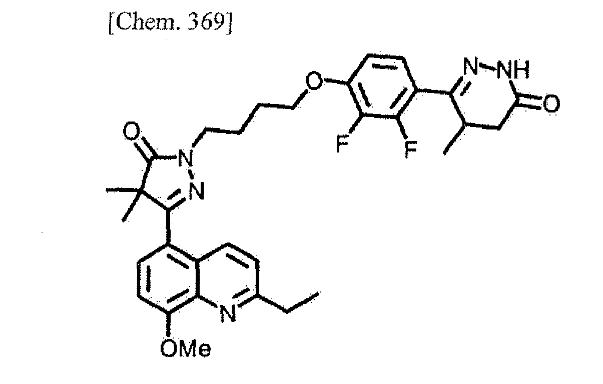

The reaction was carried out in the same manner as in Example 193 using the compound of Example 183 and the compound of Example 176 to obtain the desired product as a colorless amorphous.
HRESIMS(+): 592.27853 (Calculated value as C₃₂H₃₆F₂N₅O₄ 592.27353)
¹H-NMR (CDCl₃, 400 MHz): δ 1.20 (3H, d, J=7.3 Hz), 1.41 (3H, t, J=7.3 Hz), 1.43 (6H, s), 1.93-2.06 (4H, m), 2.44 (1H, dd, J=3.0, 17.1 Hz), 2.74 (1H, dd, J=6.7, 17.1 Hz), 3.09 (2H, q, J=7.3 Hz), 3.23-3.27 (1H, m), 3.93 (2H, t, J=6.7 Hz), 4.13 (3H, s), 4.17 (2H, t, J=6.1 Hz), 6.76-6.80 (1H, m), 7.06 (1H, d, J=8.6 Hz), 7.26-7.30 (1H, m), 7.45 (1H, d, J=9.2 Hz), 7.57 (1H, d, J=8.6 Hz), 8.52 (1H, brs), 8.77 (1H, d, J=9.2 Hz).

### <Examples 210 and 211 >

### (+)-5-(2-Ethyl-8-methoxyquinolin-5-yl)-2-[4-[2,3-difluoro-4-(4-methyl-6-oxo-1,4,5,6-tetrahydropyridazin-3-yl)phenoxy]butyl]-4,4-dimethyl-2,4-dihydro-pyrazol-3-oneand (-)-5-(2-ethyl-8-methoxyquinolin-5-yl)-2-[4-[2,3-difluoro-4-(4-methyl-6-oxo-1,4,5,6-tetrahydropyridazin-3-yl)phenoxy]butyl]-4,4-dimethyl-2,4-dihydro-pyrazol-3-one

The compound of Example 209 was subjected to resolution by HPLC (Daicel Chiralpak AS-H column, Eluent: 100% EtOH, Flow: 3.0 mL/min, Detection: UV 293 nm) to obtain a (+) form (Example 210) and a (-) form (Example 211), respectively, as a colorless amorphous.
Example 210 [α]_{D}²³=+91.2 (c 0.53, CHCl₃)
Example 211 [α]_{D}²³=-76.1 (c 0.64, CHCl₃)

### <Example 212>

### 5-(8-Methoxyquinolin-5-yl)-2-[4-[4-(4-methyl-6-oxo-1,4,5,6-tetrahydropyridazin-3-yl)phenoxy]butyl]-4,4-dimethyl-2,4-dihydro-pyrazol-3-one

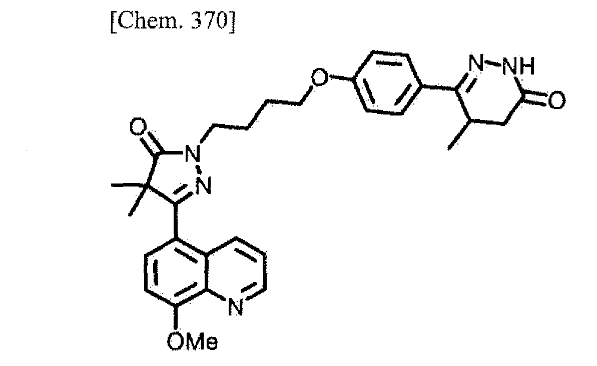

The reaction was carried out in the same manner as in Example 193 using the compound of Example 185 and the compound of Example 92 to obtain the desired product as a white solid.
HRESIMS (+): 528.26308 (Calculated value as C₃₀H₃₄N₅O₄ 528.26108)
¹H-NMR (CDCl₃, 400 MHz): δ 1.13 (3H, d, J=6.1 Hz), 1.43 (6H, s), 1.88-1.93 (2H, m), 2.00-2.06 (2H, m), 2.45 (1H, dd, J=15.9, 1.2 Hz), 2.71 (1H, dd, J=15.9, 6.7 Hz), 3.29-3.34 (1H, m), 3.93 (2H, t, J=6.7 Hz), 4.08 (2H, t, J=6.1 Hz), 4.15 (3H, s), 6.92 (2H, d, J=8.6 Hz), 7.09 (1H, d, J=8.6 Hz), 7.50 (1H, dd, J=8.6, 4.3 Hz), 7.65 (1H, d, J=8.6 Hz), 7.67 (2H, d, J,8.6 Hz), 8.39 (1H, brs), 8.89 (1H, dd, J=8.6, 1.8 Hz), 8.97 (1H, dd, J=4.3, 1.8 Hz).

### <Example 213>

### 2-[4-[2,3-Difluoro-4-(4,4-dimethyl-5-oxo-4,5-dihydro-1H-pyrazol-3-yl)phenoxy]butyl]-5-(8-methoxyquinolin-5-yl)-4,4-dimethyl-2,4-dihydro-pyrazol-3-one

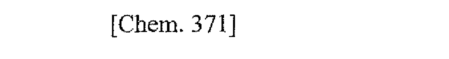

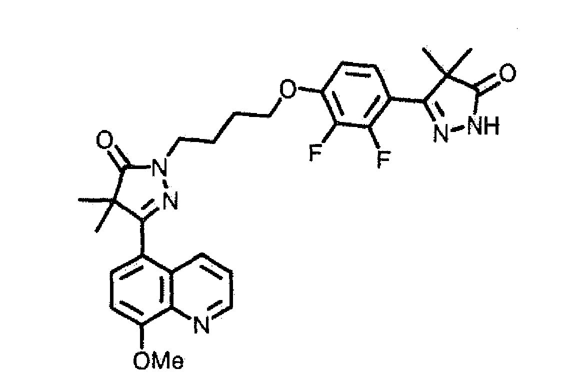

The reaction was carried out in the same manner as in Example 193 using the compound of Example 185 and the compound of Example 155 to obtain the desired product as a white amorphous solid.
HRESIMS (+): 564.24379 (Calculated value as C₃₀H₃₂F₂N₅O₄ 564.24223)
¹H-NMR (CDCl₃, 400 MHz): δ 1.40 (6H, s), 1.45 (6H, s), 1.92-1.99 (2H, m), 2.02-2.09 (2H, m), 3.94 (2H, t, J=6.7 Hz), 4.15 (3H, s), 4.18 (2H, t, J=6.1 Hz), 6.78-6.82 (1H, m), 7.09 (1H, d, J=8.6 Hz), 7.31-7.35 (1H, m), 7.53 (1H, dd, J=8.6, 4.3 Hz), 7.65 (1H, d, J=8.6 Hz), 8.62 (1H, brs), 8.90 (1H, dd, J=8.6, 1.8 Hz), 8.99 (1H, dd, J=4.3, 1.8 Hz).

### <Example 214>

### 2-[4-[2,3-Difluoro-4-(4-methyl-6-oxo-1,4,5,6-tetrahydropyridazin-3-yl)phenoxy]butyl]-5-(8-methoxyquinolin-5-yl)-4,4-dimethyl-2,4-dihydro-pyrazol-3-one

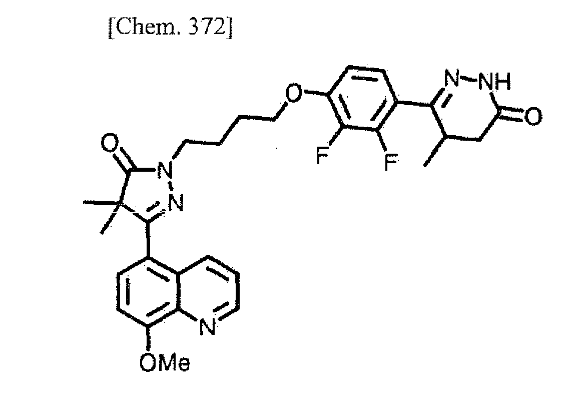

The reaction was carried out in the same manner as in Example 193 using the compound of Example 185 and the compound of Example 176 to obtain the desired product as a pale yellow amorphous solid.
HRESIMS (+): 564.24110 (Calculated value as C₃₀H₃₂F₂N₅O₄ 564.24223).
¹H-NMR (CDCl₃, 400 MHz): δ 1.20 (3H, d, J=7.3 Hz), 1.44 (6H, s), 1.93-1.98 (2H, m), 2.01-2.07 (2H, m), 2.44 (1H, dd, J=17.1, 3.1 Hz), 2.74 (1H, dd, J=17.1, 6.7 Hz), 3.24-3.28 (1H, m), 3.94 (2H, t, J=6.7 Hz), 4.13-4.17 (2H, m), 4.15 (3H, s), 6.76-6.80 (1H, m), 7.09 (1H, d, J=8.6 Hz), 7.26-7.30 (1H, m), 7.52 (1H, dd, J=7.9, 4.3 Hz), 7.65 (1H, d, J=7.9 Hz), 8.52 (1H, brs), 8.90 (1H, dd, J=8.6, 1.5 Hz), 8.99 (1H, dd, J=4.3, 1.5 Hz).

### <Example 215>

### 2-[4-[2-Fluoro-4-(4-methyl-6-oxo-1,4,5,6-tetrahydropyridazin-3-yl)phenoxy]butyl]-5-(8-methoxyquinolin-5-yl)-4,4-dimethyl-2,4-dihydro-pyrazol-3-one

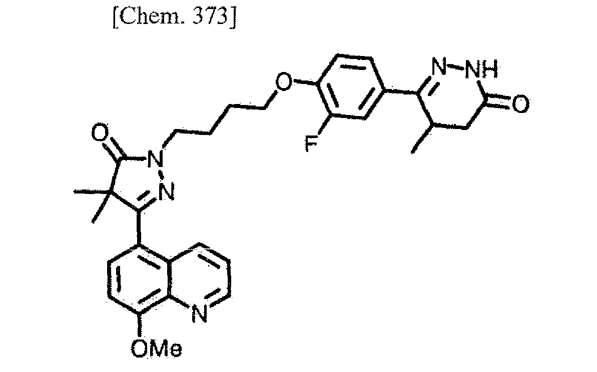

The reaction was carried out in the same manner as in Example 193 using the compound of Example 185 and the compound of Example 119 to obtain the desired product as a pale yellow amorphous solid.
HRESIMS (+): 546.24899 (Calculated value as C₃₀H₃₃FN₅O₄ 546.25166).
¹H-NMR (CDCl₃, 400 MHz): δ 1.23 (3H, d, J=7.3 Hz), 1.44 (6H, s), 1.92-1.98 (2H, m), 2.02-2.07 (2H, m), 2.46 (1H, d, J=16.5 Hz), 2.70 (1H, dd, J=16.5, 6.7 Hz), 3.23-3.30 (1H, m), 3.94 (2H, t, J=6.7 Hz), 4.11-4.17 (2H, m), 4.15 (3H, s), 6.97 (1H, t, J=8.6 Hz), 7.09 (1H, d, J=8.6 Hz), 7.39 (1H, d, J=8.6 Hz), 7.50-7.55 (2H, m), 7.65 (1H, d, J=8.6 Hz), 8.49 (1H, brs), 8.90 (1H, dd, J=8.6, 1.8 Hz), 8.98 (1H, dd, J=4.3, 1.8 Hz).

### <Example 216>

### 2-[4-[2,3-Difluoro-4-(6-oxo-1,4,5,6-tetrahydropyridazin-3-yl)phenoxy]butyl]-5-(7-methoxy-2-trifluoromethylbenzofuran-4-yl)-4,4-dimethyl-2,4-dihydro-pyrazol-3-one

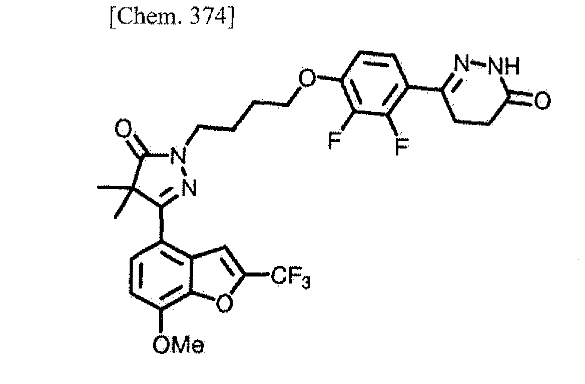

The reaction was carried out in the same manner as in Example 193 using the compound of Example 186 and the compound of Example 138 to obtain the desired product as a colorless powder.
Elemental analysis: Found value C 57.27%, H 4.39%, N 9.21 %, Calculated value as C₂₉H₂₇F₅N₄O₅ 57.43%, H 4.49%, N 9.24%.
ESIMS (+): 607 [M+H)⁺.
¹H-NMR (CDCl₃, 400 MHz): δ 1.55 (6H, s), 1.90-1.94 (2H, m), 2.01-2.06 (2H, m), 2.56-2.60 (2H, m), 2.95-2.99 (2H, m), 3.92 (2H, t, J=6.7 Hz), 4.08 (3H, s), 4.16 (2H, t, J=6.1 Hz), 6.74-6.78 (1H, m), 6.93 (1H, d, J=8.6 Hz), 7.28-7.31 (1H, m), 7.51 (1H, d, J=8.6 Hz), 7.94 (1H, d, J=1.2 Hz), 8.49 (1H, brs).

### <Example 217>

### 2-[4-[2,3-Difluoro-4-(4,4-dimethyl-5-oxo-4,5-dihydro-1H-pyrazol-3-yl)phenoxy]butyl]-5-(7-methoxy-2-trifluoromethylbenzofuran-4-yl)-4,4-dimethyl-2,4-dihydro-pyrazol-3 -one

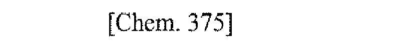

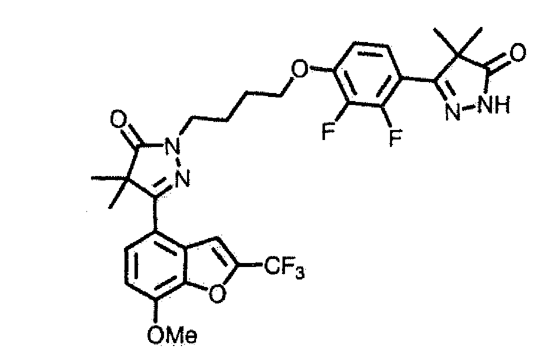

The reaction was carried out in the same manner as in Example 193 using the compound of Example 186 and the compound of Example 155 to obtain the desired product as a colorless powder.
Elemental analysis: Found value C 57.90,% H 4.69%, N 8.92%, Calculated value as C₃₀H₂₉F₅N₄O₅ C 58.06%, H 4.71%, N 9.03%.
ESIMS(+): 621 [M+H]⁺.
¹H-NMR (CDCl₃, 400 MHz): δ 1.40 (6H, s), 1.56 (6H, s), 1.92-2.07 (4H, m), 3.92 (2H, t, J=6.7 Hz), 4.08 (3H, s), 4.17 (2H, t, J=6.1 Hz), 6.79-6.81 (1H, m), 6.93 (1H, d, J=8.6 Hz), 7.31-7.36 (1H, m), 7.51 (1H, d, J=8.6 Hz), 7.95 (1H, d, J=1.2 Hz), 8.53 (1H, brs).

### <Example 218>

### 2-[4-[2,3-Difluoro-4-(4-methyl-6-oxo-1,4,5,6-tetrahydropyridazin-3-yl)phenoxy]butyl]-5-(7-methoxy-2-trifluoromethylbcnzofuran-4-yl)-4,4-dimethyl-2,4-dihydro-pyrazol-3-one

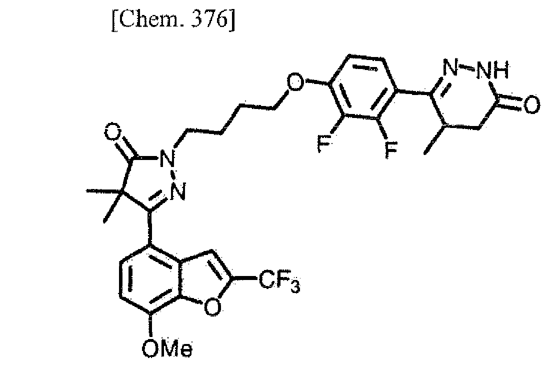

The reaction was carried out in the same manner as in Example 193 using the compound of Example 186 and the compound of Example 176 to obtain the desired product as a colorless powder.
Elemental analysis: Found value C 57.68%, H 4.57%, N 8.95%, Calculated value as C₃₀H₂₉F₅N₄O₅ C 58.06%, H 4.71%, N 9.03%.
¹H-NMR (CDCl₃, 400 MHz): δ 1.24 (3H, d, J=6.7 Hz), 1.55 (6H, s), 1.91-2.06 (4H, m), 2.44 (1H, dd, J=3.1, 17.1 Hz), 2.74 (1H, d, J=6.7, 17.1 Hz), 3.23-3.27 (1H, m), 3.92 (2H, t, J=6.7 Hz), 4.08 (3H, s), 4.16 (2H, t, J=6.1 Hz), 6.75-6.79 (1H, m), 6.93 (1H, d, J=8.6 Hz), 7.25-7.30 (1H, m), 7.51 (1H, d, J=8.6 Hz), 7.94 (1H, d, J=1.2 Hz), 8.46 (1H, brs).

### <Example 219>

### 2-[4-[2,3-Difluoro-4-(6-oxo-1,4,5,6-tetrahydropyridazin-3-yl)phenoxy]butyl]-5-(4-methoxy-2-trifluoromethyl-1H-benzo[d]imidazol-7-yl)-4,4-dimethyl-2,4-dihydro-pyrazol-3-one

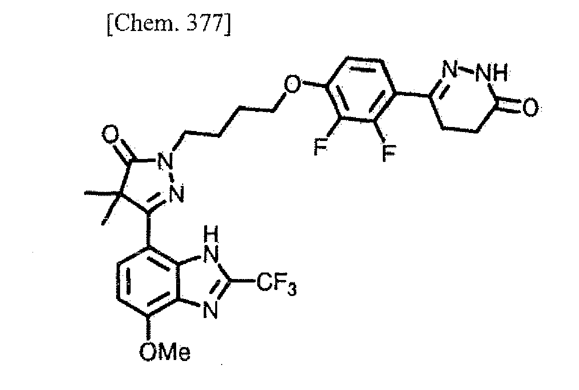

The reaction was carried out in the same manner as in Example 193 using the compound of Example 187 and the compound of Example 13 8, and then the reaction was carried out in the same manner as in Example 75 to obtain the desired product as a colorless powder.
Elemental analysis: Found value C 55.15%, H 4.43%, N 13.70%, Calculated value as C₂₈H₂₇F₅N₆O₄ C 55.45%, H 4.49%, N 13.86%.
ESIMS (+): 607 [M+H]⁺.
¹H-NMR (CDCl₃, 400 MHz): δ 1.59 (6H, s), 1.92-2.08 (4H, m), 2.56-2.61 (2H, m), 2.94-2.98 (2H, m), 3.96 (2H, t, J=6.7 Hz), 4.11 (3H, s), 4.17 (2H, t, J=6.1 Hz), 6.73-6.78 (1H, m), 6.80 (1H, d, J=8.6 Hz), 7.27-7.32 (1H, m), 7.62 (1H, d, J=8.6 Hz), 8.51 (1H, brs), 11.20 (1H, brs).

### <Example 220>

### 2-[4-[2,3-Difluoro-4-(4,4-dimethyl-5-oxo-4,5-dihydro-1H-pyrazol-3-yl)phenoxy]butyl]-5-(4-methoxy-2-trifluoromethyl-1H -benzo[d]imidazol-7-yl)-4,4-dimethyl-2,4-dihydro-pyrazol-3 -one

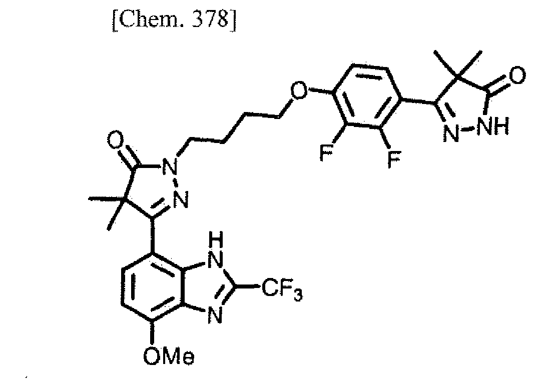

The reaction was carried out in the same manner as in Example 193 using the compound of Example 187 and the compound of Example 155, and then the reaction was carried out in the same manner as in Example 75 to obtain the desired product as a colorless powder.
Elemental analysis: Found value C 55.97%, H 4.76%, N 13.25%, Calculated value as C₂₉H₂₉F₅N₆O₄ C 56.13%, H 4.71%, N 13.54%.
ESIMS (+): 621 [M+H]⁺.
¹H-NMR (CDCl₃, 400 MHz): δ 1.39 (6H, s), 1.59 (6H, s), 1.93-2.07 (4H, m), 3.96 (2H, t, J=6.7 Hz), 4.12 (3H, s), 4.18 (2H, t, J=6.1 Hz), 6.77-6.81 (1H, m), 6.80 (1H, d, J=8.6 Hz), 7.32-7.37 (1H, m), 7.62 (1H, d, J=8.6 Hz), 8.51 (1H, brs), 11.21 (1H, brs).

### <Example 221>

### 2-[4-[2,3-Difluoro-4-(4-methyl-6-oxo-1,4,5,6-tetrahydropyridazin-3-yl)phenoxy]butyl]-5-(4-methoxy-2-trifluoromethyl-1H-benzo[d]imidazol-7-yl)-4,4-dimethyl-2,4-dihydro-pyrazol-3-one

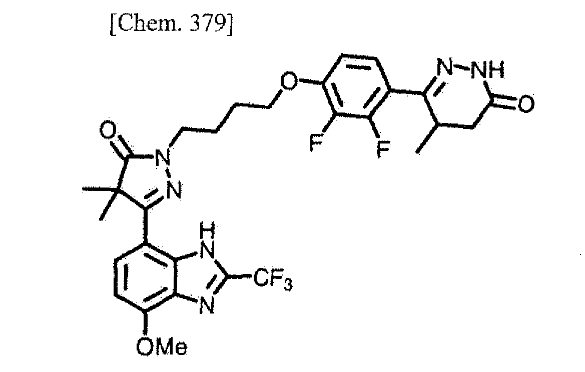

The reaction was carried out in the same manner as in Example 193 using the compound of Example 187 and the compound of Example 176, and then the reaction was carried out in the same manner as in Example 75 to obtain the desired product as a colorless powder.
Elemental analysis: Found value C 55.80%, H 4.62%, N 13.45%, Calculated value as C₂₉H₂₉F₅N₆O₄ C 56.13%, H 4.71%, N 13.54%.
ESIMS (+): 621 [M+H]⁺.
¹H-NMR (CDCl₃, 400 MHz): δ 1.20 (3H, d, J=6.7 Hz), 1.60 (6H, s), 1.92-2.07 (4H, m), 2.44 (1H, dd, J=3.1, 17.1 Hz), 2.73 (1H, dd, J=6.7, 17.1 Hz), 3.23-3.26 (1H, m), 3.96 (2H, t, J=6.7 Hz), 4.11 (3H, s), 4.17 (2H, t, J=6.1 Hz), 6.74-6.77 (1H m), 6.80 (1H, d, J=8.6 Hz), 7.26-7.31 (1H, m), 7.62 (1H, d, J=8.6 Hz), 8.54 (1H, brs), 11.21 (1H, brs).

### <Example 222>

### 2-[4-[2,3-Difluoro-4-(6-oxo-1,4,5,6-tetrahydropyridazin-3-yl)phenoxy]butyl]-5-(7-methoxy-2-trifluoromethylbenzo[b]thiophen-4-yl)-4,4-dimethyl-2,4-dihydro-pyrazol-3-one

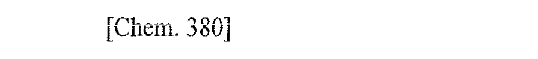

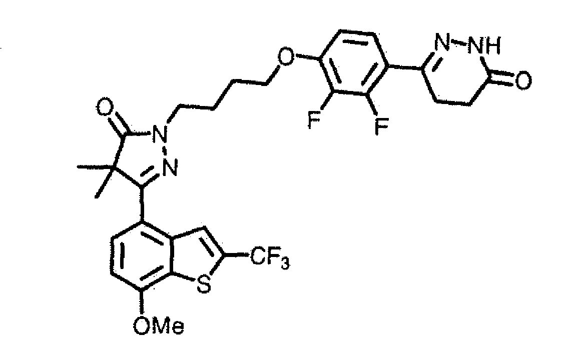

The reaction was carried out in the same manner as in Example 193 using the compound of Example 188 and the compound of Example 138 to obtain the desired product as a colorless powder.
Melting point 176-177°C
Elemental analysis: Found value C 55.65 %, H 4.35%, N 8.66%, Calculated value as C₂₉H₂₇F₅N₄O₄S C 55.94 %, H 4.37%, N 9.00%.
HRESIMS(+): 623.17615 (Calculated value as C₂₉H₂₇F₅N₄O₄S 623.17614)
¹H-NMR (400 MHz, CDCl₃): δ 1.54 (6H, s), 1.92-2.06 (4H, m), 2.56-2.60 (2H, m), 2.95-2.99 (2H, m), 3.93 (2H, t, J=6.7 Hz), 4.07 (3H, s), 4.16 (2H, t, J=6.1 Hz), 6.74-6.79 (1H, m), 6.89 (1H, d, J=8.6 Hz), 7.27-7.31 (1H, m), 7.68 (1H, d, J=8.6 Hz), 8.53 (1H, brs), 8.70 (1H, d, J=1.2 Hz).

### <Example 223>

### 2-[4-[2,3-Difluoro-4-(4,4-dimethyl-5-oxo-4,5-dihydro-1H-pyrazol-3-yl)phenoxy]butyl]-5-(7-methoxy-2-trifluoromethylbenzo[b]thiophen-4-yl)-4,4-dimethyl-2,4-dihydro-pyrazol-3-one

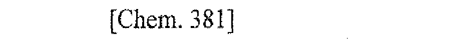

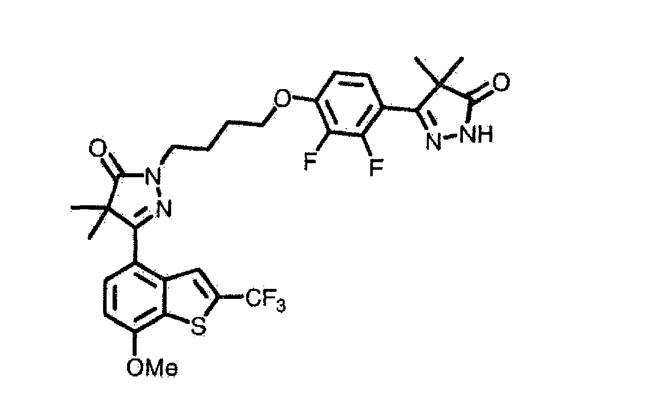

The reaction was carried out in the same manner as in Example 193 using the compound of Example 188 and the compound of Example 155 to obtain the desired product as a colorless powder.
Melting point 167-168°C
Elemental analysis: Found value C 56.35 %, H 4.64%, N 8.43%, Calculated value as C₃₀H₂₉F₅N₄O₄S C 56.60 %, H 4.59%, N 8.80%.
HRESIMS(+): 637. 18990 (Calculated value as C₃₀H₃₀F₅N₄O₄S 637.19178).
¹H-NMR (400 MHz, CDCl₃) δ 1.395 (3H, s), 1.398 (3H, s), 1.55 (6H, s), 1.93-2.07 (4H, m), 3.94 (2H, t, J=6.7 Hz), 4.07 (3H, s), 4.17 (2H, t, J=6.1 Hz), 6.77-6.82 (1H, m), 6.90 (1H, d, J=8.6 Hz), 7.30-7.33 (1H, m), 7.68 (1H, d, J=8.6 Hz), 8.57 (1H, brs), 8.70 (1H, d, J=1.2 Hz).

### <Example 224>

### 2-[4-[2,3-Difluoro-4-(4-methyl-6-oxo-1,4,5,6-tetrahydropyridazin-3-yl)phenoxy]butyl]-5-(7-methoxy-2-trifluoromethylbenzo[b]thiophen-4-yl)-4,4-dimethyl-2,4-dihydro-pyrazol-3-one

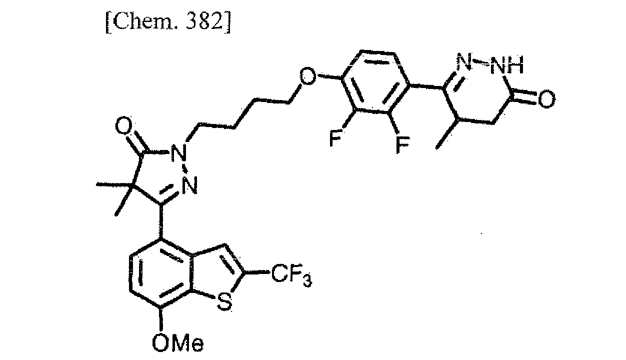

The reaction was carried out in the same manner as in Example 193 using the compound of Example 188 and the compound of Example 176 to obtain the desired product as a colorless powder.
Melting point 137-138°C
Elemental analysis: Found value C 56.39%, H 4.45%, N 8.72%, Calculated value as C₃₀H₂₉F₅N₄O₄S C 56.60 %, H 4.59%, N 8.80%.
HREIMS(+): 637. 119178 (Calculated value as C₃₀H₃₀F₅N₄O₄S 637.19178).
¹H-NMR (400 MHz, CDCl₃) δ 1.20 (3H, d, J=7.3 Hz), 1.54 (6H, s), 1.93-2.07 (4H, m), 2.44 (1H, dd, J=3.1, 17.1 Hz), 2.74 (1H, dd, J=6.7, 17.1 Hz), 3.23-3.27 (1H, m), 3.94 (2H, t, J=6.7 Hz), 4.07 (3H, s), 4.16 (2H, t, J=6.1 Hz), 6.75-6.79 (1H, m), 6.90 (1H, d, J=8.6 Hz), 7.24-7.29 (1H, m), 7.68 (1H, d, J=8.6 Hz), 8.46 (1H, brs), 8.70 (1H, t, J=1.2 Hz).

### <Example 225>

### 2-[4-[2,3-Difluoro-4-(4-methyl-6-oxo-1,4,5,6-tetrahydropyridazin-3-yl)phenoxy]butyl]-5-(5-methoxy-2-trifluoromethyl-[1,2,4]triazolo[1,5-a]pyridin-8-yl)-4,4-dimethyl-2,4-dihydro-pyrazol-3-one

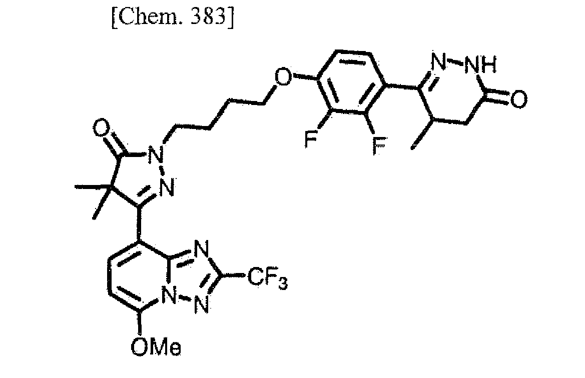

The reaction was carried out in the same manner as in Example 193 using the compound of Example 189 and the compound of Example 176 to obtain the desired product as a yellow amorphous.
HRESIMS(+): 622.22123 (Calculated value as C₂₈H₂₉F₅N₇O₄ 622.22012)
¹H-NMR (CDCl₃, 400 MHz): δ 1.22 (3H, d, J=6.7 Hz), 1.64 (6H, s), 1.95-2.01 (4H, m), 2.46 (1H, dd, J=3.1, 17.1 Hz), 2.75 (1H, dd, J=6.7, 17.1 Hz), 3.25-3.27 (1H, m), 3.92 (2H, t, 1=6.7 Hz), 4.18 (2H, t, J=6.1 Hz), 4.29 (3H, s), 6.59 (1H, d, J=8.6 Hz), 6.77-6.82 (1H, m), 7.29-7.30 (1H, m), 8.29 (1H, d, J=8.6 Hz), 8.48 (1H, s).

### <Example 226>

### 2-[4-[2,3-Difluoro-4-(6-oxo-1,4,5,6-tetrahydropyridazin-3-yl)phenoxy]butyl]-5-(5-methoxy-2-trifluoromethyl-[1,2,4]triazolo[1,5-a]pyridin-8-yl)-4,4-dimethyl-2,4-dihydro-pyrazol-3-one

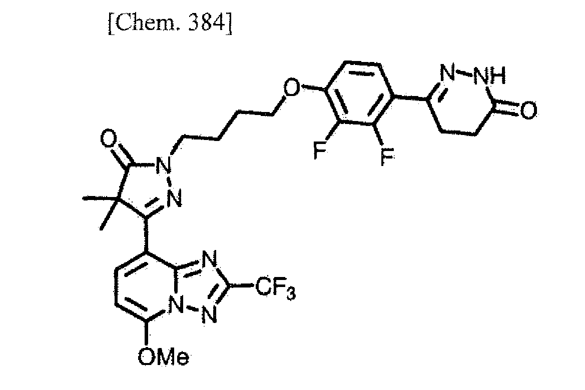

The reaction was carried out in the same manner as in Example 193 using the compound of Example 189 and the compound of Example 138 to obtain the desired product as a yellow amorphous.
HREIMS(+): 608.20550 (Calculated value as C₂₇H₂₇F₅N₇O₄ 608.20447)
¹H-NMR (CDCl₃, 400 MHz): δ 1.63 (6H, s), 1.89-2.06 (4H, m), 2.59 (2H, t, J=7.9 Hz), 2.97-2.99 (2H, m), 3.90 (2H, t, J=6.4 Hz), 4.16 (2H, t, J=6.1 Hz), 4.28 (3H, s), 6.57 (1H, d, J=8.6 Hz), 6.77-6.79 (1H, m), 7.29-7.31 (1H, m), 8.28 (1H, d, J=8.6 Hz), 8.47 (1H, s).

### <Example 227>

### 2-[4-[2,3-Difluoro-4-(4,4-dimethyl-5-oxo-4,5-dihydro-1H-pyrazol-3-yl)phenoxy]butyl]-5-(5-methoxy-2-trifluoromethyl-[1,2,4]triazolo[1,5-a]pyridin-8-yl)-4,4-dimethyl-2,4-dihydro-pyrazol-3-one

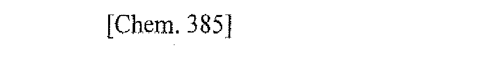

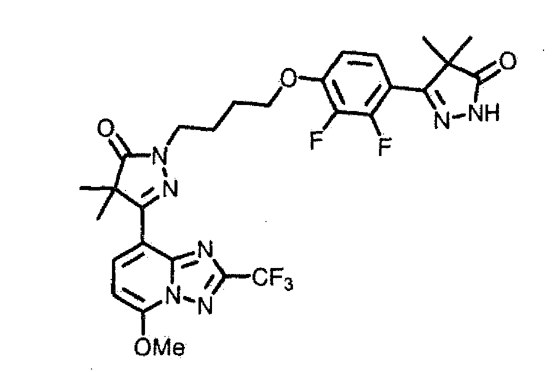

The reaction was carried out in the same manner as in Example 193 using the compound of Example 189 and the compound of Example 155 to obtain the desired product as a yellow amorphous.
HRESIMS(+): 622.21842 (Calculated value as C₂₈H₂₉F₅N₇O₄ 622.22012)
¹H-NMR (CDCl₃, 400 MHz): δ 1.39 (6H, s), 1.63 (6H, s), 1.95-2.01 (4H, m), 3.91 (2H, t, J=6.7 Hz), 4.18 (2H, t, J=6.7 Hz), 4.28 (3H, s), 6.57 (1H, d, J=8.6 Hz), 6.79-6.82 (1H, m), 7.30-7.37 (1H, m), 8.28 (1H, d, J=8.6 Hz), 8.44 (1H, s).

### <Example 228>

### 2-[4-[2,3-Difluoro-4-(4-methyl-6-oxo-1,4,5,6-tetrahydropyridazin-3-yl)phenoxy]butyl]-5-(4-methoxy-2-trifluoromethylbenzo[d]thiazol-7-yl)-4,4-dimethyl-2,4-dihydro-pyrazol-3-one

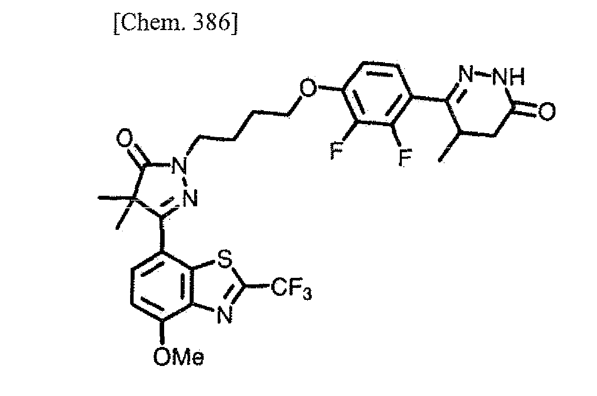

The reaction was carried out in the same manner as in Example 193 using the compound of Example 190 and the compound of Example 176 to obtain the desired product as a white powder.
Elemental analysis: Found value C 54.35%, H 4.21%, N 10.91%, Calculated value as C₂₉H₂₈F₅N₅O₄S C 54.63%, H 4.43%, N 10.98%.
¹H-NMR (CDCl₃, 400 MHz): δ 1.20 (3H, d, J=7.3 Hz), 1.59 (6H, s), 1.93-1.95 (2H, m), 2.08-2.10 (2H, m), 2.44 (1H, dd, J=3.1, 16.5 Hz), 2.74 (1H, dd, J=6.7, 16.5 Hz), 3.23-3.26 (1H, m), 3.95 (2H, t, J=6.7 Hz), 4.15 (3H, s), 4.17 (2H, t, J=6.1 Hz), 6.76-6.78 (1H, m), 7.07 (1H, d, J=8.6 Hz), 7.28-7.29 (1H, m), 7.81 (1H, d, J=8.6 Hz), 8.45 (1H, s).

### <Example 229>

### 2-[4-(2,3-Difluoro-4-(6-oxo-1,4,5,6-tetrahydropyridazin-3-yl)phenoxy]butyl]-5-(4-methoxy-2-trifluoromethylbenzo[d]thiazol-7-yl)-4,4-dimethyl-2,4-dihydro-pyrazol-3-one

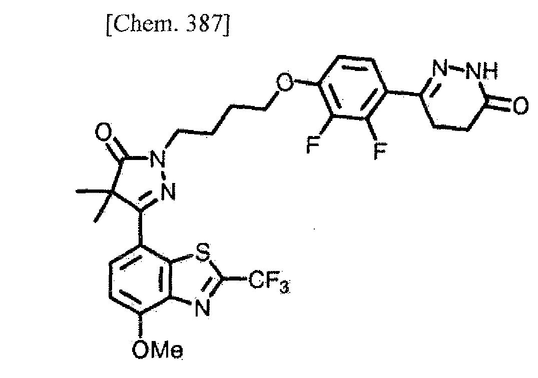

The reaction was carried out in the same manner as in Example 193 using the compound of Example 190 and the compound of Example 138 to obtain the desired product as a white powder.
Elemental analysis: Found value C 53.70%, H 4.00%, N 11.17%, Calculated value as C₂₈H₂₆F₅N₅O₄S C 53.93%, H 4.20%, N 11.23%.
¹H-NMR (CDCl₃, 400 MHz): δ 1.59 (6H, s), 1.93-1.94 (2H, m), 2.08-2.10 (2H, m), 2.58-2.59 (2H, m), 2.96-2.98 (2H, m), 3.95 (2H, t, J=6.7 Hz), 4.15 (3H, s), 4.17 (2H, t, J=6.4 Hz), 6.74-6.78 (1H, m), 7.07 (1H, d, J=8.6 Hz), 7.29-7.31 (1H, m), 7.80 (1H, d, J=8.6 Hz), 8.46 (1H, s).

### <Example 230>

### 2-[4-[2,3-Difluoro-4-(4,4-dimethyl-5-oxo-4,5-dihydro-1H-pyrazol-3-yl)phenoxy]butyl]-5-(4-methoxy-2-trifluoromethylbenzo[d]thiazol-7-yl)-4,4-dimethyl-2,4-dihydro-pyrazol-3-one

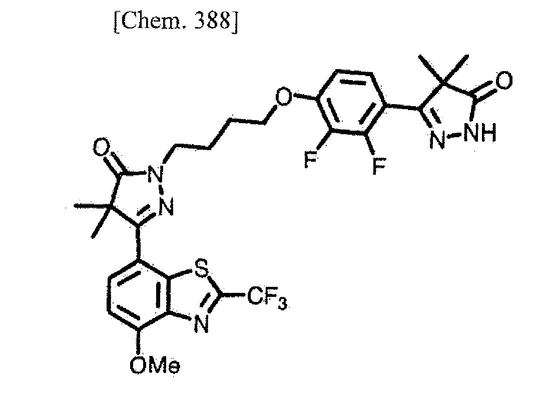

The reaction was carried out in the same manner as in Example 193 using the compound of Example 190 and the compound of Example 155 to obtain the desired product as a white powder.
Elemental analysis: Found value C 54.38%, H 4.24%, N 10.87%, Calculated value as C₂₉H₂₈F₅N₅O₄S C 54.63%, H 4.43%, N 10.98%.
¹H-NMR (CDCl₃, 400 MHz): δ 1.39 (3H, s), 1.40 (3H, s), 1.59 (6H, s), 1.94-1.96 (2H, m), 2.09-2.11 (2H, m), 3.95 (2H, t, J=6.7 Hz), 4.15 (3H, s), 4.19 (2H, t, J=6.1 Hz), 6.77-6.82 (1H, m), 7.07(1H, d, J=8.6 Hz), 7.31-7.36 (1H, m), 7.81 (1H, d, J=8.6 Hz), 8.43 (1H, s).

### <Example 231>

### 5-(2-Ethyl-5-methoxyindolidin-8-yl)-2-[4-[2,3-difluoro-4-(4-methyl-6-oxo-1,4,5,6-tetrahydropyridazin-3-yl)phenoxy]butyl]-4,4-dimethyl-2,4-dihydro-pyrazol-3-one

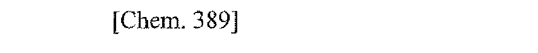

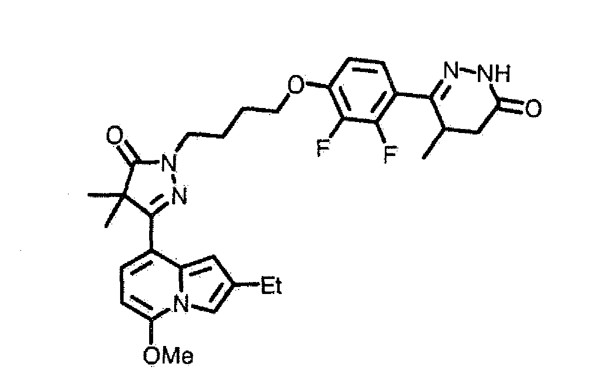

The reaction was carried out in the same manner as in Example 193 using the compound of Example 191 and the compound of Example 176 to obtain the desired product as a yellow amorphous.
¹H-NMR (CDCl₃, 400 MHz): δ 1.22 (3H, d, J=7.3 Hz), 1.34 (3H, t, J=7.3 Hz), 1.55 (6H, s), 1.95-1.37 (2H, m), 2.08-2.10 (2H, m), 2.46 (1H, dd, J=17.1, 3.1 Hz), 2.72-2.81 (3H, m), 3.26-3.28 (1H, m), 3.94 (2H, t, J=6.7 Hz), 4.11 (3H, s), 4.18 (2H, t, J=6.4 Hz), 5.83 (1H, d, J=7.3 Hz), 6.78-6.80 (1H, m), 7.17 (1H, d, J=7.3 Hz), 7.21 (1H, d, J=1.8 Hz), 7.27-7.30 (1H, m), 7.35 (1H, d, J=1.8 Hz), 8.48 (1H, s).
HRESIMS (+): 580.27654 (Calculated value as C₃₁H₃₆F₂N₅O₄ 580.27353).

### <Example 232>

### 5-(2-Ethyl-5-methoxyindolidin-8-yl)-2-[4-[2,3-difluoro-4-(6-oxo-1,4,5,6-tetrahydropyridazin-3-yl)phenoxy]butyl]-4,4-dimethyl-2,4-dihydro-pyrazol-3-one

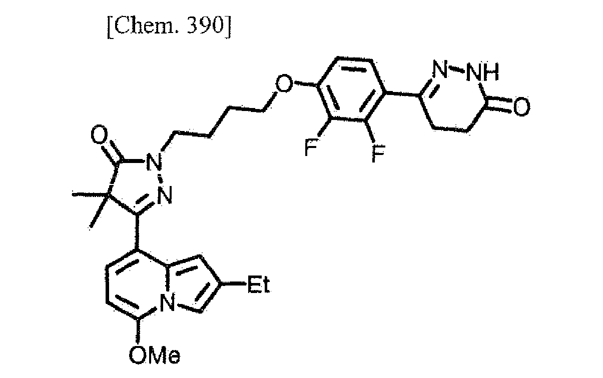

The reaction was carried out in the same manner as in Example 193 using the compound of Example 191 and the compound or Example 138 to obtain the desired product as a yellow amorphous.
¹H-NMR (CDCl₃, 400 MHz): δ 1.34 (3H, t, J=7.6 Hz), 1.55 (6H, s), 1.92-2.00 (2H, m), 2.05-2.12 (2H, m), 2.60 (2H, t, J=8.3 Hz), 2.78 (2H, q, J=7.6 Hz), 2.98-3.00 (2H, m), 3.94 (2H, t, J=6.7 Hz), 4.11 (3H, s), 4.18 (2H, t, J=6.1 Hz), 5.83 (1H, d, J=7.9 Hz), 6.76-6.81 (1H, m), 7.17 (1H, d, J=7.9 Hz), 7.20 (1H, d, J=1.8 Hz), 7.29-7.33 (1H, m), 7.35 (1H, d, J=1.8 Hz), 8.50 (1H, s).
HRESIMS (+): 566.25533 (Calculated value as C₃₀H₃₄F₂N₅O₄ 566.25788).

### <Example 233>

### 5-(2-Ethyl-5-methoxyindolidin-8-yl)-2-[4-[2,3-difluoro-4-(4,4-dimethyl-5-oxo-4,5-dihydro-1H-pyrazol-3-yl)phenoxy]butyl]-4,4-dimethyl-2,4-dihydro-pyrazol-3-one

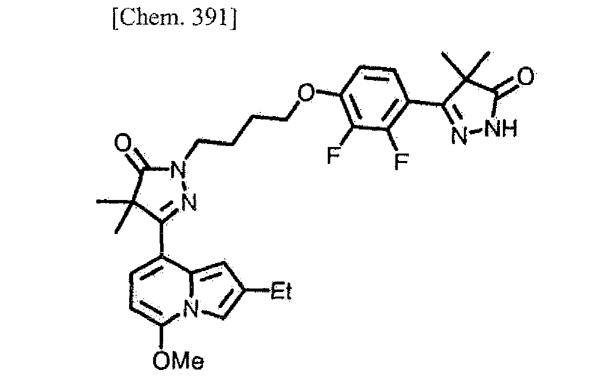

The reaction was carried out in the same manner as in Example 193 using the compound of Example 191 and the compound of Example 155 to obtain the desired product as a yellow amorphous.
¹H-NMR (CDCl₃, 400 MHz): δ 1.32 (3H, t, J=7.3 Hz), 1.39 (3H, s), 1.40 (3H, s), 1.53 (6H, s), 1.94-1.98 (2H, m), 2.06-2.08 (2H, m), 2.76 (2H, q, J=7.3 Hz), 3.93 (2H, t, J=6.7 Hz), 4.10 (3H, s), 4.18 (2H, t, J=6.4 Hz), 5.82 (1H, d, J=7.9 Hz), 6.77-6.82 (1H, m), 7.16 (1H, d, J=7.9 Hz), 7.20 (1H, d, J=1.8 Hz), 7.29-7.33 (1H, m), 7.33 (1H, d, J=1.8 Hz), 8.48 (1H, s). HREIMS (+): 580.27185 (Calculated value as C₃₁H₃₆F₂N₅O₄ 580.27353).

### <Example 234>

### 2-[4- [2,3 -Difluoro-4-(4-methyl-6-oxo-1,4,5,6-tetrahydropyridazin-3-yl)phenoxy]butyl]-5-(8-methoxy-2-trifluoromethylimidazo[1,2-a]pyridin-5-yl)-4,4-dimethyl-2,4-dihydro-pyrazol-3-one

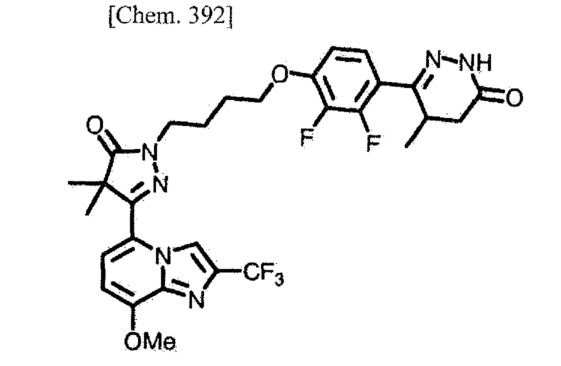

The reaction was carried out in the same manner as in Example 193 using the compound of Example 192 and the compound of Example 176 to obtain the desired product as a colorless powder.
¹H-NMR (CDCl₃, 400 MHz): δ 1.20 (3H, d, J= 7.3 Hz), 1.59 (6H, s), 1.89-1.99 (2H, m), 2.01-2.12 (2H, m), 2.44 (1H, dd, J= 17.1, 3.1 Hz), 2.74 (1H, dd, J=17.1, 7.3 Hz), 3.19-331 (1H, m), 3.98 (2H, t, J=7.3 Hz), 4.11 (3H, s), 4.16 (2H, t, J=6.1 Hz), 6.69 (1H, d, J=8.6 Hz), 6.73-6.81 (1H, m), 7.24-7.32 (1H, m), 7.29 (1H, d, J=8.6 Hz), 8.48 (1H, brs), 9.40 (1H, s). ESIMS (+): 621 [M+H]⁺,
Elemental analysis: Found value C 55.85%, H 4.54%, N 13.51%, Calculated value as C₂₉H₂₉F₅N₆O₄ C 56.13%, H 4.71%, N 13.51%.

### <Example 235>

### 2-[4-[2,3-Difluoro-4-(6-oxo-1,4,5,6-tetrahydropyridazin-3-yl)phenoxy]butyl]-5-(8-methoxy-2-trifiuoromethylimidazo[1,2-a]pyridin-5-yl)-4,4-dimethyl-2,4-dihydro-pyrazol-3-one

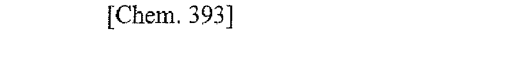

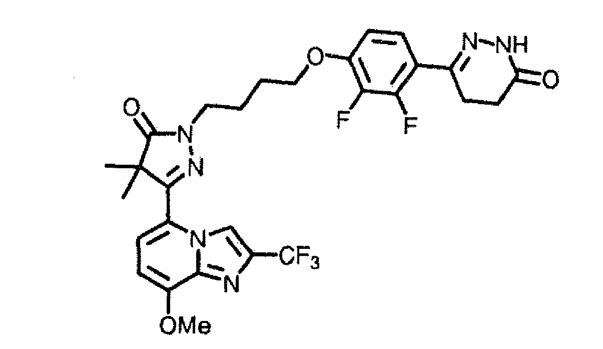

The reaction was carried out in the same manner as in Example 193 using the compound of Example 192 and the compound of Example 138 to obtain the desired product as a colorless powder.
¹H-NMR (CDCl₃, 400 MHz): δ 1.59 (6H, s), 1.89-1.99 (2H, m), 2.01-2.12 (2H, m), 2.56-2.62 (2H, m), 2.94-3.01 (2H, m), 3.98 (2H, t, J=6.7 Hz), 4.1 (3H, s), 4.16 (2H, t, J=6.1 Hz), 6.69 (1H, d, J=8.6 Hz), 6.73-6.80 (1H, m), 7.27-7.33 (1H, m), 7.29 (1H, d, J=8.6 Hz), 8.48 (1H, brs), 9.39 (1H, d, J=1.2 Hz).
ESIMS (+): 607 [M+H]⁺.
Elemental analysis: Found value C 54.42%, H 4.41%, N 13.66%, Calculated value as C₂₈H₂₇F₅N₆O, and 0.5H₂O C 54.63%, H 4.58%, N 13.65%.

### <Example 236>

### 2-[4-[2,3-Difluoro-4-(4,4-dimethyl-5-oxo-4,5-dihydro-1H-pyrazol-3-yl)phenoxy]butyl]-5-(8-methoxy-2-trifluoromethylimidazo[1,2-a]pyridin-5-yl)-4,4-dimethyl-2,4-dihydro-pyrazol-3-one

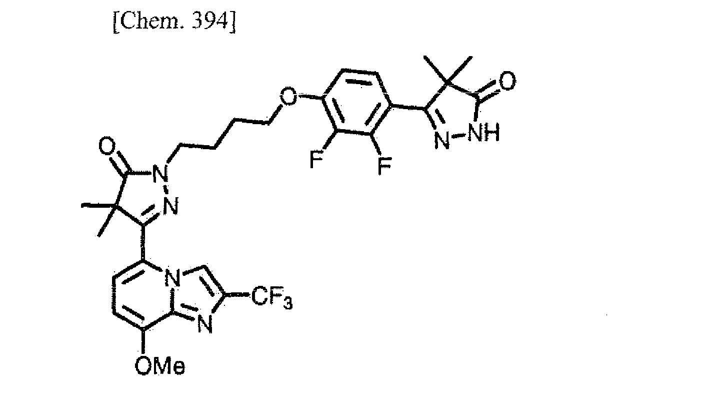

The reaction was carried out in the same manner as in Example 193 using the compound of Example 192 and the compound of Example 155 to obtain the desired product as a colorless powder.
¹H-NMR (CDCl₃, 400 MHz): δ 1.40 (3H, s), 1.40 (3H, s), 1.59 (6H, s), 1.90-2.01 (2H, m), 2.02-2.12 (2H, m), 3.99 (2H, t, J=6.7 Hz), 4.11 (3H, s), 4.18 (2H, t, J=6.1 Hz), 6.69 (1H, d, J=8.6Hz), 6.76-6.83 (1H, m), 7.29 (1H, d, J=8.6 Hz), 7.31-7.38 (1H, m), 8.52 (1H, brs), 9.40 (1H, s).
ESIMS (+): 621 [M+H]⁺.
HRESIMS(+): 621.22397 (Calculated value as C₂₉H₃₀F₃₀N₆O₄ 621.22487).

### <Example 237>

### 6-(4-Hydroxy-3-methoxyphenyl)-4,5-dihydro-2H-pyridazm-3-one

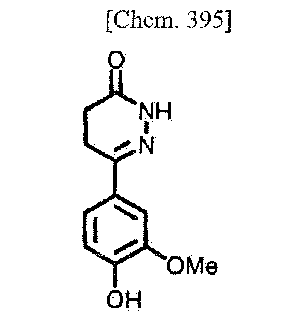

4-Acetyl-2-methoxy-1-methoxymethyloxybenzene (1.74 g) was dissolved in THF (40 mL) under an argon gas atmosphere, and lithium bistrimethylsilyl amide (1.00 mol/L THF solution, 9.93 mL) was added dropwise under ice cooling, followed by stirring at the same temperature for 30 minutes. Thereafter, tert-butyl bromoacetate (1.83 mL) was added thereto at the same temperature, followed by stirring at room temperature for 2 hours. To the reaction liquid was added a saturated aqueous ammonium chloride solution, followed by extraction with ethyl acetate. The extracted layer was washed with water and saturated brine in this order, and then dried over anhydrous sodium sulfate. The solvent was evaporated under reduced pressure, the obtained residue was dissolved in methylene chloride (10 mL), and trifluoroacetic acid (10.0 mL) was added thereto, followed by stirring at room temperature for 1 hour. The residue obtained by evaporating the solvent under reduced pressure was dissolved in ethanol (60 mL), and hydrazine monohydrate (1.20 mL) was added thereto, followed by stirring for 4 hours under the condition of heating under reflux. After evaporating the solvent under reduced pressure, to the residue was added ice water, and the resulting solid was collected by filtration. The obtained solid was washed with water, cold ethanol, and diethyl ether in that order to obtain the desired product (470 mg) as a cream color powder.
¹H-NMR (DMSO-d₆, 400 MHz): δ 2.36 (2H, dd, J=9.2, 7.9 Hz), 2.88 (2H, dd, J=9.2, 7.9 Hz), 3.78 (3H, s), 6.78 (1H, d, J=8.0 Hz), 7.14 (1H, dd, J=8.0, 1.8 Hz), 7.32 (1H, d, J=1.8 Hz), 9.37 (1H, s), 10.74 (1H, s).
EIMS (+): 220 [M]⁺.

### <Example 238>

### 5-(2-Ethyl-8-methoxyquinolin-5-yl)-2-[4-[2-methoxy-4-(4-methyl-6-oxo-1,4,5,6-tetrahydropyridazin-3-yl)phenoxy]butyl]-4,4-dimethyl-2,4-dihydro-pyrazol-3-one

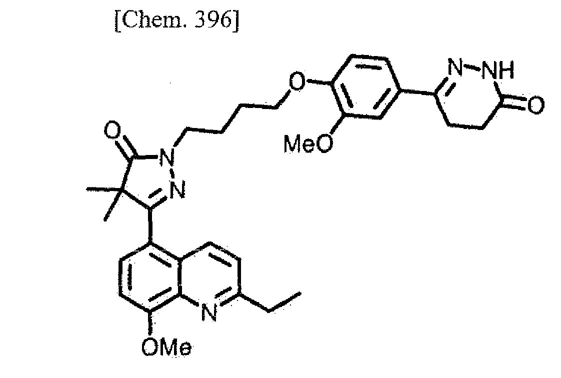

The reaction was carried out in the same manner as in Example 193 using the compound of Example 183 and the compound of Example 237 to obtain the desired product as a colorless amorphous.
HRESIMS(+): 572.28627 (Calculated value as C₃₂H₃₈N₅O₅ 572.28729)
¹H-NMR (CDCl₃, 400 MHz): δ 1.40 (3H, t, J=7.3 Hz), 1.43 (6H, s), 1.96-2.03 (4H, m), 2.60 (2H, t, J=8.6 Hz), 2.95 (2H, t, J=8.0 Hz), 3.08 (2H, q, J=7.3 Hz), 3.88 (3H, s), 3.92 (2H, t, J=7.3 Hz), 4.13 (3H, s), 4.14 (2H, t, J=6.7 Hz), 6.87 (1H, d, J=8.6 Hz), 7.06 (1H, d, J=8.6 Hz), 7.12 (1H, dd, J=1.8, 8.6 Hz), 7.39 (1H, d, J=1.8 Hz), 7.41 (1H, d, J=9.2 Hz), 7.56 (1H, d, J=8.6 Hz), 8.47 (1H, brs), 8.76 (1H, d, J=9.2 Hz).

### <Example 239>

### 6-[4-(3-Bromopropoxy)-2,3-difluorophenyl]-5-methyl-4,5-dihydro-2H-pyridazin-3-one

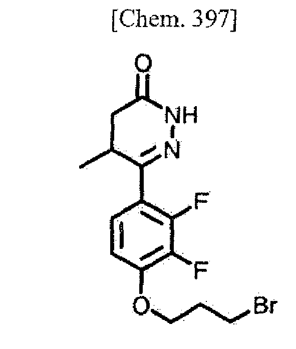

The compound of Example 176 (2.50 g) was dissolved in DMF (50 mL), and 1,3-dibromopropane (1.58 mL) and potassium carbonate (2.87 g) were added thereto, followed by stirring at 60°C for 50 minutes. To the reaction liquid was added water, followed by extraction three times with ethyl acetate. The obtained extracted layer was washed with saturated brine chloride and dried over anhydrous sodium sulfate. After evaporating the solvent under reduced pressure, the residue was purified by silica gel column chromatography (hexane:ethyl acetate=1.3) to obtain the desired product (1.96 g) as a yellow powder. ¹H-NMR (CDCl₃, 400 MHz): δ 1.22 (3H, d, J=7.3 Hz), 2.35-2.41 (2H, m), 2.45 (1H, dd, J=16.5, 3.1 Hz), 2.75 (1H, dd, J=16.5, 6.7 Hz), 3.23-3.31 (1H, m), 3.63 (2H, t, J=6.1 Hz), 4.24 (2H, t, J=5.8 Hz), 6.79-6.84 (1H, m), 7.31-7.33 (1H, m), 8.51 (1H, s). EIMS (+): 521 [M+H]⁺.

### <Example 240>

### 6-[4-(3-Bromopropoxy)-2,3-difluorophenyl]-2-t-butoxycarbonyl-5-methyl-4,5-dihydro-2H-pyridazin-3-one

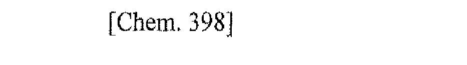

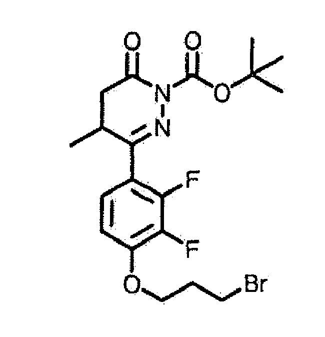

The reaction was carried out in the same manner as in Example 166 using the compound of Example 239 to obtain the desired product as a yellow powder.
¹H-NMR (CDCl₃, 400 MHz): δ 1.22 (3H, d, J=7.3 Hz), 1.60 (9H, s), 2.34-2.40 (2H, m), 2.54 (1H, dd, J=16.2, 3.4 Hz), 2.82 (1H, dd, J=16.2, 6.4 Hz), 3.27-3.34 (1H, m), 3.63 (2H, t, J=6.4 Hz), 4.24 (2H, t, J=5.8 Hz), 6.81 (1H, m), 7.44-7.49 (1H, m).

### <Example 241>

### 5-(2-Ethyl-8-methoxyquinolin-5-yl)-2-[4-[2,3-difluoro-4-(4-methyl-6-oxo-1,4,5,6-tetrahydropyridazin-3-yl)phenoxy]propyl]-4,4-dimethyl-2,4-dihydro-pyrazol-3-one

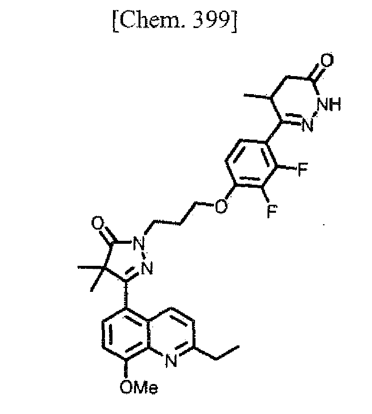

The compound of Example 71 (150 mg) was dissolved in DMF (3.0 mL) under an argon atmosphere, and 60% sodium hydride (24.0 mg) was added thereto at 0°C, followed by stirring at room temperature for 30 minutes. A solution of the compound of Example 240 (261 mg) in DMF (1.0 mL) was added thereto at 0°C, followed by stirring at room temperature for 1 hour. To the reaction liquid was added a saturated aqueous ammonium chloride solution, followed by extraction three times with ethyl acetate. The obtained extracted layer was washed with saturated brine chloride, dried over anhydrous sodium sulfate, and filtered. After evaporating the solvent of the obtained filtrate under reduced pressure, the residue was dissolved in dichloromethane (3.0 mL), and trifluoroacetic acid (1.0 mL) was added thereto, followed by stirring at room temperature for 1 hour. To the reaction liquid was added a saturated aqueous sodium hydrogen carbonate solution, followed by extraction three times with ethyl acetate. The obtained extracted layer was washed with saturated brine chloride, dried over anhydrous sodium sulfate, and filtered. After evaporating the solvent of the obtained filtrate under reduced pressure, the residue was purified by silica gel column chromatography (ethyl acetate→ethyl acetate:methanol=20:1) to obtain the desired product (32.6 mg) as a yellow amorphous.
¹H-NMR (CDCl₃, 400 MHz): δ 1.20 (3H, d, J=7.3 Hz), 1.40 (3H, t, J=7.6 Hz), 1.43 (6H, s), 2.33-2.40 (2H, m), 2.44 (1H, dd, J=17.1, 3.1 Hz), 2.74 (1H, dd, J=17.1, 6.7 Hz), 3.08 (2H, q, J=7.5 Hz), 3.24-3.26 (1H, m), 4.08 (2H, t, J=6.4 Hz), 4.13 (3H, s), 4.22 (2H, t, J=6.1 Hz), 6.77-6.80 (1H, m), 7.06 (1H, d, J=8.6 Hz), 7.28-7.30 (1H, m), 7.38 (1H, d, J=8.6 Hz), 7.57 (1H, d, J=8.6 Hz), 8.48 (1H, s), 8.77 (1H, d, J=8.6 Hz).
HRESIMS (+) 578.257943 (Calculated value as C₃₁H₃₄F₂N₅O₄ 578.25788).

### <Example 242>

### 5-(2-Ethyl-8-methoxyquinolin-5-yl)-2-[4-[2,3-difluoro-4-(4-methyl-6-oxo-1,4,5,6-tetrahydropyridazin-3-yl)phenoxy]ethyl]-4,4-dimethyl-2,4-dihydro-pyrazol-3-one

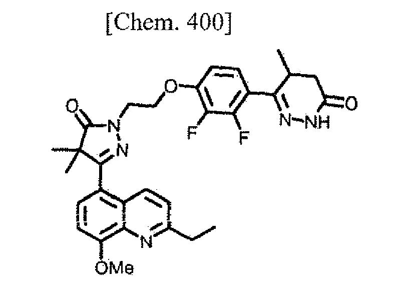

The reaction was carried out in the same manner as in Example 241 using the compound of Example 71 and the compound of Example 177 to obtain the desired product as a colorless amorphous.
¹H-NMR (CDCl₃, 400 MHz): δ 1.20 (3H, d, J=7.3 Hz), 1.39 (3H, t, J=7.6 Hz), 1.43 (3H, s), 1.43 (3H, s), 2.45 (1H, dd, J=17.1, 3.1 Hz), 2.73 (1H, dd, J=17.1, 6.7 Hz), 3.08 (2H, q, J= 7.5 Hz), 3.25-3.26 (1H, m), 4.12 (3H, s), 4.29 (2H, t, J=5.5 Hz), 4.49 (2H, t, J=5.5 Hz), 6.83-6.87 (1H, m), 7.05 (1H, d, J=8.6 Hz), 7.30-7.3 7 (1H, m), 7.3 6 (2H, d, J=8.6 Hz), 7.56 (1H, d, J=8.6 Hz), 8.48 (1H, s), 8.69 (1H, d, J=8.6 Hz).
HRESIMS (+) 564.24014 (Calculated value as C₃₀H₃₂F₂N₅O₄ 564.24223).

### <Test Example 1> Phosphodiesterase inhibitory activity

RT-PCR was each performed to isolate the cDNA of a PDE3A catalytic domain (hereinafter simply referred to as "cat") and PDE4Bcat from a human-derived RNA. The isolated cDNA fragments were inserted into Sf9 insect cells using the Gateway system (manufactured by Invitrogen Corporation) and the Bac-to-Bac (registered trademark) Baculovirus Expression system (manufactured by Invitrogen Corporation) to express these PDE proteins. These recombinant PDE3Acat, PDE4Bcat, PDE5Acat, and PDE10Al were purified from the culture supernatants or cell extracts of Sf9 cells expressing high levels of the PDE protein by ion exchange chromatography and used for the experiments as shown below.

A 4 mmol/L solution of each test compound was stepwisely diluted four-fold with a 15% DMSO solution to prepare solutions at concentrations of 15 nmol/L to 4 mmol/L (the final concentrations used in the experiments were 1.5 nmol/L to 400 µmol/L). 10 µL of the prepared test compound solutions, [³H] cAMP diluted with a buffer solution [40 mmol/L Tris-HCl (pH: 7.4), 10 mmol/L MgCl₂], and 40 µL of the recombinant human-derived PDE protein at 2 × 10⁻⁶ units (wherein 1 unit is defined as an amount of PDE that degrades 1 µmol/L of cAMP in one minute under the conditions of a pH of 7.5 and 30°C) were added to a 96-well plate, and the mixtures were reacted at 30°C for 20 minutes. Then, the mixtures were reacted at 65°C for 2 minutes, 25 µL of a 1 mg/mL 5'-nucleotidase (Crotalus atrox venom, manufactured by Sigma) was then added thereto, and the mixtures were reacted at 30°C for 10 minutes. After completion of the reaction, 200 µL of a solution of Dowex [300 mg/mL Dowex 1 × 8-400 (manufactured by Sigma Aldrich), 33% ethanol] was added thereto, and the mixtures were mixed and shaken at 4°C for 20 minutes. Subsequently, 200 µL of MicroScint 20 (manufactured by Packard) was added thereto, and measurement was performed using a scintillation counter (Topcount, manufactured by Packard). IC₅₀ values were calculated using GraphPad Prism v3.03 (manufactured by GraphPad Software).

Further, the indications were as follows: 1 µmol/L>IC₅₀ value ≥0.1 µmol/L (+), 0.1 µmol/L>IC₅₀ value ≥0.01 µmol/L(++), and 0.01 µmol/L>IC₅₀ value (+++).

The results are shown in Table 1.

### [Table 1]

**Table 1**

| Example No. | IC₅₀(µmol/L) PDE3 | IC₅₀ (µmol/L) PDE4 | Example No. | IC₅₀(µmol/L) PDE3 | IC₅₀(µmol/L) PDE4 |
|---|---|---|---|---|---|
| 193 | ++ | ++ | 217 | + | +++ |
| 194 | ++ | ++ | 218 | +++ | +++ |
| 195 | +++ | + | 219 | +++ | +++ |
| 196 | +++ | ++ | 220 | ++ | +++ |
| 197 | +++ | ++ | 221 | +++ | +++ |
| 198 | +++ | ++ | 222 | +++ | + |
| 199 | ++ | +++ | 223 | ++ | ++ |
| 200 | + | ++ | 224 | +++ | ++ |
| 201 | + | ++ | 225 | +++ | ++ |
| 202 | ++ | ++ | 226 | +++ | ++ |
| 203 | +++ | ++ | 227 | ++ | + |
| 204 | + | + | 228 | +++ | +++ |
| 205 | +++ | ++ | 229 | +++ | +++ |
| 206 | + | ++ | 230 | ++ | +++ |
| 207 | +++ | ++ | 231 | +++ | + |
| 208 | ++ | + | 232 | +++ | ++ |
| 209 | +++ | ++ | 233 | ++ | + |
| 210 | +++ | ++ | 234 | +++ | +++ |
| 211 | +++ | ++ | 235 | +++ | +++ |
| 212 | +++ | ++ | 236 | ++ | +++ |
| 213 | +++ | ++ | 238 | + | + |
| 214 | +++ | ++ | 241 | +++ | + |
| 215 | +++ | ++ | 242 | +++ | ++ |
| 216 | +++ | +++ | | | |

### <Test Example 2> Histamine-induced bronchoconstriction reaction in guinea pigs

Guinea pigs were anesthetized with pentobarbital (30 mg/kg, i.p.). A cannula for intravenous administration, a cannula for collecting blood and measuring blood pressure, and a tracheal cannula were inserted into the left external jugular vein, right internal carotid artery, and trachea, respectively. The guinea pigs were maintained on artificial respiration under conditions of 60 times/min and 10 mL/kg/stroke. The overflowing air from the side branch of the tracheal cannula was measured by a bronchospasm transducer (Ugo-Basile) and recorded on a computer via Power Lab (ADInstruments Japan). The guinea pigs were immobilized with gallamine (10 mg/kg, i.v.), and histamine (12.5 µg/kg, i.v.) was administered at 10-minute intervals. After the histamine-induced bronchoconstriction became stable, the compound (0.3 mg/kg, i.v.) was administered. The histamine-induced bronchoconstriction reaction was measured 30 seconds after the compound administration to examine the bronchoconstriction inhibitory activity of the compound. The bronchoconstriction was recorded as the airflow value, and the results were represented by the ratio of the maximum value of the histamine-induced airflow 30 seconds after administration to the maximum value of the airflow before administration. Also, the test compounds dissolned in DMSO were used.

Further, the indications were as follows: inhibition rate ≥90% (+++), 90%>inhibition rate≥80% (++), and 80%>inhibition rate≥60% (+).

The results are shown in Table 2.

### [Table 2]

**Table 2**

| Example No. | Inhibition rate | Example No. | Inhibition rate |
|---|---|---|---|
| 193 | +++ | 213 | +++ |
| 194 | +++ | 214 | +++ |
| 196 | +++ | 215 | +++ |
| 197 | +++ | 219 | +++ |
| 198 | +++ | 220 | +++ |
| 199 | +++ | 221 | +++ |
| 200 | +++ | 228 | ++ |
| 202 | + | 229 | +++ |
| 203 | +++ | 234 | ++ |
| 205 | +++ | 235 | ++ |
| 207 | +++ | 236 | + |
| 209 | +++ | 238 | + |
| 211 | +++ | 241 | +++ |
| 212 | +++ | | |

### <Test Example 3> LPS acute inflammation model in rats

10 mg/kg of the compounds were orally administered to rats one hour before inhalation of a lipopolysaccharide from E. coli serotype 055:B5 (LPS), and the rats were made to inhale 50 ml of the LPS solution nebulized using a nebulizer for 30 minutes. Then, 3 hours after LPS inhalation, the rats were euthanized with 20% urethane (5 ml/rat, i.p.). 5 ml of physiological saline for bronchoalveolar lavage was injected into the bronchial tubes and alveoli through the airway, and the bronchial tubes and alveoli were washed three times using a 5 mL syringe. This operation was repeated twice, and the solution was collected as bronchoalveolar lavage fluid (BALF). The collected BALF was centrifuged at 1200 rpm and 4°C for 10 minutes (Hirtachi; himac CR 5 DL). The pellet was re-suspended in 10 ml of a 0.1% bovine serum albumin-physiological saline, and an equivalent amount of Turk's solution was added thereto to stain leukocytes. The total number of leukocytes was counted under a microscope to calculate the inhibition rate. Further, the indications were as follows: inhibition rate≥80% (+++), 80%>inhibition rate≥60% (++), and 60%>inhibition rate≥40% (+).

The results are shown in Table 3.

### [Table 3]

**Table 3**

| Example No. | Inhibition rate |
|---|---|
| 197 | +++ |
| 205 | +++ |
| 206 | + |
| 207 | + |
| 209 | +++ |
| 210 | + |
| 211 | ++ |

As described above, the compounds represented by the general formula (1) of the present invention have a PDE inhibitory activity, and the effectiveness of the compounds has been confirmed in the experimental models of various animals.

### Industrial Availability

As described above, according to the present invention, it has been found that a novel pyrazolone derivative and an addition salt thereof have excellent PDE inhibitory action. Such a compound having PDE inhibitor action is useful as an agent for treating angina pectoris, cardiac failure, hypertension, or the like, as a platelet aggregation inhibitor, as an agent for preventing or treating bronchial asthma, chronic obstructive pulmonary disease (COPD), interstitial pneumonitis, allergic rhinitis, atopic dermatitis, rheumatoid arthritis, multiple sclerosis, Crohn's disease, or inflammatory bowel disease, as an agent for preventing or treating various psychiatric disorders such as Huntington's disease, Alzheimer, dementia, Parkinson's disease, depression, schizophrenia, and the like, as an agent for preventing or treating obesity, metabolic syndrome, and the like, and as an agent for treating male erectile dysfunction.

## Claims

1. A pyrazolone derivative, optically active compound thereof, pharmaceutically acceptable salt thereof, or hydrate thereof, wherein the pyrazolone derivative is represented by the following general formula (1): 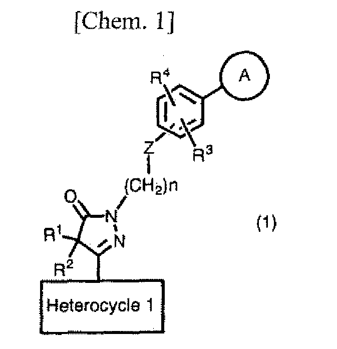 [wherein R¹ and R² are the same as or different from each other and represent an alkyl group having 1 to 6 carbon atoms,
R³ and R⁴ are the same as or different from each other and represent a hydrogen atom, a halogen atom, or an alkoxy group having 1 to 6 carbon atoms,
Z represents an oxygen atom or a sulfur atom,
A represents a substituent represented by the general formula: 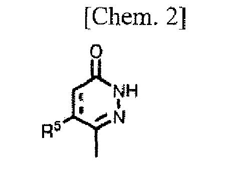 (wherein R⁵ represents a hydrogen atom or an alkyl group having 1 to 6 carbon atoms, and 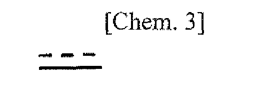 represents a single bond or a double bond) or a substituent represented by the general formula: 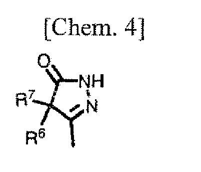 (wherein R⁶ and R⁷ are the same as or different from each other and represent an alkyl group having 1 to 6 carbon atoms),
Heterocycle I represents a substituent represented by the following general formula (2): 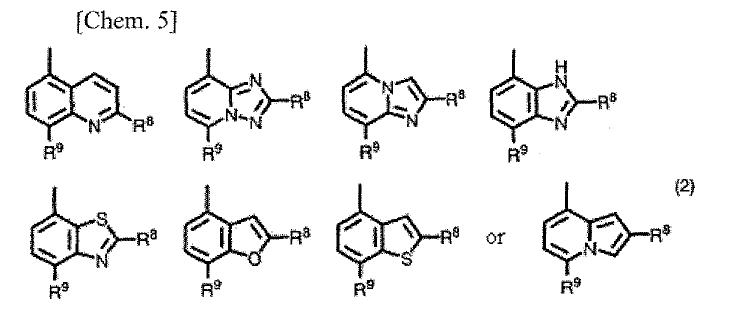 (wherein R⁸ represents a hydrogen atom or an alkyl group having 1 to 6 carbon atoms which may be substituted with halogen atom(s), and R⁹ represents an alkoxy group having I to 6 carbon atoms), and
n represents an integer of 1 to 5].

2. The pyrazolone derivative, optically active compound thereof, pharmaceutically acceptable salt thereof, or hydrate thereof according to claim 1, wherein the compound represented by the general formula (1) is represented by the general formula (1a): 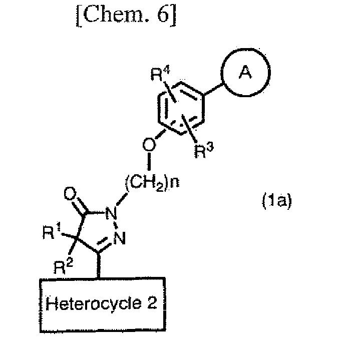 [wherein Heterocycle 2 represents the following general formula (2a): 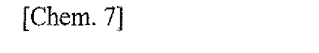 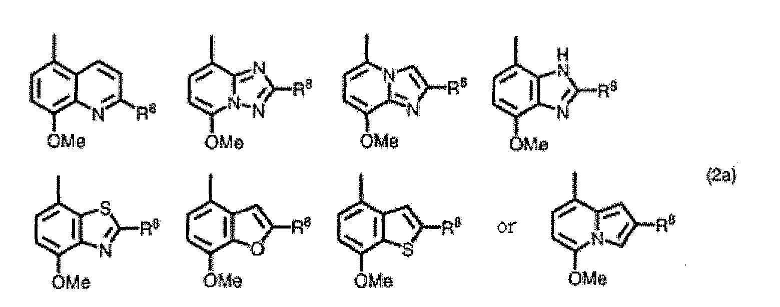 (wherein R⁸ is as defined above), and R¹, R², R³, R⁴, A, and n are as defined above].

3. The pyrazolone derivative, optically active compound thereof, pharmaceutically acceptable salt thereof, or hydrate thereof according to claim 1, wherein the compound represented by the general formula (1) is
5-(8-methoxy-2-methylquinolin-5-yl)-2-[4-[4-(4-methyl-6-oxo-1,4,5,6-tetrahydropyridazin-3-yl)phenoxy]butyl]-4,4-dimethyl-4,4-dihydro-pyrazol-3-one,
5-(2-ethyl-8-methoxyquinolin-5-yl)-2-[4-[2-fluoro-4-(4,4-dimethyl-5-oxo-4,5-dihydro-1H-pyrazol-3-yl)phenoxy]butyl]-4,4-dimethyl-2,4-dihydro-pyrazol-3-one,
5-(2-ethyl-8-methoxyquinolin-5-yl)-2-[4-[2,3-difluoro-4-(4,4-dimethyl-5-oxo-4,5-dihydro-1H-pyrazol-3-yl)phenoxy]butyl]-4,4-dimethyl-2,4-dihydro-pyrazol-3-one,
5-(2-ethyl-8-methoxyquinolin-5-yl)-2-[4-[2-fluoro-4-(4-methyl-6-oxo-1,4,5,6-tetrahydropyridazin-3-yl)phenoxy]butyl]-4,4-dimethyl-2,4-dihydro-pyrazol-3-one,
5-(2-ethyl-8-methoxyquinolin-5-yl)-2-[4-[2,3-difluoro-4-(4-methyl-6-oxo-1,4,5,6-tetrahydropyridazin-3-yl)phenoxy]butyl]-4,4-dimethyl-2,4-dihydro-pyrazol-3-one,
(-)-5-(2-ethyl-8-methoxyquinolin-5-yl)-2-[4-[2,3-difluoro-4-(4-methyl-6-oxo-1,4,5,6-tetrahydropyridazin-3-yl)phenoxy]butyl]-4,4-dimethyl-2,4-dihydro-pyrazol-3-one,
2-[4-[2,3-difluoro-4-(6-oxo-1,4,5,6-tetrahydropyridazin-3-yl)phenoxy]butyl]-5-(4-methoxy-2-trifluoromethyl-1H-benzo[d]imidazol-7-yl)-4,4-dimethyl-2,4-dihydro-pyrazol-3-one,
2-[4-[2,3-difluoro-4-(4,4-dimethyl-5-oxo-4,5-dihydro-1H-pyrazol-3-yl)phenoxy]butyl]-5-(4-methoxy-2-trifluoromethyl-1H-benzo[d]imidazol-7-yl)-4,4-dimethyl-2,4-dihydro-pyrazol-3-one,
2-[4-[2,3-difluoro-4-(4-methyl-6-oxo-1,4,5,6-tetrahydropyridazin-3-yl)phenoxy]butyl]-5-(4-methoxy-2-trifluoromethyl-1H-benzo[d]imidazol-7-yl)-4,4-dimethyl-2,4-dihydro-pyrazol-3-one,
2-[4-[2,3-difluoro-4-(6-oxo-1,4,5,6-tetrahydropyridazin-3-yl)phenoxy]butyl]-5-(4-methoxy-2-trifluoromethylbenzo[d]thiazol-7-yl)-4,4-dimethyl-2,4-dihydro-pyrazol-3-one,
2-[4-[2,3-difluoro-4-(6-oxo-1,4,5,6-tetrahydropyridazin-3-yl)phenoxy]butyl]-5-(8-methoxy-2-trifluoromethylimidazo[1,2-a]pyridin-5-yl)-4,4-dimethyl-2,4-dihydro-pyrazol-3-one,
2-[4-[2,3-difluoro-4-(4,4-dimethyl-5-oxo-4,5-dihydro-1H-pyrazol-3-yl)phenoxy]butyl]-5-(4-methoxy-2-trifluoromethylbenzo[d]thiazol-7-yl)-4,4-dimethyl-2,4-dihydro-pyrazol-3-one, or
5-(2-ethyl-8-methoxyquinolin-5-yl)-2-[4-[2,3-difluoro-4-(4-methyl-6-oxo-1,4,5,6-tetrahydropyridazin-3-yl)phenoxy]propyl]-4,4-dimethyl-2,4-dihydro-pyrazol-3-one.

4. A phosphodiesterase (PDE) inhibitor comprising, as an active ingredient, the pyrazolone derivative, optically active compound thereof, pharmaceutically acceptable salt thereof, or hydrate thereof according to any one of claims 1 to 3.

5. A pharmaceutical agent comprising, as an active ingredient, the pyrazolone derivative, optically active compound thereof, pharmaceutically acceptable salt thereof, or hydrate thereof according to any one of claims 1 to 3.

6. The pharmaceutical agent according to claim 5, which is an agent for preventing or treating angina pectoris, cardiac failure, hypertension, bronchial asthma, chronic obstructive pulmonary disease (COPD), interstitial pneumonitis, allergic rhinitis, atopic dermatitis, rheumatoid arthritis, multiple sclerosis, Crohn's disease, inflammatory bowel disease, Huntington's disease, Alzheimer, dementia, Parkinson's disease, depression, schizophrenia, obesity, or metabolic syndrome.
